# EUROPEAN PATENT APPLICATION

(11) **EP 4 733 325 A2**
(43) Date of publication of application: **29.04.2026**
(21) Application number: 25208409.0
(22) Date of filing: 05.07.2019
(51) Int. Cl.: C07K 16/30

(54) **MULTI-SPECIFIC WNT SURROGATE MOLECULES AND USES THEREOF**

(30) Priority: 05.07.2018 US 201862694339 P; 19.12.2018 US 201862782122 P; 28.01.2019 US 201962797772 P
(62) Divisional of application: 19830971.8
(71) Applicant: Surrozen Operating, Inc., South San Francisco, CA 94080 (US)
(72) Inventor: LI, Yang, San Francisco 94080 (US)
(74) Representative: Cooley (UK) LLP

(57) **Abstract**

The present disclosure provides Wnt pathway agonists and related compositions, which may be used in any of a variety of therapeutic methods for the treatment of diseases.

## Description

### CROSS REFERENCE TO RELATED APPLICATIONS

This application claims priority to U.S. Provisional Application No. 62/694,339, filed on July 5, 2018, U.S. Provisional Application No. 62/782,122, filed on December 19, 2018, and U.S. Provisional Application No. 62/797,772, filed on January 28, 2019, each of which is incorporated by reference herein in its entirety.

### Statement Regarding Sequence Listing

The Sequence Listing associated with this application is provided in text format in lieu of a paper copy, and is hereby incorporated by reference into the specification. The name of the text file containing the Sequence Listing is SRZN_008_03WO_ST25.txt. The text file is 878 KB, was created on July 5, 2019, and is being submitted electronically via EFS-Web.

### BACKGROUND

### Technical Field

The present disclosure relates generally to Wnt signaling pathway agonist molecules, compositions, and methods of using the same. Such molecules are useful, for example, in modulating Wnt signaling pathways.

### Description of the Related Art

Wnt ("Wingless-related integration site" or "Wingless and Int-1" or "Wingless-Int") ligands and their signals play key roles in the control of development, homeostasis and regeneration of many essential organs and tissues, including bone, liver, skin, stomach, intestine, kidney, central nervous system, mammary gland, taste bud, ovary, cochlea and many other tissues (reviewed, e.g., by Clevers, Loh, and Nusse, 2014; 346:1248012). Modulation of Wnt signaling pathways has potential for treatment of degenerative diseases and tissue injuries.

One of the challenges for modulating Wnt signaling as a therapeutic is the existence of multiple Wnt ligands and Wnt receptors, Frizzled 1-10 (Fzd1-10), with many tissues expressing multiple and overlapping Fzds. Canonical Wnt signals also involve Low-density lipoprotein (LDL) receptor-related protein 5 (LRP5) or Low-density lipoprotein (LDL) receptor-related protein 6 (LRP6) as co-receptors, which are broadly expressed in various tissues, in addition to Fzds. Ratios of Fzd to LRP binding moieties have not been previously explored to modulate signaling levels, and to confer tissue and/or functional specificity.

The Wnt signaling pathway is subdivided into canonical (β-catenin dependent) and non-canonical (β-catenin independent) pathways. The non-canonical pathway can be further divided into two distinct branches - the Planar Cell Polarity (PCP) pathway and the Wnt/Ca²⁺ pathway. Binding of certain Wnt ligands with certain Fzd receptors, or combinations of Fzd receptors can trigger the different pathways, and/or confer tissue and functional specificity.

Accordingly, there is clearly a need in the art for binding moieties that specifically bind to one or more Fzd, LRP5, or LRP6 to modulate the different Wnt signaling pathways. Also a need exists to create binding moieties with certain ratios of co-receptors (e.g., Fzd and LRP receptors) to modulate signaling levels, and to confer tissue and/or functional specificity. The present disclosure addresses these needs.

### BRIEF SUMMARY

In various embodiments, the present disclosure provides Wnt surrogate molecules and related uses thereof.

In one aspect, the present disclosure provides a multispecific Wnt surrogate molecule, wherein the Wnt surrogate molecule comprises: (i) a plurality of regions that each specifically bind to a set of one or more Fzd receptor epitopes (Fzd binding regions), wherein at least two Fzd binding regions bind to the same or different sets of one or more Fzd receptor epitopes; and (ii) one or more regions that specifically bind to a Low-density lipoprotein (LDL) receptor-related protein 5 (LRP5) and/or a LDL receptor-related protein 6 (LRP6) (LRP5/6 binding regions).

In some embodiments, at least two Fzd binding regions bind to different sets of one or more Fzd receptors, different sets of one or more epitopes within the same set of one or more Fzd receptors, or a combination thereof.

In some embodiments, each Fzd binding region binds to one or more of Frizzled 1 (Fzd1), Frizzled 2 (Fzd2), Frizzled 3 (Fzd3), Frizzled 4 (Fzd4), Frizzled 5 (Fzd5), Frizzled 6 (Fzd6), Frizzled 7 (Fzd7), Frizzled 8 (Fzd8), Frizzled 9 (Fzd9), and Frizzled 10 (Fzd10).

In some embodiments, at least one Fzd binding region binds to: (i) Fzd1, Fzd2, Fzd7, and Fzd9; (ii) Fzd1, Fzd2, and Fzd7; (iii) Fzd5 and Fzd8; (iv) Fzd5, Fzd7, and Fzd8; (v) Fzd1, Fzd4, Fzd5, and Fzd8; (vi) Fzd1, Fzd2, Fzd5, Fzd7, and Fzd8; (vii) Fzd4 and Fzd9; (viii) Fzd9 and Fzd10; (ix) Fzd5, Fzd8, and Fzd10; (x) Fzd4, Fzd5, and Fzd8; or (xi) Fzd1, Fzd5, Fzd7, and Fzd8.

In some embodiments, the plurality of Fzd binding regions comprises: (i) a first Fzd binding region that binds to a first set of one or more Fzd receptors, and (ii) a second Fzd binding region that binds to a second, different set of one or more Fzd receptors. In some embodiments, the first Fzd binding region binds to one or more of Fzd1, Fzd2, Fzd3, Fzd4, Fzd5, Fzd6, Fzd7, Fzd8, Fzd9, and Fzd10, and the second Fzd binding region binds to one or more of Fzd1, Fzd2, Fzd3, Fzd4, Fzd5, Fzd6, Fzd7, Fzd8, Fzd9, and Fzd10. In some embodiments, the first Fzd binding region binds to Fzd4 and the second Fzd binding region binds to Fzd9.

In some embodiments, the plurality of Fzd binding regions comprises: (i) a first Fzd binding region that binds to a first set of one or more epitopes within a set of one or more Fzd receptors, and (ii) a second Fzd binding region that binds to a second, different set of one or more epitopes within the same set of one or more Fzd receptors.

In some embodiments, the Wnt surrogate binds to at least one Fzd receptor that induces non-canonical Wnt signaling; and the second Fzd binding region binds to at least one Fzd receptor that induces canonical Wnt signaling. In a further embodiment, the Wnt surrogate binding to the first Fzd receptor and second Fzd receptor results in canonical Wnt signaling; or non-canonical Wnt signaling.

In some embodiments, at least one Fzd binding region binds monospecifically to a single Fzd receptor. In some embodiments, the at least one Fzd binding region binds monospecifically to Fzd1, Fzd2, Fzd3, Fzd4, Fzd5, Fzd6, Fzd7, Fzd8, Fzd9, or Fzd10.

In some embodiments, at least one Fzd binding region binds to a region of a Fzd receptor that (i) does not include the cysteine rich domain (CRD) of the Fzd receptor or (ii) includes less than the entire CRD of the FZD receptor or iii) partially overlap with the CRD of the FZD receptor.

In some embodiments, the at least one Fzd binding region binds to a hinge region of the Fzd receptor. In some embodiments, the hinge region comprises an amino acid sequence having at least 90% identity, at least 95% identity, or at least 98% identity to any of the sequences set forth in SEQ ID NO:98-107.

In some embodiments, the at least one Fzd binding region binds to an N-terminal region upstream of the CRD of the Fzd receptor. In some embodiments, the N-terminal region comprises an amino acid sequence having at least 90% identity, at least 95% identity, or at least 98% identity to SEQ ID NO:108.

In some embodiments, at least one of the Fzd binding regions comprises one or more antigen-binding fragments of an antibody. In some embodiments, the one or more antigen-binding fragments are selected from the group consisting of: IgG, scFv, Fab, and VHH or sdAbs. In some embodiments, the one or more antigen-binding fragments are humanized.

In some embodiments, at least one Fzd binding region comprises an amino acid sequence having at least 90% identity to any of the sequences set forth in Table 1A, Table 1B, SEQ ID NOs: 1-73, or an antigen-binding fragment thereof.

In some embodiments, the one or more LRP5/6 binding regions comprises one or more antigen-binding fragments of an antibody. In some embodiments, the one or more antigen-binding fragments are selected from the group consisting of: IgG, scFv, Fab, and VHH or sdAbs. In some embodiments, the one or more antigen-binding fragments are humanized. In some embodiments, the one or more LRP5/6 binding regions comprise an amino acid sequence having at least 90% identity to any of the sequences set forth in Table 2A, Table 2B, or SEQ ID NOs: 74-97, or an antigen-binding fragment thereof.

In some embodiments, the Wnt surrogate molecule comprises two or more LRP5/6 binding regions.

In some embodiments, the Fzd binding regions and the LRP5/6 binding regions are in a ratio of Fzd:LRP5/6.

In some embodiments, the Fzd binding regions and the LRP5/6 binding regions are in a ratio of Fzdₙ:LRP5/6ₙ (Fₙ:Lₙ), wherein F and L are integers between 1 and 9, inclusive, and n is an integer between 1 and 4 inclusive.

In some embodiments, the Fzd binding regions and the LRP5/6 binding regions are in a ratio of Fzd:LRP5/6 selected from the group consisting of: 1:1, 2:1, 3:1, 4:1, 5:1, 6:1, 2:1 2:3, 2:5, 2:7, 7:2, 5:2, 3:2, 3:4, 3:5, 3:7, 3:8, 8:3, 7:3, 5:3, 4:3, 4:5, 4:7, 4:9, 9:4, 7:4, 5:4, 6:7, 7:6, 1:2, 1:3, 1:4, 1:5, 1:6, 2:1 (with two Fzd binders and one LRP binder), 1:2 (with one Fzd binder and two LRP binders), 2:1:1 (with two different LRP binders), 1:1:2 (with two different Fzd binders), 1:1:1 (two different Fzd binders and one LRP binder or one Fzd binder and two different LRP binders) and 1:1:1:1 (all different Fzd and LRP binders).

In some embodiments, the ratio of Fzd binding regions to LRP5/6 binding regions (Fzd:LRP5/6) comprises 2 Fzd binding regions and 2 LRP5/6 binding regions; 2 Fzd binding regions and 1 LRP5/6 binding region; or 1 Fzd binding region and 2 LRP5/6 binding regions.

In some embodiments, the ratio of Fzd binding regions to LRP5/6 binding regions (Fzd:LRP5/6) comprises a first Fzd binding region, a second Fzd binding region, and 1 LRP5/6 binding region; or a first Fzd binding regions, a second Fzd binding regions, a first LRP5/6 binding region, and a second LRP5/6 binding region. In further embodiments, the first Fzd and second Fzd binding regions bind to different Fzd receptors, or bind to the same Fzd receptor on different regions/epitope, and the first LRP5/6 and second LRP5/6 binding regions bind to different epitopes or to different LRP proteins.

In some embodiments, the LRP binding regions comprise a first LRP binding region that binds to a first set of one or more LRP receptors, and a second LRP binding region that binds to a second, different set of one or more LRP receptors.

In some embodiments, the Wnt surrogate molecule comprises a structural format selected from the group consisting of: hetero-Ig, diabody (DART), tandem diabody (DART), diabody-Fc, Fabs-in-tandem, Fabs-in-tandem IgG (FIT-Ig), Fv-IgG, and tandem scFv.

In some embodiments, the Wnt surrogate molecule comprises: (i) a first light chain and a first heavy chain forming a first Fzd binding region, and (ii) a second light chain and a second heavy chain forming a second Fzd binding region, wherein the first and second Fzd binding regions bind to different sets of one or more Fzd receptor epitopes.

In some embodiments, the Wnt surrogate molecule comprises a first LRP5/6 binding region fused to an N-terminus of the first light chain, a C-terminus of the first light chain, an N-terminus of the first heavy chain, or a C-terminus of the first heavy chain. In some embodiments, the Wnt surrogate molecule comprises second LRP5/6 binding region fused to an N-terminus of the second light chain, a C-terminus of the second light chain, an N-terminus of the second heavy chain, or a C-terminus of the second heavy chain.

In some embodiments, the first and second heavy chains are connected to each other. In some embodiments, the first heavy chain comprises a first CH3 domain, the second heavy chain comprises a second CH3 domain, and the first and second CH3 domains are connected to each other. In some embodiments, the first and second CH3 domains are connected to each other via knobs-into-holes mutations. In some embodiments, the first heavy chain and/or the second heavy chain comprise an amino acid sequence having at least 90% identity, at least 95% identity, or at least 98% identity to any of the sequences set forth in SEQ ID NOs:110, 112, 114, 116, 118, 120, or 122 (or shown in Table 5 or Table 6A), and (ii) the first light chain and/or the second light chain comprise an amino acid sequence having at least 90% identity to any of the sequences set forth in SEQ ID NOs:109, 111, 113, 115, 117, 119, or 121 (or shown in Table 5 or Table 6A). In some embodiments, the Wnt surrogate molecule comprises one or more sequences (e.g., two or three sequences) having at least 90%, at least 95%, at least 98% or at least 99% sequence identity to a sequence disclosed in Table 5 or Table 6A. In particular embodiments, the Wnt surrogate molecule comprises the sequences set forth for any Wnt surrogate molecule disclosed in Table 5 or Table 6A, or sequences having at least 90%, at least 95%, at least 98%, or at least 99% identity to such sequences.

In another aspect, the Wnt surrogate molecule has a structure as set forth in Table 6B.

In some embodiments, the Wnt surrogate molecule modulates a Wnt signaling pathway in a cell, optionally a mammalian cell. In some embodiments, the Wnt surrogate molecule increases signaling via the Wnt signaling pathway in the cell. In some embodiments, the Wnt signaling pathway is a canonical Wnt signaling pathway. In some embodiments, the Wnt signaling pathway is a non-canonical Wnt signaling pathway.

In another aspect, the present disclosure provides a pharmaceutical composition comprising a pharmaceutically acceptable excipient, diluent, or carrier, and a Wnt surrogate molecule according to any of the embodiments herein.

In another aspect, the present disclosure provides a method for agonizing a Wnt signaling pathway in a cell, comprising contacting the cell with a Wnt surrogate molecule according to any of the embodiments herein, wherein the Wnt surrogate molecule is an agonist of a Wnt signaling pathway.

In another aspect, the present disclosure provides a method for treating a subject having a disease or disorder, comprising administering to the subject an effective amount of a pharmaceutical composition of any of the embodiments herein, wherein the Wnt surrogate molecule is an agonist of a Wnt signaling pathway.

In some embodiments, the disease or disorder is associated with reduced or impaired Wnt signaling, and/or wherein the subject would benefit from increased Wnt signaling. In some embodiments, the disease or disorder is selected from the group consisting of: bone fractures, stress fractures, vertebral compression fractures, osteoporosis, osteoporotic fractures, non-union fractures, delayed union fractures, spinal fusion, pre-operative optimization for spine surgeries, osteonecrosis, osseointegration of implants or orthopedic devices, osteogenesis imperfecta, bone grafts, tendon repair, tendon-bone integration, tooth growth and regeneration, maxillofacial surgery, dental implantation, periodontal diseases, maxillofacial reconstruction, osteonecrosis of the jaw, hip or femoral head, avascular necrosis, alopecia, hearing loss, vestibular hypofunction, macular degeneration, age-related macular degeneration (AMD), vitreoretinopathy, retinopathy, diabetic retinopathy, diseases of retinal degeneration, Fuchs' dystrophy, cornea diseases, stroke, traumatic brain injury, Alzheimer's disease, multiple sclerosis, muscular dystrophy, muscle atrophy caused by sarcopenia or chachexia, diseases affecting blood brain barrier (BBB), spinal cord injuries, spinal cord diseases, oral mucositis, short bowel syndrome, inflammatory bowel diseases (IBD), metabolic syndrome, diabetes, dyslipidemia, pancreatitis, exocrine pancreatic insufficiency, wound healing, diabetic foot ulcers, pressure sores, venous leg ulcers, epidermolysis bullosa, dermal hypoplasia, myocardial infarction, coronary artery disease, heart failure, hematopoietic cell disorders, immunodeficiencies, graft versus host diseases, acute kidney injuries, chronic kidney diseases, chronic obstructive pulmonary diseases (COPD), idiopathic pulmonary fibrosis, acute liver failure of all causes, acute liver failure drug-induced, alcoholic liver diseases, chronic liver failure of all causes, cirrhosis, liver fibrosis of all causes, portal hypertension, chronic liver insufficiency of all causes, nonalcoholic steatohepatitis (NASH), nonalcoholic fatty liver disease (NAFLD) (fatty liver), alcoholic hepatitis, hepatitis C virus-induced liver diseases (HCV), hepatitis B virus-induced liver diseases (HBV), other viral hepatitis (e.g., hepatitis A virus-induced liver diseases (HAV) and hepatitis D virus-induced liver diseases (HDV)), primary biliary cirrhosis, autoimmune hepatitis, livery surgery, liver injury, liver transplantation, "small for size" syndrome in liver surgery and transplantation, congenital liver disease and disorders, any other liver disorder or defect resulting from genetic diseases, degeneration, aging, drugs, and injuries.

In some embodiments, the disease or disorder is a bone disease or disorder. In some embodiments, the Wnt surrogate molecule binds: (i) Fzd1, Fzd2, and Fzd7; or (ii) Fzd1, Fzd2, Fzd5, Fzd7, and Fzd8.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
FIG. 1. Schematic diagrams of illustrative formats of Wnt surrogate molecules. Different VHH, Fv, or scFv, Diabody, or Fabs containing various VL and VH regions directed against different Fzd receptors and Lrp receptors are combined in various ratios. The different colors represent different binders (which can bind to the same target or different targets).
FIG. 2A. Schematic diagram of a Fzd receptor including a cysteine rich domain (CRD), hinge region, and N-terminal region.
FIG. 2B. Schematic diagram of a Wnt surrogate molecule with binding specificity for the Fzd receptor hinge region.
FIG. 2C. Binding kinetics of 1791-3 and 1291-3 Wnt surrogate molecules.
FIG. 2D. In vitro activity of 1791-3 and 1291-3 Wnt surrogate molecules.
FIG. 3A. Schematic diagrams of monospecific and multispecific Wnt surrogate molecules.
FIG. 3B. In vitro activity of Wnt surrogate molecules in 293STF cells.
FIG. 3C. In vitro activity of Wnt surrogate molecules in 293STF cells overexpressing Fzd4 (293STF Fzd4OE).
FIG. 3D. In vitro activity of Wnt surrogate molecules in 293STF cells overexpressing Fzd9 (293STF Fzd9OE).

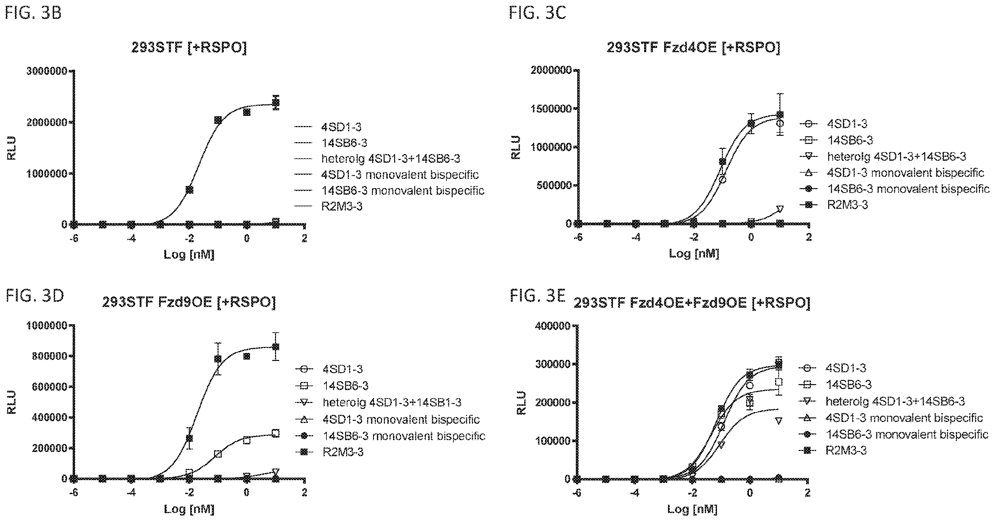

FIG. 3C. In vitro activity of Wnt surrogate molecules in 293STF cells overexpressing Fzd4 and Fzd9 (293STF Fzd4OE + Fzd9OE).

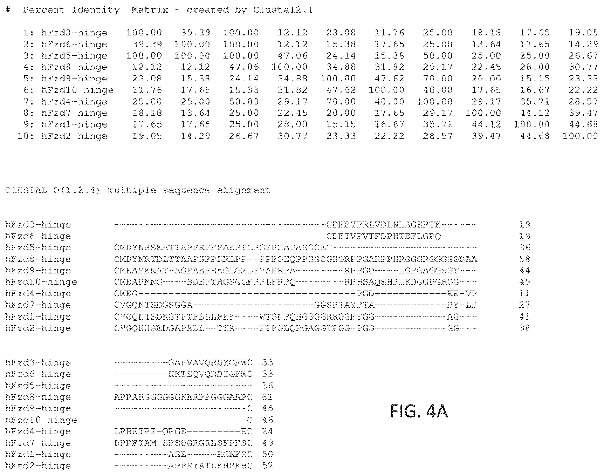

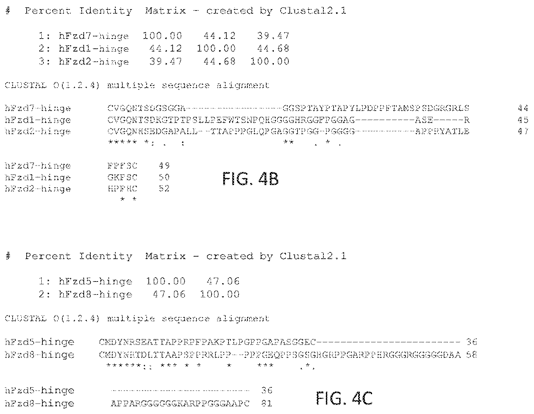

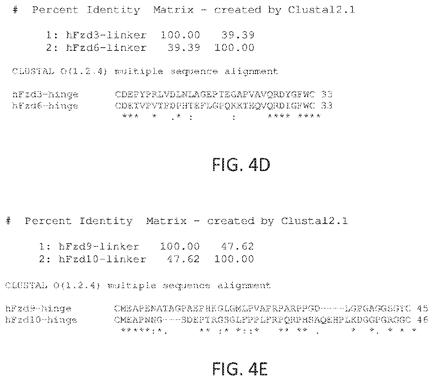

FIGS. 4A-4E show sequence alignments of the hinge region of various Fzds (SEQ ID NOs: 2251-2260).

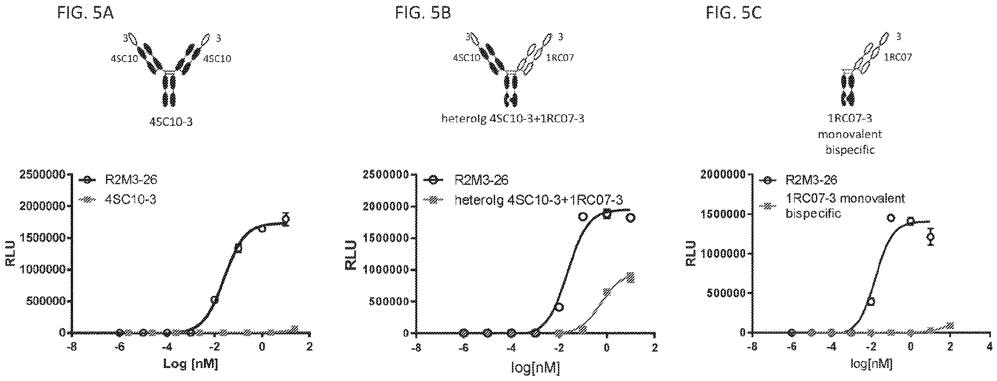

FIGS. 5A-5C show the structure of heterologous molecules containing soluble ligands for different Fzd receptors together with Lrp5, and their in vitro activity in 293STF cells.

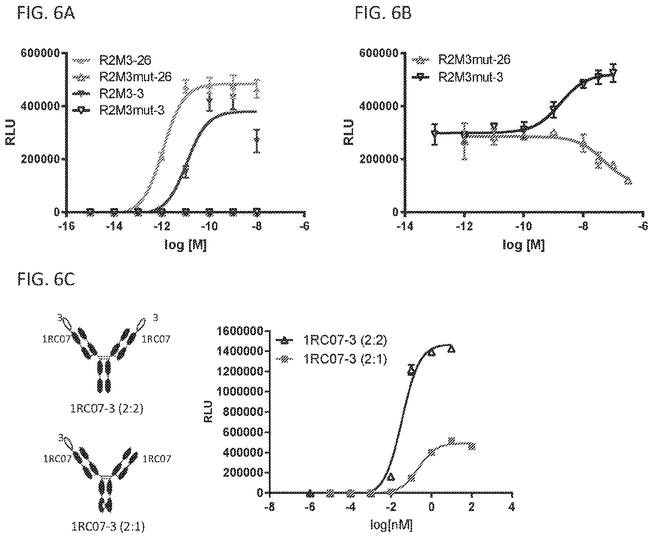

FIGS. 6A-6E show the structures of Wnt surrogate molecules with different ratios of Fzd to Lrp binders and their impact on Wnt3a activation of beta-catenin-dependent signaling.

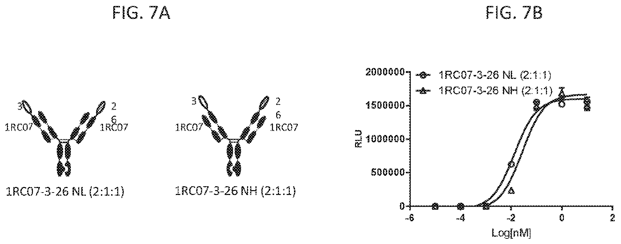

FIGS. 7A-7D show structures containing heterodimerizion of two different Lrp binders together with Fzd binders and their in vitro activity in 293STF cells.

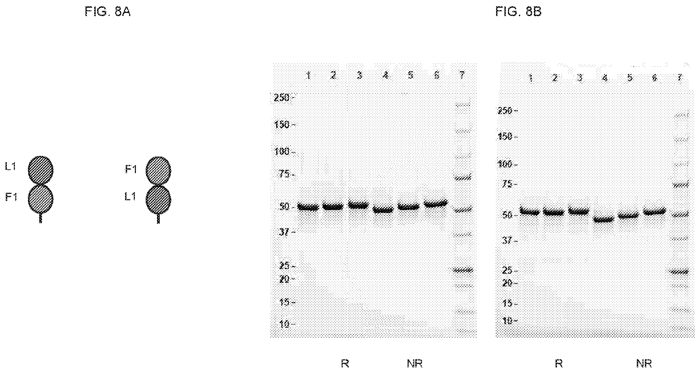

FIGS. 8A-8J show 1:1 bivalent bispecific L1/F1 tandem scFv molecules are not efficient in activating β-catenin dependent WNT signaling. (A) Diagram of 1:1 bivalent bispecific L1/F1 tandem scFv constructs. Each circle represents a scFv domain, the thin black line at the end of each molecule represent the 6xHis tag. (B) Ni resin purified tandem scFv molecules were separated on 4-15% SDS-PAGE gel. Left panel, from left to right: L1-F1 tandem scFv with 5-mer, 10-mer and 15-mer linkers under reducing (R, lanes 1-3) or nonreducing (NR, lanes 4-6) conditions. Right panel, from left to right, F1-L1 tandem scFv with 5-mer, 10-mer and 15-mer linkers under reducing (R, lanes 1-3) or nonreducing (NR, lanes 4-6) conditions. C) and D) The dose dependent STF activity of the Ni resin purified tandem scFv with L1 fused to the N-terminus of F1 (C), or F1 fused to the N-terminus of L1 (D). E) The dose dependent STF activity of the monomer peak fractions from the size exclusive column (SEC) polished tandem scFv with L1 fused to the N-terminus of F1. F) The dose dependent STF activity of the monomer peak fractions from the SEC polished tandem scFv with F1 fused to the N-terminus of L1. G) and H) The STF activity across the SEC fractions of various tandem scFv. The arrows on each panel indicate the position of the monomeric tandem scFv protein. I) The elution profile of the protein standard on the SEC column, thick arrow indicates the expected position of the monomeric form of tandem scFv molecules. J) The addition of anti-His antibody to L1:5:F1 induced significant activation of β-catenin dependent WNT signaling.

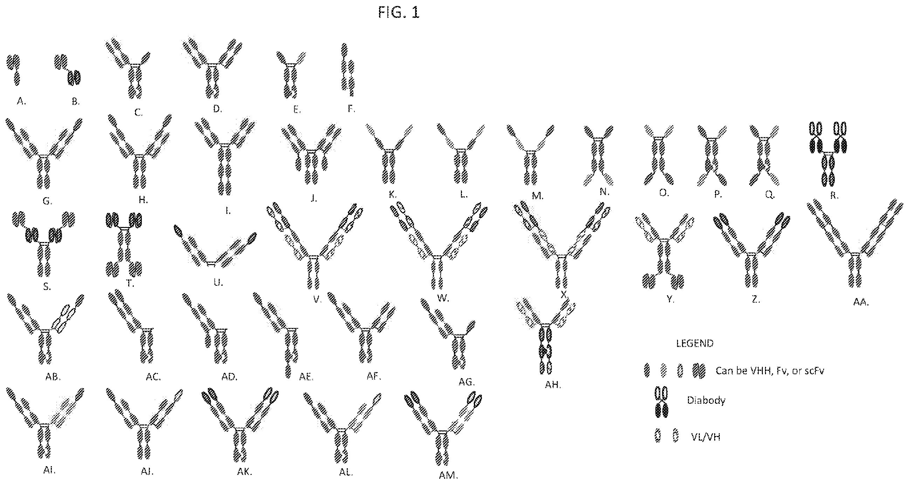

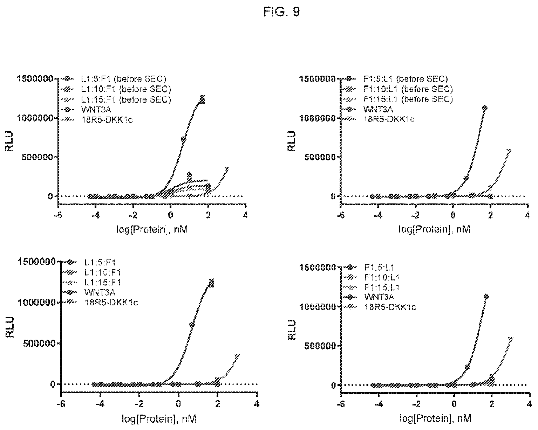

FIG. 9. 1:1 bivalent bispecific L1/F1 tandem scFv molecules are not efficient in activating β-catenin dependent WNT signaling. The dose dependent STF activity of the tandem scFvs, purified either from Ni column alone or additionally purified from SEC column, with L1 fused to the N-terminus of F1 and F1 fused to the N-terminus of L1 comparing to recombinant WNT3A and the surrogate WNT, 18R5-DKK1c. These data are identical to Fig. 1C-1F, except with the positive control molecule data included.

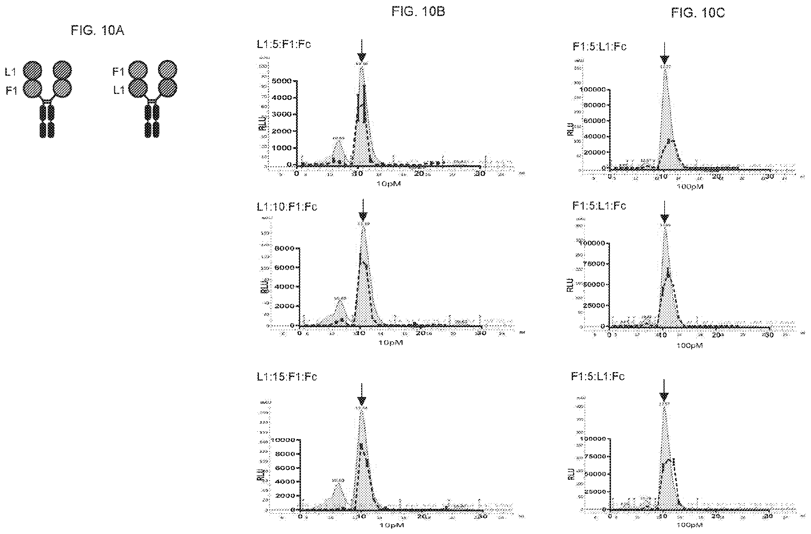

FIGS. 10A-10F. Increasing the valency of L1 and F1 tandem scFv by fusing to a Fc domain significantly increased the activity in Wnt signal. A) Diagram of F1 and L1 tandem scFv fused to Fc domain to generate the 2:2 tetravalent bispecific formats. B-C) The STF activity across the SEC fractions of the tandem scFv-Fc molecules. D) The elution profile of the protein standard on the SEC column, thick arrow indicates the expected position of the monomeric form of tandem scFv-Fc molecules. E-F) The dose dependent STF activity of the tandem scFv-Fc molecules from the protein peak fractions corresponding to the monomeric forms of the molecules from SEC column.

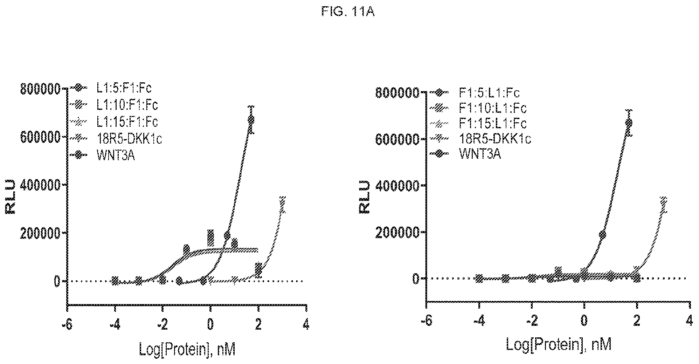

FIGS. 11A-11B. The STF activity and Octet binding profiles of the 2:2 tetravalent bispecific F1/L1 molecules. A) The dose dependent STF activity of the F1/L1 bivalent tandem scFv molecules from the protein peak fractions corresponding to the monomeric forms comparing to the recombinant WNT3A and 18R5-DKK1c. These data are identical to Fig. 2E and 2F, except with the positive control molecule data included. B) The binding affinity of the various 2:2 tetravalent bispecific tandem scFv molecules to FZD1 and LRP6E1E2 measured on the Octet.

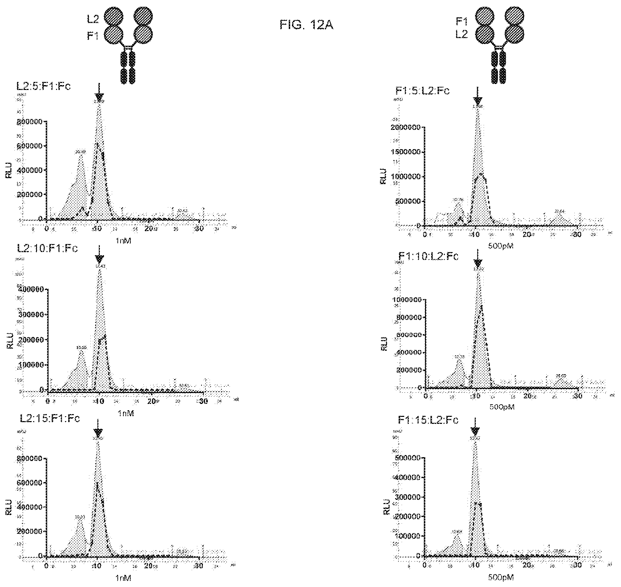

FIGS. 12A-12E. The 2:2 tetravalent bispecific molecules, consisting of the two F1 and two L2 binding arms, are highly potent in inducing Wnt signaling. A) Diagrams representing the 2:2 tetravalent bispecific molecule formats consisting of the FZD binder F1 and the LRP6E3E4 binder L2. The STF activities across the SEC fractions of these various 2:2 molecules are shown below the format diagrams. B-C) The dose dependent STF activities of the 2:2 tetravalent bispecific molecules consisting of F1 and L2 binding arms in both orientations, from the protein peak fractions corresponding to the monomeric forms of each molecules from SEC column. D) The activity of the 2:2 tetravalent bispecific molecules from the combination of F1 and L2 needs the presence of both FZD and LRP6 binding arms, as the substitution of either binding arm by the neutral anti-GFP scFv fragment resulted in no activity. E) Interaction of the molecules from D) to their respective receptors were determined by Octet. The binders L1, L2, and F1 in the IgG1 format were also included as comparators.

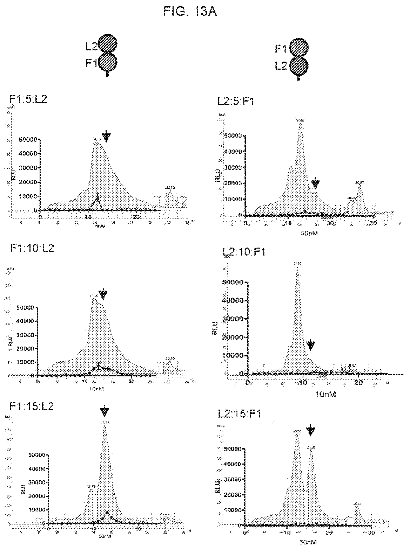

FIGS. 13A-13B. 1:1 bivalent bispecific L2/F1 tandem scFv molecules are not efficient in activating β-catenin dependent WNT signaling. A) The STF activity across the SEC fractions of the 1:1 bivalent bispecific tandem scFv molecules between F1/L2 in both orientations. The molecular format diagrams are also shown on top. The arrows in each panel indicate the position of the monomeric forms of the proteins. B) The dose dependent STF activities of the molecules shown in A) from the protein peak fractions corresponding to the monomeric forms of each molecule from SEC column.

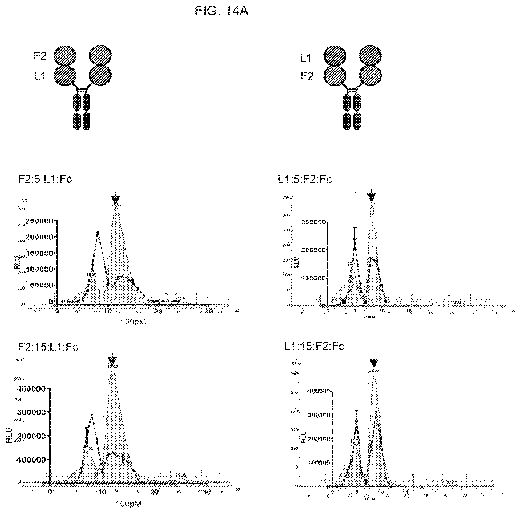

FIGS. 14A-14I. The 2:2 tetravalent bispecific molecules, consisting of the two F2 and two L1 or L2 binding arms, activate Wnt signaling. A) The molecular format diagrams of the 2:2 tetravalent bispecific molecules consisting of F2 and L1 binding arms and the STF activities across the SEC column of these various surrogate WNT molecules. The arrows in each panel indicate the position of the monomeric forms of the proteins. B) The dose dependent STF activities of the 2:2 tetravalent bispecific molecules consisting of F2 and L1 binding arms in both orientations, from the protein peak fractions corresponding to the monomeric forms of each molecules from SEC column. These surrogate WNT agonists have higher potency but lower efficacy compared to WNT3A in activating Wnt signal. C) and D) The 1:1 bivalent bispecific molecular formats and STF activities across SEC column fractions for molecules consisting of F2 and L2 combinations in both orientations. The F2-L2 orientation does not appear to be active while the reverse L2-F2 orientation seems to be active in the 1:1 format. The arrows in each panel indicate the position of the monomeric forms of the proteins. E) The dose dependent STF activities of the 1:1 bivalent bispecific L2-F2 molecules from the protein peak fractions corresponding to the monomeric forms of each molecules from SEC column. F) and H) The 2:2 tetravalent bispecific molecular formats and STF activities across SEC column fractions for molecules consisting of F2 and L2 combinations in both orientations. The arrows in each panel indicate the position of the monomeric forms of the proteins. G) and I) The dose dependent STF activities of the 2:2 tetravalent bispecific L2/F2 molecules of F) and H) from the protein peak fractions corresponding to the monomeric forms of each molecules from SEC column.

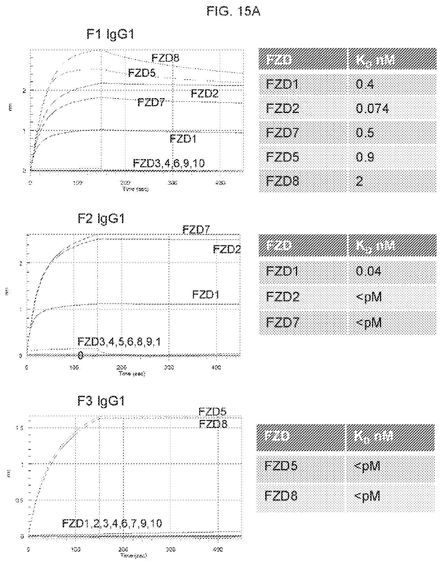

FIGS. 15A-15B. FZD specific profile of F1, F2, F3, and the STF activities of the 1:1 bivalent bispecific F2/L1 molecules. A) The binding affinity and specificity of F1, F2, and F3 to all 10 FZDs measured on Octet. B) The STF activity across the SEC fractions of the 1:1 bivalent bispecific tandem scFv molecules between F2/L1 in both orientations. The molecular format diagrams are also shown on top. The arrows in each panel indicate the position of the monomeric forms of the proteins. The 1:1 bivalent bispecific format is ineffective in inducing Wnt/β-catenin signaling.

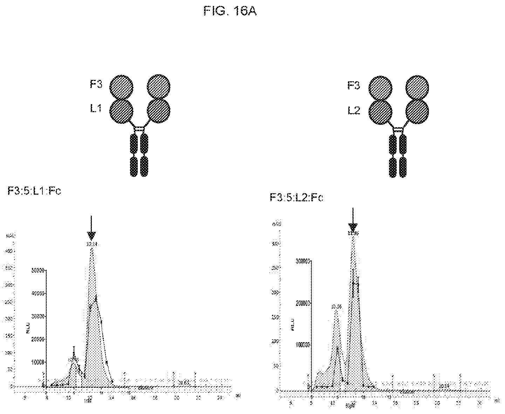

FIGS 16A-16D. The 2:2 tetravalent bispecific molecules, consisting of the two F3 and two L1 or L2 binding arms, activate Wnt signaling. A) Diagrams representing the 2:2 tetravalent bispecific molecule formats consisting of the FZD binder F3 and the LRP6 binders L1 or L2 where the FZD binder is attached to the N-terminus of the LRP binders. The STF activities across the SEC fractions of these two 2:2 molecules are shown below the format diagrams. B) Diagrams representing the 2:2 tetravalent bispecific molecule formats consisting of the FZD binder F3 and the LRP6 binders L1 or L2 where the LRP binder is attached to the N-terminus of the FZD binder, the reverse orientation of molecules in A). The STF activities across the SEC fractions of these two 2:2 molecules are shown below the format diagrams. C,D) The dose dependent STF activities of the 2:2 tetravalent bispecific molecules from the protein peak fractions corresponding to the monomeric forms of each molecules from SEC column. C) and D) correspond to molecules from A) and B), respectively.

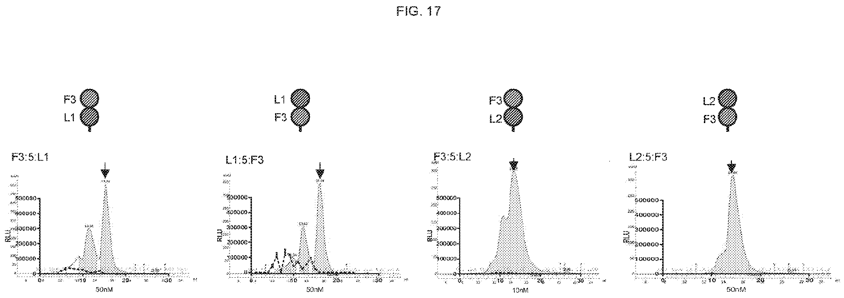

FIG. 17. The 1:1 bivalent bispecific L1/F3 or L2/F3 tandem scFv molecules are not efficient in activating β-catenin dependent WNT signaling. The STF activity across the SEC fractions of the 1:1 bivalent bispecific tandem scFv molecules between F3/L1 or F3/L2 in both orientations. The molecular format diagrams are also shown on top. The arrows in each panel indicate the position of the monomeric forms of the proteins. The 1:1 bivalent bispecific format is ineffective in inducing Wnt/β-catenin signaling.

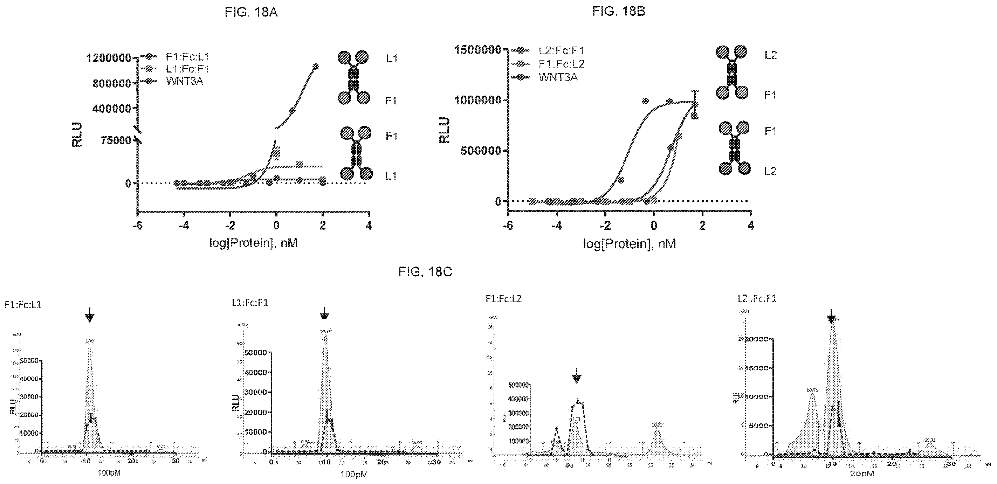

FIGS. 18A-18B. The 2:2 tetravalent bispecific dumbbell format has similar activity to the 2:2 tetravalent bispecific tandem scFv-Fc format. A) The dose dependent STF activity of the protein peak fractions corresponding to the monomeric forms of each molecules from SEC column. The surrogate WNT agonists tested here are the combination of F1 and L1 in the 2:2 tetravalent bispecific dumbbell format. This format is active, however, show a much lower efficacy compare to WNT3A. There is a preference for L1 to be on the N-terminus of Fc. B) The dose dependent STF activity of the protein peak fractions corresponding to the monomeric forms of each molecules from SEC column. The surrogate WNT agonists tested here are the combination of F1 and L2 in the 2:2 tetravalent bispecific dumbbell format. There is also a preference for L2 to be on the N-terminus of Fc.

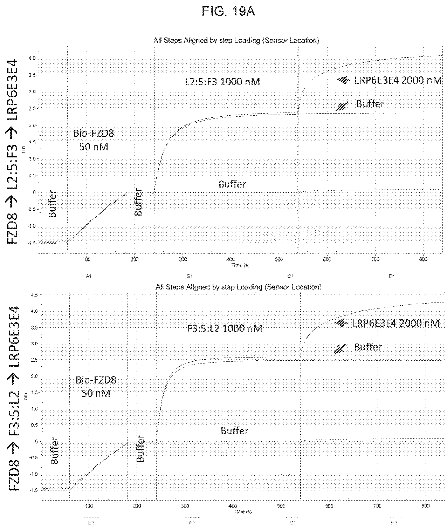

FIGS. 19A-19C. Various 1:1 bivalent bispecific tandem scFv molecules show little to no activity. A) Sequencial binding of FZD8, followed by 1:1 bivalent bispecific tandem scFv molecules, F3:5:L2 and L2:5:F3, then followed by additional of LRP6E3E4 on Octet show that the 1:1 tandem scFv molecules can simultaneously engage both FZD and LRP. B) The diagram of various molecules with the 1:1 stoichiometry between FZD and LRP binders. C) The dose response of the various molecules in B) showed no induction of STF signals.

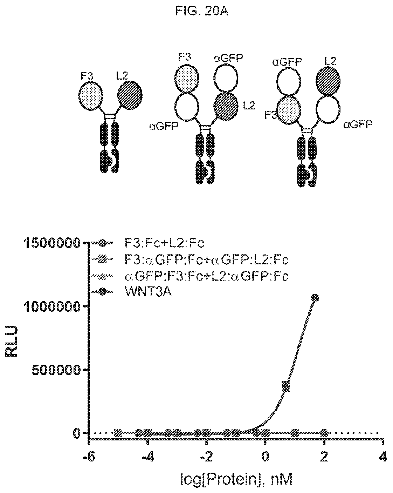

FIG 20A-20B. Various 1:1 bispecific scFv molecules show little to no activity. The diagrams of various combinations of 1:1 bivalent bispecific scFv molecules between F3/L2 and F2/L2 binder pairs. B) The dose response of the various molecules depicted in A,B) showed no induction of STF signals.

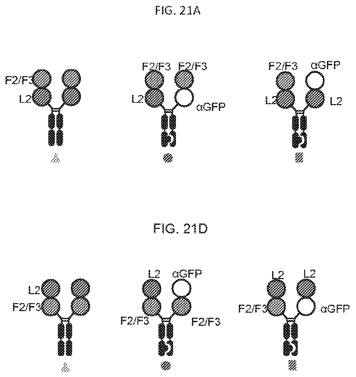

FIGS. 21A-21K. Exploring different stoichiometries of FZD and LRP binders and combining binders of different receptor specificities or epitopes in the 2:2 tetravalent multispecific formats. A,D) The diagrams of molecules with different stoichiometries between FZD and LRP binders, such as 2 FZD binders and 1 LRP binders (2:1) or 1 FZD and 2 LRP binders (1:2). B,C) Dose response of molecules in A) in STF reporter assays. E,F) Dose response of molecules in D) in STF reporter assays. G) Molecular formats of 2:2 tetravalent trispecific molecule where the two FZD binders are of different FZD specificity (1:1:2). H) Dose response of molecules in G) in STF reporter assays. I) Molecular formats of 2:2 tetravalent trispecific molecule where the two FZD binders are of different FZD specificity together with only one LRP binder (1:1:1:0). J) Dose response of the molecules in I) in STF reporter assays. K) Molecular formats of 2:2 tetravalent trispecific molecule where the two FZD binders and the two LRP binders are all of different FZD or LRP specificities (1:1:1:1). H) Dose response of the molecules in K) in STF reporter assays.

### DETAILED DESCRIPTION

The present disclosure relates to multispecific Wnt surrogate molecules that specifically bind to a plurality of different Fzd receptors and epitopes and to LRP5 and/or LRP6 in order to modulate a Wnt signaling pathway. In particular embodiments, the Wnt surrogate molecules activate a Wnt signaling pathway or increase signaling via a Wnt signaling pathway. In certain aspects, the Wnt surrogate molecules of the present disclosure have: (i) a plurality of regions that each specifically bind to a set of one or more Frizzled (Fzd) receptors and/or epitopes, referred to herein as "Fzd binding regions;" and (ii) one or more regions that specifically bind to a LRP5 and/or a LRP6, referred to herein as "LRP5/6 binding regions." Certain embodiments encompass specific structural formats or arrangements of the Fzd binding regions and the LRP5/6 binding regions that are advantageous in modulating Wnt signaling pathways and related biological effects, e.g., for the treatment of diseases and disorders associated with Wnt signaling.

In particular embodiments, the Wnt surrogate molecules disclosed herein include multiple Fzd binding regions with binding specificities for different Fzd receptors and/or epitopes. For example, a Wnt surrogate molecule may include at least two Fzd binding regions that each bind to different sets of one or more Fzd receptors, different sets of one or more epitopes within the same set of one or more Fzd receptors, or a combination thereof. Each Fzd binding region may be monospecific, bispecific, trispecific, etc. for a different Fzd receptor epitope or plurality of Fzd receptor epitopes. Such multispecific Wnt surrogate molecules are capable of selectively activating specific combinations of Fzd receptors, while reducing or eliminating activation of non-targeted Fzd receptors. Embodiments of the present disclosure are advantageous for selectively modulating Wnt signaling in a target cell type and/or for the treatment of a specific disease or disorder, e.g., by reducing off-target effects.

Embodiments of the invention pertain to the use of Wnt surrogate molecules for the diagnosis, assessment and treatment of diseases and disorders associated with Wnt signaling pathways. In certain embodiments, the subject Wnt surrogate molecules are used to modulate a Wnt signaling pathway in a cell or tissue. In certain embodiments, the subject Wnt surrogate molecules are used in the treatment or prevention of diseases and disorders associated with aberrant or deregulated (e.g., reduced) Wnt signaling, or for which modulating, e.g., increasing, Wnt signaling would provide a therapeutic benefit.

The practice of the present disclosure will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, e.g., Current Protocols in Molecular Biology or Current Protocols in Immunology, John Wiley & Sons, New York, N.Y.(2009); Ausubel et al., Short Protocols in Molecular Biology, 3rd ed., Wiley & Sons, 1995; Sambrook and Russell, Molecular Cloning: A Laboratory Manual (3rd Edition, 2001); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984) and other like references.

As used in this specification and the appended claims, the singular forms "a," "an" and "the" include plural references unless the content clearly dictates otherwise.

As used herein, "A and/or B" encompasses one or more of A or B, and combinations thereof such as A and B.

Throughout this specification, unless the context requires otherwise, the word "comprise", or variations such as "comprises" or "comprising", will be understood to imply the inclusion of a stated element or integer or group of elements or integers but not the exclusion of any other element or integer or group of elements or integers.

Each embodiment in this specification is to be applied *mutatis mutandis* to every other embodiment unless expressly stated otherwise.

Standard techniques may be used for recombinant DNA, oligonucleotide synthesis, and tissue culture and transformation (e.g., electroporation, lipofection). Enzymatic reactions and purification techniques may be performed according to manufacturer's specifications or as commonly accomplished in the art or as described herein. These and related techniques and procedures may be generally performed according to conventional methods well known in the art and as described in various general and more specific references that are cited and discussed throughout the present specification. Unless specific definitions are provided, the nomenclature utilized in connection with, and the laboratory procedures and techniques of, molecular biology, analytical chemistry, synthetic organic chemistry, and medicinal and pharmaceutical chemistry described herein are those well-known and commonly used in the art. Standard techniques may be used for recombinant technology, molecular biological, microbiological, chemical syntheses, chemical analyses, pharmaceutical preparation, formulation, and delivery, and treatment of subjects.

Embodiments of the present disclosure relate to antibodies and antigen-binding fragments thereof that bind to one or more Fzd receptors. Sequences of illustrative antibodies, or antigen-binding fragments, or complementarity determining regions (CDRs) thereof, are set forth in SEQ ID NOs:1-73, Tables 1A and 1B, and Table 5.

Embodiments of the present disclosure relate to antibodies and antigen-binding fragments thereof that bind to LRP5 and/or LRP6. Sequences of illustrative antibodies, or antigen-binding fragments, or complementarity determining regions (CDRs) thereof, are set forth in SEQ ID NOs:74-97, Tables 2A and 2B, and Table 5.

As is well known in the art, an antibody is an immunoglobulin molecule capable of specific binding to a target, such as a carbohydrate, polynucleotide, lipid, polypeptide, etc., through at least one epitope recognition site, located in the variable region of the immunoglobulin molecule. As used herein, the term encompasses not only intact polyclonal or monoclonal antibodies, but also fragments thereof (such as dAb, Fab, Fab', F(ab')₂, Fv), single chain (scFv), Nanobodies^{®} (Nabs; also referred to as VHH or single-domain antibodies (sdAbs)), synthetic variants thereof, naturally occurring variants, fusion proteins comprising an antibody or an antigen-binding fragment thereof, humanized antibodies, chimeric antibodies, and any other modified configuration of the immunoglobulin molecule that comprises an antigen-binding site or fragment (epitope recognition site) of the required specificity. "Diabodies," multivalent or multispecific fragments constructed by gene fusion (WO94/13804; P. Holliger et al., Proc. Natl. Acad. Sci. USA 90 6444-6448, 1993) are also a particular form of antibody contemplated herein. Minibodies comprising a scFv joined to a CH3 domain are also included herein (S. Hu et al., Cancer Res., 56, 3055-3061, 1996). See e.g., Ward, E. S. et al., Nature 341, 544-546 (1989); Bird et al., Science, 242, 423-426, 1988; Huston et al., PNAS USA, 85, 5879-5883, 1988); PCT/US92/09965; WO94/13804; P. Holliger et al., Proc. Natl. Acad. Sci. USA 90 6444-6448, 1993; Y. Reiter et al., Nature Biotech, 14, 1239-1245, 1996; S. Hu et al., Cancer Res., 56, 3055-3061, 1996.

The term "antigen-binding fragment" as used herein refers to a polypeptide fragment that contains at least one CDR of an immunoglobulin heavy and/or light chain, or of a VHH or sdAb, that binds to the antigen of interest, in particular to one or more Fzd receptors, or to LRP5 and/or LRP6. In this regard, an antigen-binding fragment of the herein described antibodies may comprise 1, 2, 3, 4, 5, or all 6 CDRs of a VH and VL sequence set forth herein from antibodies that bind one or more Fzd receptors or LRP5 and/or LRP6. In particular embodiments, an antigen-binding fragment may comprise all three VH CDRs or all three VL CDRs. Similarly, an antigen-binding fragment thereof may comprise all three CDRs of a VHH or sdAb. An antigen-binding fragment of a Fzd-specific antibody is capable of binding to a Fzd receptor. An antigen-binding fragment of a LRP5/6-specific antibody is capable of binding to a LRP5 and/or LRP6 receptor. As used herein, the term encompasses not only isolated fragments but also polypeptides comprising an antigen-binding fragment of an antibody disclosed herein, such as, for example, fusion proteins comprising an antigen-binding fragment of an antibody disclosed herein, such as, e.g., a fusion protein comprising a VHH or sdAb that binds one or more Fzd receptors and a VHH or sdAb that binds LRP5 and/or LRP6.

The term "antigen" refers to a molecule or a portion of a molecule capable of being bound by a selective binding agent, such as an antibody, and additionally capable of being used in an animal to produce antibodies capable of binding to an epitope of that antigen. In certain embodiments, a binding agent (e.g., a Wnt surrogate molecule or binding region thereof) is said to specifically bind an antigen when it preferentially recognizes its target antigen in a complex mixture of proteins and/or macromolecules. In certain embodiments, a Wnt surrogate molecule or binding region thereof (e.g., an antibody or antigen-binding fragment thereof) is said to specifically bind an antigen when the equilibrium dissociation constant is ≤10⁻⁷ or ≤10⁻⁸ M. In some embodiments, the equilibrium dissociation constant may be ≤10⁻⁹ M or ≤10⁻¹⁰ M.

In certain embodiments, antibodies and antigen-binding fragments thereof as described herein include a heavy chain and a light chain CDR set, respectively interposed between a heavy chain and a light chain framework region (FR) set which provide support to the CDRs and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (e.g., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRs. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures-regardless of the precise CDR amino acid sequence. Further, certain FR residues are known to participate in noncovalent interdomain contacts which stabilize the interaction of the antibody heavy and light chains.

The structures and locations of immunoglobulin CDRs and variable domains may be determined by reference to Kabat, E. A. et al., Sequences of Proteins of Immunological Interest. 4th Edition. US Department of Health and Human Services. 1987, and updates thereof, now available on the Internet (immuno.bme.nwu.edu). The Abgenesis software from Distributed Bio (South San Francisco, CA) was used to map the specificity determining regions of the antibodies disclosed herein, which include the Kabat definition of CDRs. (Padlan et al. FASEB J. 9, 133-139 (1995).

A "monoclonal antibody" refers to a homogeneous antibody population wherein the monoclonal antibody is comprised of amino acids (naturally occurring and non-naturally occurring) that are involved in the selective binding of an epitope. Monoclonal antibodies are highly specific, being directed against a single epitope. The term "monoclonal antibody" encompasses not only intact monoclonal antibodies and full-length monoclonal antibodies, but also fragments thereof (such as Fab, Fab', F(ab')₂, Fv), single chain (scFv), VHH or sdAbs, variants thereof, fusion proteins comprising an antigen-binding fragment of a monoclonal antibody, humanized monoclonal antibodies, chimeric monoclonal antibodies, and any other modified configuration of the immunoglobulin molecule that comprises an antigen-binding fragment (epitope recognition site) of the required specificity and the ability to bind to an epitope, including Wnt surrogate molecules disclosed herein. It is not intended to be limited as regards the source of the antibody or the manner in which it is made (e.g., by hybridoma, phage selection, recombinant expression, transgenic animals, etc.). The term includes whole immunoglobulins as well as the fragments etc. described above under the definition of "antibody".

The proteolytic enzyme papain preferentially cleaves IgG molecules to yield several fragments, two of which (the F(ab) fragments) each comprise a covalent heterodimer that includes an intact antigen-binding site. The enzyme pepsin is able to cleave IgG molecules to provide several fragments, including the F(ab')₂ fragment which comprises both antigen-binding sites. An Fv fragment for use according to certain embodiments of the present disclosure can be produced by preferential proteolytic cleavage of an IgM, and on rare occasions of an IgG or IgA immunoglobulin molecule. Fv fragments are, however, more commonly derived using recombinant techniques known in the art. The Fv fragment includes a noncovalent V_{H}::V_{L} heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

In certain embodiments, single chain Fv or scFV antibodies are contemplated. For example, Kappa bodies (III et al., Prot. Eng. 10: 949-57 (1997)); minibodies (Martin et al., EMBO J 13: 5305-9 (1994)); diabodies (Holliger et al., PNAS 90: 6444-8 (1993)); or Janusins (Traunecker et al., EMBO J 10: 3655-59 (1991) and Traunecker et al., Int. J. Cancer Suppl. 7: 51-52 (1992)), may be prepared using standard molecular biology techniques following the teachings of the present application with regard to selecting antibodies having the desired specificity. In still other embodiments, bispecific or chimeric antibodies may be made that encompass the ligands of the present disclosure. For example, a chimeric antibody may comprise CDRs and framework regions from different antibodies, while bispecific antibodies may be generated that bind specifically to one or more Fzd receptors through one binding domain and to a second molecule through a second binding domain. These antibodies may be produced through recombinant molecular biological techniques or may be physically conjugated together.

A single chain Fv (scFv) polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated-but chemically separated-light and heavy polypeptide chains from an antibody V region into an scFv molecule which will fold into a three dimensional structure substantially similar to the structure of an antigen-binding site. See, e.g., U.S. Patent Nos. 5,091,513 and 5,132,405, to Huston et al.; and U.S. Patent No. 4,946,778, to Ladner et al.

In certain embodiments, an antibody as described herein is in the form of a diabody. Diabodies are multimers of polypeptides, each polypeptide comprising a first domain comprising a binding region of an immunoglobulin light chain and a second domain comprising a binding region of an immunoglobulin heavy chain, the two domains being linked (e.g., by a peptide linker) but unable to associate with each other to form an antigen binding site: antigen binding sites are formed by the association of the first domain of one polypeptide within the multimer with the second domain of another polypeptide within the multimer (WO94/13804).

A dAb fragment of an antibody consists of a VH domain (Ward, E. S. et al., Nature 341, 544-546 (1989)).

Where bispecific antibodies are to be used, these may be conventional bispecific antibodies, which can be manufactured in a variety of ways (Holliger, P. and Winter G., Current Opinion Biotechnol. 4, 446-449 (1993)), e.g., prepared chemically or from hybrid hybridomas, or may be any of the bispecific antibody fragments mentioned above. Diabodies and scFv can be constructed without an Fc region, using only variable domains, potentially reducing the effects of anti-idiotypic reaction.

Bispecific diabodies, as opposed to bispecific whole antibodies, may also be particularly useful because they can be readily constructed and expressed in *E*. *coli.* Diabodies (and many other polypeptides such as antibody fragments) of appropriate binding specificities can be readily selected using phage display (WO94/13804) from libraries. If one arm of the diabody is to be kept constant, for instance, with a specificity directed against antigen X, then a library can be made where the other arm is varied and an antibody of appropriate specificity selected. Bispecific whole antibodies may be made by knobs-into-holes engineering (J. B. B. Ridgeway et al., Protein Eng., 9, 616-621 (1996)).

In certain embodiments, the antibodies described herein may be provided in the form of a UniBody^{®}. A UniBody^{®} is an IgG4 antibody with the hinge region removed (see GenMab Utrecht, The Netherlands; see also, e.g., US20090226421). This proprietary antibody technology creates a stable, smaller antibody format with an anticipated longer therapeutic window than current small antibody formats. IgG4 antibodies are considered inert and thus do not interact with the immune system. Fully human IgG4 antibodies may be modified by eliminating the hinge region of the antibody to obtain half-molecule fragments having distinct stability properties relative to the corresponding intact IgG4 (GenMab, Utrecht). Halving the IgG4 molecule leaves only one area on the UniBody^{®} that can bind to cognate antigens (*e.g*., disease targets) and the UniBody^{®} therefore binds univalently to only one site on target cells.

In certain embodiments, the antibodies of the present disclosure may take the form of a VHH or sdAb. VHH or sdAb technology was originally developed following the discovery and identification that *camelidae* (e.g., camels and llamas) possess fully functional antibodies that consist of heavy chains only and therefore lack light chains. These heavy-chain only antibodies contain a single variable domain(V_{HH}) and two constant domains (C_{H}2, C_{H}3). The cloned and isolated single variable domains have full antigen binding capacity and are very stable. These single variable domains, with their unique structural and functional properties, form the basis of "VHH or sdAbs". VHH or sdAbs are encoded by single genes and are efficiently produced in almost all prokaryotic and eukaryotic hosts, e.g., *E. coli* (see, e.g., U.S. Pat. No. 6,765,087), molds (for example *Aspergillus* or *Trichoderma*) and yeast (for example *Saccharomyces, Kluyvermyces, Hansenula* or *Pichia* (see, e.g., U.S. Pat. No. 6,838,254). The production process is scalable and multi-kilogram quantities of VHH or sdAbs have been produced. VHH or sdAbs may be formulated as a ready-to-use solution having a long shelf life. The Nanoclone^{®} method (see, e.g., WO 06/079372) is a proprietary method for generating VHH or sdAbs against a desired target, based on automated high-throughput selection of B-cells. VHH or sdAbs are single-domain antigen-binding fragments of camelid-specific heavy-chain only antibodies. VHH or sdAbs typically have a small size of around 15 kDa.

In certain embodiments, the antibodies or antigen-binding fragments thereof as disclosed herein are humanized. This refers to a chimeric molecule, generally prepared using recombinant techniques, having an antigen-binding site derived from an immunoglobulin from a non-human species and the remaining immunoglobulin structure of the molecule based upon the structure and/or sequence of a human immunoglobulin. The antigen-binding site may comprise either complete variable domains fused onto constant domains or only the CDRs grafted onto appropriate framework regions in the variable domains. Epitope binding sites may be wild type or modified by one or more amino acid substitutions. This eliminates the constant region as an immunogen in human individuals, but the possibility of an immune response to the foreign variable region remains (LoBuglio, A. F. et al., (1989) Proc Natl Acad Sci USA 86:4220-4224; Queen et al., PNAS (1988) 86:10029-10033; Riechmann et al., Nature (1988) 332:323-327). Illustrative methods for humanization of the anti-Fzd antibodies disclosed herein include the methods described in U.S. Pat. No. 7,462,697.

Another approach focuses not only on providing human-derived constant regions, but also modifying the variable regions as well so as to reshape them as closely as possible to human form. It is known that the variable regions of both heavy and light chains contain three complementarity-determining regions (CDRs) which vary in response to the epitopes in question and determine binding capability, flanked by four framework regions (FRs) which are relatively conserved in a given species and which putatively provide a scaffolding for the CDRs. When nonhuman antibodies are prepared with respect to a particular epitope, the variable regions can be "reshaped" or "humanized" by grafting CDRs derived from nonhuman antibody on the FRs present in the human antibody to be modified. Application of this approach to various antibodies has been reported by Sato, K., et al., (1993) Cancer Res 53:851-856; Riechmann, L., et al., (1988) Nature 332:323-327; Verhoeyen, M., et al., (1988) Science 239:1534-1536; Kettleborough, C. A., et al., (1991) Protein Engineering 4:773-3783; Maeda, H., et al., (1991) Human Antibodies Hybridoma 2:124-134; Gorman, S. D., et al., (1991) Proc Natl Acad Sci USA 88:4181-4185; Tempest, P. R., et al., (1991) Bio/Technology 9:266-271; Co, M. S., et al., (1991) Proc Natl Acad Sci USA 88:2869-2873; Carter, P., et al., (1992) Proc Natl Acad Sci USA 89:4285-4289; and Co, M. S. et al., (1992) J Immunol 148:1149-1154. In some embodiments, humanized antibodies preserve all CDR sequences (for example, a humanized mouse antibody which contains all six CDRs from the mouse antibodies). In other embodiments, humanized antibodies have one or more CDRs (one, two, three, four, five, six) which are altered with respect to the original antibody, which are also termed one or more CDRs "derived from" one or more CDRs from the original antibody.

In certain embodiments, the antibodies of the present disclosure may be chimeric antibodies. In this regard, a chimeric antibody is comprised of an antigen-binding fragment of an antibody operably linked or otherwise fused to a heterologous Fc portion of a different antibody. In certain embodiments, the heterologous Fc domain is of human origin. In other embodiments, the heterologous Fc domain may be from a different Ig class from the parent antibody, including IgA (including subclasses IgA1 and IgA2), IgD, IgE, IgG (including subclasses IgG1, IgG2, IgG3, and IgG4), and IgM. In further embodiments, the heterologous Fc domain may be comprised of CH2 and CH3 domains from one or more of the different Ig classes. As noted above with regard to humanized antibodies, the antigen-binding fragment of a chimeric antibody may comprise only one or more of the CDRs of the antibodies described herein (e.g., 1, 2, 3, 4, 5, or 6 CDRs of the antibodies described herein), or may comprise an entire variable domain (VL, VH or both).

### Wnt Surrogates

The disclosure provides, in certain aspects, Wnt surrogate molecules that bind both one or more Fzd receptors and one or both of LRP5 and/or LRP6. Wnt surrogate molecules may also be referred to as "Wnt surrogates" or "Wnt mimetics." In particular embodiments, the Wnt surrogate molecules bind one or more human Fzd receptors and one or both of a human LRP5 and/or a human LRP6.

In certain embodiments, a Wnt surrogate molecule is capable of modulating or modulates Wnt signaling events in a cell contacted with the Wnt surrogate molecule. In certain embodiments, the Wnt surrogate molecule increases Wnt signaling, e.g., via the canonical Wnt/β-catenin pathway. In certain embodiments, the Wnt surrogate molecule specifically modulates the biological activity of a human Wnt signaling pathway.

Wnt surrogate molecules of the present disclosure are biologically active in binding to one or more Fzd receptors and to one or more of LRP5 and LRP6, and in activation of Wnt signaling, i.e., the Wnt surrogate molecule is a Wnt agonist. The term "Wnt agonist activity" refers to the ability of an agonist to mimic the effect or activity of a Wnt protein binding to an Fzd receptor and/or LRP5 or LRP6. The ability of the Wnt surrogate molecules and other Wnt agonists disclosed herein to mimic the activity of Wnt can be confirmed by a number of assays. Wnt agonists typically initiate a reaction or activity that is similar to or the same as that initiated by the receptor's natural ligand. In particular, the Wnt agonists disclosed herein activate, enhance or increase the canonical Wnt/β-catenin signaling pathway. As used herein, the term "enhances" refers to a measurable increase in the level of Wnt/β-catenin signaling compared with the level in the absence of a Wnt agonist, e.g., a Wnt surrogate molecule disclosed herein. In particular embodiments, the increase in the level of Wnt/β-catenin signaling is at least 10%, at least 20%, at least 50%, at least two-fold, at least five-fold, at least 10-fold, at least 20-fold, at least 50-fold, or at least 100-fold as compared to the level of Wnt/β-catenin signaling in the absence of the Wnt agonist, e.g., in the same cell type. Methods of measuring Wnt/β-catenin signaling are known in the art and include those described herein. Wnt surrogate molecules disclosed herein are multispecific, i.e., they specifically bind to two or more different epitopes. At least one epitope is within one or more Fzd receptors and at least one epitope binds to LRP5 and/or LRP6. In particular embodiments, multispecific Wnt surrogate molecules are multispecific with respect to Fzd receptor binding, i.e., they specifically bind to two or more different types of Fzd receptors, two or more different epitopes within a single type of Fzd receptor, or a combination thereof. For example, a multispecific Wnt surrogate molecule may bind to two or more of Fzd1, Fzd2, Fzd3, Fzd4, Fzd5, Fzd6, Fzd7, Fzd8, Fzd9, and Fzd10. In certain embodiments, a multispecific Wnt surrogate molecule binds to: (i) Fzd1, Fzd2, Fzd7, and Fzd9; (ii) Fzd1, Fzd2, and Fzd7; (iii) Fzd5 and Fzd8; (iv) Fzd5, Fzd7, and Fzd8; (v) Fzd1, Fzd4, Fzd5, and Fzd8; (vi) Fzd1, Fzd2, Fzd5, Fzd7, and Fzd8; (vii) Fzd4 and Fzd9; (viii) Fzd9 and Fzd10; (ix) Fzd5, Fzd8, and Fzd10; (x) Fzd4, Fzd5, and Fzd8; or (xi) Fzd1, Fzd5, Fzd7 and Fzd8.

In certain embodiments, a Wnt surrogate molecule that is multispecific with respect to Fzd binding includes at least one Fzd binding region that binds to a plurality of different Fzd receptor epitopes, e.g., epitopes within different Fzd receptors, different epitopes within the same Fzd receptor, or combinations thereof. For example, the Wnt surrogate molecule may include at least one Fzd binding region that binds to two or more Fzd receptors, e.g., two or more of Fzd1, Fzd2, Fzd3, Fzd4, Fzd5, Fzd6, Fzd7, Fzd8, Fzd9, and Fzd10. As another example, the Wnt surrogate molecule may include at least one Fzd binding region that binds to: (i) Fzd1, Fzd2, Fzd7 and Fzd9; (ii) Fzd1, Fzd2 and Fzd7; (iii) Fzd5 and Fzd8; (iv) Fzd5, Fzd7 and Fzd8; (v) Fzd1, Fzd4, Fzd5 and Fzd8; (vi) Fzd1, Fzd2, Fzd5, Fzd7 and Fzd8; (vii) Fzd4 and Fzd9; (viii) Fzd9 and Fzd10; (ix) Fzd5, Fzd8 and Fzd10; (x) Fzd4, Fzd5 and Fzd8; or (xi) Fzd1, Fzd5, Fzd7 and Fzd8.

Alternatively, or in combination, in certain embodiments, a Wnt surrogate that is multispecific with respect to Fzd binding includes at least two Fzd binding regions that each bind to different sets of one or more Fzd receptor epitopes, e.g., epitopes within different Fzd receptors, different epitopes within the same Fzd receptor, or combinations thereof. A set of one or more Fzd receptor epitopes may include one, two, three, four, five, six, seven, eight, nine, ten, or more Fzd receptor epitopes, such that each Fzd binding region may be monospecific, bispecific, trispecific, tetraspecific, etc.

In certain embodiments, a multispecific Wnt surrogate includes two or more Fzd binding regions, wherein one or more of these Fzd binding regions specifically binds only one Fzd receptor or receptor epitope. In certain embodiments, two or more, three or more, or four or more Fzd binding regions within the mutispecific Wnt surrogate each specifically bind only one Fzd receptor or receptor epitope, wherein at least two or more, at least three or more, or at least four or more of the Fzd binding regions specifically bind different Fzd receptors and/or receptor epitopes.

In certain embodiments, the Wnt surrogate molecule includes a first Fzd binding region that binds to a first set of one or more Fzd receptor epitopes, and a second Fzd binding region that binds to a second, different set of one or more Fzd receptor epitopes. For example, the first Fzd binding region may bind to a first set of one or more Fzd receptors, and the second Fzd binding region may bind to a second, different set of one or more Fzd receptors. Alternatively or in combination, the first Fzd binding region may bind to a first set of one or more epitopes within a set of one or more Fzd receptors, and the second Fzd binding region may bind to a second, different set of one or more epitopes within the same set of one or more Fzd receptors. In certain embodiments, the first Fzd binding region binds to one or more of Fzd1, Fzd2, Fzd3, Fzd4, Fzd5, Fzd6, Fzd7, Fzd8, Fzd9, and Fzd10. In certain embodiments, the second Fzd binding region binds to one or more of Fzd1, Fzd2, Fzd3, Fzd4, Fzd5, Fzd6, Fzd7, Fzd8, Fzd9, and Fzd10.

In certain embodiments, the Wnt surrogate molecule includes a first Fzd binding region and a second Fzd binding region, wherein: the first Fzd binding region binds to Fzd1 and the second Fzd binding region binds to Fzd2; the first Fzd binding region binds to Fzd1 and the second Fzd binding region binds to Fzd3; the first Fzd binding region binds to Fzd1 and the second Fzd binding region binds to Fzd4; the first Fzd binding region binds to Fzd1 and the second Fzd binding region binds to Fzd5; the first Fzd binding region binds to Fzd1 and the second Fzd binding region binds to Fzd6; the first Fzd binding region binds to Fzd1 and the second Fzd binding region binds to Fzd7; the first Fzd binding region binds to Fzd1 and the second Fzd binding region binds to Fzd8; the first Fzd binding region binds to Fzd1 and the second Fzd binding region binds to Fzd9; or the first Fzd binding region binds to Fzd1 and the second Fzd binding region binds to Fzd10.

In certain embodiments, the Wnt surrogate molecule includes a first Fzd binding region and a second Fzd binding region, wherein: the first Fzd binding region binds to Fzd2 and the second Fzd binding region binds to Fzd3; the first Fzd binding region binds to Fzd2 and the second Fzd binding region binds to Fzd4; the first Fzd binding region binds to Fzd2 and the second Fzd binding region binds to Fzd5; the first Fzd binding region binds to Fzd2 and the second Fzd binding region binds to Fzd6; the first Fzd binding region binds to Fzd2 and the second Fzd binding region binds to Fzd7; the first Fzd binding region binds to Fzd2 and the second Fzd binding region binds to Fzd8; the first Fzd binding region binds to Fzd2 and the second Fzd binding region binds to Fzd9; or the first Fzd binding region binds to Fzd2 and the second Fzd binding region binds to Fzd10.

In certain embodiments, the Wnt surrogate molecule includes a first Fzd binding region and a second Fzd binding region, wherein: the first Fzd binding region binds to Fzd3 and the second Fzd binding region binds to Fzd4; the first Fzd binding region binds to Fzd3 and the second Fzd binding region binds to Fzd5; the first Fzd binding region binds to Fzd3 and the second Fzd binding region binds to Fzd6; the first Fzd binding region binds to Fzd3 and the second Fzd binding region binds to Fzd7; the first Fzd binding region binds to Fzd3 and the second Fzd binding region binds to Fzd8; the first Fzd binding region binds to Fzd3 and the second Fzd binding region binds to Fzd9; or the first Fzd binding region binds to Fzd3 and the second Fzd binding region binds to Fzd10.

In certain embodiments, the Wnt surrogate molecule includes a first Fzd binding region and a second Fzd binding region, wherein: the first Fzd binding region binds to Fzd4 and the second Fzd binding region binds to Fzd5; the first Fzd binding region binds to Fzd4 and the second Fzd binding region binds to Fzd6; the first Fzd binding region binds to Fzd4 and the second Fzd binding region binds to Fzd7; the first Fzd binding region binds to Fzd4 and the second Fzd binding region binds to Fzd8; the first Fzd binding region binds to Fzd4 and the second Fzd binding region binds to Fzd9; or the first Fzd binding region binds to Fzd4 and the second Fzd binding region binds to Fzd10.

In certain embodiments, the Wnt surrogate molecule includes a first Fzd binding region and a second Fzd binding region, wherein: the first Fzd binding region binds to Fzd5 and the second Fzd binding region binds to Fzd6; the first Fzd binding region binds to Fzd5 and the second Fzd binding region binds to Fzd7; the first Fzd binding region binds to Fzd5 and the second Fzd binding region binds to Fzd8; the first Fzd binding region binds to Fzd5 and the second Fzd binding region binds to Fzd9; or the first Fzd binding region binds to Fzd5 and the second Fzd binding region binds to Fzd10.

In certain embodiments, the Wnt surrogate molecule includes a first Fzd binding region and a second Fzd binding region, wherein: the first Fzd binding region binds to Fzd6 and the second Fzd binding region binds to Fzd7; the first Fzd binding region binds to Fzd6 and the second Fzd binding region binds to Fzd8; the first Fzd binding region binds to Fzd6 and the second Fzd binding region binds to Fzd9; or the first Fzd binding region binds to Fzd6 and the second Fzd binding region binds to Fzd10.

In certain embodiments, the Wnt surrogate molecule includes a first Fzd binding region and a second Fzd binding region, wherein: the first Fzd binding region binds to Fzd7 and the second Fzd binding region binds to Fzd8; the first Fzd binding region binds to Fzd7 and the second Fzd binding region binds to Fzd9; or the first Fzd binding region binds to Fzd7 and the second Fzd binding region binds to Fzd10.

In certain embodiments, the Wnt surrogate molecule includes a first Fzd binding region and a second Fzd binding region, wherein: the first Fzd binding region binds to Fzd8 and the second Fzd binding region binds to Fzd9; or the first Fzd binding region binds to Fzd8 and the second Fzd binding region binds to Fzd10. In certain embodiments, the Wnt surrogate molecule includes a first Fzd binding region and a second Fzd binding region, wherein the first Fzd binding region binds to Fzd9 and the second Fzd binding region binds to Fzd10.

For each of the combinations of Fzd receptors disclosed above, it is understood that, in certain embodiments, the first or second binding region may specifically bind only the indicated Fzd, or it may also bind additional Fzds. For example, wherein the first Fzd binding region binds to Fzd1 and the second Fzd binding region binds to Fzd2, the first Fzd binding region may specifically bind only Fzd1, or it may also bind to one or more other Fzds in addition to Fzd1. Similarly, the second Fzd binding region may specifically bind only Fzd2, or it may also bind to one or more other Fzds in addition to Fzd2. However, the first and second Fzd binding regions bind to different sets of Fzd receptors.

In certain embodiments, the first and second binding regions may specifically bind to different epitopes within the same Fzd receptor. For example, the first binding region may bind to a first epitope in Fzd1, and the second binding region may bind to a second, different epitope in Fzd1. The first binding region may bind to a first epitope in Fzd2, and the second binding region may bind to a second, different epitope in Fzd2. The first binding region may bind to a first epitope in Fzd3, and the second binding region may bind to a second, different epitope in Fzd3. The first binding region may bind to a first epitope in Fzd4, and the second binding region may bind to a second, different epitope in Fzd4. The first binding region may bind to a first epitope in Fzd5, and the second binding region may bind to a second, different epitope in Fzd5. The first binding region may bind to a first epitope in Fzd6, and the second binding region may bind to a second, different epitope in Fzd6. The first binding region may bind to a first epitope in Fzd7, and the second binding region may bind to a second, different epitope in Fzd7. The first binding region may bind to a first epitope in Fzd8, and the second binding region may bind to a second, different epitope in Fzd8. The first binding region may bind to a first epitope in Fzd9, and the second binding region may bind to a second, different epitope in Fzd9. The first binding region may bind to a first epitope in Fzd10, and the second binding region may bind to a second, different epitope in Fzd10.

For each of the combinations of Fzd receptor epitopes disclosed above, it is understood that, in certain embodiments, the first or second binding regions may specifically bind only the indicated epitope, or may also bind additional epitopes. For example, wherein the first binding region binds to a first epitope in Fzd1, and the second binding region binds to a second, different epitope in Fzd1, the first binding region may specifically bind only the first epitope in Fzd1, or it may also bind to one or more other epitopes in Fzd1 or other Fzd receptors. Similarly, the second Fzd binding region may specifically bind only the second epitope in Fzd1, or it may also bind to one or more other epitopes in Fzd1 or other Fzd receptors. In embodiments where the first and second binding regions specifically bind to the same Fzd receptor or receptors, the first and second binding regions bind to different epitopes within the same receptor(s).

In particular embodiments, multispecific Wnt surrogate molecules are multispecific with respect to LRP5/6 binding, i.e., they specifically bind to two or more different epitopes within LRP5 and/or LRP6. In certain embodiments, a multispecific Wnt surrogate molecule includes a first LRP5/6 binding region that binds to a first epitope within LRP5 and/or LRP6, and a second LRP5/6 binding region that binds to a second, different epitope within LRP5 and/or LRP6.

For each of the combinations of the LRP receptor epitopes, it is understood that, in certain embodiments, the first or second binding regions may specifically bind only the indicated epitope, or may also bind additional epitopes. For example, wherein the first binding region binds to a first epitope in LRP5E1E2, and the second binding region binds to a second, different epitope in LRP5E1E2, the first binding region may specifically bind only the first epitope in LRP5E1E2, or it may also bind to one or more other epitopes in LRP5E1E2 or other LRP receptors. Similarly, the second LRP binding region may specifically bind only the second epitope in LRP5E1E2, or it may also bind to one or more other epitopes in LRP5E1E2 or other LRP receptors. In embodiments where the first and second binding regions specifically bind to the same LRP receptor or receptors, the first and second binding regions bind to different epitopes within the same receptor(s). Similar epitope binding can occur for LRP5E3E4, LRP6E1E2, and LRP6E3E4.In particular embodiments, multispecific Wnt surrogate molecules are multispecific with respect to both Fzd binding and LRP5/6 binding. For example, a Wnt surrogate molecule may include a plurality of Fzd binding regions that each specifically bind to a different set of one or more Fzd receptors, and a plurality of LRP5/6 binding regions that each specifically bind to a different epitope within LRP5 and/or LRP6. It shall be appreciated that the various embodiments of Fzd binding regions and LRP5/6 binding regions disclosed herein may be combined in many ways to generate multispecific Wnt surrogate molecules with any desired combination of Fzd and LRP5/6 binding specificity.

In particular embodiments, Wnt surrogate molecules disclosed herein are multivalent, e.g., they comprise two or more regions that each specifically bind to the same epitope, e.g., two or more regions that bind to an epitope within one or more Fzd receptors and/or two or more regions that bind to an epitope within LRP5 and/or LRP6. In particular embodiments, they comprise two or more regions that bind to an epitope within one or more Fzd receptors and two or more regions that bind to an epitope within LRP5 and/or LRP6.

In particular embodiments, Wnt surrogate molecule comprise Fzd binding regions and LRP5/6 binding regions in a ratio of Fzdₙ:LRP5/6ₙ (Fₙ:Lₙ), wherein F and L are integers between 1 and 9, inclusive, and n is an integer between 1 and 4 inclusive.

In certain embodiments, Wnt surrogate molecules comprise a ratio of the number of regions that bind one or more Fzd receptors to the number of regions that bind LRP5 and/or LRP6 of or about: 1:1, 2:1, 3:1, 4:1, 5:1, 6:1, 2:1 2:3, 2:5, 2:7, 7:2, 5:2, 3:2, 3:4, 3:5, 3:7, 3:8, 8:3, 7:3, 5:3, 4:3, 4:5, 4:7, 4:9, 9:4, 7:4, 5:4, 6:7, 7:6, 1:2, 1:3, 1:4, 1:5, 1:6, 2:1 (with two Fzd binders and one LRP binder), 1:2 (with one Fzd binder and two LRP binders), 2:1:1 (with two different LRP binders), 1:1:2 (with two different Fzd binders), 1:1:1 (two different Fzd binders and one LRP binder or one Fzd binder and two different LRP binders) and 1:1:1:1 (all different Fzd and LRP binders). Varying the ratios of Fzd binding moieties to LRP5/6 binding moieties can confer tissue and/or functional specificity and modulate signaling levels. In certain embodiments, Wnt surrogate molecules are multispecific and multivalent.

Wnt surrogate molecules disclosed herein may have any of a variety of different structural formats or configurations. Wnt surrogate molecules may comprise polypeptides and/or non-polypeptide binding moieties, e.g., small molecules. In particular embodiments, Wnt surrogate molecules comprise both a polypeptide region and a non-polypeptide binding moiety. In certain embodiments, Wnt surrogate molecules may comprise a single polypeptide, or they may comprise two or more, three or more, or four or more polypeptides. In certain embodiments, the Wnt surrogates comprises one, two, three, or four polypeptides, e.g., linked or bound to each other or fused to each other.

When the Wnt surrogate molecules comprise a single polypeptide, they may be a fusion protein comprising one or more Fzd binding regions (also referred to herein as "Fzd binding domains") and one or more LRP5/6 binding regions (also referred to herein as "LRP5/6 binding domains"). The binding regions may be directly fused or they may be connected via a linker, e.g., a polypeptide or chemical linker, including but not limited to any of those disclosed herein.

When the Wnt surrogate molecules comprise two or more polypeptides, the polypeptides may be linked via covalent bonds, such as, e.g., disulfide bonds, and/or noncovalent interactions. For example, heavy chains of human immunoglobulin IgG interact at the level of their CH3 domains directly, whereas, at the level of their CH2 domains, they interact via the carbohydrate attached to the asparagine (Asn) N84.4 in the DE turn.

Wnt surrogate polypeptides may be engineered to facilitate binding between two polypeptides. For example, knobs-into-holes amino acid modifications may be introduced into two different polypeptides to facilitate their binding. Knobs-into-holes amino acid (AA) changes is a rational design strategy developed in antibody engineering, used for heterodimerization of the heavy chains, in the production of bispecific IgG antibodies. AA changes are engineered in order to create a knob on the CH3 of the heavy chains from a first antibody and a hole on the CH3 of the heavy chains of a second antibody. The knob may be represented by a tyrosine (Y) that belongs to the 'very large' IMGT volume class of AA, whereas the hole may be represented by a threonine (T) that belongs to the 'small' IMGT volume class. Other means of introducing modifications into polypeptides to facilitate their binding are known and available in the art. For example, specific amino acids may be introduced and used for cross-linking, such as Cysteine to form an intermolecular disulfide bond.

In particular embodiments, the Wnt surrogate molecules comprise one or more binding regions derived from an antibody or antigen-binding fragment thereof, e.g., antibody heavy chains or antibody light chains or fragments thereof. In certain embodiments, one or more polypeptides of a Wnt surrogate molecule are antibodies or antigen-binding fragments thereof. In certain embodiments, Wnt surrogates comprise two antibodies or antigen-binding fragments thereof, e.g., one that binds one or more Fzd receptors and one that binds LRP5 and/or LRP6. In certain embodiments, Wnt surrogates comprise three antibodies or antigen-binding fragments thereof, e.g., one that binds a first set of one or more Fzd receptor epitopes, one that binds a second, different set of one or more Fzd receptor epitopes, and one that binds LRP5 and/or LRP6. In certain embodiments, Wnt surrogates comprise four antibodies or antigen-binding fragments thereof, e.g., one that binds a first set of one or more Fzd receptor epitopes, one that binds a second, different set of one or more Fzd receptor epitopes, one that binds a first epitope within LRP5 and/or LRP6, and one that binds a second, different epitope within LRP5 and/or LRP6.

In certain embodiments, a Wnt surrogate molecule includes a polypeptide comprising two antibody heavy chain regions (e.g., hinge regions) bound together via one or more disulfide bond. In certain embodiments, a Wnt surrogate molecule includes a polypeptide comprising an antibody light chain region (e.g., a C_{L} region) and an antibody heavy chain region (e.g., a C_{H}1 region) bound together via one or more disulfide bonds.

Wnt surrogate molecules may have a variety of different structural formats, including but not limited to those shown in FIG. 1.

In one embodiment, a Wnt surrogate molecule comprises an scFv or antigen-binding fragment thereof fused to a VHH or sdAb or antigen-binding fragment thereof. In certain embodiments, the scFv specifically binds one or more Fzd receptor epitopes, and the VHH or sdAb specifically binds LRP5 and/or LRP6. In certain embodiments, the scFv specifically binds LRP5 and/or LRP6, and the VHH or sdAb specifically binds one or more Fzd receptor epitopes. In particular embodiments, the scFv or antigen-binding fragment thereof is fused directly to the VHH or sdAb or antigen-binding fragment thereof, whereas in other embodiments, the two binding regions are fused via a linker moiety. In particular embodiments, the VHH or sdAb is fused to the N-terminus of the scFV, while in other embodiments, the VHH or sdAb is fused to the C-terminus of the scFv. In particular embodiments, the scFv is described herein or comprises any of the CDR sets described herein. In particular embodiments, the VHH or sdAb is described herein or comprises any of the CDR sets disclosed herein.

In various embodiments, including but not limited to those depicted in FIG. 1, a Wnt surrogate molecule comprises one or more Fabs or antigen-binding fragment thereof and one or more VHH or sdAbs or antigen-binding fragment thereof (or alternatively, one or more scFvs or antigen-binding fragment thereof). In certain embodiments, the Wnt surrogate comprises two or more Fabs, each of which specifically binds to different sets of one or more Fzd receptor epitopes, and the VHH or sdAb (or scFv) specifically binds LRP5 and/or LRP6. In certain embodiments, the Wnt surrogate comprises a Fab that specifically binds LRP5 and/or LRP6, and two or more VHH or sdAb (or scFv), each of which specifically binds to different sets of one or more Fzd receptor epitopes. In certain embodiments, the VHH or sdAbs (or scFvs) are fused to the N-terminus of the Fab, while in some embodiments, the VHH or sdAbs (or scFvs) are fused to the C-terminus of the Fab. In particular embodiments, the Fab is present in a full IgG format, and the VHH or sdAbs (or scFvs) are fused to the N-terminus and/or C-terminus of the IgG light chain. In particular embodiments, the Fab is present in a full IgG format, and the VHH or sdAbs (or scFvs) are fused to the N-terminus and/or C-terminus of the IgG heavy chain. In particular embodiments, two or more VHH or sdAbs (or scFvs) are fused to the IgG at any combination of these locations, where each of the two or more VHH or sdAbs (or scFvs) bind different sets of Fzds.

Fabs may be converted into a full IgG format that includes both the Fab and Fc fragments, for example, using genetic engineering to generate a fusion polypeptide comprising the Fab fused to an Fc region, i.e., the Fab is present in a full IgG format. The Fc region for the full IgG format may be derived from any of a variety of different Fcs, including but not limited to, a wild-type or modified IgG1, IgG2, IgG3, IgG4 or other isotype, e.g., wild-type or modified human IgG1, human IgG2, human IgG3, human IgG4, human IgG4Pro (comprising a mutation in core hinge region that prevents the formation of IgG4 half molecules), human IgA, human IgE, human IgM, or the modified IgG1 referred to as IgG1 LALAPG. The L234A, L235A, P329G (LALA-PG) variant has been shown to eliminate complement binding and fixation as well as Fc-γ dependent antibody-dependent cell-mediated cytotoxity (ADCC) in both murine IgG2a and human IgG1. These LALA-PG substitutions allow a more accurate translation of results generated with an "effectorless" antibody framework scaffold between mice and primates. In particular embodiments of any of the IgG disclosed herein, the IgG comprises one or more of the following amino acid substitutions: N297G, N297A, N297E, L234A, L235A, or P236G.

Non-limiting examples of bivalent and bispecific Wnt surrogate molecules that are bivalent towards both the one or more Fzd receptor epitopes and the LRP5 and/or LRP6 are provided as the top four structures depicted in FIG. 1, where the VHH or sdAbs or scFvs are depicted as single solid ovals in red, blue or yellow , and the Fab or IgG is depicted in blue. As shown, the VHH or sdAbs (or scFvs) may be fused to the N-termini of both light chains, to the N-termini of both heavy chains, to the C-termini of both light chains, or to the C-termini of both heavy chains. It is further contemplated, e.g., that VHH or sdAbs (or scFvs) could be fused to both the N-termini and C-termini of the heavy and/or light chains, to the N-termini of the light chains and the heavy chains, to the C-termini of the heavy and light chains, to the N-termini of the heavy chains and C-termini of the light chains, or to the C-termini of the heavy chains and the N-termini of the light chains. In other related embodiments, two or more VHH or sdAbs (or scFvs) may be fused together, optionally via a linker moiety, and fused to the Fab or IgG at one or more of these locations. In particular embodiments, the two or more VHH or sdAbs (or scFvs) may each bind to a different set of one or more Fzd receptor epitopes.

In a related embodiment, the Wnt surrogate molecule has a hetero-Ig format, whereas the Fab is present as a half antibody, and one or more VHH or sdAb (or scFv) is fused to one or more of the N-terminus of the Fc, the N-terminus of the Fab, the C-terminus of the Fc, or the C-terminus of the Fab. In particular embodiments, two or more VHH or sdAbs (or scFvs) are fused to the N-terminus of the Fc, the N-terminus of the Fab, the C-terminus of the Fc, or the C-terminus of the Fab, wherein each of the VHH or sdAbs (or scFvs) binds a different set of one or more Fzd receptor epitopes. A bispecific but monovalent to each receptor version of this format is depicted in FIG. 1C, 1D, 1E, 1F, which may be modified to include two or more Fzd binding regions, wherein at least two of the Fzd binding regions bind to different sets of one or more Fzd receptor epitopes. In certain embodiments, the Fab or antigen-binding fragment (or IgG) thereof is fused directly to the VHH or sdAb (or scFv) or antigen-binding fragment thereof, whereas in other embodiments, the binding regions are fused via a linker moiety. In particular embodiments, the Fab is described herein or comprises any of the CDR sets described herein. In particular embodiments, the VHH or sdAbs or scFvs are described herein or comprises any of the CDR sets disclosed herein.

In various embodiments, including but not limited to those depicted in FIG. 1V, 1W, 1X, 1AA, a Wnt surrogate molecule comprises one or more Fabs or antigen-binding fragment thereof that binds one or more Fzd receptor epitopes and one or more Fabs or antigen-binding fragment thereof that binds LRP5 and/or LRP6. In certain embodiments, it comprises two Fab or antigen-binding fragments thereof that bind different sets of one or more Fzd receptor epitopes and/or two Fab or antigen-binding fragments thereof that bind LRP5 and/or LRP6. In particular embodiments, one or more of the Fabs are present in a full IgG format, and in certain embodiments, both Fabs are present in a full IgG format. In certain embodiments, the Fabs in full IgG format specifically binds one or more Fzd receptor epitopes, and the other Fabs specifically binds LRP5 and/or LRP6. In certain embodiments, the Fabs specifically bind different sets of one or more Fzd receptor epitopes, and the Fabs in full IgG format specifically binds LRP5 and/or LRP6. In certain embodiments, the Fabs specifically binds LRP5 and/or LRP6, and the Fabs in full IgG format specifically bind different sets of one or more Fzd receptor epitopes. In certain embodiments, the Fab is fused to the N-terminus of the IgG, e.g., to the heavy chain or light chain N-terminus, optionally via a linker. In certain embodiments, the Fab is fused to the N-terminus of the heavy chain of the IgG and not fused to the light chain. In particular embodiments, the two heavy chains can be fused together directly or via a linker. An example of such a bispecific and bivalent with respect to both receptors is shown in FIG. 1V, 1W, 1X and 1AA. In other related embodiments, two or more VHH or sdAbs may be fused together, optionally via a linker moiety, and fused to the Fab or IgG at one or more of these locations. In a related embodiment, the Wnt surrogate molecule has a hetero-IgG format, whereas one of the Fab is present as a half antibody, and the other Fab is fused to one or more of the N-terminus of the Fc, the N-terminus of the Fab, or the C-terminus of the Fc. A bispecific but monovalent to each receptor version of this format is depicted in FIG. 1D, which may be modified to includes one or more additional Fab, wherein two or more Fab bind to different sets of Fzd receptor epitopes. In certain embodiments, the Fab or antigen-binding fragment thereof is fused directly to the other Fab or IgG or antigen-binding fragment thereof, whereas in other embodiments, the binding regions are fused via a linker moiety. In particular embodiments, the one or both of the two Fabs are described herein or comprise any of the CDR sets described herein.

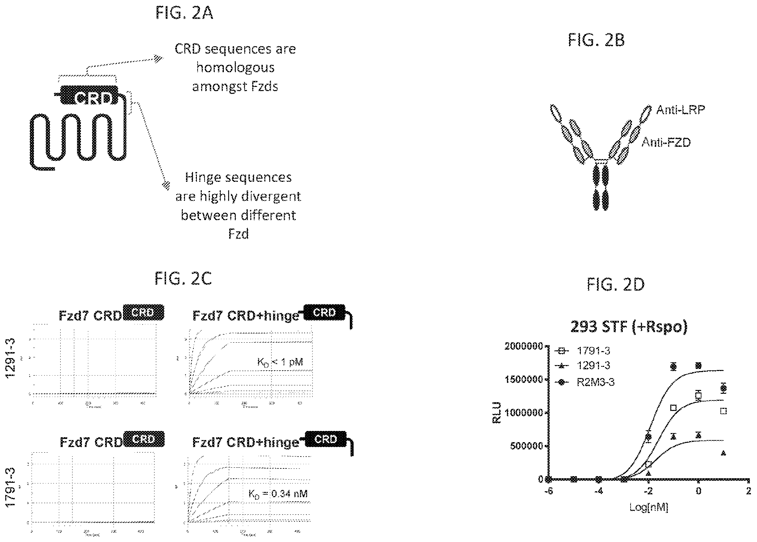

In certain embodiments, Wnt surrogate molecules have a format as described in PCT Application Publication No. WO2017/136820, e.g., a Fabs-in-tandem IgG (FIT-IG) format. Shiyong Gong, Fang Ren, Danqing Wu, Xuan Wu & Chengbin Wu (2017). FIT-IG also include the formats disclosed in "Fabs-in-tandem immunoglobulin is a novel and versatile bispecific design for engaging multiple therapeutic targets" mAbs, 9:7, 1118-1128, DOI: 10.1080/19420862.2017.1345401. In certain embodiments, FIT-IGs combine the functions of two antibodies into one molecule by re-arranging the DNA sequences of two parental monoclonal antibodies into two or three constructs and co-expressing them in mammalian cells. Examples of FIT-IG formats and constructs are provided in FIGS. 1A and 1B and FIGS. 2A and 2B of PCT Application Publication No. WO2017/136820. In certain embodiments, FIT-IGs require no Fc mutation, no scFv elements, and no linker or peptide connector. The Fab-domains in each arm work "in tandem" forming a tetravalent bi-specific antibody with four active and independent antigen binding sites that retain the biological function of their parental antibodies In particular embodiments, Wnt surrogates comprises a Fab and an IgG. In certain embodiments, the Fab binder LC is fused to the HC of the IgG, e.g., by a linker of various length in between. In various embodiment, the Fab binder HC can be fused or unfused to the LC of the IgG. A variation of this format has been called Fabs-in-tandem IgG (or FIT-Ig). In certain embodiments, the FIT-Ig comprises two or more Fzd binding domains, wherein at least two or the Fzd binding regions bind to different sets of one or more Fzd receptor epitopes, e.g., different sets of one or more Fzd receptors or different sets of one or more epitopes within the same Fzd receptor(s).

In particular embodiments, Wnt surrogate molecules comprise two or more VHH or sdAbs (or scFvs), including at least one that binds one or more Fzd receptor epitopes and at least one that binds LRP5 and/or LRP6. In certain embodiments, one of the binding regions is a VHH or sdAb and the other is an scFv. In particular embodiments, the Wnt surrogate molecules comprises three or more VHH or sdAbs (or scFvs), including at least two that binds different sets of one or more Fzd receptor epitopes and at least one that binds LRP5 and/or LRP6. Wnt surrogate molecules comprising two or more VHH or sdAbs (or scFvs) may be formatted in a variety of configurations, including but not limited to those depicted in FIG. 1K, 1L, 1M, 1N, 1O, 1P, 1Q, 1S, 1T. In certain bispecific, bivalent formats, two or more VHH or sdAbs (or scFvs) are fused in tandem or fused to two different ends of an Fc, optionally via one or more linkers. Where linkers are present, the linker and its length may be the same or different between the VHH or sdAb (or scFv) and the other VHH or sdAb (or scFv), or between the VHH or sdAb and Fc. For example, in certain embodiments, the VHH or sdAb is fused to the N-terminus and/or C-terminus of the IgG heavy chain. In particular embodiments, two or more VHH or sdAbs are fused to the IgG at any combination of these locations. Non-limiting examples of bivalent and bispecific Wnt surrogate molecules of this format are depicted as the structures depicted in FIG. 1K, 1L, 1M, 1N, 1O, 1P, 1Q., where the first VHH or sdAb is depicted in blue, the Fc or IgG is depicted in blue, and the second VHH or sdAb is depicted as red. In various embodiments, both VHH or sdAbs may be fused to the N-termini of the Fc, to the C-termini of the Fc, or one or more VHH or sdAb may be fused to either or both of an N-terminus or C-terminus of the Fc. In a related embodiment, the Wnt surrogate molecule has a hetero-IgG format, whereas one VHH or sdAb is present as a half antibody, and the other is fused to the N-terminus of the Fc or the C-terminus of the Fc. A bispecific but monovalent to each receptor version of this format is depicted in FIG. 1E. In certain embodiments, the VHH or sdAb is fused directly to the other VHH or sdAb, whereas in other embodiments, the binding regions are fused via a linker moiety. In particular embodiments, the VHH or sdAbs are described herein or comprises any of the CDR sets described herein. In various embodiments, any of these formats may comprise one or more scFvs in place of one or more VHH or sdAbs.

In certain embodiments, a Wnt surrogate molecule is formatted as a diabody. As shown in FIG. 1R, the binders against Fzd and LRP can also be linked together in a diabody (or DART) configuration. The diabody can also be in a single chain configuration. If the diabody is fused to an Fc, this will create a bivalent bispecific format. Without fusion to Fc, this would be a monovalent bispecific format. In certain embodiments, a diabody is a noncovalent dimer scFv fragment that consists of the heavy-chain variable (VH) and light-chain variable (VL) regions connected by a small peptide linker. Another form of diabody is a single-chain (Fv)2 in which two scFv fragments are covalently linked to each other. In particular embodiments, the diabody comprises two or more Fzd binding regions, wherein at least two of the Fzd binding regions bind to different sets of one or more Fzd receptor epitopes.

In certain embodiments, two or more diabodies, scFvs, and/or VHH or sdAbs can be fused in tandem in a multivalent format, with or without being fused to an Fc (FIG. 1A, 1B, 1F, 1U). In particular embodiments, at least one of the diabodies, scFvs, and/or VHH or sdAbs binds one or more Fzd receptor epitopes, and at least one of the diabodies, scFvs, and/or VHH or sdAbs binds LRP5 and/or LRP6.

In various embodiments, including but not limited those depicted in FIG. 1G or 1H, a Wnt surrogate molecule comprises two or more Fabs or antigen-binding fragments thereof that each bind a different set of one or more Fzd receptor epitopes, and one or more VHH or sdAbs or antigen-binding fragments thereof (or, alternatively or in combination, one or more scFvs or antigen-binding fragments thereof), e.g., that bind LRP5/6. In certain embodiments, a first Fab specifically binds a first set of one or more Fzd receptor epitopes, a second Fab specifically binds a second, different set of one or more Fzd receptor epitopes, and the VHH or sdAb (or scFv) specifically binds LRP5 and/or LRP6. In certain embodiments, the VHH or sdAb (or scFv) is fused to the N-terminus of the Fabs, while in some embodiments, the VHH or sdAb (or scFv) is fused to the C-terminus of the Fabs. In particular embodiments, the Wnt surrogate molecule has a hetero-Ig format, as depicted in FIG. 1G, 1H, 1AG in which the first and second Fabs are each present as a half antibody, and one or more VHH or sdAbs (or scFvs) are fused to one or more of the N-terminus of the Fc, the N-terminus of the Fab, the C-terminus of the Fc (e.g., FIG. 1Y), or the C-terminus of the Fab. The first and second Fabs may be connected to each other via knobs-into-holes mutations in their respective Fcs, e.g., within the CH3 domain.

As discussed, Wnt surrogate molecules, in various embodiments, comprise one or more antibodies or antigen-binding fragments thereof disclosed herein. Thus, in particular embodiments, a Wnt surrogate comprises two polypeptides, wherein each polypeptide comprises an VHH or sdAb or scFv that binds LRP5/6 and an VHH or sdAb or scFv that binds one or more Fzd receptor epitopes, optionally wherein one of the binding domains is an scFv and the other is an VHH or sdAb. In particular embodiments, each polypeptide comprises a Fzd binding region that binds a different set of one or more Fzd receptor epitopes. In certain embodiments, a Wnt surrogate comprises three polypeptides, wherein the first polypeptide comprises an antibody heavy chain and the second polypeptide comprises an antibody light chain, wherein the antibody heavy chain and light chain bind LRP5/6 or one or more Fzd receptor epitopes, and wherein the third polypeptide comprises a VHH or sdAb fused to a heavy chain Fc region, wherein the VHH or sdAb binds to either LRP5/6 or one or more Fzd receptor epitopes. In other embodiments, Wnt polypeptides comprise four polypeptides, including two heavy chain polypeptides and two light chain polypeptides, wherein the two heavy chains and two light chains bind LRP5/6 or one or more Fzd receptor epitopes, and further comprise one or more VHH or sdAb or scFv fused to one or more of the heavy chains and/or light chains, wherein the VHH or sdAb or scFv binds to LRP5/6 or one or more Fzd receptor epitopes. In another illustrative embodiment, a Wnt surrogate comprises at least four polypeptides, including two heavy chain polypeptides and two light chain polypeptides that bind either LRP5/6 or one or more Fzd receptor epitopes, wherein the Wnt surrogate further comprises a Fab that binds either LRP5/6 or one or more Fzd receptor epitopes. For example, the Fab may comprise two polypeptides, each fused to one of the two heavy chain polypeptides, and two polypeptides, each fused to one of the two light chain polypeptides, or it may comprise two polypeptides each fused to one of the two heavy chain polypeptides and two additional polypeptides, each bound to one of the two polypeptides fused to the heavy chain polypeptides, thus making a second Fab. Other configurations may be used to produce the Wnt surrogates disclosed herein. In particular embodiments of any of these formats, they comprise at least two or more Fzd binding regions, which each bind to a different set of Fzd receptor epitopes.

In some embodiments, the differing ratios of Fzd binding regions to LRP binding regions (Fzd:LRP) is represented in FIG. 1AB, 1AC, 1AD, 1AE, 1AF, 1AG, 1AH, 1AI, 1AJ, 1AK, 1AL, 1AM. In particular embodiments, one or more Fabs bind to one or more Fzd receptors or to different epitopes in the same Fzd receptor, and one or more VHH or sdAbs (or scFvs) bind to one or more LRP receptors or different epitopes in the same LRP receptor.

In particular embodiments, a Wnt surrogate molecule includes a first light chain and first heavy chain forming a first Fzd binding region, and a second light chain and second heavy chain forming a second Fzd binding region, with the first and second Fzd binding regions binding to different sets of one or more Fzd receptor epitopes. In certain embodiments, the first and second heavy chains are connected to each other. For example, the first heavy chain may include a first CH3 domain, the second heavy chain may include a second CH3 domain, and the first and second CH3 domains may be connected to each other, e.g., via knobs-into-holes mutations. In certain embodiments, the first heavy chain and/or the second heavy chain comprise an amino acid sequence having at least 90%, at least 95%, at least 98%, or at least 99% identity to any of the sequences set forth in SEQ ID NOs:110, 112, 114, 116, 118, 120, and 122. In certain embodiments, the first light chain and/or the second light chain comprise an amino acid sequence having at least 90%, at least 95%, at least 98%, or at least 99% identity to any of the sequences set forth in SEQ ID NOs:109, 111, 113, 115, 117, 119, and 121. In some embodiments, one or more heavy chain comprises an amino acid sequence having at least 90%, at least 95%, at least 98%, or at least 99% identity to any of the sequences disclosed in Table 5. In some embodiments, one or more light chain comprises an amino acid sequence having at least 90%, at least 95%, at least 98%, or at least 99% identity to any of the sequences disclosed in Table 5.

In certain embodiments, the Wnt surrogate molecule includes a first LRP5/6 binding region and/or a second LRP5/6 binding region, each of which may be or include a Fab or scFv. The first and second LRP5/6 binding regions may bind to the same epitope within LRP5/6, or may bind to different epitopes within LRP5/6. The first LRP5/6 binding region may be fused to an N-terminus of the first light chain, a C-terminus of the first light chain, an N-terminus of the first heavy chain, or a C-terminus of the first heavy chain. The second LRP5/6 binding region may be fused to an N-terminus of the second light chain, a C-terminus of the second light chain, an N-terminus of the second heavy chain, or a C-terminus of the second heavy chain.

In particular embodiments, a Wnt surrogate molecule comprises an Fzd binding region, e.g., an anti-Fzd antibody, or antigen-binding fragment thereof, fused or bound to a polypeptide that specifically binds to one or more Fzd receptors. In particular embodiments, the polypeptide that specifically binds to one or more Fzd receptors is an antibody or antigen-binding fragment thereof. In certain embodiments, it is an antibody or antigen-binding fragment thereof disclosed herein or in U.S. Provisional Patent Application No. 62/607,877, titled, "Anti-Frizzled antibodies and Methods of Use," Attorney Docket No. SRZN-004/00US, filed on December 19, 2017, which is incorporated herein by reference in its entirety.

In particular embodiments, at least one Fzd binding region of a Wnt surrogate molecule includes one or more antigen-binding fragments of an antibody. For example, the one or more antigen-binding fragments may be or be derived from an IgG, scFv, Fab, or VHH or sdAb. In certain embodiments, the one or more antigen-binding fragements are humanized.

In particular embodiments, the Fzd binding region comprises the three heavy chain CDRs and/or the three light chain CDRs disclosed for any of the illustrative antibodies or fragments thereof that bind to one or more Fzd receptors provided in Table 1A. In particular embodiments, the Fzd binding region comprises the three heavy chain CDRs and/or the three light chain CDRs disclosed for any of the illustrative antibodies or fragments thereof that bind to one or more Fzd receptors provided in Table 1A, wherein the CDRs collectively comprise one, two, three, four, five, six, seven, or eight amino acid modifications, e.g., substitutions, deletions, or additions. In certain embodiments, the Fzd binding region is a VHH or sdAb or was derived from a VHH or sdAb, so Table 1A only includes the three heavy chain CDRs. In particular embodiments, the Fzd binding region comprises the three CDR HC sequences provided in Table 1A or variants wherein the CDRs collectively comprise one, two, three, four, five, six, seven or eight amino acid modifications.

In particular embodiments, the Fzd binding region comprises the heavy chain fragment and/or light chain fragment of any of the illustrative antibodies or fragments thereof that bind to one or more Fzd receptors provided in Table 1B or SEQ ID NOs:1-73 (or an antigen-binding fragment or variant of either). In certain embodiments, the Fzd binding region is a Fab or was derived from a Fab, so the heavy chain of Table 1B includes VH and CH1 sequences, but not CH2 or CH3 sequences. In certain embodiments, the Fzd binding region is a VHH or sdAb or was derived from a VHH or sdAb, so Table 1B includes the VHH domain. In certain embodiments, the Fzd binding region is a polypeptide, e.g., an antibody or antigen-binding fragment thereof, that competes with any of these antibodies for binding to one or more Fzd receptors.

In particular embodiments, the Fzd binding region includes an amino acid sequence having at least 90%, at least 95%, at least 98%, or at least 99% identity to any of the sequences set forth in Table 1A, Table 1B, or SEQ ID NOs: 1-73, or an antigen-binding fragment thereof. Binding characteristics of clones listed in Table 1B were determined and are shown in Table 1B. Heavy chain CDRs are designated CDRH1, CDRH2 and CDRH3, and light chain CDRs are designated CDRL1, CDRL2, and CDRL3.

**Table 1A: Anti-Fzd Antibody Clone IDs and CDR sequences**

| **Clone ID** | **Initial Binding** | **CDRH1** | **SID NO.** | **CDRH2** | **SID NO.** | **CDRH3** | **SID NO.** |
|---|---|---|---|---|---|---|---|
| **001S-A01** | **Fzd1** | **YTFTSYGIS** | **391** | **GWISAYNGNTNYA** | **570** | **CARASAWTPYGAFDIW** | **752** |
| **001S-B01** | **Fzd1** | **GSISSGGYSWS** | **283** | **GSIYHSGSTYYN** | **547** | **CARFYYDILTGYSYFDYW** | **818** |
| **001S-E01** | **Fzd1** | **GSISNYYWS** | **282** | **GEIDRSGDTNYN** | **488** | CARVRARRFLVSDRSAF DIW | **945** |
| **001S-F01** | **Fzd1** | **GSISGNNYYZG** | **281** | **GSIYFTGGTYYN** | **546** | **CARVMLITDAFDIW** | **942** |
| **001S-G01** | **Fzd1** | **GSISSSSYYWG** | **285** | **GYIYYSGSTYYN** | **589** | **CARATYGGDAFDIW** | **760** |
| **001S-H01** | **Fzd1** | **GSISSGGYYWS** | **284** | **GYIYYSGSTYYN** | **589** | CARHAGFYGLADYFDY W | **875** |
| **001S-A02** | **Fzd1** | **GSISSGGYYWS** | **284** | **GYIYYSGSTYYN** | **589** | CARGKGYSYGYGKDWF DPW | **845** |
| **001S-E02** | **Fzd1** | **GSISGNNYYWG** | **280** | **GSIYFTGGTYYN** | **546** | **CARVMLITDAFDIW** | **942** |
| **001S-G02** | **Fzd1** | **GAISGTSYFWG** | **266** | **GSIYYTGNTYYN** | **548** | **CARIGIAVAAPVDHW** | **882** |
| **001S-H02** | **Fzd1** | **GSISSSSYYWG** | **285** | **GYIYYSGSTYYN** | **589** | **CARATYGGDAFDIW** | **760** |
| **001S-A03** | **Fzd1** | **GSISSGGYYWS** | **284** | **GYIYYSGSTYYN** | **589** | CARVRDYYDSSGYYYDY FDYW | **946** |
| **0015-803** | **Fzd1** | **ASFSGHYWT** | **158** | **GEIDHTGSTNYE** | **487** | CARGGQGGYDWGHYH GLDVW | **835** |
| **001S-H08** | **Fzd5** | **RAFTDNVMA** | **329** | **ATISGGGGSTFDD** | **466** | **CAAASSLTSTPYDLW** | **678** |
| **001S-A09** | **Fzd5** | **RSFRTNALG** | **333** | **AAISWTGGSTYYA** | **422** | **CNTVTYTGGSYKNYW** | **1005** |
| **001S-B09** | **Fzd5** | **SIDSINAMA** | **356** | **AALTSGGITYHA** | **428** | **CNVITIVRGMGPRAYW** | **1006** |
| **001S-C09** | **Fzd5** | **SIFSINAMG** | **357** | **ATIQSGGRTNYA** | **465** | **CNVITIVRGMGPRAYW** | **1006** |
| **001S-C07** | **Fzd8** | **YTFTSYGIS** | **391** | **GWISAYNGNTNYA** | **570** | **CARDGTPFYSGSYYGSW** | **772** |
| **001S-D07** | **Fzd8** | **GTFSSYAIS** | **295** | **GRIIPILGIANYA** | **529** | CARVPTSPYDILTGPFDY W | **944** |
| **001S-E07** | **Fzd8** | **ASVSSNSAAWN** | **159** | **GRTYYRSKWYNDYA** | **542** | **CARWKNYFDPW** | **953** |
| **001S-H07** | **Fzd8** | **FTFSSYAMS** | **228** | **STISGGGGSTYYA** | **646** | **CAKDLVPWGSSAFNIW** | **704** |
| **004S-E05** | **Fzd5** | **FTFSTYEMN** | **243** | **SGVSWNGSRTHYV** | **618** | CARGQSEKWWSGLYG MDVW | **856** |
| **004S-E03** | **Fzd5** | **GTFSTYAIS** | **298** | **GWINSGNGNTKYS** | **565** | CWTGLLWFGESTDAFDI W | **1031** |
| **004S-G06** | **Fzd5** | **GTFTYRYLH** | **307** | **GGIIPIFGTGNYA** | **501** | CASSMVRVPYYYGMDV W | **964** |
| **001S-D09** | **Fzd8** | **GPFNLFAMG** | **272** | **AGISRTGGNTGYA** | **445** | CASKTTINSGWSREYHY W | **958** |
| **001S-E09** | **Fzd8** | **GPFNLFAMG** | **272** | **AGISRTGGNTGYA** | **445** | CASKTTINSGWSREYHY W | **958** |
| **001S-F09** | **Fzd8** | **GFFSSFTMG** | **268** | **AAISRNGVYTRFA** | **409** | **CNALAPGVRGSW** | **987** |
| **001S-G09** | **Fzd8** | **SLFRLNGMG** | **360** | **ATISTRGTTHYA** | **467** | **CTDEESW** | **1011** |
| **001S-H09** | **Fzd8** | **GPFNLLAMG** | **273** | **AGISRTGGNTGYA** | **445** | CASKTTINSGWSREYHY W | **958** |
| **001S-A10** | **Fzd8** | **SVVNFVVMG** | **364** | **AAITSGGSTNYA** | **425** | **CNRVGSREYSYW** | **1001** |
| **001S-B10** | **Fzd8** | **RTSDLYTMG** | **352** | AAIGYKVKWNGERT YYL | **404** | **CNAVTYNGYTIW** | **994** |
| **001S-G12** | **Fzd1** | **SIFSSNTIY** | **359** | **ALITTSGNTNYA** | **455** | CNAGAPAWTYRMGTYY PQFGSW | **986** |
| **002S-A01** | **Fzd1** | **STFSTYAMG** | **362** | **AAISGSGENTYYA** | **408** | **CVKFGMNLGYSGYDYW** | **1028** |
| **002S-B01** | **Fzd1** | **STFSNYAMG** | **361** | **AAISWGGGSTFYS** | **411** | CAAGPIARWYRGDMDY W | **681** |
| **002S-C01** | **Fzd1** | **RMFSNYAMG** | **331** | **AAISSGGSGTYYS** | **410** | CAAGPIARWYRGDMDY W | **681** |
| **002S-D01** | **Fzd1** | **RTDGGYVMG** | **337** | **ATVTWRTGTTYYA** | **469** | CAAGPIARWYRGDMDY W | **681** |
| **002S-E01** | **Fzd1** | **RTFSSAAMG** | **345** | **AAISWSGSTAYYA** | **421** | **CATLTPYGTVASY** | **974** |
| **002S-F01** | **Fzd1** | **RTFSSYAMG** | **347** | **AAVNWSGGSTYYA** | **430** | CAAVFLSRNYEIQEYYRY Q | **689** |
| **002S-G01** | **Fzd1** | **RTFSSYAMG** | **347** | **AAISWSGGSTYYA** | **418** | CAAGPIARWYRGDMDY W | **681** |
| **002S-H01** | **Fzd1** | **RSFSTYPMG** | **336** | **TVISGSGGSTYYS** | **676** | CAAGPIARWYRGDMDY W | **681** |
| **002S-A02** | **Fzd1** | **RRFTTYGMG** | **332** | **AAVTWRSGSTYYA** | **436** | **CYLEGPLDVYW** | **1032** |
| **002S-B02** | **Fzd1** | **RTFNRHVMG** | **341** | **AAISWSGDSTYYA** | **415** | **CAKLGGSSWLREYDYW** | **724** |
| **002S-C02** | **Fzd1** | **RTFRAYAMG** | **342** | **SAISWSGGSTYYA** | **603** | CAAGPIARWYRGDMDY W | **681** |
| **002S-D02** | **Fzd1** | **RTFSEYAMG** | **343** | **AAISWSGGSTHYA** | **417** | **CNADSLRGIDYW** | **984** |
| **002S-E02** | **Fzd1** | **FTFREYAMT** | **199** | **SGISRDGGRTSYS** | **613** | CAPRVLVTAPSGGMDY W | **734** |
| **002S-F02** | **Fzd1** | **GDFTNYAMA** | **267** | **AAVNWRGDGTYYS** | **429** | CAAVFLSRNYEIQEYYRY Q | **689** |
| **002S-G02** | **Fzd1** | **RTFGTWAMG** | **340** | **AAISYNGFSTYYS** | **424** | CAAGPIARWYRGDMDY W | **681** |
| **002S-H02** | **Fzd1** | **RTFSSYAMG** | **347** | **AAISWSGGSTYYA** | **418** | CAAGPIARWYRGDMDY W | **681** |
| **002S-D03** | **Fzd1** | **RTFGSYAMG** | **339** | **AAISWSGGSTYYA** | **418** | CAAGPIARWYRGDMDY W | **681** |
| **002S-E03** | **Fzd1** | **SIFSIYAMG** | **358** | **AVVATGGATNYA** | **481** | CNMRGNWYREGRPAEF LSW | **1000** |
| **002S-F03** | **Fzd1** | **RTSSSYAMG** | **353** | **AAISWSGGSTYYA** | **418** | CAAGPIARWYRGDMDY W | **681** |
| **002S-G03** | **Fzd1** | **RTFGSYAMG** | **339** | **AAISWSGGSTYYA** | **418** | CAAGPIARWYRGDMDY W | **681** |
| **002S-H03** | **Fzd1** | **QTFTAYAMG** | **327** | **AAISWSGSATHYA** | **420** | CNAWVLVAGSRGTSAD YW | **996** |
| **002S-A04** | **Fzd1** | **RTFSSYAMG** | **347** | **AAISWSGRSTYYA** | **419** | CAAGPIARWYRGDMDY W | **681** |
| **002S-804** | **Fzd1** | **RTFSSYAMG** | **347** | **AAISWSGGSTYYA** | **418** | CAAGPNYSWFMPSSSRL IW | **682** |
| **002S-C04** | **Fzd1** | **RRFTTYGMG** | **332** | **AAVTWRAGSTYYA** | **435** | CSADKLDYLDDQPFKTW DYW | **1010** |
| **002S-D04** | **Fzd1** | **GTSSTYAMG** | **309** | **AAINRSGGSTYYA** | **405** | CAAVFLSRNYEIQEYYRY Q | **689** |
| **002S-E04** | **Fzd1** | **GTFSTYAMG** | **300** | **AAISWSGDSTYYL** | **416** | CAAGPIARWYRGDMDY W | **681** |
| **004S-H04** | **Fzd5** | **GTFSSYAIS** | **295** | **GWISTYNGATNYA** | **577** | CARGGAGRFGEGMDV W | **826** |
| **001S-A04** | **Fzd5** | **YTFTSYGIS** | **391** | **GWISAYNGNTNYA** | **570** | CASSKEKATYYYGMDV W | **963** |
| **001S-D03** | **Fzd5** | **GTFSSYAIS** | **295** | **GRIIPILGIANYA** | **529** | **CARLDPGYYYGMDVW** | **886** |
| **001S-F03** | **Fzd5** | **GTFSSYAIS** | **295** | **GGIIPIFGTANYA** | **499** | **CARVIFSTVTTTNDIW** | **939** |
| **004S-E04** | **Fzd5** | **YTFSGYYLH** | **374** | **GTVTPILGTANYA** | **549** | **CARVDGSGYYGIDYW** | **933** |
| **004S-A06** | **Fzd5** | **GSFSNYAIS** | **278** | **GRIIPILGSANYA** | **530** | **CARTYLKAFDIW** | **930** |
| **004S-F04** | **Fzd5** | **YTFTNNFMH** | **383** | **GRINPNSGGTNYA** | **537** | **CARDRFDNWFDPW** | **788** |
| **001S-C03** | **Fzd5** | **GTFSSYAIS** | **295** | **GRIIPILGIANYA** | **529** | CAREGRSRVYGGNSFDY W | **808** |
| **003S-A01** | **Fzd1** | **YIFTDYYMH** | **368** | **GGIIPIFGTANYA** | **499** | CARMSSDYYDSSGYYRR GMDVW | **895** |
| **003S-E01** | **Fzd1** | **YIFTDYYMH** | **368** | **GGIIPIFGTANYA** | **499** | CARAWKGLWFGEGTFD YW | **761** |
| **003S-F01** | **Fzd1** | **GTFSSYAIS** | **295** | **GWINAGNGNTTYA** | **558** | **CARLAFDIW** | **885** |
| **003S-A02** | **Fzd1** | **YTFTGYYMH** | **379** | **GWINAGNGNTTYA** | **558** | **CAKDRGNYGDYLDYW** | **707** |
| **003S-C02** | **Fzd1** | **FTFSNSDMN** | **214** | **ALISYDGSHTYYA** | **454** | **CTRGSRIGWFDPW** | **1015** |
| **003S-E02** | **Fzd1** | **GTFSSYTIS** | **296** | **GGIIPISGKTDYA** | **505** | **CARARGGDSPLSL** | **749** |
| **003S-F02** | **Fzd1** | **GTFRSYAIN** | **292** | **GGIIPIFGTANYA** | **499** | CARGGWRPDYYGSGSY YSFDYW | **840** |
| **003S-G02** | **Fzd1** | **FTFGTYWVT** | **196** | **SGITGSGGRTFYA** | **616** | **CARMKDWFGAFDIW** | **894** |
| **003S-C03** | **Fzd1** | **FTFSRYAMS** | **220** | **SYISGDSGYTNYA** | **658** | **CARGLVIATNWFDPW** | **849** |
| **003S-D03** | **Fzd1** | **YTFTSYYMH** | **392** | **GWINTYNGNTNYP** | **567** | **CAESLTSTADW** | **691** |
| **003S-E03** | **Fzd1** | **YIFTDYYMH** | **368** | **GWVNPTTGNTGYA** | **586** | **CARNVEGATSFPEFDYW** | **898** |
| **003S-H03** | **Fzd1** | **GTFSSYAIS** | **295** | **GGIIPIFGTANYA** | **499** | **CAKDIGSSWYYYMDVW** | **701** |
| **003S-A04** | **Fzd1** | **FTFGTYWVT** | **196** | **SGITGSGGRTFYA** | **616** | **CARMKDWFGAFDIW** | **894** |
| **003S-C04** | **Fzd1** | **FAVSSSYMS** | **168** | **ASIWFDGSNQDYA** | **463** | **CAPNESGNVDYW** | **733** |
| **003S-D04** | **Fzd1** | **FTFSSYAMH** | **227** | **SAISGSGGSTYYA** | **600** | CARDHGSSWYQNTDAF DIW | **774** |
| **003S-G04** | **Fzd1** | **FRFISHPIH** | **177** | **GRVIPILGVTNYA** | **545** | CASSSDYGDYLKEPNYG MDVW | **966** |
| **003S-D05** | **Fzd2** | **FTFSNYAMT** | **216** | **SAIGTGGGTYYA** | **595** | **CATAYRRPGGLDVW** | **969** |
| **003S-E05** | **Fzd2** | **FTFSSYTMS** | **236** | **GRIKSKANGGTTDYA** | **535** | **CARGSSSWYDW** | **863** |
| **003S-A06** | **Fzd2** | **FTFADYGMH** | **188** | **SYISSGSYTIYYS** | **659** | **CARGTFDWLLSPSYDYW** | **865** |
| **003S-C06** | **Fzd2** | **FTFSNYGMH** | **217** | **SAISNSGGSTYYA** | **601** | **CTSSFLTGSQPSGYW** | **1018** |
| **003S-G06** | **Fzd2** | **FTFSDYGMH** | **207** | **SSTSGSGGNSKYS** | **642** | CARHNPGYMGYYYGM DVW | **877** |
| **003S-H06** | **Fzd2** | **GTFSSYTIS** | **296** | **GLVDPEDGETIYA** | **520** | CTILPAAAAGTYYYYGM DVW | **1012** |
| **003S-B07** | **Fzd2** | **FTFSDHYMS** | **205** | **SSITRTPSGGTTEYA** | **639** | **CARDGGYW** | **768** |
| **003S-D07** | **Fzd2** | **YTFTNNFMH** | **383** | **GIINPSGGSTSYA** | **513** | CARATSLGRRYCSSTSCY PRDAFDIW | **759** |
| **003S-E07** | **Fzd2** | **YTFTNNFMH** | **383** | **GWINPNSGGTKYA** | **563** | CARSVGEVGATMLGIGV WYWFDPW | **926** |
| **003S-A08** | **Fzd2** | **FTFSNYAMT** | **216** | **SAIGTGGGTYYA** | **595** | **CATAYRRPGGLDVW** | **969** |
| **003S-C08** | **Fzd2** | **LTVSTNFMS** | **324** | **AGIGWDSTNIGYA** | **440** | **CARDLVAARPSNWDYW** | **782** |
| **003S-E08** | **Fzd2** | **FTFRNSAMH** | **201** | **STISGSGGSTYYS** | **647** | **CARGGGYSSSW** | **829** |
| **003S-G09** | **Fzd4** | **FTFDHNPMN** | **194** | **SAIGAGGGTYYA** | **593** | **CASPTVTRR** | **960** |
| **003S-C10** | **Fzd4** | **GTFSSYAIS** | **295** | **GWINAGNGNTTYA** | **558** | **CARHYYGSGSYPDW** | **880** |
| **003S-D10** | **Fzd4** | **FNFGIYSMT** | **172** | **SYISGDSGYTNYA** | **658** | **CARVGPGGWFDPW** | **936** |
| **003S-E10** | **Fzd4** | **FTFSSYAMH** | **227** | **AGISASGGSTYYA** | **442** | **CARPSTTGTKAFDIW** | **901** |
| **003S-A11** | **Fzd4** | **GTFSSYAIS** | **295** | **GWINAGNGNTTYA** | **558** | **CARHYYGSGSYPDW** | **880** |
| **003S-G11** | **Fzd4** | **GTFSSYAIS** | **295** | **GRIIPIFGTVNYA** | **528** | **CARGARLDYW** | **820** |
| **003S-H11** | **Fzd4** | **YTFTGYYMH** | **379** | **GGIIPIFGTPHYA** | **502** | **CASTDPSSGLDYW** | **967** |
| **003S-C12** | **Fzd4** | **GTFSSYAIS** | **295** | **GWINPNSGGTNYA** | **564** | **CARGGSSDVR** | **838** |
| **003S-F12** | **Fzd4** | **FTFSSYAMH** | **227** | **SVISTSGDTVLYT** | **652** | **CARGGSSDVR** | **838** |
| **004S-B01** | **Fzd4** | **GTFSSYAIS** | **295** | **GIINPSGGSTSYA** | **513** | **CAKDGVVR** | **698** |
| **004S-C01** | **Fzd4** | **FTFSNHYTS** | **213** | **STISSSGGRTFYA** | **650** | **CARASRIDGGWPIIDHL** | **754** |
| **004S-D01** | **Fzd4** | **FTFTNYAMS** | **248** | **SAISGSGGSTYYA** | **600** | **CARATGFGTVVFDYW** | **757** |
| **004S-E01** | **Fzd4** | **GTFSSYAIS** | **295** | **GWINAGNGNTTYA** | **558** | **CARHYYGSGSYPDW** | **880** |
| **004S-F01** | **Fzd4** | **GTFSSYAIS** | **295** | **GWINAGNGNTTYA** | **558** | **CARDGVE** | **773** |
| **004S-H01** | **Fzd4** | **FTFSNYAMH** | **215** | **ALMSPDGTIIYYA** | **456** | **CAKGIVGDYGAFDIW** | **717** |
| **004S-B02** | **Fzd4** | **FTFSSYGMH** | **230** | **SSINNSSRTVFYA** | **630** | **CAKDHLAVADAHGR** | **700** |
| **004S-E02** | **Fzd4** | **FTFSSYAMH** | **227** | **AVISYDGSNEYYA** | **474** | **CAGGEVYEL** | **692** |
| **004S-F02** | **Fzd4** | **FTFSTYAMH** | **242** | **AVISSDGNNKYYT** | **473** | **CAAPDVVVTADGYYW** | **685** |
| **004S-G02** | **Fzd4** | **FTFANYAMN** | **190** | **ALISYDGGTKYYA** | **453** | **CAKTLVTSHALHIW** | **728** |
| **004S-H02** | **Fzd4** | **FTFANYAMH** | **189** | **ALISYDGGNKYYA** | **452** | **CAKTLVTSHALHIW** | **728** |
| **001S-E03** | **Fzd5** | **GSFSGYYWH** | **276** | **GEINHSGSTNYN** | **489** | CARGRRLVRFTVTSAFDI W | **858** |
| **001S-B05** | **Fzd5** | **GTFSSYAIS** | **295** | **GGIIPILGIANYA** | **504** | CARIPKPRGYSYGDNGS W | **883** |
| **004S-A07** | **Fzd6** | **GNFKNYGIT** | **271** | **GRIIPALGTANYA** | **525** | CARQYCSGGSCYPDAFDI R | **908** |
| **004S-B07** | **Fzd6** | **FTFSSYSMN** | **233** | **GVISKDGDNKYYA** | **553** | **CASSRDGYNRLAFDIW** | **965** |
| **004S-A08** | **Fzd6** | **GTFSSYAIS** | **295** | **GRIIPILGIANYA** | **529** | **CARDGGDYGMDVW** | **767** |
| **004S-B08** | **Fzd6** | **YTFTNNFMH** | **383** | **GRINPNSGGTNYA** | **537** | CASQNYYGSGSYPGFDY W | **961** |
| **004S-D08** | **Fzd6** | **YTFTYRYLH** | **394** | **GGIIPIFGTANYA** | **499** | **CATHDSSGYYSFDYW** | **973** |
| **004S-E08** | **Fzd6** | **FSVSSNYMN** | **187** | **SAIGTGGGTYYA** | **595** | CTTRTYDSSGYYETQNYY MDVW | **1024** |
| **004S-G08** | **Fzd6** | **FTFSDYYMS** | **208** | **AAISYDESNKFYA** | **423** | **CARSAVAGAFDIW** | **916** |
| **004S-A09** | **Fzd6** | **FTFRDYAMN** | **198** | **SGISWNSGSIGYA** | **615** | CARRSGYSGSVYYYYGM DVW | **913** |
| **004S-B09** | **Fzd6** | **FTFSSFGMH** | **221** | **AGINWNGGSVVYA** | **441** | **CARGPSHQHTFDIW** | **854** |
| **004S-C09** | **Fzd6** | **YTFTNNFMH** | **383** | **GGFDPEDGETIYA** | **492** | **CARVGRGYSFDYW** | **937** |
| **004S-E09** | **Fzd6** | **DTFSNYVIS** | **163** | **GRISAYNGYKSYA** | **538** | **CARSSGYVGWFDPW** | **924** |
| **004S-F09** | **Fzd6** | **FTFSNYYTS** | **218** | **SYISGAGGSTEYA** | **657** | **CARLPRRSGKGSAFDIW** | **888** |
| **004S-H09** | **Fzd6** | **GTFSSYTIS** | **296** | **GWMNPNSGNTGYA** | **583** | **CARVGATSAGGMDVW** | **935** |
| **004S-C10** | **Fzd6** | **YIFTDYYMH** | **368** | **GLVDPEDGETIYA** | **520** | **CAHSDFFSGLSFGDW** | **693** |
| **004S-D10** | **Fzd6** | **FTFSNSDMN** | **214** | **SSISTSGGSTYYA** | **637** | **CARGSYW** | **864** |
| **004S-E10** | **Fzd6** | **TTLNKYAIS** | **365** | **GRITPVVGVTNYA** | **539** | **CALSSSWYGGFDYW** | **731** |
| **004S-F10** | **Fzd6** | **GFTFSDHY** | **269** | **ALVGYDGSQQFYG** | **458** | **CNTGIPMLYW** | **1003** |
| **004S-G10** | **Fzd6** | **FTFSDYYMS** | **208** | **SAISGSGFTYYA** | **599** | **CARVSRGFAFDYW** | **948** |
| **004S-A11** | **Fzd6** | **GTFSSYAIS** | **295** | **GRIIPILGIANYA** | **529** | **CARESVNNYYYMDVW** | **813** |
| **004S-C11** | **Fzd6** | **FTFSSYAMH** | **227** | **ALTSYDGSKKFYA** | **457** | **CAKTGRGYAFDIW** | **726** |
| **004S-E11** | **Fzd6** | **FTFSSYNMN** | **232** | **GRIKSKANGGTTDYA** | **535** | **CAKAGQQLDW** | **696** |
| **004S-H11** | **Fzd6** | **FTFTSSAMQ** | **249** | **GGIIPIFGTANYA** | **499** | CATVQTNYYDSSGRFSY RAHYFDYW | **977** |
| **004S-A12** | **Fzd6** | **YTFTNNFMH** | **383** | **GRINPNSGGTNYA** | **537** | CARGQGYSSGWYRGDA FDIW | **855** |
| **004S-D12** | **Fzd6** | **FAFDDYAMH** | **165** | **GFIRSKAYGGTTEYA** | **490** | **CAKDRGYSSGWYLDYW** | **708** |
| **005S-H01** | **Fzd7** | **FNFSSYTMR** | **173** | **SVIYGGGNTNYA** | **653** | **CARGGSGGNLSYW** | **836** |
| **005S-A02** | **Fzd7** | **GTFSSYAIS** | **295** | **GMIIPFLGITNYA** | **521** | **CTRPYDAFDIW** | **1016** |
| **005S-C02** | **Fzd8** | **YTFASYGMH** | **373** | **GWINAGNGNTTYA** | **558** | CARLSVWKWEQVTNWF DPW | **890** |
| **005S-E02** | **Fzd8** | **GTFTSYAIS** | **305** | **GWINAGNGNTKYS** | **557** | CTTGLFPYYRYNWNNDA FDIW | **1022** |
| **005S-A03** | **Fzd8** | **GTFSSYAIS** | **295** | **GWMNPNSGNTGYA** | **583** | CAKWHIGATGNWFDP W | **729** |
| **005S-H03** | **Fzd8** | **YTFTNNFMH** | **383** | **GGIFPIYGISTYA** | **494** | **CARDRPTSSWYAFDYW** | **792** |
| **005S-F04** | **Fzd8** | **FSFSSTAMS** | **181** | **SYISSSGSITHYA** | **670** | **CARYGDYGDYW** | **954** |
| **005S-H04** | **Fzd8** | **YTFTNNFMH** | **383** | **GWINAGNGNTTYA** | **558** | **CARVATGNAFDIW** | **932** |
| **005S-B05** | **Fzd8** | **FTFSSYWMH** | **239** | **AGISGSGKTTFYA** | **444** | **CARGGLLFDYW** | **831** |
| **005S-F05** | **Fzd8** | **FTFTSSAVQ** | **251** | **GWMNPNSGNTGYA** | **583** | **CARRTAVAGTIDYW** | **914** |
| **005S-G05** | **Fzd8** | **GTFSSYAIS** | **295** | **GWISPYNGNTNYA** | **573** | CARGGWTNYGGNLDY W | **841** |
| **005S-H05** | **Fzd8** | **YTFTSYYMH** | **392** | **GRINPNSGGTNYA** | **537** | **CARVPDFWSGYLDYW** | **943** |
| **005S-D06** | **Fzd8** | **YTFTYRYLH** | **394** | **GGIIPIFGTANYA** | **499** | **CARDSYPYGMDVW** | **800** |
| **005S-F06** | **Fzd8** | **GTFSSYAIS** | **295** | **GRVIPILGVTNYA** | **545** | **CAREYLGSFDIW** | **815** |
| **005S-A07** | **Fzd9** | **FTFTGSAVQ** | **247** | **GGILPIYGTTKYA** | **509** | **CARGARLYGFDYW** | **822** |
| **005S-B07** | **Fzd9** | **FTFTSSAVQ** | **251** | **GWMNPNSGNTGYA** | **583** | CARGRGQQWLTGYYG MDVW | **857** |
| **005S-C07** | **Fzd9** | **FTFSSYSMN** | **233** | **SYIENDGSITTYA** | **654** | CARAPYYYGSGSLFRLDY W | **748** |
| **005S-D07** | **Fzd9** | **GTFNSYAIA** | **291** | **GGIIPIFGTANYA** | **499** | **CARAGSGYYNFDYW** | **740** |
| **005S-F07** | **Fzd9** | **FSFSSYGMH** | **182** | **AYINSRGSLMYYA** | **483** | **CAKTKLPIW** | **727** |
| **005S-G07** | **Fzd9** | **GSFSGYAIN** | **274** | **GGIIPIFGTANYA** | **499** | **CATGYYYDYYFDYW** | **972** |
| **005S-H07** | **Fzd9** | **GTFTNNFMH** | **303** | **GLVDPEDGETIYA** | **520** | CARTYRIVGATPRYYYYG MDVW | **931** |
| **005S-B08** | **Fzd9** | **YIFTDYYMH** | **368** | **GWINPNSGGTIYA** | **562** | CARGPRDSGYYPGGAFD IW | **853** |
| **005S-D08** | **Fzd9** | **FAFSSHWMH** | **166** | **SAIDGSGGSTYYA** | **592** | CARDRQLGWAHWYFDL W | **794** |
| **005S-G08** | **Fzd9** | **YTFTGYYMH** | **379** | **GWINAGNGNTTYA** | **558** | **CARDRDYW** | **787** |
| **005S-C09** | **Fzd9** | **FTFSSYGMH** | **230** | **SAIGTGGGTYYA** | **595** | CALLVGAARGISYYYYYG MDVW | **730** |
| **005S-D09** | **Fzd9** | **YTFTSYAMH** | **389** | **GWINAGNGNTTYA** | **558** | CARDRPYSSGWYYPAFD IW | **793** |
| **005S-E09** | **Fzd9** | **FNLRRYNMN** | **175** | **SRISNSGSLVYYA** | **627** | CARDADSSGYYRYDAFDI W | **762** |
| **005S-A10** | **Fzd9** | **YTFTDYYMH** | **376** | **GIINPSGGSTSYA** | **513** | CARHVYGSGTYNNWFD PW | **878** |
| **005S-D10** | **Fzd9** | **YTFTSYYMH** | **392** | **GWMSPNSANTGYA** | **583** | CARGGPIHYYYYYMDV W | **834** |
| **005S-H10** | **Fzd9** | **GAFSTSSIS** | **265** | **GRIIPVLGTANYA** | **534** | **CAKGGWRSSFDPW** | **715** |
| **005S-B11** | **Fzd9** | **YTFTSYDIN** | **390** | **GGFDPEDGETIYA** | **492** | **CAKAGDWGLYGMDVW** | **695** |
| **005S-C11** | **Fzd9** | **FTFTGSAVQ** | **247** | **GGILPIYGTTKYA** | **509** | **CARGARLYGFDYW** | **822** |
| **005S-D11** | **Fzd9** | **YTFTNNFMH** | **383** | **GWINPNSGDTKFA** | **561** | CAREANYDILTGYIRPDA FDIW | **806** |
| **005S-E11** | **Fzd9** | **GTFSSYAIS** | **295** | **GWINAGNGNTKYS** | **557** | CTTTEYSSSPDYYYGMD VW | **1025** |
| **005S-G11** | **Fzd9** | **GTFTRNSIS** | **304** | **GGIIPIFGTANYA** | **499** | **CARSSDLRIFDYW** | **922** |
| **005S-H11** | **Fzd10** | **YTFASYDIH** | **372** | **GWINAGNGNTTYA** | **558** | **CARDGIWDIFDYW** | **769** |
| **005S-E12** | **Fzd10** | **YIFTDYYMH** | **368** | **GVIFPVYPTPDYA** | **551** | **CARGGSTGYYGMDVW** | **839** |
| **005S-F12** | **Fzd10** | **GTFSSYAIS** | **295** | **GRIVPIVDVVKYA** | **541** | **CARDTCSSTSCSPDYW** | **801** |
| **006S-A01** | **Fzd10** | **FTFSSYSMN** | **233** | **SAIGTGGGTYYA** | **595** | CAREGWFGESPFGMDV W | **810** |
| **006S-F01** | **Fzd10** | **YTFTRYAVH** | **385** | **GWISTFNDNTNYA** | **576** | **CASPTGMTTNFDYW** | **959** |
| **006S-H01** | **Fzd10** | **YIFTDYYMH** | **368** | **GGIIPIFGTANYA** | **499** | CAKGSYYYDSSGYYWDA FDIW | **723** |
| **006S-A02** | **Fzd10** | **YIFTDYYMH** | **368** | **GGIIPLFGTTDYA** | **507** | **CARDITGADGMDVW** | **775** |
| **006S-D02** | **Fzd10** | **GTFSSYAIS** | **295** | **GRIIPTVGTANYA** | **533** | **CARDVCSGGSCSPDVW** | **802** |
| **006S-E02** | **Fzd10** | **FTFTSSATQ** | **250** | **GGIIPIFGTANYA** | **499** | CARDGSSGWYSPNAFDI W | **770** |
| **006S-H02** | **Fzd10** | **FTFRMYGMH** | **200** | **SRISPDGRTTTYA** | **628** | **CARSPRWYDAFDIW** | **920** |
| **006S-A03** | **Fzd10** | **YIFTDYYMH** | **368** | **GWINAGNGNTTYA** | **558** | CARDPIMFGDQPGWFD PW | **784** |
| **006S-B03** | **Fzd10** | **GTFSSYAIS** | **295** | **GWINAGNGNTKYA** | **556** | CAREGYDFWSGPYAFDI W | **811** |
| **006S-C03** | **Fzd10** | **GTFSSNVIS** | **293** | **GGIIPIFGTANYA** | **499** | **CARGGYYYGMDVW** | **843** |
| **014S-B01** | **Fzd1** | **YIFTDYYMH** | **368** | **GGIIPIFGTANYA** | **499** | CARMSSDYYDSSGYYRR GMDVW | **895** |
| **014S-D01** | **Fzd4** | **GTFSSYAIS** | **295** | **GWINAGNGNTTYA** | **558** | **CARHYYGSGSYPDW** | **880** |
| **014S-E01** | **Fzd4** | **GTFSSYAIS** | **295** | **GWINAGNGNTTYA** | **558** | **CARHYYGSGSYPDW** | **880** |
| **014S-G01** | **Fzd4** | **GTFSSYAIS** | **295** | **GWMNPNNGNTTYA** | **581** | **CARHYYGSGNYRDW** | **879** |
| **014S-A02** | **Fzd4** | **FTFSSNAMH** | **223** | **SGISGSGGSTYYA** | **608** | CAKPGIAAAGTNNWFD PW | **725** |
| **014S-B02** | **Fzd4** | **FTFSSYAMH** | **227** | **SGISGSGSSTYYA** | **611** | **CARPSTTSFGMDVW** | **902** |
| **014S-C02** | **Fzd5** | **YTFTSYYMH** | **392** | **GRINPNSGGTNYA** | **537** | **CARVPDFWSGYLDYW** | **943** |
| **014S-D02** | **Fzd5** | **GTFSTYAIS** | **299** | **GIINPSGGSTSYA** | **513** | CARAKGSGWYVGSAFDI W | **744** |
| **014S-E02** | **Fzd5** | **FTFSDSYMS** | **206** | **GFIRSKAYGGTTEYA** | **490** | CARATQELLLPYGMDV W | **758** |
| **014S-F02** | **Fzd5** | **YTFTSYYMH** | **392** | **GRINPNSGGTNYA** | **537** | **CARVPDFWSGYLDYW** | **943** |
| **014S-G02** | **Fzd6** | **YTFTSYYMN** | **392** | **GIISPSGGSTSYA** | **516** | **CARWGDYGDLYYFDYW** | **951** |
| **014S-H02** | **Fzd6** | **YIFTDYYMH** | **368** | **GRINPNSGGTNYA** | **537** | **CARARSSGWTDAFDIW** | **751** |
| **014S-A03** | **Fzd6** | **GTFSSYAIS** | **295** | **GWINAGNGNTTYA** | **558** | **CARHYYGSGSYPDW** | **880** |
| **014S-B03** | **Fzd6** | **FTFSSYNMN** | **232** | **GRIKSKANGGTTDYA** | **535** | **CARAGDSPDYW** | **739** |
| **014S-E03** | **Fzd8** | **GTFSSYAIS** | **295** | **GWISPYNGYTKYA** | **574** | CARAMWSYGQQNAFDI W | **745** |
| **014S-G03** | **Fzd8** | **FTFTSSAVQ** | **251** | **GWMNPNSGNTGYA** | **583** | **CARRTAVAGTIDYW** | **914** |
| **014S-H03** | **Fzd8** | **YTFTSSAIH** | **387** | **GRINPNSGGTNYA** | **537** | **CARVKWELAIDYW** | **940** |
| **014S-B04** | **Fzd8** | **YIFTDYYMH** | **368** | **GWMNPNSGNTGYA** | **583** | CARGGSRYDFWSGHWY FDLW | **837** |
| **014S-E04** | **Fzd8** | **YTFTGYYMH** | **379** | **GRINPNSGGTNYA** | **537** | CARDVPKLVTRGVAYG MDVW | **804** |
| **014S-F04** | **Fzd8** | **YSFTTYGMN** | **371** | **GWINAGNGNTTYA** | **558** | **CARAAAGSYGGGYW** | **736** |
| **014S-G04** | **Fzd8** | **FTFSSYGMS** | **231** | **SAISGSGGSTYYA** | **600** | **CARDLTPFTQQQLVLGLL** | **780** |
| **014S-H04** | **Fzd8** | **FTFTSSAVQ** | **251** | **GRIVPAIGFTQYA** | **540** | **CARSGYNRRGYFDYW** | **919** |
| **014S-A05** | **Fzd8** | **GTFSSYAIS** | **295** | **GGIIPIFGTANYA** | **499** | **CARVTLGASVDAFDIW** | **949** |
| **014S-B05** | **Fzd8** | **GTFSSYAIS** | **295** | **GWVSPNTGNTVYA** | **587** | **CTTDRRYSTYFDLW** | **1021** |
| **014S-C05** | **Fzd8** | **YTFASYGMH** | **373** | **GWINAGNGNTTYA** | **558** | CARLSVWKWEQVTNWF DPW | **890** |
| **014S-D05** | **Fzd8** | **GTFTSYAIS** | **305** | **GWINAGNGNTKYS** | **557** | CTTGLFPYYRYNWNNDA FDIW | **1022** |
| **014S-F05** | **Fzd9** | **FTFTGSAVQ** | **247** | **GGILPIYGTTKYA** | **509** | **CARGARLYGCDYW** | **821** |
| **014S-G05** | **Fzd9** | **FTFSSSWMH** | **226** | **SAIGTGGGTYYA** | **595** | CARKVKGYCSGGSCYGY W | **884** |
| **014S-H05** | **Fzd9** | **FTFSNYAMT** | **216** | **STISGSGVSTFYA** | **648** | **CARHGRIAADIW** | **876** |
| **014S-A06** | **Fzd9** | **FTFZZSZVQ** | **254** | **GGILPIYGTTKYA** | **509** | **CARGARLYGFDYW** | **822** |
| **014S-B06** | **Fzd9** | **FTFSSYSMN** | **233** | **SYIENDGSITTYA** | **654** | CARAPYYYGSGSLFRLDY W | **748** |
| **014S-C06** | **Fzd10** | **FTFTGSAVQ** | **247** | **GGILPIYGTTKYA** | **509** | **CARGARLYGFDYW** | **822** |
| **014S-D06** | **Fzd10** | **FTFSRYAMH** | **219** | **SGIGVGGGTYYA** | **605** | **CARDAYNWFDPR** | **763** |
| **014S-F06** | **Fzd10** | **YIFTDYYMH** | **368** | **GVIFPVYPTPDYA** | **551** | **CARGGSTGYYGMDVW** | **839** |
| **014S-G06** | **Fzd10** | **FTFSSYAMH** | **227** | **SAIGAGGGTYYA** | **593** | **CARDAYNWFDPW** | **764** |
| **014S-H06** | **Fzd10** | **FTFSSYDMN** | **229** | **SAIGTGGGTYYA** | **595** | **CARDAYNWFDPW** | **764** |
| **014S-A07** | **Fzd10** | **FTFSNAQMS** | **210** | **SAIGTGGGTYYA** | **595** | **CAREGSYYDWYFDLW** | **809** |
| **017S-E08** | **Fzd8** | **IIFSPNDMG** | **313** | **ALISSGGSTSYA** | **450** | **CHFGVASVGLNYW** | **980** |
| **017S-H08** | **Fzd8** | **RTFSSFVMG** | **346** | **AAVSASGGYTWYA** | **432** | **CNLAQRGETYW** | **998** |
| **017S-A09** | **Fzd8** | **LAFNGYTMG** | **317** | **AAISWSDNTYYA** | **414** | CAAGFPTVFVVDGEYDY W | **680** |
| **017S-B09** | **Fzd8** | **FTLDYYAIS** | **255** | **ADITSGGSTNYA** | **437** | **CNAVTYNGYTIW** | **994** |
| **017S-C09** | **Fzd8** | **LTFSDYTVG** | **319** | **ASSTGGGVFENYA** | **464** | **CNAVTYNGYTIW** | **994** |
| **018S-D06** | **Fzd4** | **RIFSSYAQA** | **330** | **PRIPSDSTTFYA** | **590** | **CEVHNFGATYW** | **979** |
| **018S-E06** | **Fzd4** | **RTFSNYVMG** | **344** | **AVISRSGGNTYYT** | **472** | **CNAVSTDWTTDYW** | **992** |
| **018S-F06** | **Fzd4** | **RTFSTYGMG** | **349** | **AAISWSDNTYYA** | **414** | **CNSFPLRLHDW** | **1002** |
| **018S-G06** | **Fzd5** | **LAIDDYYMV** | **318** | **SYISTSDGSTYYA** | **673** | **CNAVTYNGYSIW** | **993** |
| **018S-H06** | **Fzd5** | **LAFNGYTMG** | **317** | **AQISWTGGSTDYA** | **460** | **CNADYGTWYGIGW** | **985** |
| **018S-A07** | **Fzd5** | **LAFNGYTMG** | **317** | **AAISWMSNTYYA** | **412** | **CNMGLGYSEYRPLGYW** | **999** |
| **018S-B07** | **Fzd5** | **SAFSNYAMG** | **355** | **AAITWSGARTYYA** | **426** | **CNAVWKFGTTHW** | **995** |
| **018S-C07** | **Fzd7** | **LTIDDYYVV** | **323** | **SYISAGDGFTYYA** | **656** | **CNAVTYNGYTIW** | **994** |
| **017S-F09** | **Fzd4** | **GSFSGYYWS** | **277** | **GEINHSGSTNYN** | **489** | CARDLRFYSSSWRRVGM DVW | **778** |
| **017S-G09** | **Fzd4** | **YTITTYAIH** | **396** | **GWINADTGDTAYS** | **555** | **CARGWTTISSLGVW** | **872** |
| **017S-H09** | **Fzd5** | **NIFRIYAIA** | **326** | **AALTGQRTTNYA** | **427** | **CNTVTYNAGCYKKYW** | **1004** |
| **017S-A10** | **Fzd5** | **LAFNGYTMG** | **317** | **ASITWNGRYTYYA** | **462** | CNARLDAVYGHSRYDS W | **988** |
| **017S-B10** | **Fzd5** | **NFFSNYPLG** | **325** | **GAISRTGSGTFYA** | **484** | **CAAGVTGSWRYW** | **684** |
| **017S-C10** | **Fzd8** | **RSFSNYRVA** | **335** | **AVSWSVGMTYYA** | **479** | **CNAVTYNGYTIW** | **994** |
| **017S-D10** | **Fzd8** | **GTFGSYAVG** | **288** | **GLISRNAGNTLYA** | **518** | **CNAVNGRLNYW** | **991** |
| **017S-E10** | **Fzd8** | **RTFSSYSLA** | **348** | **AAVSASGANTYYA** | **431** | CAAPQSPNMYIRTDQL WWYKYW | **687** |
| **018S-D07** | **Fzd1** | **RSFSTYPMG** | **336** | **TVISGSGGSTYYA** | **675** | **CAAGPTLPFRYW** | **683** |
| **018S-E07** | **Fzd1** | **RAFSNYAMG** | **328** | **AAINWSGDSAYYA** | **406** | **CNARLSFAGGMGYW** | **989** |
| **018S-F07** | **Fzd1** | **IKSMFDMNFMG** | **314** | **AFITRGGTTRYG** | **438** | **CNAVSTDWTRDYW** | **992** |
| **018S-G07** | **Fzd1** | **LTIDDYYMV** | **322** | **SYIGTSDGTTYYA** | **655** | **CNAVTYNGYTIW** | **994** |
| **018S-H07** | **Fzd4** | **RVFSSYAQA** | **354** | **AGIASDSTTFYA** | **439** | **CKVHNFGATYW** | **983** |
| **018S-A08** | **Fzd4** | **RIFSSYAQA** | **330** | **ASIPSDGTTFYA** | **461** | **CKVHNFEATYW** | **982** |
| **018S-B08** | **Fzd4** | **LTFSTYGMG** | **321** | **AAINWSGRSTVYA** | **407** | **CNSFPLRLHDW** | **1002** |
| **018S-C08** | **Fzd4** | **RTLSSYVVG** | **351** | **ALISLSGASTYYA** | **449** | **CNAVSTDWTTDYW** | **992** |
| **018S-D08** | **Fzd5** | **IKSMFDMNFMG** | **314** | **AFITRGGTTRYG** | **438** | **CNAVSTDWTRDYW** | **992** |
| **018S-E08** | **Fzd5** | **RTDGMQAMG** | **338** | **GAITWSLGSAFYA** | **486** | **CNVLAQNDGDYRTYG** | **1007** |
| **018S-F08** | **Fzd5** | **RTFSSFVMG** | **346** | **AAVSASGGYTWYA** | **432** | **CNAVWKFGTTHW** | **995** |
| **018S-G08** | **Fzd5** | **RTFSSFVMG** | **346** | **AAVSASGGYTWYA** | **432** | **CNAVCKFGTTHW** | **990** |
| **018S-H08** | **Fzd5** | **RTFSSFVMG** | **346** | **AAVTASGGYAWYA** | **434** | **CNAVWKFGTTHW** | **995** |
| **018S-A09** | **Fzd8** | **ITFSFNSVG** | **316** | **AVFIAGYGAYYA** | **470** | **CNGVTYNGYTIW** | **997** |
| **018S-B09** | **Fzd8** | **HDFSSTYGVG** | **310** | **ATISWGGTNIA** | **468** | CAAQKPYYNGHFYADDK HYDHW | **688** |
| **018S-C09** | **Fzd8** | **ITFGFDSVG** | **315** | **AVFNAGYRAYYA** | **471** | **CNAVTYNGYTIW** | **994** |
| **018S-D09** | **Fzd8** | **RTFSWYSMG** | **350** | **AAVSWSGVSTYYP** | **433** | **CNAVTYNGYTIW** | **994** |
| **018S-E09** | **Fzd8** | **ITFSFNSVG** | **316** | **AVFIAGYGAYYA** | **470** | **CIGVTYNGYTIG** | **981** |
| **018S-F09** | **Fzd8** | **RTDGMQAMG** | **338** | **GAITWSLGIAFYA** | **485** | **CNVLAQNDGDYRTYW** | **1008** |
| **018S-G09** | **Fzd8** | **HDFSSTYGVG** | **311** | **AAISWRGTNIA** | **413** | CAAQKPYYNGHFYADDK HYDHW | **688** |
| **021S-A01** | **Fzd8** | **DSVSSNSAAWN** | **160** | **GRAYYKSRWYYDYA** | **524** | **CVRDLRPSGDLNFDYW** | **1029** |
| **021S-C01** | **Fzd1** | **GSISSGGYSWS** | **283** | **GSIYHSGSTYYN** | **547** | **CARFYYDILNGYSYFDYW** | **817** |
| **021S-D01** | **Fzd1** | **FTFSSYGMH** | **230** | **AVISYDGSNKYYA** | **475** | CAKGSVFGLKAGGYADY W | **721** |
| **021S-E02** | **Fzd8** | **YTFTSYGIS** | **391** | **GWISAYNGNTNYA** | **570** | **CARDGTPFYSGSYYGSW** | **772** |
| **021S-G02** | **Fzd8** | **DSVSSNSGAWN** | **162** | **GRTYYRSKYYNGYA** | **544** | **PRLDYW** | **1034** |
| **021S-A03** | **Fzd8** | **DSVSSNSAAWN** | **160** | **GRTYYRSKWYNDYA** | **542** | **CARSQATGERFDYW** | **921** |
| **022S-H06** | **Fzd4** | **FTFSSYAMS** | **228** | **SVISTSGGTVLYT** | **652** | **CADGSGTSHR** | **690** |
| **022S-A11** | **Fzd10** | **YIFTDYYMH** | **368** | **GGIFPIFGTANYA** | **493** | CAKGSYYYDNSGYYWD AFDIW | **722** |
| **OMP-18R5** | | **GFTFSHYTLS** | **270** | **VISGDGSYTYYADSV KG** | **677** | **NFIKYVFAN** | **1033** |
| **027S-H02** | **Fzd5** | **FTFSSYAMS** | **228** | **SAISGSGGSTYYA** | **600** | **CAKGLWGPLLNW** | **718** |
| **027S-B03** | **Fzd8** | **DSVSSNSATWN** | **161** | **GRTYYRSKWYSDYA** | **543** | **CTRGNWNVGLANW** | **1014** |
| **027S-E01** | **Fzd5** | **RSFSIYNTA** | **334** | **AAISWSGGSTYYA** | **418** | **CNVITIVRGMGPRAYW** | **1006** |
| **004S-D05** | **Fzd5** | **LTFSIYAMH** | **320** | **SAISGDGALTYYA** | **597** | CARGVYPYSSKHKPSYYY YGMDVW | **870** |
| **004S-D04** | **Fzd5** | **YDFTTYGIH** | **367** | **GGVIPAFGATDYS** | **511** | **CARGYYYGMDVW** | **874** |
| **004S-B05** | **Fzd5** | **GTFSSYAIS** | **295** | **GWINAGNGNTTYA** | **558** | **CASGLGYFDYW** | **957** |
| **004S-G03** | **Fzd5** | **YTFTNNFMH** | **383** | **GGIIPIFGTPHYA** | **502** | **CARTLTTPPYYYGMDVW** | **929** |
| **004S-F03** | **Fzd5** | **FTFSNSDMN** | **214** | **SAIGTGGDTYYA** | **594** | **CTRDLYGGYRDYW** | **1013** |
| **004S-C04** | **Fzd5** | **YIFTGYYMH** | **369** | **GRINPNSGGTNYA** | **537** | CARGGEYSSGWTYYYYY GMDVW | **827** |
| **004S-B06** | **Fzd5** | **YTFTYRYLH** | **394** | **GMINPIGGSINYA** | **522** | **CARDVMDVW** | **803** |
| **004S-F06** | **Fzd5** | **FSVGSNYMT** | **186** | **SSISSGNSYIYYA** | **634** | CARGPKTMWEDRPDY W | **851** |
| **004S-A04** | **Fzd5** | **FTFSTYSMI** | **245** | **GFIRSKDYGGTTEYA** | **491** | CARLTGGAVAGTHRDY W | **892** |
| **004S-A05** | **Fzd5** | **FTFSSYVMS** | **237** | **SAIGTGGGTYYA** | **595** | **CARGSSGYYVAW** | **862** |
| **004S-F05** | **Fzd5** | **FTFSNHYMS** | **212** | **AGVSIDANKKYYA** | **447** | **CARDQNDSWYRSDYW** | **785** |
| **003S-C01** | **Fzd1** | **GTFSSYAIS** | **295** | **GRINPNSGGTNYA** | **537** | CARGSGYDFFDYGMDV W | **861** |
| **003S-H01** | **Fzd1** | **DTFSNYVLS** | **164** | **GLVDPEDGETIYA** | **520** | **CAKASTPMVQGAPDYW** | **697** |
| **003S-H02** | **Fzd1** | **GTFNRYAIT** | **289** | **GGIIPIFGTANYA** | **499** | CATTQGVYSSSWYGGG RAFDIW | **976** |
| **003S-H04** | **Fzd1** | **YTFTYRYLH** | **394** | **GRINPNSGGTNYA** | **537** | **CWGGSYYGDYW** | **1030** |
| **003S-A05** | **Fzd2** | **FTFSSYAMH** | **227** | **SSISWNSGRVDYA** | **638** | **CARGSGIAASGSYW** | **860** |
| **003S-B05** | **Fzd2** | **FTFSNAWMS** | **211** | **STIAGSGGRTYYS** | **643** | CAKDSIGRRGRGAPQPY YYYGMDVW | **709** |
| **003S-F05** | **Fzd2** | **FSFSTYTMS** | **184** | **SRINGDGSSTRYA** | **624** | CARAIVGATGLNRFKAF DIW | **743** |
| **003S-G05** | **Fzd2** | **STFTNAWMS** | **363** | **SAIGTGGGTYYA** | **595** | CARDRVTLRGGYSYGTD AFDIW | **796** |
| **003S-H05** | **Fzd2** | **FTLSTYNMN** | **257** | **SRINYDGSATTYA** | **626** | CARDRDIVVVPAQRGEG GFDPW | **786** |
| **003S-A07** | **Fzd2** | **FTFSSYAMS** | **228** | **SAISGSGGSTYYA** | **600** | **CAKGGRDGYKGYFDYW** | **714** |
| **003S-C07** | **Fzd2** | **FSFRSYSMS** | **178** | **SAIGTGGGTYYA** | **595** | **CTTTTVTTSW** | **1026** |
| **003S-F07** | **Fzd2** | **FSFSSYGMS** | **183** | **SHISSGGATIDYA** | **619** | **CARDGGYW** | **768** |
| **003S-G07** | **Fzd2** | **FTFSSYWMH** | **239** | **SYISGDSGYTNYA** | **658** | CARDNGYCSGGSCYATY YGMDVR | **783** |
| **003S-B08** | **Fzd2** | **FTFSSZZMH** | **240** | **AVISYDGSNRZYA** | **476** | CARSYYDSSGYPRKDAF DIW | **927** |
| **003S-F08** | **Fzd2** | **ZSVSSNYMS** | **401** | **SRINSDGSTISYA** | **625** | CARARLLGGYYTPDRMD VW | **750** |
| **003S-H08** | **Fzd2** | **FTFNRHALS** | **197** | **ALISSNGDHKYYT** | **451** | **CARDLMVGRNKLDYW** | **777** |
| **003S-A09** | **Fzd2** | **FTFSSSNMN** | **225** | **SGISGSGSSTYYA** | **611** | **CARGRVWSSRDYW** | **859** |
| **003S-B09** | **Fzd2** | **FNIRRZNMZ** | **174** | **SAIGTGGGTYYA** | **595** | **CARGDSGSYRDYW** | **823** |
| **003S-C09** | **Fzd2** | **FTFSSSAMH** | **224** | **SGISGSGTTTYYR** | **612** | **CARRLIAVAGAEFDPW** | **912** |
| **003S-F09** | **Fzd4** | **FTFSNSDMN** | **214** | **GRIKSKAYGGTTEYA** | **536** | **CARQYYFDYW** | **909** |
| **003S-H09** | **Fzd4** | **FTFSSFGMH** | **221** | **SVISSGGSPYYA** | **651** | **CATASGDFDYW** | **968** |
| **003S-A10** | **Fzd4** | **FTFDDYAMH** | **191** | **AIVSYDGTYKYYS** | **448** | **CARQTRGGTTDGW** | **907** |
| **003S-B10** | **Fzd4** | **FTFSSHSTH** | **222** | **SAISASGDSTFYA** | **596** | **CARPIVGATAFDIW** | **900** |
| **003S-G10** | **Fzd4** | **FTZSSYSMN** | **264** | **SYSSGNSGYTNYA** | **674** | **CARGVVGSGAFDIW** | **868** |
| **003S-B11** | **Fzd4** | **FTFSDYYMS** | **208** | **SAIDGAGRTYYT** | **591** | **CARAIPGDYDYW** | **742** |
| **003S-C11** | **Fzd4** | **FTFTSYAMH** | **252** | **GGIIPIFGIANYA** | **496** | **CARTGRGYYGMDVW** | **928** |
| **003S-D11** | **Fzd4** | **FTFSSYSMS** | **234** | **SYISGDSGYTNYA** | **658** | **CARAGVATIAFDYW** | **741** |
| **003S-F11** | **Fzd4** | **FTFDDYGMH** | **192** | **SAISGSGGSTYYA** | **600** | **CTTPNYYDSR** | **1023** |
| **003S-E12** | **Fzd4** | **GTFSSYAIS** | **295** | **GWINAGNGNTTYA** | **558** | **CARHYYGSGSYPDW** | **880** |
| **004S-A01** | **Fzd4** | **FTFSTYGMH** | **244** | **SYISSSSSAIYYA** | **671** | **CARGGLDGPIDYR** | **830** |
| **004S-G01** | **Fzd4** | **FTVSSHSMG** | **260** | **SLVSFDGSKEHYA** | **621** | **CARLGSTPDYW** | **887** |
| **004S-C02** | **Fzd4** | **FTFSSYGMH** | **230** | **AVISYDGSNKYYA** | **475** | **CASDPVTAATR** | **956** |
| **004S-D02** | **Fzd4** | **FSFSSYGMS** | **183** | **SGISGSGRSTYYA** | **610** | **CAKDGYW** | **699** |
| **004S-A03** | **Fzd4** | **FTFSSYAMH** | **227** | **SGINWNGGSTGYA** | **606** | **CARPAGSAQNWFDPW** | **899** |
| **004S-B03** | **Fzd4** | **FSFSRYGMS** | **180** | **SGVGGSGGSTZYA** | **617** | **CARDGSW** | **771** |
| **004S-C03** | **Fzd4** | **YTFTSYAIS** | **388** | **GIINPSGGSTSYA** | **513** | **CARQIGWELMPDIW** | **906** |
| **004S-C05** | **Fzd5** | **ZZZTDYYZQ** | **403** | **GGMNZNRGNTGYA** | **510** | **CANGSYAQHLW** | **732** |
| **004S-G05** | **Fzd5** | **FTFSSYWMH** | **239** | **STISPSGLYIYQA** | **649** | **CAKDKVPYSYGPNFDYW** | **703** |
| **004S-E06** | **Fzd5** | **FFFSGYWMS** | **169** | **ANIKQDGSEKYYV** | **459** | **CARVFPLHDYW** | **934** |
| **004S-C06** | **Fzd5** | **FPFSTFSMN** | **176** | **AGISWNSGTIDYA** | **446** | CARSGPAAMVYYYYGM DVW | **918** |
| **004S-E07** | **Fzd6** | **FTLSSHHMN** | **256** | **SAIGTGGGTYYA** | **595** | **CAAPDYW** | **686** |
| **004S-F07** | **Fzd6** | **FSFSKKYMT** | **179** | **SSIDGNGDHVFYA** | **629** | CARPYYYDSSGYDPMGD YW | **904** |
| **004S-C08** | **Fzd6** | **FTVSSNYMN** | **261** | **SAIGTGGGTYYA** | **595** | **CAQGTYW** | **735** |
| **004S-F08** | **Fzd6** | **FTFDDYYMN** | **193** | **SAVSGNGGGTFYA** | **604** | **CARGGNYGSGDYW** | **833** |
| **004S-G09** | **Fzd6** | **GTLNNHTLS** | **308** | **GRIIPIFGTANYA** | **526** | **CARDRRGYGMDVW** | **795** |
| **004S-B10** | **Fzd6** | **FTFSDYYMS** | **208** | **SGINWNSAKIGYV** | **607** | **CARIGAGGAFDIW** | **881** |
| **004S-H10** | **Fzd6** | **FIFSDYYMS** | **170** | **AVITSGGTFKYYA** | **477** | **CARNGIAAAEDYW** | **896** |
| **004S-B11** | **Fzd6** | **FTFSSSWMH** | **226** | **SGISWNSGSIGYA** | **615** | **CARYSSGGSLDYW** | **955** |
| **004S-D11** | **Fzd6** | **YZFZZZYMH** | **400** | **GRINPNSGGTNYA** | **537** | **CARARSSGWTDAFDIW** | **751** |
| **004S-F11** | **Fzd6** | **FTFSSYAMS** | **228** | **SSISGGGRHTYYA** | **632** | **CARPYSSSRQGDYW** | **903** |
| **004S-G11** | **Fzd6** | **YIFTDYYMH** | **368** | **GWINPNSGGTNYA** | **564** | **CARDRPGFDPW** | **790** |
| **004S-B12** | **Fzd6** | **FTFSSYWIH** | **238** | **SYISGDSGYTNYA** | **658** | **CAKGIRWFDPW** | **716** |
| **004S-C12** | **Fzd6** | **YIFTDYYMH** | **368** | **GWMNPNSGNTGYA** | **583** | **CASSHYAPGMDVW** | **962** |
| **004S-F12** | **Fzd7** | **FTVGNNYMS** | **258** | **SSITTTSTLYA** | **640** | **CARGKEGRYSNYEAAW** | **844** |
| **005S-B01** | **Fzd7** | **FTFRSYGMH** | **202** | **SLISGSGDNTNYA** | **620** | **CARREPLYSSRRGAFDIW** | **910** |
| **005S-C01** | **Fzd7** | **FTFSSYSMS** | **234** | **SAISGSGGSTYYA** | **600** | **CTRTIVGATPHYW** | **1017** |
| **005S-F01** | **Fzd7** | **FTVSSNYMS** | **262** | **SAISGSGATTTYA** | **598** | CAKGAGYGSGSWQAA W | **711** |
| **005S-B02** | **Fzd8** | **YSFTNYAMH** | **370** | **GRIIPIFGTAZYA** | **527** | CARGTFLEWLLTNYGMD VW | **866** |
| **005S-D02** | **Fzd8** | **GTFSSYVIS** | **297** | **GWIGPHNGNTNYA** | **554** | **CATGWPRYYYGMDVW** | **971** |
| **005S-G02** | **Fzd8** | **YTFTSYYMH** | **392** | **GGIIPIFGTAZYA** | **500** | CARLPYYDFWSGYYGGR TGFDYW | **889** |
| **005S-H02** | **Fzd8** | **YTFTYRYLH** | **394** | **GWINAGNGNTTYA** | **558** | CARASLYYDYVWGSYRH YYFDYW | **753** |
| **005S-803** | **Fzd8** | **GTFSSYAIS** | **295** | **GIINPSGGRTTYA** | **512** | CATSFGGGWIVVDTSLW YW | **975** |
| **005S-C03** | **Fzd8** | **GSFSGYAIS** | **275** | **GGIIPIFGTANYA** | **499** | **CRVDAFDIW** | **1009** |
| **005S-E03** | **Fzd8** | **FTFTSSAVQ** | **251** | **GGIIPIFGTANYA** | **499** | **CARSSGWQNRFAFDIW** | **923** |
| **005S-F03** | **Fzd8** | **YTFTYRYLH** | **394** | **GWINAGNGNTKYS** | **557** | CATDLPVRKGFTYYDILT GSYGMDVW | **970** |
| **005S-B04** | **Fzd8** | **YTFTNNFMH** | **383** | **GGIIPIFGTANHA** | **498** | CARGLRYFDWPQGIYYY YGMDVW | **848** |
| **005S-C04** | **Fzd8** | **YTFTSYYMH** | **392** | **GRINPNSGGTNYA** | **537** | **CARGGLLFDYW** | **831** |
| **005S-D04** | **Fzd8** | **FTFSTYSMS** | **246** | **STIGTGGGTYYA** | **645** | **CARVGWLRFLDYW** | **938** |
| **005S-G04** | **Fzd8** | **GTFSSYAIS** | **295** | **GWMSPSSGNAGYA** | **585** | **CARNNFLRAFDIW** | **897** |
| **005S-A05** | **Fzd8** | **FAFSSYAMS** | **167** | **SRIDTDGSTTVYA** | **622** | **CARAPSYSSGWYVRW** | **747** |
| **005S-C05** | **Fzd8** | **YTFTYYAMH** | **395** | **GIINPSGGSTSYA** | **513** | **CARELLPMTTVTSPFIW** | **812** |
| **005S-E05** | **Fzd8** | **GTFSSYAIS** | **295** | **GGIIPIFGTANYA** | **499** | **CAIRAFDIW** | **694** |
| **005S-C06** | **Fzd8** | **ZTFSZYDMH** | **402** | **SSISSSSHYKYYA** | **635** | CARVRSKAVAGTLPKRLF DIW | **947** |
| **005S-E06** | **Fzd8** | **YTFTSYYMH** | **392** | **GWMNPNSGNTGYA** | **583** | CARGNPTSGHIVVVPAA TFSDYW | **850** |
| **005S-G06** | **Fzd8** | **GTFSZZTIS** | **302** | **GWMNPDSGKTGYA** | **579** | **CARWAFPIPNAFDIW** | **950** |
| **005S-H06** | **Fzd8** | **YTFTNNFMH** | **383** | **GGIFPIYGISTYA** | **494** | **CARDRPSSSWYAFDYW** | **791** |
| **005S-A08** | **Fzd9** | **GTFSZYAIS** | **301** | **GGIIPIFGTANYA** | **499** | CARGGLLRFGDGWGMG MDVW | **832** |
| **005S-C08** | **Fzd9** | **YTFTDYHMH** | **375** | **GWINAGNGNTTYA** | **558** | CARASSWYLHYYYGMD VW | **755** |
| **005S-E08** | **Fzd9** | **FIFSZYAMS** | **171** | **SSISAAGAYKYYA** | **631** | CARRGYSSGWRDAFDI W | **911** |
| **005S-F08** | **Fzd9** | **YTFTSYYMH** | **392** | **GWINAGNGNTTYA** | **558** | **CAKDVNYW** | **710** |
| **005S-H08** | **Fzd9** | **GTFSSYAIS** | **295** | **GRIIPILGTPNYA** | **531** | **CARDRLAFDYW** | **789** |
| **005S-A09** | **Fzd9** | **FAFSSHWMH** | **166** | **SAISVSGGTTFYA** | **602** | CARWGKRLRGSPYYFDY W | **952** |
| **005S-F09** | **Fzd9** | **FTFSIYGMH** | **209** | **SGISWNSGNIGYA** | **614** | CARGPLPTKIGGHYMDV W | **852** |
| **005S-B10** | **Fzd9** | **FTFSTXWMS** | **241** | **AVMYSGGTTYYA** | **478** | CARLSYYYDSSGPKGDAF DIW | **891** |
| **005S-C10** | **Fzd9** | **FSLSSYGMH** | **185** | **SSISSSSSYIYYA** | **636** | **CARSGMVKWLRSFDYW** | **917** |
| **005S-E10** | **Fzd9** | **FTFTSSAMQ** | **249** | **GVINPGSGGTSYN** | **552** | CARGYGDYVWGENYFD YW | **873** |
| **005S-F10** | **Fzd9** | **YTLSNYGIS** | **397** | **GWISAYNGDTKYA** | **568** | **CARFDYFGGMDVW** | **816** |
| **005S-G10** | **Fzd9** | **YTFTRYAVH** | **385** | **GGIIPFFNTVNYA** | **495** | CAADRSPYYYDSSGYYP DAFDIW | **679** |
| **005S-A11** | **Fzd9** | **FTFSSYDMN** | **229** | **SGISWNSGYIGYA** | **615** | **CAKGSLLLGYYGMDVW** | **720** |
| **005S-B12** | **Fzd10** | **FTZSSYDMH** | **263** | **SSISGLGGSTYZA** | **633** | **CAREAGTTGGWFDPW** | **805** |
| **005S-D12** | **Fzd10** | **FTFSDHYMD** | **204** | **STIGPAGDTYYP** | **644** | **CARASTSGDYSLW** | **756** |
| **006S-B01** | **Fzd10** | **YTFTNYCTR** | **384** | **GLVCPSDGSTSYA** | **519** | **CARRTSASDIW** | **915** |
| **006S-C01** | **Fzd10** | **FTFTZSAVQ** | **253** | **GGFDPEDGETIYA** | **492** | **CTTDPLELPWYW** | **1020** |
| **006S-E01** | **Fzd10** | **YTFTGYYMH** | **379** | **GIINPSSGRTDYA** | **514** | CARDLTYYYDSSGHSPLG AFDIW | **781** |
| **006S-G01** | **Fzd10** | **FTFSDFGMN** | **203** | **AGISGGGGSTDYA** | **443** | CARDSDFWYYYGMDV W | **797** |
| **006S-B02** | **Fzd10** | **VSFSGYAMH** | **366** | **AYINSGSSEMNYA** | **482** | **CAREEWELFGMDVW** | **807** |
| **006S-G02** | **Fzd10** | **YTVTSYAMH** | **399** | **GGIIPIFGTAKYA** | **497** | **CAKGGQWLYGMDVW** | **713** |
| **014S-A01** | **Fzd1** | **YTFTSYYMH** | **392** | **GWVSPSSGNTAYA** | **588** | CARDEGAGYYYYYMDV W | **766** |
| **014S-C01** | **Fzd2** | **FTFSNYAMT** | **216** | **SAIGTGGGTYYA** | **595** | **CATAYRRPGGLDVW** | **969** |
| **014S-F01** | **Fzd4** | **FTFSSYAMH** | **227** | **SVISTSGDTVLYT** | **652** | **CARGGSSDVR** | **838** |
| **014S-H01** | **Fzd4** | **FTFSNYGMH** | **217** | **SYISSSSSTIYYA** | **672** | CARAALGYCTGGVCPPV DYW | **737** |
| **014S-C03** | **Fzd7** | **YTFTNNFMH** | **383** | **GIIZPGGGRTIYA** | **517** | **CAKGDYGALDYW** | **712** |
| **014S-D03** | **Fzd7** | **FNFSSYTMR** | **173** | **SVIYGGGNTNYA** | **653** | **CARGGSGGNLSYW** | **836** |
| **014S-F03** | **Fzd8** | **YTFTNNFMH** | **383** | **GGIIPLFGTANYA** | **506** | **CARLVVRGGYGMDVW** | **893** |
| **014S-A04** | **Fzd8** | **GTFSSYAIS** | **295** | **GWISSFNGNTKYA** | **575** | CARADDYYDSSGYYYGF DYW | **738** |
| **014S-C04** | **Fzd8** | **FTFSSYTMN** | **235** | **SRINGDGSNTNYA** | **623** | **CARGWAGFDYW** | **871** |
| **014S-D04** | **Fzd8** | **HTFSGYHIH** | **312** | **GWINAGNGNTTYA** | **558** | CARDLSPMVRGVISGM DVW | **779** |
| **014S-E05** | **Fzd8** | **YTFTNNFMH** | **383** | **GIISPGGGRTIYA** | **515** | **CAKGDYGALDYW** | **712** |
| **014S-E06** | **Fzd10** | **FTFGNYDMN** | **195** | **SSLSWNSGTIVYA** | **641** | CARDSSSGWYASYYGM DVW | **798** |
| **027S-C02** | **Fzd5** | **YTLTTWYMX** | **398** | **GWMNPNSGNTAYA** | **582** | **CARGALGMDVW** | **819** |
| **027S-E03** | **Fzd8** | **YTFTGHYMH** | **378** | **GWMNPNSGNTGYA** | **583** | **CARGTGGFDYW** | **867** |
| **027S-F03** | **Fzd8** | **YTFTGHYIH** | **377** | **GWMNPISGNTGYA** | **580** | **CARSTPFDPW** | **925** |
| **027S-G03** | **Fzd8** | **YTFTHSYIH** | **380** | **GWINAKSGGTFYA** | **559** | CARGDYDFWSGYHEYYY YGMDVW | **824** |
| **027S-H03** | **Fzd8** | **YTFTSYYMH** | **392** | **GWINPNSGGTNYA** | **564** | **CARAPLDGSGSYYVDW** | **746** |
| **027S-A04** | **Fzd8** | **YTFTNHFMH** | **382** | **GWISPNRGGTNYA** | **571** | **CARDCSGGSCYSHFDYW** | **765** |
| **027S-B04** | **Fzd8** | **FTVGSWYMS** | **259** | **SAIGTGGGTYYA** | **595** | **CAKDITPYGDYSILSHW** | **702** |
| **027S-C04** | **Fzd8** | **YTFTSHWMH** | **386** | **GGIIPIFGTTNYA** | **503** | CARDSSSWYSYYYYYMD VW | **799** |
| **027S-D04** | **Fzd8** | **YTFTTYFMH** | **393** | **GWIYPNSGGTKYA** | **578** | **CTTDLRYDSSGPAAFDIW** | **1019** |
| **027S-E04** | **Fzd8** | **FTFSDHYMS** | **205** | **SGISGSGGTTYYA** | **609** | **CATYGDFGYFDLW** | **978** |
| **027S-C05** | **Fzd8** | **GSFSTSVFG** | **279** | **GRIIPLFGTTNYA** | **532** | **CVKDRAWGFDYW** | **1027** |
| **027S-D05** | **Fzd8** | **YTFTSYYMH** | **392** | **GWINPKSGGTNYA** | **560** | **CARGGFVFDYW** | **828** |
| **027S-E05** | **Fzd8** | **GTFSSYAIS** | **295** | **GMINPSGGSTTYA** | **523** | CARQAGLHCSSTSCYLG NWFDPW | **905** |
| **027S-F05** | **Fzd8** | **GTFNRYGIS** | **290** | **GGIIPRLGATDYA** | **508** | **CAKGNWAFDIW** | **719** |
| **027S-G05** | **Fzd8** | **GTFSSYAIS** | **295** | **GWISPYNGNTKYA** | **572** | CARGVWTTPMGGGGN WFDPW | **869** |
| **027S-H05** | **Fzd8** | **GTFGNYGIN** | **286** | **GWINPNSGGTNYA** | **564** | **CARETTDYYYGMDVW** | **814** |
| **027S-A06** | **Fzd8** | **GTFSSYAIN** | **294** | **GVIDPSTGGTNYA** | **550** | CARVLPGDSSGWYRGYY YYYGMDVW | **941** |
| **027S-C06** | **Fzd8** | **GTFTSYPIS** | **306** | **GWINTYNGNTIYA** | **566** | **CARDLDSGFDLW** | **776** |
| **027S-D06** | **Fzd8** | **GTFSSYAIS** | **295** | **GWISAYNGHTNYA** | **569** | **CARGGYSYGTVFDYW** | **842** |
| **027S-E06** | **Fzd8** | **YTFTKDYMH** | **381** | **GGIIPIFGTANYA** | **499** | CARGLPPAAGGGGYFQ HW | **847** |
| **027S-F06** | **Fzd8** | **FTFSSYAMH** | **227** | **AVTWYDGSNKYYA** | **480** | CAKDLVPYCSGGSCPPSG W | **705** |
| **027S-G06** | **Fzd5** | **YTFTDYYMH** | **376** | **GWMSPNSGNAGFA** | **584** | **CARGKSGSFDYW** | **846** |
| **027S-H06** | **Fzd5** | **YTFTGHYIH** | **377** | **GIINPSGGSTSYA** | **513** | CARGFCSGGSCLWYGM DVW | **825** |
| **027S-A07** | **Fzd5** | **GTFGSYAIT** | **287** | **GGIIPIFGTANYA** | **499** | **CAKDNGWYFDLW** | **706** |
| **029S-B01** | **Fzd1** | **GTFSSYAIS** | **295** | **GRINPHNGNTNYA** | **1461** | CARATRVSAAGTVHFQH W | **1472** |
| **029S-D01** | **Fzd2** | **YTFTRYYIH** | **1452** | **GWMNPNSGNTGYA** | **583** | **CARVRFLEEMDVW** | **1494** |
| **029S-C02** | **Fzd2** | **GTFSSYGIS** | **1457** | **GIINPSGGSTSYA** | **513** | CARGDIVATMGMKKVD YYYYMDVW | **1478** |
| **029S-F02** | **Fzd2** | **YTFTRYYLH** | **1453** | **GWMNPNSGNTGYA** | **583** | **CARGIGYW** | **1481** |
| **029S-H02** | **Fzd2** | **GTFSTYAIS** | **298** | **GDIIPIFGSANYA** | **1458** | **CARELGLGWFDPW** | **1476** |
| **030S-A02** | **Fzd7** | **YTFTDYYMH** | **1449** | **GWMNPNSGSTGYA** | **1467** | **CARGDINYGNFDYW** | **1477** |
| **030S-B02** | **Fzd3** | **YTFTDYYMH** | **1449** | **GWMNPNSGNTGYA** | **583** | **CARQGGSYSMGLDPW** | **1488** |
| **029S-E03** | **Fzd3** | **YTFTGYYMH** | **379** | **GWINPNSGNTGYA** | **1463** | **CARSYYGVIDAFDIW** | **1492** |
| **029S-G03** | **Fzd3** | **YTFTNYYMH** | **1451** | **GWMNPNSGNTGYA** | **583** | CAREDDFWSGGGMDV W | **1474** |
| **030S-E03** | **Fzd3** | **FTFSDYYMS** | **1438** | **SAISGSGHSTYYA** | **1468** | **CAREGLRGWSIFDIW** | **1475** |
| **029S-D05** | **Fzd3** | **YTFTDHYFH** | **1447** | **GWANPSSGNTGTA** | **1462** | **CARSRLRWDWYFDLW** | **1491** |
| **030S-H03** | **Fzd3** | **FSFSSHAMS** | **1437** | **SAIGTGGGTYYA** | **595** | **CANPKHYW** | **1470** |
| **0295-B06** | **Fzd3** | **YTFSRHYIH** | **1446** | **GWMNPNSGNTGYA** | **583** | CARGGHTGYSSGWYNH W | **1479** |
| **029S-E06** | **Fzd3** | **YWFTASYMH** | **1455** | **GWMKPDSGNTGYA** | **1465** | **CARRSSSWGWYFDLW** | **1489** |
| **029S-H06** | **Fzd3** | **YTFAKYYIH** | **1444** | **GWMNPNSGNTGYA** | **583** | **CARHKRHTPYAFDIW** | **1487** |
| **029S-G07** | **Fzd3** | **YTFTDSYIH** | **1448** | **GWISAYNGNTNYA** | **570** | **CARGSGYFDLW** | **1486** |
| **029S-H08** | **Fzd3** | **YTFTGHYMH** | **378** | **GWMNPNSGNTGYA** | **583** | CARVGDYDRFNWYFDL W | **1493** |
| **029S-F09** | **Fzd3** | **GTFSSYAIT** | **1456** | **GWISAYNGNTNYA** | **570** | CARANRGLRKNYYYGM DVW | **1471** |
| **030S-F04** | **Fzd3** | **YTFTSSYIH** | **1454** | **GIINPSGGGAVYA** | **1459** | **CARWTTVVTGAAFDIW** | **1495** |
| **029S-A10** | **Fzd3** | **YSFTGYYLH** | **1442** | **GWINPNSGGTNYA** | **564** | CARDHGTMIAVAGTFDY YYYMDVW | **1473** |
| **029S-B11** | **Fzd3** | **YTFNGYYMH** | **1445** | **GIVNPSGGGTNYA** | **1460** | CARGGNYGRWLQPWYF DLW | **1480** |
| **029S-D11** | **Fzd3** | **HTFTSHYMH** | **1440** | GWMNPNSANAGY A | **1466** | **CARGLGYFDLW** | **1483** |
| **030S-H05** | **Fzd7** | **YSFTNYYMH** | **1443** | **GWMNPNSGNTGYA** | **583** | **CARSPDFWSGEGYFDLW** | **1490** |
| **030S-A06** | **Fzd7** | **YMFTGHDMH** | **1441** | **GRIIPILGIANYA** | **529** | CARGIHGDYGLDYYYMD VW | **1482** |
| **029S-C12** | **Fzd7** | **YTFTGYYMH** | **379** | **GWMNPNSGNTGYA** | **583** | **CARGMEYW** | **1484** |
| **030S-C06** | **Fzd7** | **YTFTGYYIH** | **1450** | **GWMDPNSGYTGYA** | **1464** | CARGPADFWSGYKNDY FDFW | **1485** |
| **4A12** | **Fzd5** | **GYTFTNYDIN** | **1439** | WIYPRDGSTKYNEKF KG | **1469** | **CVRSAWGFAY** | **1496** |
| **1791** | **Fzd7** | **TYAMH** | **2190** | RIRSKSNNYAKNY DDSVKD | **2193** | **ENYGGRFDY** | **2196** |
| **1291** | **Fzd7** | **SYAMS** | **2191** | TISDGGSYTRYPDK LKG | **2194** | **VGGRRDYFDY** | **2197** |
| **18R5** | **Fzd7** | **GFTFSHYTLS** | **2192** | VISGDGSYTYYADS VKG | **2195** | **NFIKYVFAN** | **2198** |

| **Clone ID** | **Initial Binding** | **CDRL1** | **SID NO.** | **CDRL2** | **SID NO.** | **CDRL3** | **SID NO.** |
|---|---|---|---|---|---|---|---|
| **001S-A01** | **Fzd1** | **SGSSSNIGSHTVS** | **1156** | **SNYQRPS** | **1256** | **CAAWDGSLFGHWVF** | **1265** |
| **001S-B01** | **Fzd1** | RSSRSLLDTDDGN TYLD | **1142** | **TLSHRAS** | **1259** | **CMQSIQLPWTF** | **1295** |
| **001S-E01** | **Fzd1** | **SGNTLGSHYVS** | **1155** | **QDSKRPS** | **1246** | **CQVWDSSTVVF** | **1431** |
| **001S-F01** | **Fzd1** | RSSQSLLHSNGYN YLD | **1138** | **LGSNRAS** | **1237** | **CMQGTHWPYTF** | **1289** |
| **001S-G01** | **Fzd1** | TRSSSNIGAGYDV H | **1161** | **GNSIRPS** | **1220** | **CGTWDSSLSAWVF** | **1267** |
| **001S-H01** | **Fzd1** | RSSQSLLHSNGYN YLD | **1138** | **LGSKRAS** | **1236** | **CMQALQIPPTF** | **1280** |
| **001S-A02** | **Fzd1** | **QASQDIGKYLN** | **1041** | **DASNLET** | **1185** | **CQQNDYLPLTF** | **1332** |
| **001S-E02** | **Fzd1** | RSSQSLLHSNGYN YLD | **1138** | **LGSNRAS** | **1237** | **CMQGTHWPYTF** | **1289** |
| **001S-G02** | **Fzd1** | **RASQSVGTYLT** | **1110** | **DASNRAT** | **1188** | **CMQATQFPLTF** | **1284** |
| **001S-H02** | **Fzd1** | TRSSSNIGAGYDV H | **1161** | **GNSIRPS** | **1220** | **CGTWDSSLSAWVF** | **1267** |
| **001S-A03** | **Fzd1** | **RASRSISSYFN** | **1128** | **AASSLQS** | **1175** | **CQQADTFPPTF** | **1314** |
| **001S-B03** | **Fzd1** | **SGDKVGHKYAS** | **1154** | **EDSQRPS** | **1199** | **CQAWDSSTDVVF** | **1301** |
| **001S-H08** | **Fzd5** | | | | | | |
| **001S-A09** | **Fzd5** | | | | | | |
| **001S-B09** | **Fzd5** | | | | | | |
| **001S-C09** | **Fzd5** | | | | | | |
| **001S-C07** | **Fzd8** | **QGDSLRTYYAS** | **1052** | **GKNNRPS** | **1219** | **CNSRDNSGKHKVF** | **1300** |
| **001S-D07** | **Fzd8** | RSSQSLLHSNGFN YVD | **1136** | **FGSYRAS** | **1206** | **CMQNLQTPWTF** | **1291** |
| **001S-E07** | **Fzd8** | **RASQGIRSDLA** | **1070** | **AASTLES** | **1177** | **CLQDYSYPRTF** | **1273** |
| **001S-H07** | **Fzd8** | **RASQSVSSYLA** | **1121** | **GASSRAT** | **1213** | **CQQYGSSPPTF** | **1410** |
| **004S-E05** | **Fzd5** | **RASQGISSALA** | **1076** | **AASALQS** | **1165** | **CQQTYSTPRTF** | **1394** |
| **004S-E03** | **Fzd5** | **RASQSISSYLN** | **1098** | **AASSLQS** | **1175** | **CQQSYSTPLTF** | **1372** |
| **004S-G06** | **Fzd5** | **RASQSISSYLN** | **1098** | **AASSLQS** | **1175** | **CQQSYSTPLTF** | **1372** |
| **001S-D09** | **Fzd8** | | | | | | |
| **001S-E09** | **Fzd8** | | | | | | |
| **001S-F09** | **Fzd8** | | | | | | |
| **001S-G09** | **Fzd8** | | | | | | |
| **001S-H09** | **Fzd8** | | | | | | |
| **001S-A10** | **Fzd8** | | | | | | |
| **001S-B10** | **Fzd8** | | | | | | |
| **001S-G12** | **Fzd1** | | | | | | |
| **002S-A01** | **Fzd1** | | | | | | |
| **002S-B01** | **Fzd1** | | | | | | |
| **002S-C01** | **Fzd1** | | | | | | |
| **0025-D01** | **Fzd1** | | | | | | |
| **002S-E01** | **Fzd1** | | | | | | |
| **002S-F01** | **Fzd1** | | | | | | |
| **002S-G01** | **Fzd1** | | | | | | |
| **002S-H01** | **Fzd1** | | | | | | |
| **002S-A02** | **Fzd1** | | | | | | |
| **002S-B02** | **Fzd1** | | | | | | |
| **002S-C02** | **Fzd1** | | | | | | |
| **002S-D02** | **Fzd1** | | | | | | |
| **002S-E02** | **Fzd1** | | | | | | |
| **002S-F02** | **Fzd1** | | | | | | |
| **002S-G02** | **Fzd1** | | | | | | |
| **002S-H02** | **Fzd1** | | | | | | |
| **002S-D03** | **Fzd1** | | | | | | |
| **002S-E03** | **Fzd1** | | | | | | |
| **002S-F03** | **Fzd1** | | | | | | |
| **002S-G03** | **Fzd1** | | | | | | |
| **002S-H03** | **Fzd1** | | | | | | |
| **002S-A04** | **Fzd1** | | | | | | |
| **002S-B04** | **Fzd1** | | | | | | |
| **002S-C04** | **Fzd1** | | | | | | |
| **002S-D04** | **Fzd1** | | | | | | |
| **002S-E04** | **Fzd1** | | | | | | |
| **004S-H04** | **Fzd5** | **RASQSISSYLN** | **1098** | **AASSLQS** | **1175** | **CQQSYSTPLTF** | **1372** |
| **001S-A04** | **Fzd5** | GLSSGSVSTNYYP S | **1035** | **YTNTRSS** | **1263** | **CLLYLGRGIWVF** | **1271** |
| **001S-D03** | **Fzd5** | TGTSSDVGGYNSV S | **1159** | **DVTKRPS** | **1196** | **CFSYAGSRF** | **1266** |
| **001S-F03** | **Fzd5** | **TRSSGSIASNYVQ** | **1160** | **ENDKRPS** | **1202** | **CQSYDYDHRWVF** | **1430** |
| **004S-E04** | **Fzd5** | **RASQSISSYLN** | **1098** | **AASSLQS** | **1175** | **CQQSYSTPLTF** | **1372** |
| **004S-A06** | **Fzd5** | **RASQSISSYLN** | **1098** | **AASSLQS** | **1175** | **CQQSYSTPLTF** | **1372** |
| **004S-F04** | **Fzd5** | **RASQGISSALA** | **1076** | **AASTLQS** | **1179** | **CQQSYNTPWTF** | **1351** |
| **001S-C03** | **Fzd5** | RSSQSLLRRNGHN YVD | **1139** | **MGSNRAP** | **1238** | **CMHGLHPPFTF** | **1279** |
| **003S-A01** | **Fzd1** | **RASQGISNNLN** | **1072** | **GASTLQS** | **1215** | **CQQADSFPPTF** | **1312** |
| **003S-E01** | **Fzd1** | **RASQSISSYLN** | **1098** | **AASSLQS** | **1175** | **CQQSYSTPLTF** | **1372** |
| **003S-F01** | **Fzd1** | **RASQSISSYLN** | **1098** | **AASSLQS** | **1175** | **CQQSYSTPLTF** | **1372** |
| **003S-A02** | **Fzd1** | **RASQGISNYLA** | **1074** | **EVSSVQG** | **1204** | **CQQSYSTPLAF** | **1370** |
| **003S-C02** | **Fzd1** | **RASQSIGRWLA** | **1084** | **AASRLQS** | **1171** | **CQQGFNFPLTF** | **1325** |
| **003S-E02** | **Fzd1** | **RASQGISNNLN** | **1072** | **TASSLQS** | **1258** | **CLQDYSYPYTF** | **1274** |
| **003S-F02** | **Fzd1** | **RASQSVSSDLA** | **1115** | **GASTRAT** | **1217** | **CQQYETWPVLTF** | **1405** |
| **003S-G02** | **Fzd1** | **RASESVSSSSFA** | **1056** | **GASTRAT** | **1217** | **CQQYNNWPPNYTF** | **1420** |
| **003S-C03** | **Fzd1** | **QANQDISNYLN** | **1038** | **AASSLQS** | **1175** | **CQQTYNPPRTF** | **1389** |
| **003S-D03** | **Fzd1** | **RASQGISNNLN** | **1072** | **AASSLQR** | **1174** | **CQQSYSTPFTF** | **1368** |
| **003S-E03** | **Fzd1** | **RASQGISNNLN** | **1072** | **SASNLQS** | **1252** | **CQQSYSPPPYTF** | **1364** |
| **003S-H03** | **Fzd1** | **RASQSISSYLN** | **1098** | **AASSLQS** | **1175** | **CQQSYSTPLTF** | **1372** |
| **003S-A04** | **Fzd1** | **RTSERSSISSFA** | **1148** | **GASTRAT** | **1217** | **CQQYNNWPRNYTF** | **1420** |
| **003S-C04** | **Fzd1** | **RASQGISNNLN** | **1072** | **KASSLEN** | **1225** | **CQQSYSTPHTF** | **1369** |
| **003S-D04** | **Fzd1** | **QASQDIGNYLN** | **1042** | **DVSNLER** | **1195** | **CQHLNSYPPGDTF** | **1304** |
| **003S-G04** | **Fzd1** | **RASQSISSYLN** | **1098** | **AASSLQS** | **1175** | **CQQSYSTPLTF** | **1372** |
| **003S-D05** | **Fzd2** | RSSQSLLHSDGKT YLY | **1134** | **LGSNRAS** | **1237** | **CMQNTHWPLTR** | **1293** |
| **003S-E05** | **Fzd2** | KSSQSLLHSDGKT YLY | **1036** | **LGSNRAS** | **1237** | **CMQNTHWPLTR** | **1293** |
| **003S-A06** | **Fzd2** | **RASQGISNNLN** | **1072** | **AASRLES** | **1170** | **CQQSYSTPLTF** | **1372** |
| **003S-C06** | **Fzd2** | **RASQDISSYLA** | **1065** | **AASSLQS** | **1175** | **CQQSYRTPLTF** | **1353** |
| **003S-G06** | **Fzd2** | **RASQSVSSNLA** | **1116** | **DASNRAT** | **1188** | **CQHRTSWPLTF** | **1307** |
| **003S-H06** | **Fzd2** | **RASQRVGNNLA** | **1083** | **DASIRAT** | **1184** | **CQQYKDWPTF** | **1415** |
| **003S-B07** | **Fzd2** | **RASQSVGSYLA** | **1109** | **GSSNRAA** | **1221** | **CQQYGTSLLTF** | **1414** |
| **003S-D07** | **Fzd2** | **QASQGISNNLN** | **1049** | **LGSDRAS** | **1233** | **CQQSYSTPFTF** | **1368** |
| **003S-E07** | **Fzd2** | **RASQSISSYLN** | **1098** | **AASSLQS** | **1175** | **CQQSYSTPFTF** | **1368** |
| **003S-A08** | **Fzd2** | RSSQSLLHSDGKT YLY | **1134** | **LGFNRAS** | **1232** | **CMQNTHWPLTR** | **1293** |
| **003S-C08** | **Fzd2** | **RASQGIRNDLG** | **1069** | **GASTLQR** | **1214** | **CQQSYSTPRVTF** | **1374** |
| **003S-E08** | **Fzd2** | RSSRSLLHSDGKTY LY | **1143** | **LGSNRAS** | **1237** | **CMQSSHWPKTF** | **1298** |
| **003S-G09** | **Fzd4** | **RASQSISSYLN** | **1098** | **AASSLQS** | **1175** | **CQQSYSTPLTF** | **1372** |
| **003S-C10** | **Fzd4** | **RASQSISSYLN** | **1098** | **AASSLQS** | **1175** | **CQQSYSTPLTF** | **1372** |
| **003S-D10** | **Fzd4** | **RASQGISSYLA** | **1076** | **AASNLLG** | **1167** | **CQQTYSTPWTF** | **1396** |
| **003S-E10** | **Fzd4** | **RASQSIGSNLD** | **1085** | **AASTLET** | **1178** | **CQQSYSVPDTF** | **1380** |
| **003S-A11** | **Fzd4** | **RASQSISZYZN** | **1103** | **ZASSLQS** | **1264** | **CQQSYSTPLTF** | **1372** |
| **003S-G11** | **Fzd4** | **RASQSISSYLN** | **1098** | **AASSLQS** | **1175** | **CQQSYSTPLTF** | **1372** |
| **003S-H11** | **Fzd4** | **RASQSIGSNLD** | **1085** | **DASSLES** | **1189** | **CQQSFIMPLTF** | **1341** |
| **003S-C12** | **Fzd4** | **RASQSISSYLN** | **1098** | **AASSLQS** | **1175** | **CQQSYSTPLTF** | **1372** |
| **003S-F12** | **Fzd4** | **RASQSISSYLN** | **1098** | **AASSLQS** | **1175** | **CQQSYSTPLTF** | **1372** |
| **004S-B01** | **Fzd4** | **RAIQSISSYLN** | **1054** | **AASSLQS** | **1175** | **CQQSYSTPLTF** | **1372** |
| **004S-C01** | **Fzd4** | **RASQDIRDELA** | **1062** | **AASTLQS** | **1179** | **CQQADSFPLTF** | **1311** |
| **004S-D01** | **Fzd4** | **RASQSISSYLN** | **1098** | **AASSLQS** | **1175** | **CQQSYSTPLTF** | **1372** |
| **004S-E01** | **Fzd4** | **ZACLRIISYLN** | **1163** | **FASSLQS** | **1205** | **CQQSYSTPLTF** | **1372** |
| **004S-F01** | **Fzd4** | **RASQGISNWLA** | **1073** | **DASSLQS** | **1190** | **CQQSHITPYTF** | **1344** |
| **004S-H01** | **Fzd4** | **RASQSISSYLN** | **1098** | **AASSLQS** | **1175** | **CQQSYSTPLTF** | **1372** |
| **004S-B02** | **Fzd4** | **RASQSISSYLN** | **1098** | **AASSLQS** | **1175** | **CQQSYSTPLTF** | **1372** |
| **004S-E02** | **Fzd4** | **RASQSISSYLN** | **1098** | **AASSLQS** | **1175** | **CQQSYSTPLTF** | **1372** |
| **004S-F02** | **Fzd4** | **RASQGISSALA** | **1076** | **AASTLQS** | **1179** | **CQQANTVPFTF** | **1322** |
| **004S-G02** | **Fzd4** | **RASQSISSYLN** | **1098** | **AASSLQS** | **1175** | **CQQSYSTPLTF** | **1372** |
| **004S-H02** | **Fzd4** | **RASQSISSYLN** | **1098** | **AASSLQS** | **1175** | **CQQSYSTPLTF** | **1372** |
| **001S-E03** | **Fzd5** | TGSSSNIGAGFGV H | **1158** | **SDRNRPS** | **1255** | **CQSYDSSLRASVF** | **1429** |
| **001S-B05** | **Fzd5** | RSSQSLLHSNGNT YLD | **1137** | **LGSDRTS** | **1234** | **CMQSLQTPYTF** | **1297** |
| **004S-A07** | **Fzd6** | **RASQDIRSALA** | **1063** | **QASSLIS** | **1245** | **CQQSYSMPQTF** | **1361** |
| **004S-B07** | **Fzd6** | **QASQDIRNYLN** | **1043** | **AASSLQS** | **1175** | **CQQSSRFWTF** | **1347** |
| **004S-A08** | **Fzd6** | **RASQSISSYLN** | **1098** | **AASSLQS** | **1175** | **CQQSYSTPLTF** | **1372** |
| **004S-B08** | **Fzd6** | **RASQSISSYLN** | **1098** | **AASSLQS** | **1175** | **CQQSYSTPLTF** | **1372** |
| **004S-D08** | **Fzd6** | **RASQSISSYLN** | **1098** | **AASSLQS** | **1175** | **CQQSYSTPLTF** | **1372** |
| **004S-E08** | **Fzd6** | RSSRSLLHSNGNT YLQ | **1144** | **LGSNRAS** | **1237** | **CVQTTQSPLTF** | **1434** |
| **004S-G08** | **Fzd6** | **RASQSISSYLN** | **1098** | **AASSLQS** | **1175** | **CQQSYSTPLTF** | **1372** |
| **004S-A09** | **Fzd6** | **RASQSISSYLN** | **1098** | **AASSLQS** | **1175** | **CQQSYSTPLTF** | **1372** |
| **004S-B09** | **Fzd6** | **RASQGISSALA** | **1076** | **AASSLQS** | **1175** | **CQQSYSHTAFTF** | **1357** |
| **004S-C09** | **Fzd6** | **RASQSISSYLN** | **1098** | **AASSLQS** | **1175** | **CQQSYSTPLTF** | **1372** |
| **004S-E09** | **Fzd6** | **RASQSISSYLN** | **1098** | **AASSLQS** | **1175** | **CQQSYSTPLTF** | **1372** |
| **004S-F09** | **Fzd6** | **RASQSISSYLN** | **1098** | **AASSLQS** | **1175** | **CQQSYSTPLTF** | **1372** |
| **004S-H09** | **Fzd6** | **RASQSISSYLN** | **1098** | **AASSLQS** | **1175** | **CQQSYSTPLTF** | **1372** |
| **004S-C10** | **Fzd6** | **RASQSISSYLN** | **1098** | **AASSLQS** | **1175** | **CQQSYSTPLTF** | **1372** |
| **004S-D10** | **Fzd6** | **RASQNINNYLA** | **1081** | **RASTLQS** | **1249** | **CQQYSSYPYTI** | **1425** |
| **004S-E10** | **Fzd6** | **RASQSISSYLN** | **1098** | **AASSLQS** | **1175** | **CQQSYSTPLTF** | **1372** |
| **004S-F10** | **Fzd6** | **RASQSISSYLN** | **1098** | **AASSLQS** | **1175** | **CQQSYSTPLTF** | **1372** |
| **004S-G10** | **Fzd6** | **RASQSISSYLN** | **1098** | **AASSLQS** | **1175** | **CQQSYSTPLTF** | **1372** |
| **004S-A11** | **Fzd6** | **RASQSISSYLN** | **1098** | **AASSLQS** | **1175** | **CQQSYSTPLTF** | **1372** |
| **004S-C11** | **Fzd6** | **RASQSISSYLN** | **1098** | **AASSLQS** | **1175** | **CQQSYSTPLTF** | **1372** |
| **004S-E11** | **Fzd6** | **RASQSISSYLN** | **1098** | **AASSLQS** | **1175** | **CQQSYSTPLTF** | **1372** |
| **004S-H11** | **Fzd6** | **RASQSISRWLA** | **1094** | **AASSLQS** | **1175** | **CQQYVSYPLTF** | **1426** |
| **004S-A12** | **Fzd6** | **RASQSISSYLN** | **1098** | **AASSLQS** | **1175** | **CQQSYSTPLTF** | **1372** |
| **004S-D12** | **Fzd6** | **RASQSISSYLN** | **1098** | **AASSLQS** | **1175** | **CQQSYSTPLTF** | **1372** |
| **005S-H01** | **Fzd7** | **RASQSISSYLN** | **1098** | **AASSLQS** | **1175** | **CQQSYSTPLTF** | **1372** |
| **005S-A02** | **Fzd7** | **RASQSISSYLN** | **1098** | **AASSLQS** | **1175** | **CQQSYSTPLTF** | **1372** |
| **005S-C02** | **Fzd8** | **RASQSISSYLN** | **1098** | **AASSLQS** | **1175** | **CQQSYSTPLTF** | **1372** |
| **005S-E02** | **Fzd8** | **RASQSISSYLN** | **1098** | **AASSLQS** | **1175** | **CQQSYSTPLTF** | **1372** |
| **005S-A03** | **Fzd8** | **RASQSISSYLN** | **1098** | **AASSLQS** | **1175** | **CQQSYSTPLTF** | **1372** |
| **005S-H03** | **Fzd8** | **RASQGISNNLN** | **1072** | **DASTLQT** | **1193** | **CQQSFSAPITF** | **1342** |
| **005S-F04** | **Fzd8** | **RASQSISSYLN** | **1098** | **AASSLQS** | **1175** | **CQQSYSTPLTF** | **1372** |
| **005S-H04** | **Fzd8** | **RASQSISSYLN** | **1098** | **AASSLQS** | **1175** | **CQQSYSTPLTF** | **1372** |
| **005S-B05** | **Fzd8** | **QASQDISNYLN** | **1046** | **KASSLES** | **1226** | **CQQSYSTPRTF** | **1373** |
| **005S-F05** | **Fzd8** | **RASQSISSYLN** | **1098** | **AASSLQS** | **1175** | **CQQSYSTPLTF** | **1372** |
| **005S-G05** | **Fzd8** | **RASQSISSYLN** | **1098** | **AASSLQS** | **1175** | **CQQSYSTPLTF** | **1372** |
| **005S-H05** | **Fzd8** | **RASQGISRTLZ** | **1075** | **AASSLQS** | **1175** | **CQQTYSMPITF** | **1392** |
| **005S-D06** | **Fzd8** | **RASQSISSYLN** | **1098** | **AASSLQS** | **1175** | **CQQSYSTPLTF** | **1372** |
| **005S-F06** | **Fzd8** | **RASQSVGSNLA** | **1108** | **GASSRAT** | **1213** | **CQQYGSSPPFTF** | **1409** |
| **005S-A07** | **Fzd9** | **RASQSVSRNLA** | **1114** | **GASTRAT** | **1217** | **CQQRSNWPITF** | **1335** |
| **005S-B07** | **Fzd9** | **RASQGISSALA** | **1076** | **GASTLQS** | **1215** | **CLQDYNYPFTF** | **1272** |
| **005S-C07** | **Fzd9** | **RASQSISSYLN** | **1098** | **AASSLQS** | **1175** | **CQQSYSTPLTF** | **1372** |
| **005S-D07** | **Fzd9** | **RASQSINRWLA** | **1090** | **AASSLQS** | **1175** | **CQQTYNIPITF** | **1388** |
| **005S-F07** | **Fzd9** | **RASQSINRNYLG** | **1089** | **AASSRVT** | **1176** | **CQQYDSWPPTF** | **1402** |
| **005S-G07** | **Fzd9** | **RASQGISNNLN** | **1072** | **AASSLQS** | **1175** | **CQHYYNLPLTF** | **1309** |
| **005S-H07** | **Fzd9** | **RASQTINNQLA** | **1125** | **KASNLET** | **1224** | **CQQANSFPVTF** | **1318** |
| **0055-B08** | **Fzd9** | **RASQSISSYLN** | **1098** | **AASSLQS** | **1175** | **CQQSYSTPLTF** | **1372** |
| **005S-D08** | **Fzd9** | **RASQSISSYLN** | **1098** | **AASSLQS** | **1175** | **CQQSYSTPLTF** | **1372** |
| **005S-G08** | **Fzd9** | **QTSQDINNNLN** | **1053** | **KASSLES** | **1226** | **CQQSYSSPPTF** | **1366** |
| **005S-C09** | **Fzd9** | **QASQDISNYLN** | **1046** | **AASTLQS** | **1179** | **CLQHKSFPTF** | **1276** |
| **005S-D09** | **Fzd9** | **RASQSVSSNQLA** | **1117** | **GASTRAT** | **1217** | **CQQRYNWPPSITF** | **1339** |
| **005S-E09** | **Fzd9** | **RASQSVSSNLA** | **1116** | **DASNRAT** | **1188** | **CQQRNNWLYTF** | **1334** |
| **005S-A10** | **Fzd9** | **RASQSISSYLN** | **1098** | **AASSLQS** | **1175** | **CQQSYSTPLTF** | **1372** |
| **005S-D10** | **Fzd9** | **RASQGISNNLN** | **1072** | **AASTLQS** | **1179** | **CQQTNLFPYTF** | **1385** |
| **005S-H10** | **Fzd9** | **RASQSVSSNLA** | **1116** | **GASTRAT** | **1217** | **CQQYNSWPLTF** | **1421** |
| **005S-B11** | **Fzd9** | **RASQSISRWLA** | **1094** | **AASSLQS** | **1175** | **CQQTNTFPFTF** | **1386** |
| **005S-C11** | **Fzd9** | **RASQSVSRKLA** | **1113** | **GASTRAT** | **1217** | **CQQRSNWPITF** | **1335** |
| **005S-D11** | **Fzd9** | **RASQSLRSKLA** | **1106** | **GASTRAT** | **1217** | **CQQYANSPWTF** | **1401** |
| **005S-E11** | **Fzd9** | **QASQDISNYLN** | **1046** | **GASTLQS** | **1215** | **CQQLSRYPSLF** | **1331** |
| **005S-G11** | **Fzd9** | **RASQSVSSNLA** | **1116** | **GASNRPT** | **1209** | **CQQYGSSPYTF** | **1413** |
| **005S-H11** | **Fzd10** | **RASQSISSYLN** | **1098** | **AASSLQS** | **1175** | **CQQSYSTPLTF** | **1372** |
| **005S-E12** | **Fzd10** | **RASQSVGRWMA** | **1107** | **AASSLQS** | **1175** | **CQQANTFPFTF** | **1321** |
| **005S-F12** | **Fzd10** | **RASQSISSYLN** | **1098** | **AASSLQS** | **1175** | **CQQSYSTPLTF** | **1372** |
| **006S-A01** | **Fzd10** | **RASQSISSYLN** | **1098** | **AASSLQS** | **1175** | **CQQSYSTPLTF** | **1372** |
| **006S-F01** | **Fzd10** | **RASQSISSYLN** | **1098** | **AASSLQS** | **1175** | **CQQSYSTPLTF** | **1372** |
| **006S-H01** | **Fzd10** | **RASQGISNNLN** | **1072** | **AASNLET** | **1166** | **CQQTSSTPLTF** | **1387** |
| **006S-A02** | **Fzd10** | **RASQSISSYLN** | **1098** | **AASSLQS** | **1175** | **CQQSYSTPLTF** | **1372** |
| **006S-D02** | **Fzd10** | **RASQGISNNLN** | **1072** | **DASSLES** | **1189** | **CQQTYNTPRTF** | **1390** |
| **006S-E02** | **Fzd10** | **RASQGISNNLN** | **1072** | **AASSLQS** | **1175** | **CLQHNGYPITF** | **1277** |
| **006S-H02** | **Fzd10** | RSSQSLLHSNGYN YLD | **1138** | **RVSSRFS** | **1251** | **CMQGTHWPPTF** | **1288** |
| **006S-A03** | **Fzd10** | **RASESVSSNLA** | **1055** | **GASSRAT** | **1213** | **CQQYNKSPSF** | **1419** |
| **006S-B03** | **Fzd10** | **RASQTISRYLN** | **1126** | **EVSSLQG** | **1203** | **CQQSYSTPWTF** | **1378** |
| **006S-C03** | **Fzd10** | **RASQSISSYLN** | **1098** | **AASSLQS** | **1175** | **CQQSYSTPLTF** | **1372** |
| **014S-B01** | **Fzd1** | **RASQGISNNLN** | **1072** | **GASTLQS** | **1215** | **CQQADSFPPTF** | **1312** |
| **0145-D01** | **Fzd4** | **RASQSISSHZN** | **1096** | **AASSLQS** | **1175** | **CQQSYSTPLTF** | **1372** |
| **014S-E01** | **Fzd4** | **RASQSIZZYZN** | **1105** | **AASSLQS** | **1175** | **CQQSYSTPLTF** | **1372** |
| **014S-G01** | **Fzd4** | **RASQSISSYLN** | **1098** | **AASSLQS** | **1175** | **CQQSYSTPFTF** | **1368** |
| **014S-A02** | **Fzd4** | **RASQGISSALA** | **1076** | **GASTVES** | **1218** | **CQQSYSTPRTF** | **1373** |
| **014S-B02** | **Fzd4** | **RASQSVSSNLA** | **1116** | **GASTRAT** | **1217** | **CQQYDTPLRTF** | **1403** |
| **014S-C02** | **Fzd5** | **RASQGISSALA** | **1076** | **AASSLQS** | **1175** | **CQQTYSMPITF** | **1392** |
| **014S-D02** | **Fzd5** | **RASQSISSYLN** | **1098** | **AASSLQS** | **1175** | **CQQSYSTPLTF** | **1372** |
| **014S-E02** | **Fzd5** | **RASQSISSYLN** | **1098** | **AASSLQS** | **1175** | **CQQSYSTPLTF** | **1372** |
| **014S-F02** | **Fzd5** | **RASQGVSTZLS** | **1079** | **AASSLQS** | **1175** | **CQQTYSMPITF** | **1392** |
| **014S-G02** | **Fzd6** | **RASQGISSALA** | **1076** | **ATSTLQS** | **1183** | **CQQVNSYPPTF** | **1399** |
| **014S-H02** | **Fzd6** | **RASQSVSSWLA** | **1120** | **AASTLQT** | **1180** | **CQQSYSTPTF** | **1376** |
| **014S-A03** | **Fzd6** | **RASQSISSYLN** | **1098** | **AASSLQS** | **1175** | **CQQSYSTPLTF** | **1372** |
| **014S-B03** | **Fzd6** | **RASQSISSYLN** | **1098** | **AASSLQS** | **1175** | **CQQSYSTPLTF** | **1372** |
| **014S-E03** | **Fzd8** | **RASQSISSYLN** | **1098** | **AASSLQS** | **1175** | **CQQSYSTPLTF** | **1372** |
| **014S-G03** | **Fzd8** | **RASQSISSYLN** | **1098** | **AASSLQS** | **1175** | **CQQSYSTPLTF** | **1372** |
| **014S-H03** | **Fzd8** | **RASQSISSYLN** | **1098** | **AASSLQS** | **1175** | **CQQSYSTPLTF** | **1372** |
| **014S-B04** | **Fzd8** | **RASQGISNYLA** | **1074** | **AASSLQS** | **1175** | **CQQSYSTPFTF** | **1368** |
| **014S-E04** | **Fzd8** | **RASQSISSYLN** | **1098** | **AASSLQS** | **1175** | **CQQSYSTPLTF** | **1372** |
| **014S-F04** | **Fzd8** | **RASQGISNNLN** | **1072** | **EASSVAS** | **1197** | **CQQSYTSTPLNSF** | **1381** |
| **014S-G04** | **Fzd8** | **RASQSVSGYLA** | **1112** | **GASTRAA** | **1216** | **CQQYNYWPPAF** | **1423** |
| **014S-H04** | **Fzd8** | **RASQSISSYLN** | **1098** | **AASSLQS** | **1175** | **CQQSYSTPLTF** | **1372** |
| **014S-A05** | **Fzd8** | **RASQGISNNLN** | **1072** | **DASSLES** | **1189** | **CLQHNSLPFTF** | **1278** |
| **014S-B05** | **Fzd8** | **RASQSVSSNLA** | **1116** | **GVSNRAT** | **1223** | **CQQYNIWPRTF** | **1418** |
| **014S-C05** | **Fzd8** | **RASQSISSYLN** | **1098** | **AASSLQS** | **1175** | **CQQSYSTPLTF** | **1372** |
| **014S-D05** | **Fzd8** | **RASQSISSYLN** | **1098** | **AASSLQS** | **1175** | **CQQSYSTPLTF** | **1372** |
| **014S-F05** | **Fzd9** | **RASQZVSRZZA** | **1127** | **GASTRAT** | **1217** | **CQQRSNWPITF** | **1335** |
| **014S-G05** | **Fzd9** | **RVSQGISSALA** | **1151** | **AASSLQS** | **1175** | **CQQTFSVPWTF** | **1384** |
| **014S-H05** | **Fzd9** | **RASQSISSYLN** | **1098** | **AASSLQS** | **1175** | **CQQSYSTPLTF** | **1372** |
| **014S-A06** | **Fzd9** | **RASQSVSRNLA** | **1114** | **GASTRAT** | **1217** | **CQQRSNWPITF** | **1335** |
| **014S-B06** | **Fzd9** | **RASQSISSYLN** | **1098** | **AASSLQS** | **1175** | **CQQSYSTPLTF** | **1372** |
| **014S-C06** | **Fzd10** | **RASQSVSRNLA** | **1114** | **GASTRAT** | **1217** | **CQQRSNWPITF** | **1335** |
| **014S-D06** | **Fzd10** | **RASQSISRYLN** | **1095** | **AASSLQS** | **1175** | **CQQRYSTPLTF** | **1340** |
| **014S-F06** | **Fzd10** | **RASQSVGRWMA** | **1107** | **AASSLQS** | **1175** | **CQQANTFPFTF** | **1321** |
| **014S-G06** | **Fzd10** | **RASQSISSYLN** | **1098** | **AASSLQS** | **1175** | **CQQSYSTPLTF** | **1372** |
| **014S-H06** | **Fzd10** | **RASQSISSYLN** | **1098** | **AASSLQS** | **1175** | **CQQSYSTPLTF** | **1372** |
| **014S-A07** | **Fzd10** | **RASQNIGSRLA** | **1080** | **GASNRAS** | **1208** | **CQQYNHWPPLFTF** | **1417** |
| **017S-E08** | **Fzd8** | | | | | | |
| **017S-H08** | **Fzd8** | | | | | | |
| **017S-A09** | **Fzd8** | | | | | | |
| **017S-B09** | **Fzd8** | | | | | | |
| **017S-C09** | **Fzd8** | | | | | | |
| **018S-D06** | **Fzd4** | | | | | | |
| **018S-E06** | **Fzd4** | | | | | | |
| **018S-F06** | **Fzd4** | | | | | | |
| **018S-G06** | **Fzd5** | | | | | | |
| **018S-H06** | **Fzd5** | | | | | | |
| **018S-A07** | **Fzd5** | | | | | | |
| **018S-B07** | **Fzd5** | | | | | | |
| **018S-C07** | **Fzd7** | | | | | | |
| **017S-F09** | **Fzd4** | RSSRSLLHTSGYNY LD | **1147** | **LGSNRAS** | **1237** | **CMQGTRWPTF** | **1290** |
| **017S-G09** | **Fzd4** | RSSRSLLHTNGYN YLD | **1146** | **LGSNRAS** | **1237** | **CMQALQTPLTF** | **1281** |
| **017S-H09** | **Fzd5** | | | | | | |
| **017S-A10** | **Fzd5** | | | | | | |
| **017S-B10** | **Fzd5** | | | | | | |
| **017S-C10** | **Fzd8** | | | | | | |
| **017S-D10** | **Fzd8** | | | | | | |
| **017S-E10** | **Fzd8** | | | | | | |
| **018S-D07** | **Fzd1** | | | | | | |
| **018S-E07** | **Fzd1** | | | | | | |
| **018S-F07** | **Fzd1** | | | | | | |
| **018S-G07** | **Fzd1** | | | | | | |
| **018S-H07** | **Fzd4** | | | | | | |
| **018S-A08** | **Fzd4** | | | | | | |
| **018S-B08** | **Fzd4** | | | | | | |
| **018S-C08** | **Fzd4** | | | | | | |
| **018S-D08** | **Fzd5** | | | | | | |
| **018S-E08** | **Fzd5** | | | | | | |
| **018S-F08** | **Fzd5** | | | | | | |
| **018S-G08** | **Fzd5** | | | | | | |
| **018S-H08** | **Fzd5** | | | | | | |
| **018S-A09** | **Fzd8** | | | | | | |
| **018S-B09** | **Fzd8** | | | | | | |
| **018S-C09** | **Fzd8** | | | | | | |
| **018S-D09** | **Fzd8** | | | | | | |
| **018S-E09** | **Fzd8** | | | | | | |
| **018S-F09** | **Fzd8** | | | | | | |
| **018S-G09** | **Fzd8** | | | | | | |
| **021S-A01** | **Fzd8** | **RASQSIGSSLH** | **1087** | **YASQSVS** | **1260** | **CHQSGRVPVTF** | **1268** |
| **021S-C01** | **Fzd1** | RSSRSLLDTDDGN TYLD | **1142** | **TLSHRAS** | **1259** | **CMQSIQLPWTF** | **1295** |
| **021S-D01** | **Fzd1** | RSSQSLVHSDGNT YLS | **1140** | **KISNRFS** | **1230** | **CMQATQFPHTF** | **1283** |
| **021S-E02** | **Fzd8** | **QGDSLRTYYAS** | **1052** | **GKNNRPS** | **1219** | **CNSRDNSGKHKVF** | **1300** |
| **021S-G02** | **Fzd8** | RSSQSLLDSDDGN TYLD | **1133** | **MLSSRAP** | **1240** | **CMQRLEFPYTF** | **1294** |
| **021S-A03** | **Fzd8** | **RSSQNIFQSLN** | **1131** | **SASSLQS** | **1254** | **CQQSYNSPITF** | **1349** |
| **022S-H06** | **Fzd4** | **RASQSISSYLN** | **1098** | **AASSLQS** | **1175** | **CQQSYSTPLTF** | **1372** |
| **022S-A11** | **Fzd10** | **RASQGISNNIN** | **1071** | **AASNLET** | **1166** | **CQQTYSIPFTF** | **1391** |
| **OMP-18R5** | | **SGDKLGKKYAS or SGDNIGSFYVH** | **1152 or 1153** | **EKDNRPSG or DKSNRPSG** | **1200 or 1201** | **SSFAGNSLE or QSYANTLSL** | **1435 or 1436** |
| **027S-H02** | **Fzd5** | **RASQSVSSNYLS** | **1118** | **GASSRAP** | **1212** | **CQQRTNWPPRVTF** | **1337** |
| **027S-B03** | **Fzd8** | SGTSSNIGAGYDV H | **1157** | **GNNNRPS** | **1219** | **CSAWDDNLNGVVF** | **1432** |
| **027S-E01** | **Fzd5** | | | | | | |
| **004S-D05** | **Fzd5** | **QASQDISNYLN** | **1046** | **AASSLQS** | **1175** | **CQQSYSTPLFTF** | **1371** |
| **004S-D04** | **Fzd5** | **RASQSISSYLN** | **1098** | **AASSLQS** | **1175** | **CQQSYSTPLTF** | **1372** |
| **004S-B05** | **Fzd5** | **RASQSISSYLN** | **1098** | **AASSLQS** | **1175** | **CQQSYSTPLTF** | **1372** |
| **004S-G03** | **Fzd5** | **RASQSISSYLN** | **1098** | **AASSLQS** | **1175** | **CQQSYSTPLTF** | **1372** |
| **004S-F03** | **Fzd5** | KSSQSLLHSDGYT YLY | **1036** | **LGSNRAS** | **1237** | **CMQGLQTPWTF** | **1286** |
| **004S-C04** | **Fzd5** | **RATQTISTYLN** | **1129** | **AASRLQS** | **1171** | **CQQYYSYPWTS** | **1427** |
| **004S-B06** | **Fzd5** | **RASQGISNNLN** | **1072** | **AASALQS** | **1165** | **CQHLNNFPLTF** | **1303** |
| **004S-F06** | **Fzd5** | **RASQSISSYLN** | **1098** | **AASSLQS** | **1175** | **CQQSYSTPLTF** | **1372** |
| **004S-A04** | **Fzd5** | **RASQGISNNLN** | **1072** | **GASSLQS** | **1211** | **CQQSHSSPRTF** | **1346** |
| **004S-A05** | **Fzd5** | **RASQSISSYLN** | **1098** | **AASSLQS** | **1175** | **CQQSYSTPLTF** | **1372** |
| **004S-F05** | **Fzd5** | RSSQSLLHSDGYT YLY | **1135** | **LGSHRAS** | **1235** | **CMQGLQTPHTF** | **1285** |
| **003S-C01** | **Fzd1** | **RASQSISNNLN** | **1092** | **AASSLQS** | **1175** | **CQQSYNTPFTF** | **1350** |
| **003S-H01** | **Fzd1** | **RASQSIGSNLD** | **1085** | **AASTLQS** | **1179** | **CQQNYATPRTF** | **1333** |
| **003S-H02** | **Fzd1** | **RASQSISSYLN** | **1098** | **AASSLQS** | **1175** | **CQQSYSTPLTF** | **1372** |
| **003S-H04** | **Fzd1** | **RASQGISNNLN** | **1072** | **AASSLQS** | **1175** | **CQQANSFPITF** | **1316** |
| **003S-A05** | **Fzd2** | TSSQSLLHSDGKT YLY | **1162** | **LGSNRAS** | **1237** | **CMQGTHWPYTF** | **1289** |
| **003S-B05** | **Fzd2** | KSSQSLLHSDGKT YLY | **1036** | **LGSNRAS** | **1237** | **CMQSLQSPLTF** | **1296** |
| **003S-F05** | **Fzd2** | KSSQSLLHSDGKT YLY | **1036** | **LGSNRAS** | **1237** | **CMQNTHWPLTR** | **1293** |
| **003S-G05** | **Fzd2** | RSSRSLLHSNGNT YLR | **1145** | **LASRRAS** | **1231** | **CIQNTHWPLTR** | **1270** |
| **003S-H05** | **Fzd2** | KSSQSLLHSDGKT YLY | **1036** | **MGSYRAS** | **1239** | **CMQGTHWPLTF** | **1287** |
| **003S-A07** | **Fzd2** | KSSQSLLHSDGKT YLY | **1036** | **LGSNRAS** | **1237** | **CMQNTHWPLTL** | **1292** |
| **003S-C07** | **Fzd2** | **RTSQSVSSNLA** | **1150** | **DASNRAS** | **1187** | **CQQYGSSPYNF** | **1412** |
| **003S-F07** | **Fzd2** | **RASQSISSYLN** | **1098** | **AASSLQS** | **1175** | **CQQSYSTPLTF** | **1372** |
| **003S-G07** | **Fzd2** | **RASQAISSYLA** | **1060** | **KASTLDT** | **1228** | **CQQADTFPFTF** | **1313** |
| **003S-B08** | **Fzd2** | KSSQSLLHSDGKT YLY | **1036** | **LGSNRAS** | **1237** | **CMQTLKAPLTF** | **1299** |
| **003S-F08** | **Fzd2** | **RSSQYLSSAYLA** | **1141** | **GASSRAT** | **1213** | **CQQYGSSPTF** | **1411** |
| **003S-H08** | **Fzd2** | **QASQGISNNLN** | **1049** | **AASSLQS** | **1175** | **CQQSYSTPAFTF** | **1367** |
| **003S-A09** | **Fzd2** | RSSQSLLHSDGYT YLY | **1135** | **LGSNRAS** | **1237** | **CMQGTHWPLTF** | **1287** |
| **003S-B09** | **Fzd2** | RSSESLLHSDGKTY LY | **1130** | **LGSNRAS** | **1237** | **CTQTVQFPITF** | **1433** |
| **003S-C09** | **Fzd2** | **RASQGISNNLN** | **1072** | **SASNLQS** | **1252** | **CQQSYSTPWTF** | **1378** |
| **003S-F09** | **Fzd4** | **RASQSISSYLN** | **1098** | **AASSLQS** | **1175** | **CQQSYSTPLTF** | **1372** |
| **003S-H09** | **Fzd4** | **RASQSIGSNLN** | **1086** | **RASTLES** | **1248** | **CQQTYTTPRF** | **1398** |
| **003S-A10** | **Fzd4** | **RASQGISNNLN** | **1072** | **YASSLQS** | **1262** | **CQQSHSPPGTF** | **1345** |
| **003S-B10** | **Fzd4** | **RASQSISSYLN** | **1098** | **AASSLQS** | **1175** | **CQQSYSTPLTF** | **1372** |
| **003S-G10** | **Fzd4** | **RASQSIVSYLN** | **1104** | **DASNLQS** | **1186** | **CQQGYSAPWTF** | **1329** |
| **003S-B11** | **Fzd4** | **RASQSISSYLN** | **1098** | **AASSLQS** | **1175** | **CQQSYSTPLTF** | **1372** |
| **003S-C11** | **Fzd4** | **RASQSIGSNLD** | **1085** | **AASTLQS** | **1179** | **CQQSYSTPRTF** | **1373** |
| **003S-D11** | **Fzd4** | **RASQSISSYLN** | **1098** | **AASSLQS** | **1175** | **CQQSYSTPLTF** | **1372** |
| **003S-F11** | **Fzd4** | **RASQSISSYLN** | **1098** | **AASSLQS** | **1175** | **CQQSYSTPLTF** | **1372** |
| **003S-E12** | **Fzd4** | **ZPZQTZZSHLN** | **1164** | **PASSLQS** | **1242** | **CQQSYSTPLTF** | **1372** |
| **004S-A01** | **Fzd4** | **RASQGISNNLN** | **1072** | **AASTLQS** | **1179** | **CQQGNNFPFTF** | **1326** |
| **004S-G01** | **Fzd4** | **RASQGISNNLN** | **1072** | **AASSLQS** | **1175** | **CQQYYTYPYTF** | **1428** |
| **004S-C02** | **Fzd4** | **RASQSISSYLN** | **1098** | **AASSLQS** | **1175** | **CQQSYSTPLTF** | **1372** |
| **004S-D02** | **Fzd4** | **RASQSISSYLN** | **1098** | **AASSLQS** | **1175** | **CQQSYSTPLTF** | **1372** |
| **004S-A03** | **Fzd4** | **RASQGISNNLN** | **1072** | **DASNLET** | **1185** | **CHQSYSIPRTF** | **1269** |
| **004S-B03** | **Fzd4** | **RASQDVDTWLA** | **1066** | **DASTLET** | **1191** | **CQQGYNIPWTF** | **1328** |
| **004S-C03** | **Fzd4** | **QASQDISSYLN** | **1047** | **AASTLQS** | **1179** | **CQQAISFPLTF** | **1315** |
| **004S-C05** | **Fzd5** | **RASQSISSYLN** | **1098** | **AASSLQS** | **1175** | **CQQSYSTPLTF** | **1372** |
| **004S-G05** | **Fzd5** | **RASQSISSYLN** | **1098** | **AASSLQS** | **1175** | **CQQSYSTPLTF** | **1372** |
| **004S-E06** | **Fzd5** | **QASQDISNYLN** | **1046** | **KASSLES** | **1226** | **CQQANSFPYTF** | **1319** |
| **004S-C06** | **Fzd5** | **RSSQNVSSYLA** | **1132** | **GASTRAT** | **1217** | **CQHRANWPQTF** | **1305** |
| **004S-E07** | **Fzd6** | **RASQSISSYLN** | **1098** | **AASSLQS** | **1175** | **CQQSYSTPLTF** | **1372** |
| **004S-F07** | **Fzd6** | **QASQDITNYLN** | **1048** | **KASTLES** | **1229** | **CQQSYSAPYTF** | **1356** |
| **004S-C08** | **Fzd6** | **RASQSISSYLN** | **1098** | **AASSLQS** | **1175** | **CQQSYSTPLTF** | **1372** |
| **004S-F08** | **Fzd6** | **RASQSISZWLA** | **1102** | **EASTLQS** | **1198** | **CQQTYTPPFTF** | **1397** |
| **004S-G09** | **Fzd6** | **RASQAISNSLA** | **1058** | **DASNLET** | **1185** | **CQQAYSFPWTF** | **1324** |
| **004S-B10** | **Fzd6** | **RASQSISSYLN** | **1098** | **AASSLQS** | **1175** | **CQQSYSTPLTF** | **1372** |
| **004S-H10** | **Fzd6** | **RASQSISTYLS** | **1101** | **GASSLES** | **1210** | **CQQSYSPPFTF** | **1362** |
| **004S-B11** | **Fzd6** | **RASQSISSYLN** | **1098** | **AASSLQS** | **1175** | **CQQSYSTPLTF** | **1372** |
| **004S-D11** | **Fzd6** | **RASQSVSSWLA** | **1120** | **AASTLQT** | **1180** | **CQQSYSTPTF** | **1376** |
| **004S-F11** | **Fzd6** | **RASQSISSYLN** | **1098** | **AASSLQS** | **1175** | **CQQSYSTPLTF** | **1372** |
| **004S-G11** | **Fzd6** | **RASQSVSSYLA** | **1121** | **GASSRAT** | **1213** | **CQQYAISYTF** | **1400** |
| **004S-B12** | **Fzd6** | **RASQSISSYLN** | **1098** | **AASSLQS** | **1175** | **CQQSYSTPLTF** | **1372** |
| **004S-C12** | **Fzd6** | **RASQGISSYLA** | **1076** | **RTSTLES** | **1250** | **CQQSYSTPWTF** | **1378** |
| **004S-F12** | **Fzd7** | **RASQSISSYLN** | **1098** | **AASTLQT** | **1180** | **CQQSYSIPFTF** | **1358** |
| **005S-B01** | **Fzd7** | **RASQSISSYLN** | **1098** | **AASSLQS** | **1175** | **CQQSYSTPLTF** | **1372** |
| **005S-C01** | **Fzd7** | **RASQGISNNLN** | **1072** | **KASSLQS** | **1227** | **CQQSYSLPYTF** | **1360** |
| **005S-F01** | **Fzd7** | **RASQSVSSSYLS** | **1119** | **GASSRAT** | **1213** | **CQQRYKSYTF** | **1338** |
| **005S-B02** | **Fzd8** | **RASQSISSYLN** | **1098** | **AASSLQS** | **1175** | **CQQSYSTPLTF** | **1372** |
| **005S-D02** | **Fzd8** | **RASQSVSSNLA** | **1116** | **NTSNRAT** | **1241** | **CQHYNNWPFTF** | **1308** |
| **005S-G02** | **Fzd8** | **RASQSVSTNLA** | **1122** | **DASNRAT** | **1188** | **CQQRSNWPPQITF** | **1336** |
| **005S-H02** | **Fzd8** | **QASQDISHYLN** | **1044** | **AASSLQS** | **1175** | **CQQSYSTPLTF** | **1372** |
| **005S-B03** | **Fzd8** | **RASQSINSNLA** | **1091** | **GASSRAT** | **1213** | **CQQYGSSPYTF** | **1413** |
| **005S-C03** | **Fzd8** | **RASQSVSSSYLS** | **1119** | **DTSNRAT** | **1194** | **CQQYGSSPITF** | **1408** |
| **005S-E03** | **Fzd8** | **RTSQSISSYLN** | **1149** | **AASTSQS** | **1181** | **CQQSFSSWTF** | **1343** |
| **005S-F03** | **Fzd8** | **RASQSISSYLN** | **1098** | **AASSLQS** | **1175** | **CQQSYSTPLTF** | **1372** |
| **005S-B04** | **Fzd8** | **QASHDINIALN** | **1039** | **AASSLQS** | **1175** | **CQQSYSSPLTF** | **1365** |
| **005S-C04** | **Fzd8** | **RASQSISSYLN** | **1098** | **AASSLQS** | **1175** | **CQQSYSTPLTF** | **1372** |
| **005S-D04** | **Fzd8** | **RASQSISSYLN** | **1098** | **AASSLQS** | **1175** | **CQQSYSTPLTF** | **1372** |
| **005S-G04** | **Fzd8** | **RASQSISSYLN** | **1098** | **AASSLQS** | **1175** | **CQQSYSTPLTF** | **1372** |
| **005S-A05** | **Fzd8** | **RASQSISSYLN** | **1098** | **AASSLQS** | **1175** | **CQQSYSTPLTF** | **1372** |
| **005S-C05** | **Fzd8** | **RASQGISNNLN** | **1072** | **RASSLQS** | **1247** | **CQQANSYPLTF** | **1320** |
| **005S-E05** | **Fzd8** | **RASQSISSYLN** | **1098** | **AASSLQS** | **1175** | **CQQSYSTPLTF** | **1372** |
| **005S-C06** | **Fzd8** | **RASQSVSSSYLS** | **1119** | **AASRRAT** | **1172** | **CQQYSNWPFTF** | **1424** |
| **005S-E06** | **Fzd8** | **QASQDISNRLN** | **1045** | **SASRLQI** | **1253** | **CQQSYRTPRTF** | **1354** |
| **005S-G06** | **Fzd8** | **RASQSISSYLN** | **1098** | **AASSLQS** | **1175** | **CQQSYSTPLTF** | **1372** |
| **005S-H06** | **Fzd8** | **RASQGISNNLN** | **1072** | **DASTLQT** | **1193** | **CQQSFSAPITF** | **1342** |
| **005S-A08** | **Fzd9** | **RASQSISSKSLA** | **1097** | **GASTRAT** | **1217** | **CQQYGIAPTF** | **1407** |
| **005S-C08** | **Fzd9** | **RASQSISSYLN** | **1098** | **AASSLQS** | **1175** | **CQQSYSTPLTF** | **1372** |
| **005S-E08** | **Fzd9** | **RASQSISSYLN** | **1098** | **AASSLQS** | **1175** | **CQQSYSTPLTF** | **1372** |
| **005S-F08** | **Fzd9** | **QASQGISNYLN** | **1050** | **AASSLQS** | **1175** | **CQQTYSTPTTF** | **1395** |
| **005S-H08** | **Fzd9** | **RASQSISSYLN** | **1098** | **AASSLQS** | **1175** | **CQQSYSTPLTF** | **1372** |
| **005S-A09** | **Fzd9** | **RASQSIGSNLD** | **1085** | **RASTLQS** | **1249** | **CQQSYSTPSF** | **1375** |
| **005S-F09** | **Fzd9** | **RASQSISSYLN** | **1098** | **AASSLQS** | **1175** | **CQQSYSTPLTF** | **1372** |
| **005S-B10** | **Fzd9** | **RASQGISNNLN** | **1072** | **AASSLQS** | **1175** | **CQQGNNFPLTF** | **1327** |
| **005S-C10** | **Fzd9** | **RASQDIGSFLA** | **1061** | **AASSLQS** | **1175** | **CQKYNRAPFTF** | **1310** |
| **005S-E10** | **Fzd9** | **RASQSISSYLN** | **1098** | **AASSLQS** | **1175** | **CQQSYSTPLTF** | **1372** |
| **005S-F10** | **Fzd9** | **RASQSISSYLN** | **1098** | **AASSLQS** | **1175** | **CQQSYSTPLTF** | **1372** |
| **005S-G10** | **Fzd9** | **RASQGISNNLN** | **1072** | **QASSLDS** | **1244** | **CQQSYNVPYTF** | **1352** |
| **005S-A11** | **Fzd9** | **RASQSISNNLN** | **1092** | **DASTLKR** | **1192** | **CQQSYNTPRTF** | **1350** |
| **005S-B12** | **Fzd10** | **RASQSISSYLN** | **1098** | **AASSLQS** | **1175** | **CQQSYSTPLTF** | **1372** |
| **005S-D12** | **Fzd10** | **RASQSVSTSYLA** | **1123** | **GASTRAT** | **1217** | **CQQYGASPWTF** | **1406** |
| **006S-B01** | **Fzd10** | **RASQSISSYLN** | **1098** | **AASSLQS** | **1175** | **CQQSYSTPLTF** | **1372** |
| **006S-C01** | **Fzd10** | **RASQGISSALA** | **1076** | **SASNLQS** | **1252** | **CQQAISFPLTF** | **1315** |
| **006S-E01** | **Fzd10** | **RASQSVTSSLA** | **1124** | **GASTRAT** | **1217** | **CQQYNDWPPTF** | **1416** |
| **006S-G01** | **Fzd10** | KSSQSVLYSSNNK NYLA | **1037** | **STNTRSS** | **1257** | **CQHRNFF** | **1306** |
| **006S-B02** | **Fzd10** | **RASQSISSYLN** | **1098** | **AASSLQS** | **1175** | **CQQSYSTPLTF** | **1372** |
| **006S-G02** | **Fzd10** | **RASQSISSYLN** | **1098** | **AASSLQS** | **1175** | **CQQSYSTPLTF** | **1372** |
| **014S-A01** | **Fzd1** | **RASQSISSYLN** | **1098** | **AASSLQS** | **1175** | **CQQSYSTPLTF** | **1372** |
| **014S-C01** | **Fzd2** | RSSQSLLHSDGKT YLY | **1134** | **LGSNRAS** | **1237** | **CMQNTHWPLTR** | **1293** |
| **014S-F01** | **Fzd4** | **RASQSISSYLN** | **1098** | **AASSLQS** | **1175** | **CQQSYSTPLTF** | **1372** |
| **014S-H01** | **Fzd4** | **RASQGISNNLN** | **1072** | **AASRLQS** | **1171** | **CQQSYSPPLTF** | **1363** |
| **014S-C03** | **Fzd7** | **RASQSISSYLN** | **1098** | **AASSLQS** | **1175** | **CQQSYSTPLTF** | **1372** |
| **014S-D03** | **Fzd7** | **RASQSISSYLN** | **1098** | **AASSLQS** | **1175** | **CQQSYSTPLTF** | **1372** |
| **014S-F03** | **Fzd8** | **RASQSISSYLN** | **1098** | **AASSLQS** | **1175** | **CQQSYSTPLTF** | **1372** |
| **014S-A04** | **Fzd8** | **RASQSISSYLN** | **1098** | **AASSLQS** | **1175** | **CQQSYSTPLTF** | **1372** |
| **014S-C04** | **Fzd8** | **RASQSISSYLN** | **1098** | **AASSLQS** | **1175** | **CQQSYSTPLTF** | **1372** |
| **014S-D04** | **Fzd8** | **RASQSISSYLN** | **1098** | **AASSLQS** | **1175** | **CQQSYSTPLTF** | **1372** |
| **014S-E05** | **Fzd8** | **RASQSISSYLN** | **1098** | **AASSLQS** | **1175** | **CQQSYSTPLTF** | **1372** |
| **014S-E06** | **Fzd10** | **RASQSISRYLN** | **1095** | **AASSLQS** | **1175** | **CLQHHSYPFTF** | **1275** |
| **027S-C02** | **Fzd5** | **QASQDISNYLN** | **1046** | **AASSLHT** | **1173** | **CQESYSSPYTF** | **1302** |
| **027S-E03** | **Fzd8** | **RASQSISSYLN** | **1098** | **AASSLQS** | **1175** | **CQQSYSTPYTF** | **1379** |
| **027S-F03** | **Fzd8** | **RASHDIGTFLA** | **1057** | **AASTLQS** | **1179** | **CQQSYRTPYTF** | **1355** |
| **027S-G03** | **Fzd8** | **QATQNIKKYLN** | **1051** | **KASTLES** | **1229** | **CQQSYSTPLTF** | **1372** |
| **027S-H03** | **Fzd8** | RSSQSLLHSNGYN YLD | **1138** | **LGSNRAS** | **1237** | **CMQALQTPQTF** | **1282** |
| **027S-A04** | **Fzd8** | **RASQSISRSLA** | **1093** | **AASNLQS** | **1169** | **CQQAYSFPQTF** | **1323** |
| **027S-B04** | **Fzd8** | **RASQAISNYLN** | **1059** | **AASSLQS** | **1175** | **CQQTFSPPLTF** | **1383** |
| **027S-C04** | **Fzd8** | **RASQGINNYLA** | **1067** | **QASNLES** | **1243** | **CQQTYSSPLTF** | **1393** |
| **027S-D04** | **Fzd8** | **QASQDIDNYLN** | **1040** | **AASSLQS** | **1175** | **CQQSYSTPVTF** | **1377** |
| **027S-E04** | **Fzd8** | **RASQGIRNDLG** | **1069** | **AASTLQS** | **1179** | **CQQAYSFPWTF** | **1324** |
| **027S-C05** | **Fzd8** | **RASQGIRNDLA** | **1068** | **AASSLQR** | **1174** | **CQQSYSKPTF** | **1359** |
| **027S-D05** | **Fzd8** | **QASQDISNYLN** | **1046** | **ASSTLQT** | **1182** | **CQQSYSAPYTF** | **1356** |
| **027S-E05** | **Fzd8** | **RASQGITKSLA** | **1077** | **AASNLQL** | **1168** | **CQQYNTFPITF** | **1422** |
| **027S-F05** | **Fzd8** | **RASQSISTYLA** | **1100** | **GASTRAT** | **1217** | **CQQYGSSPTF** | **1411** |
| **027S-G05** | **Fzd8** | **RASQSISSYLN** | **1098** | **YASSLQN** | **1261** | **CQQSYSTPFTF** | **1368** |
| **027S-H05** | **Fzd8** | **RASQSIGTYLN** | **1088** | **DASNLET** | **1185** | **CQQANSFPLTF** | **1317** |
| **027S-A06** | **Fzd8** | **RASQGISNNLN** | **1072** | **KASSLES** | **1226** | **CQQANSFPITF** | **1316** |
| **027S-C06** | **Fzd8** | **RASQGVGDYLA** | **1078** | **DASNLQS** | **1186** | **CQQHNAYPLTF** | **1330** |
| **027S-D06** | **Fzd8** | **RASQDISSWLA** | **1064** | **KASTLES** | **1229** | **CQQSYGAPLTF** | **1348** |
| **027S-E06** | **Fzd8** | **RASQNVNDWLA** | **1082** | **SASNLQS** | **1252** | **CQQSYSTPFTF** | **1368** |
| **027S-F06** | **Fzd8** | **RASQSISSYLN** | **1098** | **GASNLQS** | **1207** | **CQQSYSTPLTF** | **1372** |
| **027S-G06** | **Fzd5** | **RASQSVNNTYVA** | **1111** | **GTSTRAT** | **1222** | **CQQYDTSPPTF** | **1404** |
| **027S-H06** | **Fzd5** | **RASQSISSYLN** | **1098** | **AASSLQS** | **1175** | **CQQSYTTPFTF** | **1382** |
| **027S-A07** | **Fzd5** | **RASQSISTNVN** | **1099** | **AASSLQS** | **1175** | **CQQSYSTPYTF** | **1379** |
| **029S-B01** | **Fzd1** | KSSQSVLHSSNNK NYLA | **1498** | **STNTRSS** | **1257** | **CQQYYSTPFTF** | **1544** |
| **029S-D01** | **Fzd2** | **RASQSLSSWLA** | **1512** | **DASTLQS** | **1520** | **CQQAISFPLTF** | **1315** |
| **029S-C02** | **Fzd2** | **RASQDISNNLN** | **1500** | **GASHLQT** | **1522** | **CQQANSFPVTF** | **1318** |
| **029S-F02** | **Fzd2** | **RASQGISNYLA** | **1074** | **AASRLQT** | **1515** | **CLQYNTYPWTF** | **1528** |
| **029S-H02** | **Fzd2** | RSSQSLLHSNGYN YLD | **1138** | **LGSSRAS** | **1525** | **CMQALQTPLTF** | **1281** |
| **030S-A02** | **Fzd7** | **RASQSISSYLN** | **1098** | **KASTLHN** | **1523** | **CQQAISFPLTF** | **1315** |
| **030S-B02** | **Fzd3** | **RASQSITTYLN** | **1511** | **KTSSLQS** | **1524** | **CQQGDSFPYTF** | **1531** |
| **029S-E03** | **Fzd3** | **RASQSISSYLN** | **1098** | **AASSLQT** | **1516** | **CQQSFRLPLTF** | **1532** |
| **029S-G03** | **Fzd3** | **RASQSIISYLN** | **1509** | **AASSLQS** | **1175** | **CQQSWRFPYTF** | **1535** |
| **030S-E03** | **Fzd3** | KSSQSVLYSSNNK NYLA | **1037** | **WASTRES** | **1526** | **CQQYYSTPPTF** | **1545** |
| **0295-D05** | **Fzd3** | **RASQTISSYLN** | **1513** | **DASNLET** | **1185** | **CQQSYSIPLTF** | **1536** |
| **030S-H03** | **Fzd3** | **RASQGVSTYLA** | **1505** | **AASSLQS** | **1175** | **CQQYYSSPQTF** | **1543** |
| **029S-B06** | **Fzd3** | **RASQSVSSWLA** | **1120** | **AASSLQS** | **1175** | **CQQAFRFPPTF** | **1529** |
| **029S-E06** | **Fzd3** | **RASQNINSWLA** | **1506** | **AASSLQS** | **1175** | **CQQYYSFPLTF** | **1542** |
| **029S-H06** | **Fzd3** | **RASQSISSYLN** | **1098** | **AASSLQS** | **1175** | **CQQSHSTPLTF** | **1533** |
| **029S-G07** | **Fzd3** | **RASQSISKWLA** | **1510** | **GASTLQS** | **1215** | **CQQAYSFPWTF** | **1324** |
| **029S-H08** | **Fzd3** | **RASRTVYNFLA** | **1514** | **DASNLRT** | **1519** | **CQQSYSTPPTF** | **1537** |
| **029S-F09** | **Fzd3** | **RASQSIARYLN** | **1507** | **GASSLQS** | **1211** | **CQQSYNTPWTF** | **1351** |
| **030S-F04** | **Fzd3** | **RASQGIRNDLN** | **1502** | **DASNLGT** | **1518** | **CQQSSRIPPTF** | **1534** |
| **029S-A10** | **Fzd3** | **RASQGISKYLA** | **1503** | **AASSLQS** | **1175** | **CQQSYSTPWTF** | **1538** |
| **029S-B11** | **Fzd3** | **QASQDISNYLN** | **1046** | **GASALRS** | **1521** | **CQQTKSFPLTF** | **1540** |
| **029S-D11** | **Fzd3** | **RASQDISRGLG** | **1501** | **AASTLYR** | **1517** | **CQQAYSFPWTF** | **1324** |
| **030S-H05** | **Fzd7** | **RASQSIGNYLN** | **1508** | **AASSLQS** | **1175** | **CQQANSFPLTF** | **1530** |
| **030S-A06** | **Fzd7** | **RASQAIGRRLA** | **1499** | **AASSLQS** | **1175** | **CQQYDTYWTF** | **1541** |
| **029S-C12** | **Fzd7** | **RASQGISSYLA** | **1076** | **AASTLQS** | **1179** | **CLQYNTYPWTF** | **1528** |
| **030S-C06** | **Fzd7** | **RASQGISSWLA** | **1504** | **DASSLQS** | **1190** | **CQQSYSTPYSF** | **1539** |
| **4A12** | **Fzd5** | **KASQDVGTAVA** | **1497** | **WASTRHT** | **1527** | **QQYSTYPLT** | **1546** |
| **1791** | **Fzd7** | **KASENVLNYVS** | **2199** | **GASNRYT** | **2202** | **GQSYRYP** | **2205** |
| **1291** | **Fzd7** | KSSQSLLYSSNQ KNYLAW | **2200** | **WASTRES** | **2203** | **QQYYSY** | **2206** |
| **18R5** | **Fzd7** | **SGDNIGS FYVH** | **2201** | **DKSNRPSG** | **2204** | **QSYA NTLSL** | **2207** |

**Table 1B: Anti-Fzd Antibody Clone IDs, Heavy Chain (HC) and Light Chain (LC) Seq ID Nos, and Binding Characteristics**

| **Clone ID** | **HC SID NO** | **LC SID NO** | **Confirmed Binding** |
|---|---|---|---|
| 001S-B01 | 1 | 38 | Fzd1,2,7,9 |
| 001S-E02 | 2 | 39 | Fzd1,2,7 |
| 001S-G02 | 3 | 40 | Fzd1,2,7 |
| 001S-H02 | 4 | 41 | Fzd1,2,7 |
| 001S-A03 | 5 | 42 | Fzd1,2,7,9 |
| 001S-B03 | 6 | 43 | Fzd1,2,7 |
| 004S-G06 | 7 | 44 | Fzd5,8 |
| 002S-B01 | 8 | | Fzd1 |
| 002S-C02 | 9 | | Fzd1 |
| 002S-E02 | 10 | | Fzd1 |
| 002S-G02 | 11 | | Fzd1 |
| 002S-F03 | 12 | | Fzd1 |
| 002S-A04 | 13 | | Fzd1 |
| 002S-B04 | 14 | | Fzd1 |
| 002S-D04 | 15 | | Fzd1 |
| 004S-H04 | 16 | 45 | Fzd5 |
| 001S-A04 | 17 | 46 | Fzd1,2,5,7,8 |
| 003S-E07 | 18 | 47 | Fzd2 |
| 003S-D10 | 19 | 48 | Fzd4 |
| 004S-B08 | 20 | 49 | Fzd6 |
| 004S-D08 | 21 | 50 | Fzd6 |
| 004S-C09 | 22 | 51 | Fzd6 |
| 004S-F10 | 23 | 52 | Fzd6 |
| 004S-A11 | 24 | 53 | Fzd6 |
| 004S-A12 | 25 | 54 | Fzd6 |
| 005S-B07 | 26 | 55 | Fzd9 |
| 005S-D08 | 27 | 56 | Fzd9 |
| 005S-E09 | 28 | 57 | Fzd9 |
| 005S-H10 | 29 | 58 | Fzd9 |
| 005S-B11 | 30 | 59 | Fzd9 |
| 005S-D11 | 31 | 60 | Fzd9 |
| 014S-G02 | 32 | 61 | Fzd6 |
| 014S-B04 | 33 | 62 | Fzd8 |
| 014S-B06 | 34 | 63 | Fzd9 |
| 014S-G06 | 35 | 64 | Fzd10 |
| 014S-A07 | 36 | 65 | Fzd10 |
| 017S-B09 | 37 | | Fzd8 |
| 004S-D01 | 66 | 67 | Fzd4 |
| 004S-E09 | 68 | 69 | Fzd6 |
| anti-FZD7-1791 | 70 | 71 | Fzd7 |
| anti-FZD7-1291 | 72 | 73 | Fzd7 |
| 004S-B10 | | | Fzd6 |
| 004S-C10 | | | Fzd6 |
| 004S-F10 | | | Fzd6 |
| 004S-G10 | | | Fzd6 |
| 004S-A11 | | | Fzd6 |
| 004S-B11 | | | n.b. |
| 004S-D11 | | | Fzd6 |
| 004S-E11 | | | n.b. |
| 004S-F11 | | | Fzd6 |
| 004S-G11 | | | Fzd6 |
| 004S-A12 | | | Fzd6 |
| 004S-B12 | | | Fzd6 |
| 004S-C12 | | | n.b. |
| 004S-D12 | | | n.b. |
| 004S-F12 | | | n.b. |
| 004S-F12 | | | n.b. |
| 004S-G12 | | | n.b. |
| 005S-B02 | | | n.b. |
| 005S-C02 | | | n.b. |
| 005S-D02 | | | Fzd5,8 |
| 005S-E02 | | | Fzd5,8 |
| 005S-H02 | | | Fzd5,8 |
| 005S-A03 | | | Fzd5,8 |
| 005S-C03 | | | n.s. |
| 005S-E03 | | | n.s. |
| 005S-F03 | | | Fzd8 |
| 005S-B04 | | | Fzd5,8 |
| 005S-F04 | | | n.b. |
| 005S-G04 | | | Fzd5,8 |
| 005S-H04 | | | n.b. |
| 005S-E05 | | | n.b. |
| 005S-G05 | | | Fzd5,8 |
| 005S-H05 | | | Fzd5,8 |
| 005S-D06 | | | Fzd8 |
| 005S-F06 | | | n.b. |
| 005S-G06 | | | n.b. |
| 005S-A07 | | | Fzd9,10 |
| 005S-B07 | | | Fzd9 |
| 005S-A08 | | | Fzd9 |
| 005S-B08 | | | Fzd9 |
| 005S-D08 | | | Fzd9 |
| 005S-E08 | | | Fzd9 |
| 005S-F08 | | | n.b. |
| 005S-C09 | | | Fzd9 |
| 005S-D09 | | | Fzd9 |
| 005S-E09 | | | Fzd9 |
| 005S-F09 | | | Fzd9 |
| 005S-A10 | | | Fzd9 |
| 005S-B10 | | | Fzd9 |
| 005S-E10 | | | Fzd9 |
| 005S-H10 | | | Fzd9 |
| 005S-B11 | | | Fzd9 |
| 005S-D11 | | | Fzd9 |
| 005S-G11 | | | n.b. |
| 005S-H11 | | | n.b. |
| 005S-E12 | | | Fzd10 |
| 006S-A01 | | | Fzd10 |
| 006S-H01 | | | n.b. |
| 006S-A02 | | | Fzd10 |
| 006S-D02 | | | n.b. |
| 006S-H02 | | | Fzd10 |
| 006S-A03 | | | n.b. |
| 006S-B03 | | | n.b. |
| 006S-C03 | | | n.b. |
| 014S-A01 | | | Fzd1,2,7 |
| 014S-B02 | | | n.b. |
| 014S-G02 | | | Fzd6 |
| 014S-B03 | | | n.b. |
| 014S-C03 | | | Fzd1,2,7 |
| 014S-A04 | | | n.b. |
| 014S-B04 | | | Fzd8 |
| 014S-B05 | | | Fzd5,8 |
| 014S-B06 | | | Fzd9 |
| 014S-F06 | | | n.s. |
| 014S-G06 | | | Fzd10 |
| 014S-A07 | | | Fzd10 |
| 017S-E08 | | | Fzd8 |
| 017S-H08 | | | n.b. |
| 017S-A09 | | | n.b. |
| 017S-B09 | | | Fzd8 |
| 018S-F06 | | | Fzd4 |
| 018S-H06 | | | n.b. |
| 018S-B07 | | | n.b. |
| 017S-A10 | | | n.b. |
| 017S-B10 | | | n.b. |
| 017S-D10 | | | n.b. |
| 018S-H08 | | | n.b. |
| 018S-B09 | | | Fzd5,8 |
| 021S-A01 | | | n.b. |
| 021S-E02 | | | Fzd5,8 |
| 021S-G02 | | | n.s. |
| 021S-A03 | | | n.b. |
| 029S-B01 | | | n.b. |
| 029S-D01 | | | Fzd1,2,7 |
| 029S-C02 | | | Fzd1,2,7 |
| 029S-H02 | | | Fzd1 |
| 030S-A02 | | | Fzd7 |
| 029S-E06 | | | Fzd2, 6, 3 |
| 030S-F04 | | | Fzd3 |
| 030S-H05 | | | Fzd7 |
| 030S-A06 | | | Fzd1,2,7,5 |
| 029S-C12 | | | Fzd7 |
| 030S-C06 | | | Fzd1 |
| 001S-A01 | | | Fzd1,2,7 |
| 001S-H01 | | | Fzd1,2,7 |

In certain embodiments, the Fzd binding region may be selected from any binding domain that binds an Fzd receptor epitope with an affinity of, e.g., a K_{D} of at least about 1 x 10⁻⁴ M, at least about 1 x 10⁻⁵ M, at least about 1 x 10⁻⁶ M, at least about 1 x 10⁻⁷ M, at least about 1 x 10⁻⁸ M, at least about 1 x 10⁻⁹ M, at least about 1 x 10⁻¹⁰ M, at least about 1 x 10⁻¹¹ M, at least about 1 x 10⁻¹² M, at least about 1 x 10⁻¹³ M, at least about 1 x 10⁻¹⁴ M, or at least about 1 x 10⁻¹⁵ M. In certain embodiment, the Fzd binding region may be selected from any binding domain that binds one or more Fzd receptor epitopes at high affinity, e.g., a K_{D} of less than about 1 x 10⁻⁷ M, less than about 1 x 10⁻⁸ M, less than about 1 x 10⁻⁹ M, less than about 1 x 10⁻¹⁰ M, less than about 1 x 10⁻¹¹ M, less than about 1 x 10⁻¹² M, less than about 1 x 10⁻¹³ M, less than about 1 x 10⁻¹⁴ M, or less than about 1 x 10⁻¹⁵ M. In certain embodiment, the Fzd binding region may be selected from any binding domain that binds an Fzd receptor epitope at high affinity, e.g. a K_{D} of less than or equal to about 1 x 10⁻⁴ M, less than or equal to about 1 x 10⁻⁵ M, less than or equal to about 1 x 10⁻⁶ M, less than or equal to about 1 x 10⁻⁷ M, less than or equal to about 1 x 10⁻⁸ M, less than or equal to about 1 x 10⁻⁹ M, less than or equal to about 1 x 10⁻¹⁰ M, less than or equal to about 1 x 10⁻¹¹ M, less than or equal to about 1 x 10⁻¹² M, less than or equal to about 1 x 10⁻¹³ M, less than or equal to about 1 x 10⁻¹⁴ M, or less than or equal to about 1 x 10⁻¹⁵ M in the context of a Wnt surrogate molecule.

Suitable Fzd binding regions include, without limitation, de novo designed Fzd binding proteins, antibody derived binding proteins, e.g. scFv, Fab, etc. and other portions of antibodies that specifically bind to one or more Fzd proteins, VHH or sdAb derived binding domains, knottin-based engineered scaffolds, norrin and engineered binding fragments derived therefrom, naturally occurring Fzd binding domains, and the like. An Fzd binding domain may be affinity selected to enhance binding to a desired Fzd protein or plurality of Fzd proteins, e.g. to provide tissue selectivity.

In some embodiments, the Fzd binding region binds to one, two, three, four, five or more different frizzled proteins, e.g., one or more of human frizzled proteins Fzd1, Fzd2, Fzd3, Fzd4, Fzd5, Fzd6, Fzd7, Fzd8, Fzd9, and Fzd10. In some embodiments, the Fzd binding region binds to Fzd1, Fzd2, and Fzd 7. In some embodiments, the Fzd binding region binds to Fzd1, Fzd2, Fzd5, Fzd7, and Fzd8. In other embodiments the Fzd binding region is selective for one or more frizzled protein of interest, e.g. having a specificity for the one or more desired frizzled protein of at least 10-fold, 25-fold, 50-fold, 100-fold, 200-fold or more relative to other frizzled proteins.

In certain embodiments, the Fzd binding region comprises the six CDR regions of the pan specific frizzled antibody OMP-18R5 (vantictumab). In certain embodiments, the Fzd binding region is an scFv comprising the six CDR regions of the pan-specific frizzled antibody OMP-18R5 (vantictumab). See, for example, U.S. Patent No. 8,507,442, herein specifically incorporated by reference. For example, the CDR sequences of OMP-18R5 include (i) a heavy chain CDR1 comprising GFTFSHYTLS (SEQ ID NO:270), a heavy chain CDR2 comprising VISGDGSYTYYADSVKG (SEQ ID NO:677), and a heavy chain CDR3 comprising NFIKYVFAN (SEQ ID NO:1033), and (ii) a light chain CDR1 comprising SGDKLGKKYAS (SEQ ID NO:1152) or SGDNIGSFYVH (SEQ ID NO:1153), a light chain CDR2 comprising EKDNRPSG (SEQ ID NO:1200) or DKSNRPSG (SEQ ID NO:1201), and a light chain CDR3 comprising SSFAGNSLE (SEQ ID NO:1435) or QSYANTLSL (SEQ ID NO:1436). In particular embodiments, the Fzd binding region is an antibody or derivative thereof, including without limitation scFv, minibodies, VHH or sdAbs and various antibody mimetics comprising any of these CDR sequences. In certain embodiments, these CDR sequences comprise one or more amino acid modifications.

In certain embodiments, the Fzd binding region comprises the six CDR regions of anti-FZD7-1791 or anti-FZD7-1291. Anti-FZD7-1791 and anti-FZD7-1291 are antibodies that bind to different epitopes within the hinge region of Fzd7, as described in PCT Patent Publication Nos. WO2016/205551 and WO2016/205566, herein specifically incorporated by reference. In certain embodiments, the Fzd binding region is an scFv comprising the six CDR regions of anti-FZD7-1791 or anti-FZD7-1291. For example, the CDR sequences of anti-FZD7-1791 include (i) a heavy chain CDR1 comprising TYAMH (SEQ ID NO:2190), a heavy chain CDR2 comprising RIRSKSNNYAKNYDDSVKD (SEQ ID NO:2193), and a heavy chain CDR3 comprising ENYGGRFDY (SEQ ID NO:2196), and (ii) a light chain CDR1 comprising KASENVLNYVS (SEQ ID NO:2199), a light chain CDR2 comprising GASNRYT (SEQ ID NO:2202), and a light chain CDR3 comprising GQSYRYP (SEQ ID NO:2205). The heavy chain sequence of anti-FZD7-1791 includes SEQ ID NO:70 and the light chain sequence of anti-FZD7-1791 includes SEQ ID NO:71. As another example, the CDR sequences of anti-FZD7-1291 include (i) a heavy chain CDR1 comprising SYAMS (SEQ ID NO:2191), a heavy chain CDR2 comprising TISDGGSYTRYPDKLKG (SEQ ID NO:2194), and a heavy chain CDR3 comprising VGGRRDYFDY (SEQ ID NO:2197), and (ii) a light chain CDR1 comprising KSSQSLLYSSNQKNYLAW (SEQ ID NO:2200), a light chain CDR2 comprising WASTRES (SEQ ID NO:2203), and a light chain CDR3 comprising QQYYSYP (SEQ ID NO:2206). The heavy chain sequence of anti-FZD7-1291 includes SEQ ID NO:72 and the light chain sequence of anti-FZD7-1291 includes SEQ ID NO:73. In some embodiments, In particular embodiments, the Fzd binding region is an antibody or derivative thereof, including without limitation scFv, minibodies, VHH or sdAbs and various antibody mimetics comprising any of these CDR sequences. In certain embodiments, these CDR sequences comprise one or more amino acid modifications.

In other embodiments, the Fzd binding region comprises a variable region sequence, or the CDRs thereof, from any of a number of frizzled specific antibodies, which are known in the art and are commercially available, or can be generated de novo. Any of the frizzled polypeptides can be used as an immunogen or in screening assays to develop an antibody. Non-limiting examples of frizzled binding domains include antibodies available from Biolegend, e.g., Clone CH3A4A7 specific for human frizzled 4 (CD344); Clone W3C4E11 specific for human Fzd9 (CD349); antibodies available from Abcam, e.g., ab64636 specific for Fzd7; ab83042 specific for human Fzd4; ab77379 specific for human Fzd7; ab75235 specific for human Fzd8; ab102956 specific for human Fzd9; and the like. Other examples of suitable antibodies are described in, inter alia, U.S. Patent Application No. 20140105917; U.S. Patent Application No. 20130230521; U.S. Patent Application No. 20080267955; U.S. Patent Application No. 20080038272; U.S. Patent Application No. 20030044409; etc., each herein specifically incorporated by reference.

The Fzd binding region of a Wnt surrogate molecule may be an engineered protein that is selected for structural homology to the frizzled binding region of a Wnt protein. Such proteins can be identified by screening a structure database for homologies. The initial protein thus identified, for example the microbial Bh1478 protein. The native protein is then engineered to provide amino acid substitutions that increase affinity, and may further be selected by affinity maturation for increased affinity and selectivity in binding to the desired frizzled protein. Non-limiting examples of frizzled binding moieties include the Fz27 and Fz27-B12 proteins.

In particular embodiments, a Wnt surrogate molecule comprises an LRP5/6 binding region, e.g., an anti-LRP5/6 antibody, or antigen-binding fragment thereof, fused to a polypeptide that specifically binds to one or more Fzd receptor epitopes. In particular embodiments, the polypeptide that specifically binds to LRP5/6 is an antibody or antigen-binding fragment thereof. If certain embodiments, it is an antibody or antigen-binding fragment thereof disclosed in the U.S. Provisional Patent Application No. 62/607,879, titled, "Anti-LR5/6 Antibodies and Methods of Use," Attorney Docket No. SRZN-005/00US, filed on December 19, 2017, which is incorporated herein by reference in its entirety.

In particular embodiments, at least one LRP5/6 binding region of a Wnt surrogate molecule includes one or more antigen-binding fragments of an antibody. For example, the one or more antigen-binding fragments may be or be derived from an IgG, scFv, Fab, or VHH or sdAb. In certain embodiments, the one or more antigen-binding fragments are humanized.

In particular embodiments, the LRP5/6 binding region comprises the three heavy chain CDRs and/or the three light chain CDRs disclosed for any of the illustrative antibodies or fragments thereof that bind to LRP5 and/or LRP6 provided in Table 2A. In particular embodiments, the LRP5/6 binding region comprises the three heavy chain CDRs and/or the three light chain CDRs disclosed for any of the illustrative antibodies or fragments thereof that bind to LRP5 and/or LRP6 provided in Table 2A, wherein the CDRs collectively comprise one, two, three, four, five, six, seven, or eight amino acid modifications, e.g., substitutions, deletions, or additions. In certain embodiments, the LRP5/6 binding region is a VHH or sdAb or was derived from a VHH or sdAb, so Table 2A only includes the three heavy chain CDRs. In certain embodiments, the LRP5/6 binding region comprises the three heavy chain CDRs shown in Table 2A or variants wherein the CDRs collectively comprise one, two, three, four, five, six, seven or eight amino acid modifications. In particular embodiments, the LRP5/6 binding region comprises the heavy chain fragment and/or light chain fragment of any of the illustrative antibodies or fragments thereof that bind to LRP5 and/or LRP6 provided in Table 2B or SEQ ID NOs:74-97 (or an antigen-binding fragment or variant of either). In certain embodiments, the LRP5/6 binding region is a Fab or was derived from a Fab, so Table 2B includes VH and CH1 sequence, but not CH2 or CH3 sequences. In certain embodiments, the LRP5/6 binding region is a VHH or sdAb or was derived from a VHH or sdAb, so Table 2B includes the VHH domain. In certain embodiments, the LRP5/6 binding region is a polypeptide, e.g., an antibody or antigen-binding fragment thereof, that competes with one of these antibodies for binding to LRP5 and/or LRP6.

In particular embodiments, the LRP5/6 binding region includes an amino acid sequence having at least 90%, at least 95%, at least 98%, or at least 99% identity to any of the sequences set forth in Table 2A, Table 2B, or SEQ ID NOs:74-97, or an antigen-binding fragment thereof. Binding characteristics for clones listed in Table 2B were determined and are shown in Table 2B.

**Table 2A: Anti-LRP5/6 Antibody Clone IDs and CDR sequences.**

| **Clone ID** | **Confirmed Binding** | **CDRH1** | **SID NO.** | **CDRH2** | **SID NO.** | **CDRH3** | **SID NO.** |
|---|---|---|---|---|---|---|---|
| 0015-C08 | LRP6e1e2 | YTISNYYIH | 1682 | GMINPSGGSTTYA | 1762 | CAIVRGKKWYFDLW | 1842 |
| 001S-C10 | LRP6e1e2 | RTFGTYPNG | 1632 | AAISWGGRTAYA | 1700 | CYARTVIGGFGAFRAHW | 2061 |
| 001S- D10 | LRP6e1e2 | RTFSRYAMA | 1642 | AAIRWSGGGTYYA | 1689 | CAASMEAMNSLRVNKERYYQ SW | 1836 |
| 001S-E10 | LRP6e1e2 | LTFSNAAMA | 1614 | AAISRSGANTAYS | 1696 | CTLVNEIKTWW | 2039 |
| 001S-F10 | LRP6e1e2 | RTFSSYAMA | 1645 | AAIKWSGTNTYYA | 1684 | CAASMEAMNSLRVNKERYYQ SW | 1834 |
| 001S- G 10 | LRP6e1e2 | RTFSRYVMG | 1644 | AAITWRGGSTYYA | 1706 | CATGPNSIY | 1987 |
| 001S-A11 | LRP6e1e2 | RTFGNYDMG | 1630 | AGIRWSGSTLYA | 1709 | CYARTVIGGFGAFRAHW | 2062 |
| 001S-B11 | LRP6e1e2 | RRFTTYGMG | 1623 | AAVTWRSGSTYYA | 1708 | CAAGSTVVAEFNYW | 1828 |
| 001S-C11 | LRP6e1e2 | SISSFNTMG | 1659 | AVITTGGDTSYS | 1741 | CNKVNAITKL | 2025 |
| 001S-E11 | LRP6e1e2 | RTLSRYSMG | 1651 | AAISRSGDRIYYS | 1697 | CTLVNEIKTWW | 2040 |
| 001S-F11 | LRP6e1e2 | RTFSSYAMS | 1646 | AVIGRSGGIKYYA | 1736 | CATRRPFNSYNTEQSYDSW | 1989 |
| 001S-G11 | LRP6e1e2 | SIFRLGTMY | 1655 | ASIGKSGSTNYA | 1719 | CKQHPNGYR | 2005 |
| 001S-H11 | LRP6e1e2 | RTLSSFAMG | 1652 | ATISRSGGNTYYA | 1732 | CNLREWNNSGAGYW | 2026 |
| 001S-A12 | LRP6e1e2 | IAFRYYDMG | 1608 | AAITWNGRSSDYA | 1704 | CAAVFTGRFYGRPPREKYDYW | 1838 |
| 001S-B12 | LRP6e1e2 | RLLSYYALA | 1622 | AAISRNGDKSHYS | 1694 | CTLVNEIKTWW | 2038 |
| 001S-C12 | LRP6e1e2 | RTFSNYAVG | 1641 | AAISRFGGSTYYV | 1693 | CAADRIENYLGRYYDPSEYEYW | 1824 |
| 001S-D12 | LRP6e1e2 | RTFSRYAMG | 1643 | GAISRSGNNTYYA | 1744 | CTLVNEIKTWW | 2041 |
| 001S-F12 | LRP6e1e2 | RTFRSYTMG | 1637 | AAISGSGGSTTYA | 1690 | CNADIKTTTYSPLRNYW | 2009 |
| 008S-B01 | LRP5 | TIFSINTMG | 1664 | ATMTSGGNTNYA | 1734 | CYRRQWASSWGARNYEYW | 2064 |
| 008S-C01 | LRP5 | NINSIETLG | 1617 | ANMRGGGYMKYA | 1716 | CHGRDYGSNAPQYW | 2001 |
| 008S-D01 | LRP5 | NINSIETLG | 1617 | ANMRGGGYMKYA | 1716 | CYVKLRDDDYVYR | 2065 |
| 0085-E01 | LRP5 | NINSIETLG | 1617 | ANMRGGGYMKYA | 1716 | CNAVTYNGYTIR | 2023 |
| 0085-G01 | LRP5 | NINSIETLG | 1617 | ANMRGGGYMKYA | 1716 | CYARTQRMGVVNSYW | 2060 |
| 008S-A02 | LRP5 | NINSIETLG | 1617 | ANMRGGGYMKYA | 1716 | CNAVTFGGNTIR | 2021 |
| 008S-C02 | LRP5 | NINSIETLG | 1617 | ANMRGGGYMKYA | 1716 | CNAVTYDGY | 2022 |
| 008S-D02 | LRP5 | NINSIETLG | 1617 | ANMRGGGYMKYA | 1716 | CAAQFRNDYGLRYQSTNNYW | 1832 |
| 008S-E02 | LRP5 | NINSIETLG | 1617 | ANMRGGGYMKYA | 1716 | CNANYRGNRYW | 2019 |
| 0095-C01 | LRP6e3e4 | GSFSGYYWT | 1595 | GEINHSGATNYN | 1745 | CVRYAWPEFDHW | 2053 |
| 009S-B02 | LRP6e3e4 | GSLSGYYWS | 1596 | GEINHSGSTNYN | 1746 | CVRYAWPEFDHW | 2055 |
| 009S-C02 | LRP6e3e4 | GSFSDYYWS | 1594 | GEINHSGSTNYN | 1746 | CVRYAWPEFDHW | 2054 |
| 009S-D02 | LRP6e3e4 | GTFSSYAIS | 1603 | GGIIPIFGTANYA | 1749 | CVYGRDFDYW | 2056 |
| 010S-A02 | LRP6e1e2 | HTFSSYAMG | 1607 | AAISQSGYVRYYA | 1691 | CKIYGLNGQPLGSW | 2003 |
| 010S-B02 | LRP6e1e2 | RTFNSGTMG | 1634 | AAITWRGGITYYA | 1705 | CNADGYSWDGRSGRRLELW | 2008 |
| 010S-D02 | LRP6e1e2 | RTFSSYAVG | 1647 | AAISYSGGSTKYA | 1702 | CAASVYISRRDSDYGYW | 1837 |
| 010S-E02 | LRP6e1e2 | LSSGRPFSSYVM G | 1612 | AAISWSGGSTKYA | 1701 | CKLQVRPIGYSSAYSRNYW | 2004 |
| 010S-F02 | LRP6e1e2 | RSFNSYVIG | 1625 | AAIRWSGDNTYYA | 1688 | CAASMEAMNSLRVNKERYYQ SW | 1835 |
| 009S-E02 | LRP6e1e2 | RRFTTYGMG | 1623 | AAVTWRSGSTYYA | 1708 | CAAGSTVVAEFNYW | 1829 |
| 009S-F02 | LRP6e1e2 | RTFSYYAMG | 1649 | AAISRSGGIYYA | 1698 | CNTVRPLWAW | 2029 |
| 009S-G02 | LRP6e1e2 | SIFSIYAMG | 1658 | AVITSGGKTVYA | 1740 | CYADSRSSWYDEYLEHW | 2058 |
| 009S-H02 | LRP6e1e2 | SIVRSLPMA | 1660 | ATINDAQRYYA | 1727 | CNTSPYMHDVW | 2027 |
| 009S-A03 | LRP6e1e2 | RTFSVYGVG | 1648 | AAVSASGGYTWYA | 1707 | CKAAPRWGGATAYW | 2002 |
| 010S-G02 | LRP6e1e2 | SIVRSLPMA | 1660 | ATINDAQRYYA | 1727 | CNTSPYMHDVW | 2028 |
| 010S-A03 | LRP6e1e2 | RTFRRYAMG | 1636 | ATISASGGNTAYA | 1731 | CNAPAWLYDDDYW | 2020 |
| 009S-B03 | LRP6e1e2 | RTFSNYAVG | 1641 | AAISRFGGSTYYV | 1693 | CAADRIENYLGRYYDPSEYEYW | 1825 |
| 0105-B03 | LRP6e1e2 | RTFSNYAVG | 1641 | AAISRFGGSTYYA | 1692 | CHAKQLRNGQMYTYW | 1999 |
| 010S-D03 | LRP6e1e2 | ISSVYGMG | 1609 | AAIQWSADNTFYA | 1686 | CAARTSGGLFHYRRSDHWDT W | 1833 |
| 009S-C03 | LRP6e1e2 | LPFSRYAMA | 1610 | AGMSGEGRNTKYR | 1713 | CSSRGYW | 2034 |
| 009S-D03 | LRP6e1e2 | SIFSDGAMG | 1656 | AVISGGRTGYA | 1737 | CNTYPFPIYKKGYPFW | 2030 |
| 009S-E03 | LRP6e1e2 | RRFTTYGMG | 1623 | AAVTWRSGSTYYA | 1708 | CAAGSTVVAEFNYW | 1830 |
| 009S-F03 | LRP6e1e2 | RTFSSYAMS | 1646 | AVIGRSGGIKYYA | 1736 | CATRRPFNSYNTEQSYDSW | 1990 |
| 010S-E03 | LRP6e1e2 | RSVSIYPMG | 1628 | AAINWSGDSTKYA | 1685 | CNAVVVGLSRRIDNIW | 2024 |
| 010S-F03 | LRP6e1e2 | RTFSRYVMG | 1644 | AAITWRGGSTYYA | 1706 | CATGPNSIY | 1988 |
| 009S-G03 | LRP6e1e2 | RSVSSYNMG | 1629 | AAISRRGGIIEYG | 1695 | CHAVENILGRFVDYW | 2000 |
| 009S-H03 | LRP6e1e2 | SIFSINTMG | 1657 | AVITSGGKTVYA | 1740 | CYADSRSSWYDEYLEHW | 2057 |
| 009S-A04 | LRP6e1e2 | RTLSAYDMG | 1650 | GGIRWSGGTTLYP | 1753 | CYARTVIGGFGAFRAHW | 2063 |
| 009S-B04 | LRP6e3e4 | SIFMINTMA | 1654 | ATIRPVVSETTYA | 1728 | CNAKRPWGTRDEYW | 2018 |
| 010S-G03 | LRP6e3e4 | RSFNSYTTT | 1624 | AAIRGSSGSTFYA | 1687 | CNAASTVTAWPYYGPDYW | 2006 |
| 0095-C04 | LRP6e3e4 | FRFSISTMG | 1553 | AYITGGGRTMDG | 1743 | CNAFVRSDFDRYYDYW | 2011 |
| 009S-D04 | LRP6e3e4 | TIVSIYRIN | 1665 | AGITSSGRTIYA | 1712 | CNAASTVTAWPYYGPDYW | 2007 |
| 010S-H03 | LRP6e3e4 | RIFSIYDMG | 1621 | SGIRWSGGTSYA | 1789 | CSSRGYW | 2035 |
| 009S-E04 | LRP6e3e4 | RIFAIYDIA | 1618 | AMIRPVVTEIDYA | 1715 | CNAKRPWGSRDEYW | 2012 |
| 010S-A04 | LRP6e3e4 | SLFSFNAVG | 1662 | ASISSGGRTNYA | 1722 | CSKGGVYGGTYVPDSW | 2032 |
| 009S-F04 | LRP6e3e4 | RSLSSFAMG | 1627 | ARISRGDGYTDEA | 1718 | CAAVQAVIGGTLTTAYDYW | 1839 |
| 010S-B04 | LRP6e3e4 | RVLSYYAMA | 1653 | AGITRGGATTYYS | 1711 | CAAGPNWSTRNREYDYW | 1827 |
| 009S-G04 | LRP6e3e4 | GTFSRYHMG | 1601 | SAITWSGGRTYYA | 1788 | CALTWAPTPTNRRSDYAYW | 1872 |
| 009S-H04 | LRP6e3e4 | RIFAIYDMA | 1619 | ATIRPVVSETTYA | 1728 | CNAKRPWGTRDEYW | 2017 |
| 010S-C04 | LRP6e3e4 | SLFSFNAMG | 1661 | ASISSGSRTNYA | 1723 | CSKGGVYGGTYVPDSW | 2033 |
| 010S-D04 | LRP6e3e4 | RIFAIYDIA | 1618 | ATIRPVVTQIDYA | 1730 | CNAKRPWGSRDEYW | 2015 |
| 010S-E04 | LRP6e3e4 | RTFGSDVMG | 1631 | ALTGWGDGSTTYYE | 1714 | CAAARRSGTYDIGQYLRESAYV FW | 1820 |
| 010S-F04 | LRP6e3e4 | RTFSRYAMG | 1643 | AAITRSGSNTYYA | 1703 | CAADPRGVTLPRATAYEYW | 1823 |
| 0095-A05 | LRP6e3e4 | RTFSDYSMG | 1639 | AGISWIADNRYYA | 1710 | CTAGRSRYLYGSSLNGPYDYW | 2036 |
| 010S-G04 | LRP6e3e4 | VIFALYDIA | 1666 | ATIRPVVTETDYA | 1729 | CNAKRPWGSRDEYW | 2014 |
| 010S-H04 | LRP6e3e4 | RSFSDFFMG | 1626 | ATISWSGSSANYE | 1733 | CAAAYSYSQYGSSYSYW | 1821 |
| 010S-A05 | LRP6e3e4 | LSFSSYAMG | 1611 | AAISRSGVSTYYA | 1699 | CAAKFGVLATTESRHDYW | 1831 |
| 010S-C05 | LRP6e3e4 | RTFNIDDMG | 1633 | ASIRWSGQSPYYA | 1720 | CNAETYSGNTIW | 2010 |
| 010S-D05 | LRP6e3e4 | RTFSDYSMA | 1638 | AGISWIADNRYYA | 1710 | CAGDRSRYLYGDSLRGPYGYW | 1841 |
| 010S-E05 | LRP6e3e4 | SVFTTFAKG | 1663 | ASITASSDRTFYA | 1725 | CAAYSTFNTDVASMKPDYW | 1840 |
| 010S-F05 | LRP6e3e4 | RIFSIYDIA | 1620 | ATIRPVVTETDYA | 1729 | CNAKRPWGSRDEYW | 2013 |
| 013S-G04 | LRP6e3e4 | RIFAIYDIA | 1618 | ATIRPVVSETTYA | 1728 | CNAKRPWGTRDEYW | 2016 |
| 013S-H04 | LRP6e3e4 | RTFSMYDMG | 1640 | ASIRWSSGNTWYA | 1721 | CYANIYYTRRAPEEYW | 2059 |
| 0135-A05 | LRP6e3e4 | RTFNTYAMG | 1635 | ASVSWRYDRTYYT | 1726 | CAADTNWRAGPRVGIDEYAY W | 1826 |
| 0135-B05 | LRP6e3e4 | FAFSTTAMS | 1549 | STINPGGLSKSYA | 1806 | CTKGGIQ | 2037 |
| 013S-C05 | LRP6e3e4 | NIFPIDDMS | 1616 | ATVTSGGRINYA | 1735 | CNVDRTLYGKYKEYW | 2031 |
| 013S-D05 | LRP6e3e4 | RIFSIYDMG | 1621 | SGIRWSGGTSYA | 1789 | CGSRGYW | 1998 |
| 013S-E05 | LRP6e3e4 | YTFTYRYLH | 1681 | GGIIPIFGTADYA | 1748 | CARDWELYGMDVW | 1907 |
| 013S-F05 | LRP6e3e4 | GTFSSYAIS | 1603 | GIINPSGGSTSYA | 1761 | CARAGYYDSSGYYAFDIW | 1882 |
| 0135-G05 | LRP6e3e4 | YTFTYRYLH | 1681 | GGVIPIFGTADYA | 1755 | CASDIVVDDAFDTW | 1969 |
| 0105-G06 | LRP6e3e4 | FSFETYGMS | 1555 | SGISGSGGRTHYA | 1792 | CARDLDYW | 1897 |
| 009S-B05 | LRP6e3e4 | FTFDAYAMH | 1560 | STLSGDANNAYYA | 1811 | CARGGSGWSNYYGMDVW | 1931 |
| 009S-C05 | LRP6e3e4 | YTFTYRYLH | 1681 | GRIIPVLKITNYA | 1768 | CAVVDDAFDIW | 1996 |
| 009S-D05 | LRP6e3e4 | FTLRNHWLS | 1591 | SAISGSGGSTYYA | 1786 | CATRTGYSYGFNFWAFDIW | 1991 |
| 009S-E05 | LRP6e3e4 | YTFTNNFMH | 1676 | GHVDPGDGETIYA | 1756 | CARDWGIAAAGDYYYYGMDV W | 1908 |
| 009S-F05 | LRP6e3e4 | FTFDDYGMS | 1561 | SAIGTGGGTYYA | 1781 | CARLGSYGSPYYYYGMDVW | 1959 |
| 009S-G05 | LRP6e3e4 | FTFSDYYMS | 1568 | SGVSWNGSRTHYA | 1799 | CAKDSGLV | 1852 |
| 009S-C06 | LRP6e3e4 | YTFASYDIH | 1671 | GWMNPNSGNTGY A | 1776 | CARATGSGWYTDLGYW | 1883 |
| 009S-D06 | LRP6e3e4 | FTFSSHSTH | 1573 | STISDTNSGTYYA | 1807 | CAKAQATGWSGYYTFDYW | 1843 |
| 009S-E06 | LRP6e3e4 | FTFTDYGLH | 1587 | AVISYGGSNKYYA | 1739 | CASGYSYGLYYYGMDVW | 1973 |
| 009S-F06 | LRP6e3e4 | YTFTYRYLH | 1681 | GGIIPIFGTANYA | 1749 | CATEAALDAFDIW | 1985 |
| 009S-G06 | LRP6e3e4 | YIFTDYYMH | 1669 | GWINPNSGGTNYA | 1774 | CARDFLGSTGDYW | 1892 |
| 009S-H06 | LRP6e3e4 | FTFSSSAMH | 1574 | SAIGTGGSTYYA | 1783 | CAKGGDYFYYYYGMDVW | 1856 |
| 009S-A07 | LRP6e3e4 | YTFTYRYLH | 1681 | GGIIPIFGTANYA | 1749 | CATAYGSSSLNIDYW | 1980 |
| 009S-B07 | LRP6e3e4 | YTFTGYYMH | 1675 | GWINPNSGGTNYA | 1774 | CVKDGGSFPLAYAFDIW | 2049 |
| 009S-D07 | LRP6e3e4 | FPFRYYGMS | 1551 | ARIGWNGGSIVYA | 1717 | CARDYSDRSGIDYW | 1910 |
| 009S-F07 | LRP6e3e4 | GTFSSYAIS | 1603 | GIINPSGGSTSYA | 1761 | CARAAGNFWSGYYTFDYW | 1876 |
| 009S-G07 | LRP6e3e4 | YTFTYRYLH | 1681 | GGIIPIFGTANYA | 1749 | CARGSYGMDVW | 1947 |
| 009S-H07 | LRP6e3e4 | YTFTGYYMH | 1675 | GWMNPNSGNTGY A | 1776 | CASSVVPAGPAGVYAFDIW | 1975 |
| 0095-A08 | LRP6e3e4 | GTFSSHAIN | 1602 | GWISANNGNTDYA | 1775 | CARDQDYGDYGWYYYGMDV W | 1902 |
| 011S-C01 | LRP6e3e4 | LTFTSHGMS | 1615 | SYVSDSGSSVYYA | 1818 | CARHPGSFGGYSYAWYYYYG MDVW | 1956 |
| 009S-C08 | LRP6e3e4 | FSFNTFGIH | 1556 | AVISYDGSNKYYA | 1738 | CAKSIAAAGTGYYGMDVW | 1868 |
| 009S-D08 | LRP6e3e4 | YTFTSYDIN | 1679 | GGIIPIFGTANYA | 1749 | CARGPYYFDYW | 1939 |
| 011S-F01 | LRP6e3e4 | FSFSDYYMS | 1558 | SGISESGGRTYYA | 1790 | CASAADFDYW | 1966 |
| 009S-E08 | LRP6e3e4 | YGFTGYYIH | 1668 | GWMNPNSGNTGY A | 1776 | CARGYGDYDLW | 1951 |
| 009S-F08 | LRP6e3e4 | DTFANYGFS | 1547 | GXVNAGNGNTTYA | 1777 | CAKGWLDFDYW | 1866 |
| 009S-G08 | LRP6e3e4 | FTFSDFAMT | 1566 | SYISGDSGYTNYA | 1813 | CARLGSYPGPYYYYMDVW | 1961 |
| 009S-H08 | LRP6e3e4 | YTFTDYFMN | 1673 | GIINPSGDSTRFA | 1758 | CARDDGLGGMDVW | 1888 |
| 009S-A09 | LRP6e3e4 | YTFTYRYLH | 1681 | GRIIPILGSTNYA | 1767 | CTTDLWDYW | 2047 |
| 011S-F02 | LRP6e3e4 | FTFSTYGMH | 1584 | SSISVSSGTTHYA | 1804 | CARGGSGSYYYAFDIW | 1929 |
| 011S-G02 | LRP6e3e4 | YTFTSYAMN | 1678 | GGIIPIFGTANYA | 1749 | CARDASGGSTGWYYFDSW | 1886 |
| 011S-A03 | LRP6e3e4 | FTFSSYWMH | 1580 | STISGSGGRTYYA | 1808 | CATSPYGVFTLDYW | 1993 |
| 011S-C03 | LRP6e3e4 | YTFSYRYLH | 1672 | GGIIPIFGTANYA | 1749 | CASTVTTDAFDIW | 1977 |
| 011S-D03 | LRP6e3e4 | FSFDDYGMS | 1554 | SVISSGGTIYYA | 1812 | CARHLSSGYLSYYGMDVW | 1954 |
| 011S-F03 | LRP6e3e4 | FTFSSYAMS | 1577 | SAISGSGGSTYYA | 1786 | CAKGGRDGYKGYFDYW | 1859 |
| 0115-C04 | LRP6e1e2 | GTFNSNAIS | 1598 | GWMNPNSGNTGY A | 1776 | CARDYYGSGSYNYGMDVW | 1912 |
| 0115-D04 | LRP6e1e2 | YTFTSYDIN | 1679 | GIINPSGGSTSYA | 1761 | CAREAYYYYYGMDVW | 1915 |
| 011S-H04 | LRP6e1e2 | YIFTDYYMH | 1669 | GRIIPILGRANYA | 1765 | CARGGYSTLDYW | 1932 |
| 0085-F02 | LRP5 | YTFTNYCMH | 1677 | GIINPSDGSTSHA | 1757 | CAKDMVHLIVALAIDYW | 1851 |
| 010S-C06 | LRP6e1e2 | FTFNSYSMD | 1563 | SSISPRGGSTYYA | 1802 | CAPYYYDKSAKPLRSYFDHW | 1875 |
| 010S-E06 | LRP6e3e4 | LTVSSNYMS | 1615 | SGISWNSGSIGYA | 1796 | CARGSDCSGGSCYYSFDYW | 1944 |
| 010S-F06 | LRP6e3e4 | FTFSSSWMH | 1575 | SAIGTGGGTYYA | 1781 | CAREVAVKDYYYYYMDVW | 1921 |
| 010S-H06 | LRP6e3e4 | YTFTSYDIN | 1679 | GRIIPILGRTNYA | 1766 | CAREERGATGRAFDIW | 1918 |
| 010S-A07 | LRP6e3e4 | FTFSSYAMH | 1576 | ASISSTSGSKYYA | 1724 | CAKTYYDFWSGYYTFDYW | 1870 |
| 010S-B07 | LRP6e3e4 | FTFSDYYMS | 1568 | SMISYNGGRAFYA | 1800 | CARGNPYYFDYW | 1937 |
| 010S-C07 | LRP6e3e4 | FTFSKTDMH | 1569 | STITTDSRGTYYA | 1810 | CAKGGDYYYYYYGMDVW | 1858 |
| 010S-D07 | LRP6e3e4 | YTFTYRYLH | 1681 | GGIIPIFGTANYA | 1749 | CANGLEDAYAFDIW | 1873 |
| 009S-D05 | LRP6e3e4 | FTLRNHWLS | 1591 | SAISGSGGSTYYA | 1786 | CATRTGYSYGFNFWAFDIW | 1992 |
| 009S-E05 | LRP6e3e4 | YTFTNNFMH | 1676 | GHVDPGDGETIYA | 1756 | CARDWGIAAAGDYYYYGMDV W | 1909 |
| 0105-E07 | LRP6e3e4 | YTFTYRYLH | 1681 | GGIIPIFGTANYA | 1749 | CAKDDFSLYGMDVW | 1845 |
| 009S-F05 | LRP6e3e4 | FTFDDYGMS | 1561 | SAIGTGGGTYYA | 1781 | CARLGSYGSPYYYYGMDVW | 1960 |
| 010S-F07 | LRP6e3e4 | YTFTYRYLH | 1681 | GGIIPIFGTANYA | 1749 | CARLDYGETEGNGDW | 1958 |
| 010S-G07 | LRP6e3e4 | FTFSSYAMH | 1576 | STISGSGGSTYYA | 1809 | CARAGYG RYYYG M DVW | 1880 |
| 0095-G05 | LRP6e3e4 | FTFSDYYMS | 1568 | SGVSWNGSRTHYA | 1799 | CAKDSGLV | 1853 |
| 010S-H07 | LRP6e3e4 | YTFTYRYLH | 1681 | GGIIPIFGTANYA | 1749 | CARDDSMGAFDIW | 1890 |
| 010S-A08 | LRP6e3e4 | HTFLTYDIN | 1606 | GRITPRLGIANYA | 1770 | CASYFGVMDVW | 1979 |
| 0095-A07 | LRP6e3e4 | YTFTYRYLH | 1681 | GGIIPIFGTANYA | 1749 | CATAYGSSSLNIDYW | 1981 |
| 009S-B07 | LRP6e3e4 | YTFTGYYMH | 1675 | GWINPNSGGTNYA | 1774 | CVKDGGSFPLAYAFDIW | 2050 |
| 009S-B06 | LRP6e3e4 | YTFTYRYLH | 1681 | GGIIPIFGTANYA | 1749 | CAPALTDAGSFDYW | 1874 |
| 010S-B08 | LRP6e3e4 | YTFTYRYLH | 1681 | GGIIPVFGTADYA | 1751 | CARDREQQILDYW | 1904 |
| 010S-C08 | LRP6e3e4 | FTFSTFGMH | 1582 | STITSSGGSTYYA | 1809 | CARAGIAAAPGSRNYYGMDV W | 1878 |
| 009S-C06 | LRP6e3e4 | YTFASYDIH | 1671 | GWMNPNSGNTGY A | 1776 | CARATGSGWYTDLGYW | 1884 |
| 009S-D06 | LRP6e3e4 | FTFSSHSTH | 1573 | STISDTNSGTYYA | 1807 | CAKAQATGWSGYYTFDYW | 1844 |
| 010S-D08 | LRP6e3e4 | FTFSSSWMH | 1575 | SAIGTGGGTYYA | 1781 | CAKEDYDSSGYYYYYFQHW | 1855 |
| 009S-E06 | LRP6e3e4 | FTFTDYGLH | 1587 | AVISYGGSNKYYA | 1739 | CASGYSYGLYYYGMDVW | 1974 |
| 010S-E08 | LRP6e3e4 | YSFTRTDMH | 1670 | GYISAYTGHTSYA | 1778 | CARDLGGTADYW | 1898 |
| 010S-F08 | LRP6e3e4 | LTFDDHAMH | 1613 | SYISSSGRTIFYA | 1815 | CVRGDSGWGILYYVMDVW | 2052 |
| 009S-F06 | LRP6e3e4 | YTFTYRYLH | 1681 | GGIIPIFGTANYA | 1749 | CATEAALDAFDIW | 1986 |
| 010S-G08 | LRP6e3e4 | YIFTDYYMH | 1669 | GGFDPEDGETIYA | 1747 | CARGGGPNEHDYYFDYW | 1927 |
| 010S-H08 | LRP6e3e4 | FTFZNAWMS | 1590 | SGISGSGGSTYYA | 1793 | CARGRGKKNYYYGMDVW | 1942 |
| 010S-A09 | LRP6e3e4 | FTFSTYYMS | 1586 | SGISWNGGKTHYV | 1794 | CARGGDFDYW | 1925 |
| 0105-B09 | LRP6e3e4 | GTFSSYAIS | 1603 | GWINPNSGDTNYA | 1773 | CARGEQWLVWGFDPW | 1924 |
| 009S-G06 | LRP6e3e4 | YIFTDYYMH | 1669 | GWINPNSGGTNYA | 1774 | CARDFLGSTGDYW | 1893 |
| 010S-C09 | LRP6e3e4 | YTFTYRYLH | 1681 | GGIIPIFGTANYA | 1749 | CARDEVEGGMDVW | 1891 |
| 0095-H06 | LRP6e3e4 | FTFSSSAMH | 1574 | SAIGTGGSTYYA | 1783 | CAKGGDYFYYYYGMDVW | 1857 |
| 010S-D09 | LRP6e3e4 | GTFSSYTIS | 1603 | GGIVPAYRRANYA | 1754 | CAKGGYELDYW | 1865 |
| 010S-E09 | LRP6e3e4 | GDLSIYTIN | 1593 | GWINAGNGNTTYA | 1772 | CARGGDSSGYYYYAFDIW | 1926 |
| 009S-A07 | LRP6e3e4 | YTFTYRYLH | 1681 | GGIIPIFGTANYA | 1749 | CATAYGSSSLNIDYW | 1982 |
| 009S-B07 | LRP6e3e4 | YTFTGYYMH | 1675 | GWINPNSGGTNYA | 1774 | CVKDGGSFPLAYAFDIW | 2051 |
| 009S-D08 | LRP6e3e4 | YTFTSYDIN | 1679 | GGIIPIFGTANYA | 1749 | CARGPYYFDYW | 1940 |
| 010S-F09 | LRP6e3e4 | FTFDEYAMH | 1562 | STISGSGGSTYYA | 1809 | CASAKNDFWSGYFAFDYW | 1968 |
| 010S-G09 | LRP6e3e4 | GTFNTHTIT | 1599 | GWMNPNSGNTGY A | 1776 | CARGNLDFDYW | 1936 |
| 010S-H09 | LRP6e3e4 | FTFSDHYMS | 1567 | SAISSGSDRTYYA | 1787 | CARYSGYDFDYW | 1965 |
| 010S-A10 | LRP6e3e4 | FSFSSYSMN | 1559 | SYISSSSSTIYYA | 1816 | CARGSGYYGPGYYGMDVW | 1946 |
| 009S-D07 | LRP6e3e4 | FPFRYYGMS | 1551 | ARIGWNGGSIVYA | 1717 | CARDYSDRSGIDYW | 1911 |
| 010S-B10 | LRP6e3e4 | FAFKDYYMT | 1548 | SAIGAGGGTYYA | 1779 | CARESALYSSSWYYYYYGMDV W | 1920 |
| 010S-C10 | LRP6e3e4 | FTFSSYAMS | 1577 | SAISGSGGSTYYA | 1786 | CAKGGRDGYKGYFDYW | 1860 |
| 009S-E07 | LRP6e3e4 | YTFTGYYIH | 1674 | ZHVDPEDGETIYA | 1819 | CARGPAAIGILGWFDPW | 1938 |
| 010S-D10 | LRP6e3e4 | YIFTDYYMH | 1669 | GWMNPNSGNTGY A | 1776 | CARTLSGYSSSWYVFDYW | 1964 |
| 010S-E10 | LRP6e3e4 | FTFSSYSMN | 1579 | SGISWNSGTTGYS | 1797 | CARDHSSGWRHYFDYW | 1895 |
| 010S-F10 | LRP6e3e4 | FTFSNSDMN | 1570 | SYISGNSGYTNYA | 1814 | CASGSYYSDFDYW | 1971 |
| 010S-G10 | LRP6e3e4 | GTFSSYAIS | 1603 | GRINPNGGGTIYA | 1769 | CAREGGYYFDYW | 1919 |
| 009S-F07 | LRP6e3e4 | GTFSSYAIS | 1603 | GIINPSGGSTSYA | 1761 | CARAAGNFWSGYYTFDYW | 1877 |
| 009S-G07 | LRP6e3e4 | YTFTYRYLH | 1681 | GGIIPIFGTANYA | 1749 | CARGSYGMDVW | 1948 |
| 010S-H10 | LRP6e3e4 | YTFTSYYMH | 1680 | GWINPNSGGTNYA | 1774 | CAREAAEIPVGAFDIW | 1914 |
| 010S-A11 | LRP6e3e4 | FTFSNSDMN | 1570 | SYISGNSGYTNYA | 1814 | CASGSYYSDFDYW | 1972 |
| 010S-B11 | LRP6e3e4 | FTFRNYAIH | 1564 | SAIGTGGDTYYA | 1780 | CARDGGIRDFDYW | 1894 |
| 010S-C11 | LRP6e3e4 | YTFTYRYLH | 1681 | GGIIPIFGTANYA | 1749 | CAADDLGLELHYW | 1822 |
| 009S-H07 | LRP6e3e4 | YTFTGYYMH | 1675 | GWMNPNSGNTGY A | 1776 | CASSVVPAGPAGVYAFDIW | 1976 |
| 009S-A08 | LRP6e3e4 | GTFSSHAIN | 1602 | GWISANNGNTDYA | 1775 | CARDQDYGDYGWYYYGMDV W | 1903 |
| 010S-D11 | LRP6e3e4 | YTFTYRYLH | 1681 | GGIIPVFGTANYA | 1752 | CATDEYSSSYAFDIW | 1983 |
| 010S-E11 | LRP6e3e4 | FTFSAHGMH | 1565 | SGISESGGSTYYA | 1791 | CARGRGYSYGYYAFDIW | 1943 |
| 010S-F11 | LRP6e3e4 | YTFTYRYLH | 1681 | GGIIPIFGTANYA | 1749 | CARDSDWGVVDPW | 1905 |
| 010S-G11 | LRP6e3e4 | YTFTYRYLH | 1681 | GRIIPVLKITNYA | 1768 | CAVVDDAFDIW | 1997 |
| 010S-H11 | LRP6e3e4 | YTFTYRYLH | 1681 | GGIIPIFGTANYA | 1749 | CAKDGTDGRFDPW | 1846 |
| 009S-B08 | LRP6e3e4 | FTFTSSAVQ | 1589 | GWINAGNGNTTYA | 1772 | CARRGGDVTVPAAYYAMDVW | 1963 |
| 0105-A12 | LRP6e3e4 | VTFSRYPIS | 1667 | GGIIPIFGTANYA | 1749 | CAKDSGNYGYYGMDVW | 1854 |
| 010S-B12 | LRP6e3e4 | FTFSSYDMH | 1578 | SGITSNGGATYYA | 1798 | CARGTTGKGYYYYGMDVW | 1949 |
| 0105-C12 | LRP6e3e4 | FTFSNYWIH | 1571 | SAIGTGGGTYYA | 1781 | CTTAGYKAARRSVYPRIFNFDY W | 2044 |
| 010S-D12 | LRP6e3e4 | YTFTYRYLH | 1681 | GRIIPIFGTANYA | 1763 | CAREEGVGGMDVW | 1917 |
| 010S-E12 | LRP6e3e4 | FTFSSYAMH | 1576 | SAIGAGGGTYYA | 1779 | CARGVSSGYYYYYGMDVW | 1950 |
| 010S-F12 | LRP6e3e4 | FTVSSNYMS | 1592 | SAIGTGGGTYYA | 1781 | CARAGTNWGGWYFDLW | 1879 |
| 010S-G12 | LRP6e3e4 | FALSGYYMS | 1550 | SSISSSSTYIRYA | 1803 | CATVTGYSSAGAFDIW | 1995 |
| 011S-A01 | LRP6e3e4 | FTFSTHAFH | 1583 | SAIRGSGERTYYA | 1784 | CARDLRNWGSPYWYFDLW | 1901 |
| 011S-B01 | LRP6e3e4 | GTFSHYTIS | 1600 | GWINAGNGNTKYS | 1771 | CAKGGSLDMDVW | 1864 |
| 011S-C01 | LRP6e3e4 | LTFTSHGMS | 1615 | SYVSDSGSSVYYA | 1818 | CARHPGSFGGYSYAWYYYYG MDVW | 1957 |
| 011S-D01 | LRP6e3e4 | GTISDYTVS | 1605 | GIINPSGGSTSYA | 1761 | CARGYYDFDYW | 1953 |
| 009S-C08 | LRP6e3e4 | FSFNTFGIH | 1556 | AVISYDGSNKYYA | 1738 | CAKSIAAAGTGYYGMDVW | 1869 |
| 011S-E01 | LRP6e3e4 | FPFZYYSMN | 1552 | SAISGRDGRTYYA | 1785 | CAKDLGIQLPDYYFDYW | 1847 |
| 009S-D08 | LRP6e3e4 | YTFTSYDIN | 1679 | GGIIPIFGTANYA | 1749 | CARGPYYFDYW | 1941 |
| 011S-F01 | LRP6e3e4 | FSFSDYYMS | 1558 | SGISESGGRTYYA | 1790 | CASAADFDYW | 1967 |
| 009S-E08 | LRP6e3e4 | YGFTGYYIH | 1668 | GWMNPNSGNTGY A | 1776 | CARGYGDYDLW | 1952 |
| 009S-F08 | LRP6e3e4 | DTFANYGFS | 1547 | GXVNAGNGNTTYA | 1777 | CAKGWLDFDYW | 1867 |
| 0115-G01 | LRP6e3e4 | YTFTYRYLH | 1681 | GGIIPLFGTANYA | 1750 | CTTDDYGDQYGMDVW | 2046 |
| 011S-H01 | LRP6e3e4 | YTFTYRYLH | 1681 | GGIIPIFGTANYA | 1749 | CTTDDYGDLTHLDYW | 2045 |
| 011S-A02 | LRP6e3e4 | GTFSSYAIS | 1603 | GWMNPNSGNTGY A | 1776 | CARDKGYAFDIW | 1896 |
| 011S-B02 | LRP6e3e4 | YSFTRTDMH | 1670 | GYISAYTGHTSYA | 1778 | CARDLGGTADYW | 1899 |
| 011S-C02 | LRP6e3e4 | FTFSTYSMN | 1585 | SGISWNSGRIGYA | 1795 | CARDVGAFDIW | 1906 |
| 009S-G08 | LRP6e3e4 | FTFSDFAMT | 1566 | SYISGDSGYTNYA | 1813 | CARLGSYPGPYYYYMDVW | 1962 |
| 011S-D02 | LRP6e3e4 | FTFSSYAMS | 1577 | SSISGSGGVTYYA | 1801 | CARGGNTYYYYYGMDVW | 1928 |
| 009S-H08 | LRP6e3e4 | YTFTDYFMN | 1673 | GIINPSGDSTRFA | 1758 | CARDDGLGGMDVW | 1889 |
| 011S-E02 | LRP6e3e4 | YTFTYRYLH | 1681 | GGIIPIFGTANYA | 1749 | CATDYGDYYYGMDVW | 1984 |
| 009S-A09 | LRP6e3e4 | YTFTYRYLH | 1681 | GRIIPILGSTNYA | 1767 | CTTDLWDYW | 2048 |
| 011S-F02 | LRP6e3e4 | FTFSTYGMH | 1584 | SSISVSSGTTHYA | 1805 | CARGGSGSYYYAFDIW | 1930 |
| 0115-G02 | LRP6e3e4 | YTFTSYAMN | 1678 | GGIIPIFGTANYA | 1749 | CARDASGGSTGWYYFDSW | 1887 |
| 011S-H02 | LRP6e3e4 | YTFTNNFMH | 1676 | GIINPSGGSTSYA | 1761 | CARGLYKRYSYGYGMDVW | 1935 |
| 009S-B09 | LRP6e3e4 | FSFNTYAMN | 1557 | AVTSYDGGKKNYA | 1742 | CARDAGGDYDYW | 1885 |
| 009S-C09 | LRP6e3e4 | GTFHTYGLS | 1597 | GGIIPIFGTANYA | 1749 | CARGSGWSGLDYW | 1945 |
| 011S-A03 | LRP6e3e4 | FTFSSYWMH | 1580 | STISGSGGRTYYA | 1808 | CATSPYGVFTLDYW | 1994 |
| 011S-B03 | LRP6e3e4 | GTFSZYAIS | 1604 | GIINPSGGSTNYA | 1760 | CARAGYWSGYGYYGMDVW | 1881 |
| 011S-C03 | LRP6e3e4 | YTFSYRYLH | 1672 | GGIIPIFGTANYA | 1749 | CASTVTTDAFDIW | 1978 |
| 011S-D03 | LRP6e3e4 | FSFDDYGMS | 1554 | SVISSGGTIYYA | 1812 | CARHLSSGYLSYYGMDVW | 1955 |
| 009S-F09 | LRP6e3e4 | YSFTRTDMH | 1670 | GYISAYTGHTSYA | 1778 | CARDLGGTADYW | 1900 |
| 011S-E03 | LRP6e3e4 | FTFSSYAMS | 1577 | SAISGSGGSTYYA | 1786 | CAKGGRDGYKGYFDYW | 1861 |
| 0095-G09 | LRP6e3e4 | FTFSRHSMN | 1572 | SYSSGNSGYTNYA | 1817 | CARGDLEFDYW | 1923 |
| 011S-F03 | LRP6e3e4 | FTFSSYAMS | 1577 | SAISGSGGSTYYA | 1786 | CAKGGRDGYKGYFDYW | 1862 |
| 0095-H09 | LRP6e3e4 | FTFSSYAMS | 1577 | SAISGSGGSTYYA | 1786 | CAKGGRDGYKGYFDYW | 1863 |
| 011S-G03 | LRP6e3e4 | YTFTYRYLH | 1681 | GRIIPIHGIANYA | 1764 | CAREYSYGYFRYW | 1922 |
| 0095-A10 | LRP6e3e4 | FTFTSSAMQ | 1588 | GIINPSGGSTIYA | 1759 | CASGDTYDLYSLDVW | 1970 |
| 009S-B10 | LRP6e3e4 | YIFTDYYMH | 1669 | GWINAGNGNTTYA | 1772 | CAKVASGWSWPFDIW | 1871 |
| 011S-B04 | LRP6e1e2 | YTFTSYDIN | 1679 | GIINPSGGSTSYA | 1761 | CTREHSYYYYGMDVW | 2042 |
| 011S-C04 | LRP6e1e2 | GTFNSNAIS | 1598 | GWMNPNSGNTGY A | 1776 | CARDYYGSGSYNYGMDVW | 1913 |
| 011S-D04 | LRP6e1e2 | YTFTSYDIN | 1679 | GIINPSGGSTSYA | 1761 | CAREAYYYYYGMDVW | 1916 |
| 011S-E04 | LRP6e1e2 | FTFSSZZMH | 1581 | SAIGTGGGTZYA | 1782 | CAKDLGRAAAGSMDVW | 1850 |
| 011S-F04 | LRP6e1e2 | YIFTDYYMH | 1669 | GRIIPILGRANYA | 1765 | CARGGYSTLDYW | 1933 |
| 011S-H04 | LRP6e1e2 | YIFTDYYMH | 1669 | GRIIPILGRANYA | 1765 | CARGGYSTLDYW | 1934 |
| 011S-A05 | LRP6e1e2 | FTFSSYAMH | 1576 | SAIGTGGGTYYA | 1781 | CAKDLGRAAAGSMDVW | 1848 |
| 011S-B05 | LRP6e1e2 | YZFTDYYMH | 1683 | GWMNPNSGNTGY A | 1776 | CTRVAWGLDYW | 2043 |
| 011S-C05 | LRP6e1e2 | FTFSSYAMH | 1576 | SAIGTGGGTYYA | 1781 | CAKDLGRAAAGSMDVW | 1849 |
| 1115.3 | LRP6e1e2 | GFSFSTS | 2208 | NLNGGS | 2211 | ELAGYGTPFAY | 2214 |
| 421.1 | LRP6e1e2 | GYTFTTY | 2209 | FPGNVNT | 2212 | EELQYYFDY | 2215 |
| YW211.31 .57 | LRP6e3e4 | GFTFTSY | 2210 | SPYSGS | 2213 | RARPPIRLHPRGSVMDY | 2216 |

| **Clone ID** | **Confirmed Binding** | **CDRL1** | **SID NO.** | **CDRL2** | **SID NO.** | **CDRL3** | **SID NO.** |
|---|---|---|---|---|---|---|---|
| 001S-C08 | LRP6e1e2 | RASQYISNYLN | 2098 | AASSLQS | 2110 | CQQSYITPLTF | 2160 |
| 001S-C10 | LRP6e1e2 | | | | | | |
| 001S-D10 | LRP6e1e2 | | | | | | |
| 001S-E10 | LRP6e1e2 | | | | | | |
| 001S-F10 | LRP6e1e2 | | | | | | |
| 001S-G10 | LRP6e1e2 | | | | | | |
| 001S-A11 | LRP6e1e2 | | | | | | |
| 001S-B11 | LRP6e1e2 | | | | | | |
| 001S-C11 | LRP6e1e2 | | | | | | |
| 001S-E11 | LRP6e1e2 | | | | | | |
| 001S-F11 | LRP6e1e2 | | | | | | |
| 0015-G11 | LRP6e1e2 | | | | | | |
| 001S-H11 | LRP6e1e2 | | | | | | |
| 001S-A12 | LRP6e1e2 | | | | | | |
| 001S-B12 | LRP6e1e2 | | | | | | |
| 001S-C12 | LRP6e1e2 | | | | | | |
| 0015-D12 | LRP6e1e2 | | | | | | |
| 001S-F12 | LRP6e1e2 | | | | | | |
| 0085-B01 | LRP5 | | | | | | |
| 008S-C01 | LRP5 | | | | | | |
| 008S-D01 | LRP5 | | | | | | |
| 0085-E01 | LRP5 | | | | | | |
| 008S-G01 | LRP5 | | | | | | |
| 008S-A02 | LRP5 | | | | | | |
| 008S-C02 | LRP5 | | | | | | |
| 008S-D02 | LRP5 | | | | | | |
| 008S-E02 | LRP5 | | | | | | |
| 009S-C01 | LRP6e3e4 | RASQRVSNYLN | 2089 | AASSLQG | 2110 | CQQSYSVPYTF | 2178 |
| 009S-B02 | LRP6e3e4 | RASQSISNYLN | 2090 | AASSLQS | 2110 | CQQSYSLPLTF | 2170 |
| 009S-C02 | LRP6e3e4 | RASQSISNYLN | 2090 | AASSLQS | 2110 | CQQSYSMPLTF | 2171 |
| 009S-D02 | LRP6e3e4 | SGSSSNVGNNYV S | 2105 | DNDKRPS | 2122 | CESWDSSLSSEVF | 2139 |
| 010S-A02 | LRP6e1e2 | | | | | | |
| 010S-B02 | LRP6e1e2 | | | | | | |
| 010S-D02 | LRP6e1e2 | | | | | | |
| 010S-E02 | LRP6e1e2 | | | | | | |
| 010S-F02 | LRP6e1e2 | | | | | | |
| 009S-E02 | LRP6e1e2 | | | | | | |
| 009S-F02 | LRP6e1e2 | | | | | | |
| 009S-G02 | LRP6e1e2 | | | | | | |
| 009S-H02 | LRP6e1e2 | | | | | | |
| 009S-A03 | LRP6e1e2 | | | | | | |
| 010S-G02 | LRP6e1e2 | | | | | | |
| 010S-A03 | LRP6e1e2 | | | | | | |
| 009S-B03 | LRP6e1e2 | | | | | | |
| 010S-B03 | LRP6e1e2 | | | | | | |
| 010S-D03 | LRP6e1e2 | | | | | | |
| 009S-C03 | LRP6e1e2 | | | | | | |
| 009S-D03 | LRP6e1e2 | | | | | | |
| 009S-E03 | LRP6e1e2 | | | | | | |
| 009S-F03 | LRP6e1e2 | | | | | | |
| 010S-E03 | LRP6e1e2 | | | | | | |
| 010S-F03 | LRP6e1e2 | | | | | | |
| 009S-G03 | LRP6e1e2 | | | | | | |
| 0095-H03 | LRP6e1e2 | | | | | | |
| 009S-A04 | LRP6e1e2 | | | | | | |
| 0095-B04 | LRP6e3e4 | | | | | | |
| 010S-G03 | LRP6e3e4 | | | | | | |
| 009S-C04 | LRP6e3e4 | | | | | | |
| 009S-D04 | LRP6e3e4 | | | | | | |
| 010S-H03 | LRP6e3e4 | | | | | | |
| 009S-E04 | LRP6e3e4 | | | | | | |
| 010S-A04 | LRP6e3e4 | | | | | | |
| 009S-F04 | LRP6e3e4 | | | | | | |
| 010S-B04 | LRP6e3e4 | | | | | | |
| 009S-G04 | LRP6e3e4 | | | | | | |
| 009S-H04 | LRP6e3e4 | | | | | | |
| 010S-C04 | LRP6e3e4 | | | | | | |
| 010S-D04 | LRP6e3e4 | | | | | | |
| 010S-E04 | LRP6e3e4 | | | | | | |
| 010S-F04 | LRP6e3e4 | | | | | | |
| 0095-A05 | LRP6e3e4 | | | | | | |
| 010S-G04 | LRP6e3e4 | | | | | | |
| 010S-H04 | LRP6e3e4 | | | | | | |
| 010S-A05 | LRP6e3e4 | | | | | | |
| 010S-C05 | LRP6e3e4 | | | | | | |
| 010S-D05 | LRP6e3e4 | | | | | | |
| 010S-E05 | LRP6e3e4 | | | | | | |
| 010S-F05 | LRP6e3e4 | | | | | | |
| 013S-G04 | LRP6e3e4 | | | | | | |
| 0135-H04 | LRP6e3e4 | | | | | | |
| 0135-A05 | LRP6e3e4 | | | | | | |
| 0135-B05 | LRP6e3e4 | | | | | | |
| 013S-C05 | LRP6e3e4 | | | | | | |
| 013S-D05 | LRP6e3e4 | | | | | | |
| 013S-E05 | LRP6e3e4 | RASQSISSYLN | 2094 | AASSLQS | 2110 | CQQSYSTPLTF | 2174 |
| 0135-F05 | LRP6e3e4 | RASQSISSYLN | 2094 | AASSLQS | 2110 | CQQSYSTPLTF | 2174 |
| 0135-G05 | LRP6e3e4 | RASQDISNYLN | 2079 | AASTLQS | 2113 | CQQGNSFPYTF | 2152 |
| 0105-G06 | LRP6e3e4 | QASQDISNYLN | 2077 | AASSLQS | 2110 | CQQSYRIHWTF | 2163 |
| 009S-B05 | LRP6e3e4 | RASQSISSYLN | 2094 | AASSLQS | 2110 | CQQSYSTPLTF | 2174 |
| 009S-C05 | LRP6e3e4 | | | | | | |
| 0095-D05 | LRP6e3e4 | RASQSISSYLN | 2094 | AASSLQS | 2110 | CQQSYSTPLTF | 2174 |
| 0095-E05 | LRP6e3e4 | RASQGINSYLA | 2081 | DAKGLHP | 2114 | CQQSYSAPLSF | 2166 |
| 009S-F05 | LRP6e3e4 | RASQSISSYLN | 2094 | AASSLQS | 2110 | CQQSYSTPLTF | 2174 |
| 009S-G05 | LRP6e3e4 | QASQDISNYLN | 2077 | AASTLQR | 2112 | CQQSYSAPLTF | 2167 |
| 009S-C06 | LRP6e3e4 | RASRNINRYLN | 2099 | AASSLLS | 2109 | CQQSYNVPFTF | 2162 |
| 009S-D06 | LRP6e3e4 | RASQSISSYLN | 2094 | AASSLQS | 2110 | CQQSYSTPLTF | 2174 |
| 009S-E06 | LRP6e3e4 | RASQSISSYLN | 2094 | AASSLQS | 2110 | CQQSYSTPLTF | 2174 |
| 009S-F06 | LRP6e3e4 | RASQSISSYLN | 2094 | AASSLQS | 2110 | CQQSYSTPLTF | 2174 |
| 009S-G06 | LRP6e3e4 | RASQNIGLYLN | 2088 | DASSLQR | 2121 | CQQSYSTPYTF | 2176 |
| 009S-H06 | LRP6e3e4 | RASQSISSYLN | 2094 | AASSLQS | 2110 | CQQSYSTPLTF | 2174 |
| 009S-A07 | LRP6e3e4 | RASQSISSYLN | 2094 | AASSLQS | 2110 | CQQSYSTPLTF | 2174 |
| 009S-B07 | LRP6e3e4 | RASQSISSYLN | 2094 | AASSLQS | 2110 | CQQSYSTPLTF | 2174 |
| 009S-D07 | LRP6e3e4 | RSSQSLLHSNGY NYLD | 2102 | LGSNRAS | 2131 | CMQATQFPLTF | 2146 |
| 009S-F07 | LRP6e3e4 | RASQSISSYLN | 2094 | AASSLQS | 2110 | CQQSYSTPLTF | 2174 |
| 009S-G07 | LRP6e3e4 | RASQGISNYLA | 2083 | DASNLET | 2115 | CLQDFSFPWTF | 2140 |
| 009S-H07 | LRP6e3e4 | RASQSISSYLN | 2094 | AASSLQS | 2110 | CQQSYSTPLTF | 2174 |
| 0095-A08 | LRP6e3e4 | RASQGISNYLA | 2083 | GSSTLQS | 2127 | CQQTYSIPPTF | 2181 |
| 0115-C01 | LRP6e3e4 | RASQSISSYLN | 2094 | AASSLQS | 2110 | CQQSYSTPLTF | 2174 |
| 009S-C08 | LRP6e3e4 | RASQSISSYLN | 2094 | AASSLQS | 2110 | CQQSYSTPLTF | 2174 |
| 0095-D08 | LRP6e3e4 | RASQGISNNLN | 2082 | DASSLES | 2120 | CLQHNSYPFTF | 2143 |
| 011S-F01 | LRP6e3e4 | RASQDISNYLN | 2079 | AASSLQS | 2110 | CLQDYSYPRTF | 2141 |
| 009S-E08 | LRP6e3e4 | QASQDISNYLN | 2077 | DASSLES | 2120 | CQQSYRYPTF | 2165 |
| 009S-F08 | LRP6e3e4 | QASQDISNYLN | 2077 | DASSLES | 2120 | CQQSYSTSITF | 2177 |
| 009S-G08 | LRP6e3e4 | RASQSISSYLN | 2094 | AASSLQS | 2110 | CQQSYSTPLTF | 2174 |
| 009S-H08 | LRP6e3e4 | QASQDISNYLA | 2076 | AASSLQS | 2110 | CQQSYSTPLTF | 2174 |
| 009S-A09 | LRP6e3e4 | QASQGITNYLN | 2078 | AASSLQS | 2110 | CLQDYTDPFTF | 2142 |
| 011S-F02 | LRP6e3e4 | RASQSISSYLN | 2094 | AASSLQS | 2110 | CQQSYSTPLTF | 2174 |
| 011S-G02 | LRP6e3e4 | RASQGISSYLA | 2087 | AASSLQS | 2110 | CQQAYSFPWTF | 2150 |
| 011S-A03 | LRP6e3e4 | RASQSISSYLN | 2094 | AASSLQS | 2110 | CQQSYSTPLTF | 2174 |
| 011S-C03 | LRP6e3e4 | QASQDISNYLN | 2077 | DASSLES | 2120 | CQQSYSFPPFTF | 2168 |
| 011S-D03 | LRP6e3e4 | RASQSISSYLA | 2093 | AASTLQS | 2113 | CQQSYSTPLTF | 2174 |
| 011S-F03 | LRP6e3e4 | KSSQSVLYTTTNR NHIA | 2073 | WASSRKS | 2135 | CQQYYSTPYTF | 2189 |
| 0115-C04 | LRP6e1e2 | GASQSVPRNSLA | 2066 | GASQRAT | 2124 | CQQYHNWPPEYTF | 2184 |
| 011S-D04 | LRP6e1e2 | RASQSISSYLN | 2094 | AASSLQS | 2110 | CQQSYSTPLTF | 2174 |
| 011S-H04 | LRP6e1e2 | QASQDISNYLN | 2077 | AASTLQS | 2113 | CQQSFSTPRTF | 2156 |
| 008S-F02 | LRP5 | RSSQSLLHSDGYT YLY | 2101 | TLSYRAS | 2134 | CMQALEALFTF | 2144 |
| 010S-C06 | LRP6e1e2 | RASQSISSYLN | 2094 | AASSLQS | 2110 | CQQSYSTPLTF | 2174 |
| 010S-E06 | LRP6e3e4 | RASQSISSYLN | 2094 | AASSLQS | 2110 | CQQSYSTPLTF | 2174 |
| 010S-F06 | LRP6e3e4 | RASQSISSYLN | 2094 | AASSLQS | 2110 | CQQSYSTPLTF | 2174 |
| 010S-H06 | LRP6e3e4 | RASQSISSYLN | 2094 | AASSLQS | 2110 | CQQSYSTPLTF | 2174 |
| 010S-A07 | LRP6e3e4 | RASQSISSYLN | 2094 | AASSLQS | 2110 | CQQSYSTPLTF | 2174 |
| 010S-B07 | LRP6e3e4 | RASQSISSYLN | 2094 | AASSLQS | 2110 | CQQSYSTPLTF | 2174 |
| 010S-C07 | LRP6e3e4 | RASQSISSYLN | 2094 | AASSLQS | 2110 | CQQSYSTPLTF | 2174 |
| 010S-D07 | LRP6e3e4 | RASQSISSYLN | 2094 | AASSLQS | 2110 | CQQSYSTPLTF | 2174 |
| 009S-D05 | LRP6e3e4 | RASQSISSYLN | 2094 | AASSLQS | 2110 | CQQSYSTPLTF | 2174 |
| 009S-E05 | LRP6e3e4 | RASQGINSYLA | 2081 | DAKGLHP | 2114 | CQQSYSAPLSF | 2166 |
| 0105-E07 | LRP6e3e4 | RASQSISSYLN | 2094 | AASSLQS | 2110 | CQQSYSTPLTF | 2174 |
| 009S-F05 | LRP6e3e4 | RASQSISSYLN | 2094 | AASSLQS | 2110 | CQQSYSTPLTF | 2174 |
| 010S-F07 | LRP6e3e4 | RASQSVYSNLA | 2097 | DTSNRAT | 2123 | CQQYNNWPPITF | 2185 |
| 010S-G07 | LRP6e3e4 | RVSQGISSYLN | 2103 | AASSLQS | 2110 | CQQTYTIPFTF | 2182 |
| 009S-G05 | LRP6e3e4 | QASQDISNYLN | 2077 | AASTLQR | 2112 | CQQSYSAPLTF | 2167 |
| 010S-H07 | LRP6e3e4 | QASQDISNYLN | 2077 | GTSNLQS | 2128 | CQQSYSTPYTF | 2176 |
| 0105-A08 | LRP6e3e4 | RASQSISSYLN | 2094 | AASSLQS | 2110 | CQQSYSTPLTF | 2174 |
| 0095-A07 | LRP6e3e4 | RASQSISSYLN | 2094 | AASSLQS | 2110 | CQQSYSTPLTF | 2174 |
| 0095-B07 | LRP6e3e4 | RASQSISSYLN | 2094 | AASSLQS | 2110 | CQQSYSTPLTF | 2174 |
| 0095-B06 | LRP6e3e4 | RVSQSISSYLN | 2103 | AASSLQS | 2110 | CQQSYSTPLTF | 2174 |
| 010S-B08 | LRP6e3e4 | RASQGISNNLN | 2082 | DASNLET | 2115 | CQQSYTSRLTF | 2179 |
| 010S-C08 | LRP6e3e4 | RASQSISSYLN | 2094 | AASSLQS | 2110 | CQQSYSTPLTF | 2174 |
| 009S-C06 | LRP6e3e4 | RASRNINRYLN | 2099 | AASSLLS | 2109 | CQQSYNVPFTF | 2162 |
| 009S-D06 | LRP6e3e4 | RASQSISSYLN | 2094 | AASSLQS | 2110 | CQQSYSTPLTF | 2174 |
| 010S-D08 | LRP6e3e4 | RASQSISSYLN | 2094 | AASSLQS | 2110 | CQQSYSTPLTF | 2174 |
| 009S-E06 | LRP6e3e4 | RASQSISSYLN | 2094 | AASSLQS | 2110 | CQQSYSTPLTF | 2174 |
| 010S-E08 | LRP6e3e4 | RASQSISSYLN | 2094 | ZASSLQS | 2137 | CQQSYSTPLTF | 2174 |
| 010S-F08 | LRP6e3e4 | RASQSISSYLN | 2094 | AASSLQS | 2110 | CQQSYSTPLTF | 2174 |
| 009S-F06 | LRP6e3e4 | RASQSISSYLN | 2094 | AASSLQS | 2110 | CQQSYSTPLTF | 2174 |
| 010S-G08 | LRP6e3e4 | RASQSVRSSDLA | 2096 | GSSSRAT | 2126 | CQQYGRSPRYSF | 2183 |
| 010S-H08 | LRP6e3e4 | RASQSISSYLN | 2094 | AASSLQS | 2110 | CQQSYSTPLTF | 2174 |
| 010S-A09 | LRP6e3e4 | QASQDIANYLN | 2075 | AASSLQS | 2110 | CQQSYSTPYTF | 2176 |
| 010S-B09 | LRP6e3e4 | RASQSISRYLN | 2092 | KASSLES | 2130 | CQQSYDSPWTF | 2159 |
| 009S-G06 | LRP6e3e4 | RASQNIGLYLN | 2088 | DASSLQR | 2121 | CQQSYSTPYTF | 2176 |
| 010S-C09 | LRP6e3e4 | RASQSISSYLN | 2094 | AASSLQS | 2110 | CQQSYSTPLTF | 2174 |
| 009S-H06 | LRP6e3e4 | RASQSISSYLN | 2094 | AASSLQS | 2110 | CQQSYSTPLTF | 2174 |
| 010S-D09 | LRP6e3e4 | QASQDISNYLN | 2077 | AASSLQS | 2110 | CQQIHSYPLTF | 2155 |
| 010S-E09 | LRP6e3e4 | RASQSISSYLN | 2094 | AASSLQS | 2110 | CQQSYSTPLTF | 2174 |
| 0095-A07 | LRP6e3e4 | RASQSISSYLN | 2094 | AASSLQS | 2110 | CQQSYSTPLTF | 2174 |
| 0095-B07 | LRP6e3e4 | RASQSISSYLN | 2094 | AASSLQS | 2110 | CQQSYSTPLTF | 2174 |
| 0095-D08 | LRP6e3e4 | RASQGISNNLN | 2082 | DASSLES | 2120 | CLQHNSYPFTF | 2143 |
| 010S-F09 | LRP6e3e4 | RASQSISSYLN | 2094 | AASSLQS | 2110 | CQQSYSTPLTF | 2174 |
| 010S-G09 | LRP6e3e4 | QASQDISNYLN | 2077 | DASNLET | 2115 | CQQSYSTPLTF | 2174 |
| 010S-H09 | LRP6e3e4 | RASQGISNYLN | 2084 | AASTLQS | 2113 | CQQGYGTPPMF | 2153 |
| 010S-A10 | LRP6e3e4 | RASQSISSYLN | 2094 | AASSLQS | 2110 | CQQSYSTPLTF | 2174 |
| 009S-D07 | LRP6e3e4 | RSSQSLLHSNGY NYLD | 2102 | LGSNRAS | 2131 | CMQATQFPLTF | 2146 |
| 010S-B10 | LRP6e3e4 | RASQSISSYLN | 2094 | GTSSLHT | 2129 | CQQANSFPFTF | 2148 |
| 010S-C10 | LRP6e3e4 | KSSQSILSSSSNR DSLA | 2072 | WASSRKS | 2135 | CQQYYNIPYSF | 2187 |
| 009S-E07 | LRP6e3e4 | RASQSISSYLN | 2094 | AASSLQS | 2110 | CQQSYSTPLTF | 2174 |
| 010S-D10 | LRP6e3e4 | RASQSISSYLN | 2094 | AASSLQS | 2110 | CQQSYSTPLTF | 2174 |
| 010S-E10 | LRP6e3e4 | RASQSISSYLN | 2094 | AASSLQS | 2110 | CQQSYSTPLTF | 2174 |
| 010S-F10 | LRP6e3e4 | RASQSISNYLN | 2090 | AASTLES | 2111 | CQQANSFPPTF | 2148 |
| 010S-G10 | LRP6e3e4 | RASQGISNYLA | 2083 | AASSLQS | 2110 | CQQSYSTPWTF | 2175 |
| 009S-F07 | LRP6e3e4 | RASQSISSYLN | 2094 | AASSLQS | 2110 | CQQSYSTPLTF | 2174 |
| 0095-G07 | LRP6e3e4 | RASQGISNYLA | 2083 | DASNLET | 2115 | CLQDFSFPWTF | 2140 |
| 010S-H10 | LRP6e3e4 | KSSHSLLYSSDNK NYLA | 2071 | WSSTRES | 2136 | CQQYYSTPQTF | 2188 |
| 010S-A11 | LRP6e3e4 | RASQSIZNYLN | 2095 | ZASTLES | 2138 | CQQANSFPPTF | 2148 |
| 010S-B11 | LRP6e3e4 | QASQDISNYLN | 2077 | AASTLQS | 2113 | CQQSYSTPLTF | 2174 |
| 010S-C11 | LRP6e3e4 | RASQSISSYLN | 2094 | AASSLQS | 2110 | CQQSYSTPLTF | 2174 |
| 009S-H07 | LRP6e3e4 | RASQSISSYLN | 2094 | AASSLQS | 2110 | CQQSYSTPLTF | 2174 |
| 009S-A08 | LRP6e3e4 | RASQGISNYLA | 2083 | GSSTLQS | 2127 | CQQTYSIPPTF | 2181 |
| 010S-D11 | LRP6e3e4 | RASQSVSSNLA | 2097 | GASTRAT | 2125 | CQQFDRSPLTF | 2151 |
| 010S-E11 | LRP6e3e4 | RASQSISSYLN | 2094 | AASSLQS | 2110 | CQQSYSTPLTF | 2174 |
| 010S-F11 | LRP6e3e4 | RASQSISSYLN | 2094 | AASSLQS | 2110 | CQQSYSTPLTF | 2174 |
| 010S-G11 | LRP6e3e4 | RASQSISSYLN | 2094 | AASSLQS | 2110 | CQQSYSTPLTF | 2174 |
| 010S-H11 | LRP6e3e4 | RASQDISSYLA | 2080 | SASTLQS | 2133 | CQQSNSFPYTF | 2157 |
| 009S-B08 | LRP6e3e4 | RASQSISSYLN | 2094 | ZASSLQS | 2137 | CQQSYSTPLTF | 2174 |
| 010S-A12 | LRP6e3e4 | RASQSISSYLN | 2094 | AASSLQS | 2110 | CQQSYSTPLTF | 2174 |
| 010S-B12 | LRP6e3e4 | RASQSISSYLN | 2094 | AASSLQS | 2110 | CQQSYSTPLTF | 2174 |
| 0105-C12 | LRP6e3e4 | RASQSISSYLN | 2094 | AASSLQS | 2110 | CQQSYSTPLTF | 2174 |
| 010S-D12 | LRP6e3e4 | RPSQSIGSWLA | 2100 | DASNLQS | 2116 | CQQSSSTPYTF | 2158 |
| 010S-E12 | LRP6e3e4 | RASQSISSYLN | 2094 | AASSLQS | 2110 | CQQSYSTPLTF | 2174 |
| 010S-F12 | LRP6e3e4 | RASQGISRDLA | 2085 | AASTLQS | 2113 | CQQSYSPPFTF | 2172 |
| 010S-G12 | LRP6e3e4 | RASQSISSYLN | 2094 | AASSLQS | 2110 | CQQSYSTPLTF | 2174 |
| 011S-A01 | LRP6e3e4 | RASQSISSYLN | 2094 | AASSLQS | 2110 | CQQSYSTPLTF | 2174 |
| 0115-B01 | LRP6e3e4 | RASQGISNYLA | 2083 | AASSLHS | 2108 | CQQSYRTPLTF | 2164 |
| 0115-C01 | LRP6e3e4 | RASQSISSYLN | 2094 | AASSLQS | 2110 | CQQSYSTPLTF | 2174 |
| 011S-D01 | LRP6e3e4 | RASQGISNYLA | 2083 | AASSLQS | 2110 | CQQSYSTPLTF | 2174 |
| 009S-C08 | LRP6e3e4 | RASQSISSYLN | 2094 | AASSLQS | 2110 | CQQSYSTPLTF | 2174 |
| 011S-E01 | LRP6e3e4 | RASQGISSALA | 2086 | AASTLQS | 2113 | CQQSYSSPPTF | 2173 |
| 009S-D08 | LRP6e3e4 | RASQGISNNLN | 2082 | DASSLES | 2120 | CLQHNSYPFTF | 2143 |
| 011S-F01 | LRP6e3e4 | RASQDISNYLN | 2079 | AASSLQS | 2110 | CLQDYSYPRTF | 2141 |
| 009S-E08 | LRP6e3e4 | QASQDISNYLN | 2077 | DASSLES | 2120 | CQQSYRYPTF | 2165 |
| 009S-F08 | LRP6e3e4 | QASQDISNYLN | 2077 | DASSLES | 2120 | CQQSYSTSITF | 2177 |
| 0115-G01 | LRP6e3e4 | RASQSISSYLN | 2094 | AASSLQS | 2110 | CQQSYSTPLTF | 2174 |
| 011S-H01 | LRP6e3e4 | RASQSISSYLN | 2094 | AASSLQS | 2110 | CQQSYSTPLTF | 2174 |
| 011S-A02 | LRP6e3e4 | RSSQSLLHSNGY NYLD | 2102 | AASSLQS | 2110 | CMQALQTPITF | 2145 |
| 011S-B02 | LRP6e3e4 | RZSQSZSZYLN | 2104 | AASSLQS | 2110 | CQQSYSTPLTF | 2174 |
| 011S-C02 | LRP6e3e4 | QASQDISNYLN | 2077 | AASILQS | 2107 | CQQSYSIPFTF | 2169 |
| 009S-G08 | LRP6e3e4 | RASQSISSYLN | 2094 | AASSLQS | 2110 | CQQSYSTPLTF | 2174 |
| 011S-D02 | LRP6e3e4 | RASQSISSYLN | 2094 | AASSLQS | 2110 | CQQSYSTPLTF | 2174 |
| 0095-H08 | LRP6e3e4 | QASQDISNYLA | 2076 | AASSLQS | 2110 | CQQSYSTPLTF | 2174 |
| 011S-E02 | LRP6e3e4 | RASQSISSYLN | 2094 | AASSLQS | 2110 | CQQSYSTPLTF | 2174 |
| 009S-A09 | LRP6e3e4 | QASQGITNYLN | 2078 | AASSLQS | 2110 | CLQDYTDPFTF | 2142 |
| 011S-F02 | LRP6e3e4 | RASQSISSYLN | 2094 | AASSLQS | 2110 | CQQSYSTPLTF | 2174 |
| 011S-G02 | LRP6e3e4 | RASQGISSYLA | 2087 | AASSLQS | 2110 | CQQAYSFPWTF | 2150 |
| 011S-H02 | LRP6e3e4 | RASQSISSYLN | 2094 | AASSLQS | 2110 | CQQSYSTPLTF | 2174 |
| 009S-B09 | LRP6e3e4 | QASQDISNYLN | 2077 | AASSLQS | 2110 | CQQSYNTPRTF | 2161 |
| 009S-C09 | LRP6e3e4 | QASQDISNYLN | 2077 | DASNLET | 2115 | CQQSYTTPFTF | 2180 |
| 011S-A03 | LRP6e3e4 | RASQSISSYLN | 2094 | AASSLQS | 2110 | CQQSYSTPLTF | 2174 |
| 011S-B03 | LRP6e3e4 | RASQSISSYLN | 2094 | AASSLQS | 2110 | CQQSYSTPLTF | 2174 |
| 011S-C03 | LRP6e3e4 | QASQDISNYLN | 2077 | DASSLES | 2120 | CQQSYSFPPFTF | 2168 |
| 011S-D03 | LRP6e3e4 | RASQSISSYLA | 2093 | AASTLQS | 2113 | CQQSYSTPLTF | 2174 |
| 009S-F09 | LRP6e3e4 | RASQSISSYLN | 2094 | AASSLQS | 2110 | CQQSYSTPLTF | 2174 |
| 011S-E03 | LRP6e3e4 | KSSHSLLSTSTNR NQLA | 2070 | WASSRKS | 2135 | CQQYNNWPYTF | 2186 |
| 009S-G09 | LRP6e3e4 | RASQGISNYLA | 2083 | SASSLQS | 2132 | CQQGYNTPRTF | 2154 |
| 011S-F03 | LRP6e3e4 | KSSQSVLYTTTNR NHIA | 2074 | WASSRKS | 2135 | CQQYYSTPYTF | 2189 |
| 009S-H09 | LRP6e3e4 | KSSHSLLSTSTNR NHLA | 2069 | WASSRKS | 2135 | CQQYYNIPYSF | 2187 |
| 0115-G03 | LRP6e3e4 | RASQGISSYLA | 2087 | DASNLET | 2115 | CQQANSLFTF | 2149 |
| 0095-A10 | LRP6e3e4 | RASQSISRWLA | 2091 | AASSLQS | 2110 | CQQAYSFPWTF | 2150 |
| 009S-B10 | LRP6e3e4 | RASQSISSYLN | 2094 | AASSLQS | 2110 | CQQSYSTPLTF | 2174 |
| 011S-B04 | LRP6e1e2 | RASQSISSYLN | 2094 | AASSLQS | 2110 | CQQSYSTPLTF | 2174 |
| 011S-C04 | LRP6e1e2 | GASQSVPRNSLA | 2066 | GASQRAT | 2124 | CQQYHNWPPEYTF | 2184 |
| 011S-D04 | LRP6e1e2 | RASQSISSYLN | 2094 | AASSLQS | 2110 | CQQSYSTPLTF | 2174 |
| 011S-E04 | LRP6e1e2 | WASQSVRGNYV A | 2106 | DASNRAA | 2117 | CQHRSNWPLTF | 2147 |
| 011S-F04 | LRP6e1e2 | HGSQDISNYLN | 2067 | DASNRQS | 2119 | CQQSFSTPRTF | 2156 |
| 011S-H04 | LRP6e1e2 | QASQDISNYLN | 2077 | AASTLQS | 2113 | CQQSFSTPRTF | 2156 |
| 011S-A05 | LRP6e1e2 | WASQSVRGNYV A | 2106 | DASNRAG | 2118 | CQHRSNWPLTF | 2147 |
| 011S-B05 | LRP6e1e2 | RASQSISSYLN | 2094 | AASSLQS | 2110 | CQQSYSTPLTF | 2174 |
| 011S-C05 | LRP6e1e2 | WASQSVRGNYV A | 2106 | DASNRAA | 2117 | CQHRSNWPLTF | 2147 |
| 1115.3 | LRP6e1e2 | KASQSISYNLH | 2217 | YTSQSIS | 2220 | QQSNSWPLT | 2223 |
| 421.1 | LRP6e1e2 | SANSSVRFMF | 2218 | RTSNLAS | 2221 | QQYHSYPWT | 2224 |
| YW211.31 .57 | LRP6e3e4 | RASQDVSTAVA | 2219 | SASFLYS | 2222 | QQSYTTPPT | 2225 |

**Table 2B. Anti-LRP5/6 Antibody Clone IDs, Heavy Chain (HC) Seq ID Nos, and Binding Characteristics.**

| **Clone ID** | **HC Seq ID NO** | **Confirmed Binding** |
|---|---|---|
| 001S-F11 | 74 | LRP6e1e2 |
| 009S-G02 | 75 | LRP6e1e2 |
| 009S-A03 | 76 | LRP6e1e2 |
| 009S-D03 | 77 | LRP6e1e2 |
| 009S-F03 | 78 | LRP6e1e2 |
| 009S-H03 | 79 | LRP6e1e2 |
| 009S-A04 | 80 | LRP6e1e2 |
| 009S-B04 | 81 | LRP6e3e4 |
| 009S-D04 | 82 | LRP6e3e4 |
| 009S-E04 | 83 | LRP6e3e4 |
| 009S-F04 | 84 | LRP6e3e4 |
| 009S-G04 | 85 | LRP6e3e4 |
| 009S-H04 | 86 | LRP6e3e4 |
| 009S-A05 | 87 | LRP6e3e4 |
| 013S-G04 | 88 | LRP6e3e4 |
| 013S-H04 | 89 | LRP6e3e4 |
| 013S-C05 | 90 | LRP6e3e4 |
| 013S-D05 | 91 | LRP6e3e4 |
| 013S-G04 | 92 | LRP6e3e4 |
| 013S-H04 | 93 | LRP6e3e4 |
| 013S-A05 | 94 | LRP6e3e4 |
| 013S-C05 | 95 | LRP6e3e4 |
| 013S-D05 | 96 | LRP6e3e4 |
| 008S-D01 | 97 | LRP5 |

In certain embodiments, the LRP5/6 binding region may be selected from any binding domain that binds LRP5 or LRP6 with a K_{D} of less than or equal to about 1 x 10⁻⁴ M, less than or equal to about 1 x 10⁻⁵ M, less than or equal to about 1 x 10⁻⁶ M, less than or equal to about 1 x 10⁻⁷ M, less than or equal to about 1 x 10⁻⁸ M, less than or equal to about 1 x 10⁻⁹ M, less than or equal to about 1 x 10⁻¹⁰ M, less than or equal to about 1 x 10⁻¹¹ M, less than or equal to about 1 x 10⁻¹² M, less than or equal to about 1 x 10⁻¹³ M, less than or equal to about 1 x 10⁻¹⁴ M, or less than or equal to 1 x 10⁻¹⁵ M in the context of a Wnt surrogate molecule. In certain embodiment, the LRP5/6 binding region may be selected from any binding domain that binds LRP5 or LRP6 with a K_{D} of greater than or equal to about 1 x 10⁻⁴ M, greater than or equal to about 1 x 10⁻⁵ M, greater than or equal to about 1 x 10⁻⁶ M, greater than or equal to about 1 x 10⁻⁷ M, greater than or equal to about 1 x 10⁻⁸ M, greater than or equal to about 1 x 10⁻⁹ M, greater than about 1 x 10⁻¹⁰ M, greater than or equal to about 1 x 10⁻¹¹ M, greater than or equal to about 1 x 10⁻¹² M, greater than or equal to about 1 x 10⁻¹³ M, greater than or equal to about 1 x 10⁻¹⁴ M, or greater than or equal to 1 x 10⁻¹⁵ M in the context of a Wnt surrogate molecule. In certain embodiment, the LRP5/6 binding region may be selected from any binding domain that binds LRP5 or LRP6 at high affinity, e.g. a K_{D} of less than about 1 x 10⁻⁷ M, less than about 1 x 10⁻⁸ M, less than about 1 x 10⁻⁹ M, or less than about 1 x 10⁻¹⁰ M.

Other suitable LRP5/6 binding region include, without limitation, de novo designed LRP5/6 binding proteins, antibody derived binding proteins, e.g., scFv, Fab, etc., and other portions of antibodies that specifically bind to one or more Fzd proteins; VHH or sdAb derived binding domains; knottin-based engineered scaffolds; naturally occurring LRP5/6, including without limitation, DKK1, DKK2, DKK3, DKK4, sclerostin; Wise; fusions proteins comprising any of the above; derivatives of any of the above; variants of any of the above; and biologically active fragments of any of the above, and the like. A LRP5/6 binding region may be affinity selected to enhance binding.

Members of the Dickkopf (DKK) gene family (see Krupnik et al. (1999) Gene 238(2):301-13) include DKK-1, DKK-2, DKK-3, and DKK-4, and the DKK-3 related protein Soggy (Sgy). hDKKs 1-4 contain two distinct cysteine-rich domains in which the positions of 10 cysteine residues are highly conserved between family members. Exemplary sequences of human Dkk genes and proteins are publicly available, e.g., Genbank accession number NM_014419 (soggy-1); NM_014420 (DKK4); AF177394 (DKK-1); AF177395 (DKK-2); NM_015881 (DKK3); and NM_014421 (DKK2). In some embodiments of the disclosure, the LRP6 binding moiety is a DKK1 peptide, including without limitation the C-terminal domain of human DKK1. The C-terminal domain may comprise the sequence: KMYHTKGQEGSVCLRSSDCASGLCCARHFWSKICKPVLKEGQVCTKHRRKGSH GLEIFQRCYCGEGLSCRIQKDHHQASNSSRLHTCQRH (SEQ ID NO:2249) (see Genbank accession number NP_036374) or a biologically active fragment thereof.

Binding of DKK proteins to LRP5/6 are discussed, for example in Brott and Sokol Mol. Cell. Biol. 22 (17), 6100-6110 (2002); and Li et al. J. Biol. Chem. 277 (8), 5977-5981 (2002), each herein specifically incorporated by reference. The corresponding region of human DKK2 (Genbank reference NP_055236) may comprise the sequence: 
KMSHIKGHEGDPCLRSSDCIEGFCCARHFWTKICKPVLHQGEVCTKQRKKGSHG
LEIFQRCDCAKGLSCKVWKDATYSSKARLHVCQK (SEQ ID NO:2250),
 or a biologically active fragment thereof.

Antibodies that specifically bind to LRP5 or LRP6 are known in the art and are commercially available, or can be generated de novo. LRP5, LRP6 or fragments thereof can be used as an immunogen or in screening assays to develop an antibody. Examples of known antibodies include, without limitation, those described in Gong et al. (2010) PLoS One. 5(9):e12682; Ettenberg et al. (2010) Proc Natl Acad Sci U S A. 107(35):15473-8; and those commercially available from, for example Santa Cruz biotechnology antibody clone 1A12, which was raised against synthetic LRP5/6 of human origin and binds to both the full length and proteolytic fragment of LRP6 and LRP5 of mouse and human origin; the monoclonal antibody 2B11; Cell Signaling Technology antibody specific for LRP5 (D80F2), catalog number 5731; etc.

In certain embodiments, Wnt surrogate molecules disclosed herein comprise one or more polypeptides comprising two or more binding regions. For example, the two or more binding regions may be two or more Fzd binding regions or two or more LRP5/6 binding regions, or they may comprise one or more Fzd binding regions and one or more LRP5/6 binding regions. The binding regions may be directly joined or contiguous, or may be separated by a linker, e.g. a polypeptide linker, or a non-peptidic linker, etc. The length of the linker, and therefore the spacing between the binding domains can be used to modulate the signal strength, and can be selected depending on the desired use of the Wnt surrogate molecule. The enforced distance between binding domains can vary, but in certain embodiments may be less than about 100 angstroms, less than about 90 angstroms, less than about 80 angstroms, less than about 70 angstroms, less than about 60 angstroms, or less than about 50 angstroms. In some embodiments the linker is a rigid linker, in other embodiments the linker is a flexible linker. In certain embodiments where the linker is a peptide linker, it may be from about 1, 2, 3, 4, 5, 6, 7, 8, 9, 10, 11, 12, 13, 14, 15, 16, 17, 18, 19, 20 21, 22, 23, 24, 25, 26, 27, 28, 29, 30 or more amino acids in length, and is of sufficient length and amino acid composition to enforce the distance between binding domains. In some embodiments, the linker comprises or consists of one or more glycine and/or serine residues.

In particular embodiments, a Wnt surrogate molecule comprises a polypeptide sequence having at least 90%, at least 95%, at least 98% or at least 99% identity to a polypeptide sequence disclosed in any of SEQ ID NOs: 109-124 or 125-157, or having at least 90%, at least 95%, at least 98% or at least 99% identity to an antigen-binding fragment of a polypeptide sequence disclosed in any of SEQ ID NOs:109-124 or 125-157. In certain embodiments, the Wnt surrogate molecules comprises or consists of a polypeptide sequence set forth in any of SEQ ID NOs:109-124 or 125-147, or an antigen-binding fragment thereof. In particular embodiments, the antigen-binding fragment binds one or more Fzd receptors and also binds LRP5 and/or LRP6.

Wnt surrogate molecule can be multimerized, e.g., through an Fc domain, by concatenation, coiled coils, polypeptide zippers, biotin/avidin or streptavidin multimerization, and the like. The Wnt surrogate molecules can also be joined to a moiety such as PEG, Fc, etc., as known in the art to enhance stability in vivo.

In certain embodiments, a Wnt surrogate molecule directly activates canonical Wnt signaling through binding to one or more Fzd proteins and to LRP5/6, particularly by binding to these proteins on a cell surface, e.g., the surface of a human cell. The direct activation of Wnt signaling by a Wnt surrogate molecule is in contrast to potentiation of Wnt signaling, which enhances activity only when native Wnt proteins are present.

Wnt surrogate molecules may activate Wnt signaling, e.g., by mimicking the effect or activity of a Wnt protein binding to a frizzled protein. The ability of the Wnt surrogate molecules of the present disclosure to mimic the activity of Wnt can be confirmed by a number of assays. The Wnt surrogate molecules typically initiate a reaction or activity that is similar to or the same as that initiated by the receptor's natural ligand. In particular, the Wnt surrogate molecules of the present disclosure enhance the canonical Wnt/β-catenin signaling pathway. As used herein, the term "enhances" refers to a measurable increase in the level of Wnt/β-catenin signaling compared with the level in the absence of a Wnt surrogate molecule of the present disclosure.

Various methods are known in the art for measuring the level of canonical Wnt/β-catenin signaling. These include, but are not limited to assays that measure: Wnt/β-catenin target gene expression; TCF reporter gene expression; β-catenin stabilization; LRP phosphorylation; Axin translocation from cytoplasm to cell membrane and binding to LRP. The canonical Wnt/β-catenin signaling pathway ultimately leads to changes in gene expression through the transcription factors TCF7, TCF7L1, TCF7L2 and LEF. The transcriptional response to Wnt activation has been characterized in a number of cells and tissues. As such, global transcriptional profiling by methods well known in the art can be used to assess Wnt/β-catenin signaling activation or inhibition.

Changes in Wnt-responsive gene expression are generally mediated by TCF and LEF transcription factors. A TCF reporter assay assesses changes in the transcription of TCF/LEF controlled genes to determine the level of Wnt/β-catenin signaling. A TCF reporter assay was first described by Korinek, V. et al., 1997. Also known as TOP/FOP this method involves the use of three copies of the optimal TCF motif CCTTTGATC, or three copies of the mutant motif CCTTTGGCC, upstream of a minimal c-Fos promoter driving luciferase expression (pTOPFI_ASH and pFOPFI_ASH, respectively) to determine the transactivational activity of endogenous p-catenin/TCF4. A higher ratio of these two reporter activities (TOP/FOP) indicates higher β-catenin/TCF4 activity, whereas a lower ratio of these two reporter activities indicates lower β-catenin/TCF4 activity.

Various other reporter transgenes that respond to Wnt signals exist intact in animals and therefore, effectively reflect endogenous Wnt signaling. These reporters are based on a multimerized TCF binding site, which drives expression of LacZ or GFP, which are readily detectable by methods known in the art. These reporter genes include: TOP-GAL, BAT-GAL, ins-TOPEGFP, ins-TOPGAL, LEF-EGFP, Axin2-LacZ, Axin2-d2EGFP, Lgr5tm1 (cre/ERT2), TOPdGFP.

The recruitment of dephosphorylated β-catenin to the membrane, stabilization and phosphorylation status of β-catenin, and translocation of β-catenin to the nucleus (Klapholz- Brown Z et al., PLoS One. 2(9) e945, 2007), in some cases mediated by complex formation with TCF transcription factors and TNIK are key steps in the Wnt signaling pathway. Stabilization is mediated by Disheveled family proteins that inhibit the "destruction" complex so that degradation of intracellular β-catenin is reduced, and translocation of β-catenin to the nucleus follows thereafter. Therefore, measuring the level and location of β-catenin in a cell is a good reflection of the level of Wnt/β-catenin signaling. A non-limiting example of such an assay is the "Biolmage β-Catenin Redistribution Assay" (Thermo Scientific) which provides recombinant U20S cells that stably express human β-catenin fused to the C-terminus of enhanced green fluorescent protein (EGFP). Imaging and analysis is performed with a fluorescence microscope or HCS platform allowing the levels and distribution of EGFP-β-catenin to be visualized.

Another way, in which the destruction complex is inhibited, is by removal of Axin by recruitment of Axin to the cytoplasmic tail of the Wnt co-receptor LRP. Axin has been shown to bind preferentially to a phosphorylated form of the LRP tail. Visualization of Axin translocation, for example with a GFP-Axin fusion protein, is therefore another method for assessing levels of Wnt/β-catenin signaling.

In certain embodiments, a Wnt surrogate molecule enhances or increases canonical Wnt pathway signaling, e.g., β-catenin signaling, by at least 30%, 35%, 40%, 45%, 50%, 60%, 70%, 75%, 80%, 85%, 90%, 95%, 100%, 110%, 150%, 200%, 250%, 300%, 400% or 500%, as compared to the β-catenin signaling induced by a neutral substance or negative control as measured in an assay described above, for example as measured in the TOPFlash assay. A negative control may be included in these assays. In particular embodiments, Wnt surrogate molecules may enhance β-catenin signaling by a factor of 2x, 5x, 10x, 100x, 1000x, 10000x or more as compared to the activity in the absence of the Wnt surrogate molecule when measured in an assay described above, for example when measured in the TOPFlash assay, or any of the other assays mentioned herein.

"Wnt gene product" or "Wnt polypeptide" when used herein encompass native sequence Wnt polypeptides, Wnt polypeptide variants, Wnt polypeptide fragments and chimeric Wnt polypeptides. In particular embodiments, a Wnt polypeptide is a native human full length mature Wnt protein.

For example, human native sequence Wnt proteins of interest in the present application include the following: Wnt-1 (GenBank Accession No. NM_005430); Wnt-2 (GenBank Accession No. NM_003391); Wnt-2B (Wnt-13) (GenBank Accession No. NM_004185 (isoform 1), NM_024494.2 (isoform 2)), Wnt-3 (RefSeq.: NM_030753), Wnt3a (GenBank Accession No. NM_033131), Wnt-4 (GenBank Accession No. NM_030761), Wnt-5A (GenBank Accession No. NM_003392), Wnt-5B (GenBank Accession No. NM_032642), Wnt-6 (GenBank Accession No. NM_006522), Wnt-7A (GenBank Accession No. NM_004625), Wnt-7B (GenBank Accession No. NM_058238), Wnt-8A (GenBank Accession No. NM_058244), Wnt-8B (GenBank Accession No. NM_003393), Wnt-9A (Wnt- 14) (GenBank Accession No. NM_003395), Wnt-9B (Wnt-15) (GenBank Accession No. NM_003396), Wnt-1 OA (GenBank Accession No. NM_025216), Wnt-10B (GenBank Accession No. NM_003394), Wnt-11 (GenBank Accession No. NM_004626), Wnt- 16 (GenBank Accession No. NM_016087)). Although each member has varying degrees of sequence identity with the family, all encode small (i.e., 39-46 kD), acylated, palmitoylated, secreted glycoproteins that contain 23-24 conserved cysteine residues whose spacing is highly conserved (McMahon, A P et al., Trends Genet. 1992; 8: 236-242; Miller, J R. Genome Biol. 2002; 3(1): 3001.1-3001.15). Other native sequence Wnt polypeptides of interest include orthologs of the above from any mammal, including domestic and farm animals, and zoo, laboratory or pet animals, such as dogs, cats, cattle, horses, sheep, pigs, goats, rabbits, rats, mice, frogs, zebra fish, fruit fly, worm, etc.

"Wnt pathway signaling" or "Wnt signaling" is used herein to refer to the mechanism by which a biologically active Wnt exerts its effects upon a cell to modulate a cell's activity. Wnt proteins modulate cell activity by binding to Wnt receptors, including proteins from the Frizzled (Fzd) family of proteins, proteins from the ROR family of proteins, the proteins LRP5, LRP6 from the LRP family of proteins, the protein FRL1/crypto, and the protein Derailed/Ryk. Once activated by Wnt binding, the Wnt receptor(s) will activate one or more intracellular signaling cascades. These include the canonical Wnt signaling pathway; the Wnt/planar cell polarity (Wnt/PCP) pathway; the Wnt-calcium (Wnt/Ca²⁺) pathway (Giles, RH et al. (2003) Biochim Biophys Acta 1653, 1-24; Peifer, M. et al. (1994) Development 120: 369-380; Papkoff, J. et al (1996) Mol. Cell Biol. 16: 2128-2134; Veeman, M. T. et al. (2003) Dev. Cell 5: 367-377); and other Wnt signaling pathways as is well known in the art.

For example, activation of the canonical Wnt signaling pathway results in the inhibition of phosphorylation of the intracellular protein β-catenin, leading to an accumulation of β- catenin in the cytosol and its subsequent translocation to the nucleus where it interacts with transcription factors, e.g. TCF/LEF, to activate target genes. Activation of the Wnt/PCP pathway activates RhoA, c-Jun N-terminal kinase (JNK), and nemo-like kinase (NLK) signaling cascades to control such biological processes as tissue polarity and cell movement. Activation of the Wnt/Ca²⁺ by, for example, binding of Wnt-4, Wnt-5A or Wnt-11, elicits an intracellular release of calcium ions, which activates calcium sensitive enzymes like protein kinase C (PKC), calcium-calmodulin dependent kinase II (CamKII) or calcineurin (CaCN). By assaying for activity of the above signaling pathways, the biological activity of an antibody or antigen-binding fragment thereof, e.g., a Wnt surrogate molecule, can be readily determined.

In certain embodiments, functional properties of Wnt surrogate molecules may be assessed using a variety of methods known to the skilled person, including e.g., affinity/binding assays (for example, surface plasmon resonance, competitive inhibition assays), cytotoxicity assays, cell viability assays, cell proliferation or differentiation assays in response to a Wnt, cancer cell and/or tumor growth inhibition using in vitro or in vivo models, including but not limited to any described herein. The Wnt surrogate molecules described herein may also be tested for effects on Fzd receptor internalization, in vitro and in vivo efficacy, etc. Such assays may be performed using well-established protocols known to the skilled person (see, e.g., Current Protocols in Molecular Biology (Greene Publ. Assoc. Inc. & John Wiley & Sons, Inc., NY, NY); Current Protocols in Immunology (Edited by: John E. Coligan, Ada M. Kruisbeek, David H. Margulies, Ethan M. Shevach, Warren Strober 2001 John Wiley & Sons, NY, NY); or commercially available kits.

In certain embodiments, an Fzd binding region of a Wnt surrogate molecule (e.g., an antigen-binding fragment of an anti-Fzd antibody) comprises one or more of the CDRs of the anti-Fzd antibodies described herein. In certain embodiments, a LRP5/6 binding region of a Wnt surrogate molecule (e.g., an antigen-binding fragment of an anti-LRP5/6 antibody) comprises one or more of the CDRs of the anti-LRP5/6 antibodies described herein. In this regard, it has been shown in some cases that the transfer of only the VHCDR3 of an antibody can be performed while still retaining desired specific binding (Barbas et al., PNAS (1995) 92: 2529-2533). See also, McLane et al., PNAS (1995) 92:5214-5218, Barbas et al., J. Am. Chem. Soc. (1994) 116:2161-2162.

Also disclosed herein is a method for obtaining an antibody or antigen-binding domain specific for a Fzd receptor, the method comprising providing by way of addition, deletion, substitution or insertion of one or more amino acids in the amino acid sequence of a VH domain set out herein or a VH domain which is an amino acid sequence variant of the VH domain, optionally combining the VH domain thus provided with one or more VL domains, and testing the VH domain or VH/VL combination or combinations to identify a specific binding member or an antibody antigen binding domain specific for one or more Fzd receptor and optionally with one or more desired properties. The VL domains may have an amino acid sequence which is substantially as set out herein. An analogous method may be employed in which one or more sequence variants of a VL domain disclosed herein are combined with one or more VH domains.

In particular embodiments, Wnt surrogate molecules are water soluble. By "water soluble" it is meant a composition that is soluble in aqueous buffers in the absence of detergent, usually soluble at a concentration that provides a biologically effective dose of the polypeptide. Compositions that are water soluble form a substantially homogenous composition that has a specific activity that is at least about 5% that of the starting material from which it was purified, usually at least about 10%, 20%, or 30% that of the starting material, more usually about 40%, 50%, or 60% that of the starting material, and may be about 50%, about 90% or greater. Wnt surrogate molecules disclosed herein typically form a substantially homogeneous aqueous solution at concentrations of at least 25 µM and higher, e.g., at least 25 µM, 40 µM, or 50 µM, usually at least 60 µM, 70 µM, 80 µM, or 90 µM, sometimes as much as 100 µM, 120 µM, or 150 µM. In other words, Wnt surrogate molecules disclosed herein typically form a substantially homogeneous aqueous solution at concentrations of about 0.1 mg/ml, about 0.5 mg/ml, of about 1 mg/ml or more.

An antigen or epitope that "specifically binds" or "preferentially binds" (used interchangeably herein) to an antibody or antigen-binding fragment thereof is a term well understood in the art, and methods to determine such specific or preferential binding are also well known in the art. A molecule, e.g., a Wnt surrogate molecule, is said to exhibit "specific binding" or "preferential binding" if it reacts or associates more frequently, more rapidly, with greater duration and/or with greater affinity with a particular cell or substance than it does with alternative cells or substances. A molecule or binding region thereof, e.g., a Wnt surrogate molecule or binding region thereof, "specifically binds" or "preferentially binds" to a target antigen, e.g., an Fzd receptor, if it binds with greater affinity, avidity, more readily, and/or with greater duration than it binds to other substances. For example, a Wnt surrogate molecule or binding region thereof that specifically or preferentially binds to the Fzd1 receptor is an antibody that binds to the Fzd1 receptor with greater affinity, avidity, more readily, and/or with greater duration than it binds to other Fzd receptors or non-Fzd proteins. It is also understood by reading this definition that, for example, a Wnt surrogate molecule or binding region thereof that specifically or preferentially binds to a first target may or may not specifically or preferentially bind to a second target. As such, "specific binding" or "preferential binding" does not necessarily require (although it can include) exclusive binding. Generally, but not necessarily, reference to binding means preferential binding.

In some embodiments, any of the one or more Fzd binding regions of a Wnt surrogate molecule binds to one, two, three, four, five or more different frizzled receptors, e.g., one or more of human frizzled receptors Fzd1, Fzd2, Fzd3, Fzd4, Fzd5, Fzd6, Fzd7, Fzd8, Fzd9, Fzd10. In some embodiments, any of the Fzd binding regions binds to Fzd1, Fzd2, Fzd5, Fzd7 and Fzd8. In various embodiments, any of the Fzd binding regions binds to: (i) Fzd1, Fzd2, Fzd7 and Fzd9; (ii) Fzd1, Fzd2 and Fzd7; (iii) Fzd5 and Fzd8; (iv) Fzd5, Fzd7 and Fzd8; (v) Fzd1, Fzd4, Fzd5 and Fzd8; (vi) Fzd1, Fzd2, Fzd5, Fzd7 and Fzd8; (vii) Fzd4 and Fzd9; (viii) Fzd9 and Fzd10; (ix) Fzd5, Fzd8 and Fzd10; (x) Fzd4, Fzd5 and Fzd8; or (xi) Fzd1, Fzd5, Fzd7 and Fzd8.

In some embodiments, the Fzd binding region is selective for one or more Fzd receptors of interest, e.g. having a specificity for the one or more desired Fzd receptors of at least 10-fold, 25-fold, 50-fold, 100-fold, 200-fold or more relative to other Fzd receptors. In some embodiments, any of the one or more Fzd binding regions of a Wnt surrogate molecule is multispecific and binds or specifically binds to a plurality of Fzd receptors, e.g., two or more of Fzd1, Fzd2, Fzd3, Fzd4, Fzd5, Fzd6, Fzd7, Fzd8, Fzd9, or Fzd10. For example, any of the one or more Fzd binding regions may be bispecific, trispecific, tetraspecific, and so on. In some embodiments, any of the one or more Fzd binding regions of a Wnt surrogate molecule is monospecific and binds or specifically binds to a single Fzd receptor, e.g., only one of Fzd1, Fzd2, Fzd3, Fzd4, Fzd5, Fzd6, Fzd7, Fzd8, Fzd9, or Fzd10.

In some embodiments, a monospecific Fzd binding region binds to a region of an Fzd receptor that does not include the cysteine rich domain (CRD) of the Fzd receptor, or includes less than the entire CRD of the FZD receptor. As illustrated in FIG. 2A, sequences within the CRD show strong homology between the 10 Fzd receptors, with homologies being even higher between subfamily members. Accordingly, certain embodiments of the monospecific Fzd binding regions disclosed herein do not bind to the CRD, or bind only to a subset of the CRD.

In some embodiments, a Fzd binding region, e.g., a monospecific Fzd binding region, binds to an epitope comprising at least a portion of the extracellular domain after the CRD, referred to herein as the "hinge region" of a Fzd receptor (see FIG. 2A). In particular embodiments, at least 20%, at least 30%, at least 40%, at least 50%, at least 60%, at least 70%, at least 80%, at least 90%, or 100% of the epitope is present within the hinge region of a Fzd receptor. As illustrated in FIGS. 4A-4E, the hinge regions of the extracellular domain of Fzd receptors show highly divergent sequences. Sequences of illustrative Fzd receptor hinge regions are set forth in SEQ ID NOs:98-107 and in Table 3 below. In certain embodiments, the hinge region includes an amino acid sequence having at least 90%, at least 95%, at least 98%, or at least 99% identity to any of the sequences set forth in SEQ ID NOs:98-107.

**Table 3: Fzd hinge region sequences**

| **SID NO.** | **Fzd** | **Hinge Region Sequence** |
|---|---|---|
| 98 | Fzd1 | CVGQNTSDKGTPTPSLLPEFWTSNPQHGGGHRGGFPGGA GASERGKFSC |
| 99 | Fzd2 | CVGQNHSEDGAPALLTTAPPPGLQPGAGGTPGGPGGGGAP PRYATLEHPFHC |
| 100 | Fzd3 | CDEPYPRLVDLNLAGEPTEGAPVAVQRDYGFWC |
| 101 | Fzd4 | CMEGPGDEEVPLPHKTPIQPGEEC |
| 102 | Fzd5 | CMDYNRSEATTAPPRPFPAKPTLPGPPGAPASGGEC |
| 103 | Fzd6 | CDETVPVTFDPHTEFLGPQKKTEQVQRDIGFWC |
| 104 | Fzd7 | CVGQNTSDGSGGAGGSPTAYPTAPYLPDPPFTAMSPSDGRG RLSFPFSC |
| 105 | Fzd8 | CMDYNRTDLTTAAPSPPRRLPPPPPGEQPPSGSGHGRPPGA RPPHRGGGRGGGGGDAAAPPARGGGGGGKARPPGGGAAP C |
| 106 | Fzd9 | CMEAPENATAGPAEPHKGLGMLPVAPRPARPPGDLGPGAGG SGTC |
| 107 | Fzd10 | CMEAPNNGSDEPTRGSGLFPPLFRPQRPHSAQEHPLKDGGP GRGGC |

In some embodiments, a monospecific Fzd binding region binds to an epitope comprising at least a portion of an N-terminal region upstream of the CRD of the Fzd receptor (FIG. 2A). In particular embodiments, at least 20%, at least 30%, at least 40%, at least 50%, at least 60%, at least 70%, at least 80%, at least 90%, or 100% of the epitope is present within the N-terminal region of a Fzd receptor. The sequence of an illustrative N-terminal region is set forth in SEQ ID NO:108 and in Table 4 below. In certain embodiments, the N-terminal region includes an amino acid sequence having at least 90%, at least 95%, at least 98%, or at least 99% identity to SEQ ID NO:108.

**Table 4: Fzd N-terminal region sequences**

| **SEQ ID NO** | **Fzd** | **Hinge Region Sequence** |
|---|---|---|
| 108 | Fzd1 | QAAGQGPGQGPGPGQQPPPPPQQQQSGQQYN |

In some embodiments, any of the one or more LRP5/6 binding regions of a Wnt surrogate molecule binds to one or both of LRP5/6. For convenience, the term "LRP5/6" is used to refer collectively to either or both of LRP5 and/or LRP6.

Immunological binding generally refers to the non-covalent interactions of the type which occur between an immunoglobulin molecule and an antigen for which the immunoglobulin is specific, for example by way of illustration and not limitation, as a result of electrostatic, ionic, hydrophilic and/or hydrophobic attractions or repulsion, steric forces, hydrogen bonding, van der Waals forces, and other interactions. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}. See, generally, Davies et al. (1990) Annual Rev. Biochem. 59:439-473.

In certain embodiments, the Wnt surrogate molecules or binding regions thereof described herein have an affinity of less than about 10,000, less than about 1000, less than about 100, less than about 10, less than about 1, less than about 0.1, less than about 0.01, less than about 0.001, less than about 0.0001, less than about 0.00001, or less than about 0.000001 nM, and in some embodiments, the antibodies may have even higher affinity for one or more Fzd receptor epitopes or LRP5 or LRP6 receptor.

The constant regions of immunoglobulins show less sequence diversity than the variable regions, and are responsible for binding a number of natural proteins to elicit important biochemical events. In humans, there are five different classes of antibodies including IgA (which includes subclasses IgA1 and IgA2), IgD, IgE, IgG (which includes subclasses IgG1, IgG2, IgG3, and IgG4), and IgM. The distinguishing features between these antibody classes are their constant regions, although subtler differences may exist in the V region.

The Fc region of an antibody interacts with a number of Fc receptors and ligands, imparting an array of important functional capabilities referred to as effector functions. For IgG, the Fc region comprises Ig domains CH2 and CH3 and the N-terminal hinge leading into CH2. An important family of Fc receptors for the IgG class are the Fc gamma receptors (FcγRs). These receptors mediate communication between antibodies and the cellular arm of the immune system (Raghavan et al., 1996, Annu Rev Cell Dev Biol 12:181-220; Ravetch et al., 2001, Annu Rev Immunol 19:275-290). In humans this protein family includes FcγRI (CD64), including isoforms FcγRIa, FcγRIb, and FcγRIc; FcγRII (CD32), including isoforms FcγRIIa (including allotypes H131 and R131), FcγRIIb (including FcγRIIb-1 and FcγRIIb-2), and FcγRIIc; and FcγRIII (CD16), including isoforms FcγRIIIa (including allotypes V158 and F158) and FcγRIIIb (including allotypes FcγRIIIb-NA1 and FcγRIIIb-NA2) (Jefferis et al., 2002, Immunol Lett 82:57-65). These receptors typically have an extracellular domain that mediates binding to Fc, a membrane spanning region, and an intracellular domain that may mediate some signaling event within the cell. These receptors are expressed in a variety of immune cells including monocytes, macrophages, neutrophils, dendritic cells, eosinophils, mast cells, platelets, B cells, large granular lymphocytes, Langerhans' cells, natural killer (NK) cells, and T cells. Formation of the Fc/FcγR complex recruits these effector cells to sites of bound antigen, typically resulting in signaling events within the cells and important subsequent immune responses such as release of inflammation mediators, B cell activation, endocytosis, phagocytosis, and cytotoxic attack.

The ability to mediate cytotoxic and phagocytic effector functions is a potential mechanism by which antibodies destroy targeted cells. The cell-mediated reaction wherein nonspecific cytotoxic cells that express FcγRs recognize bound antibody on a target cell and subsequently cause lysis of the target cell is referred to as antibody dependent cell-mediated cytotoxicity (ADCC) (Raghavan et al., 1996, Annu Rev Cell Dev Biol 12:181-220; Ghetie et al., 2000, Annu Rev Immunol 18:739-766; Ravetch et al., 2001, Annu Rev Immunol 19:275-290). The cell-mediated reaction wherein nonspecific cytotoxic cells that express FcγRs recognize bound antibody on a target cell and subsequently cause phagocytosis of the target cell is referred to as antibody dependent cell-mediated phagocytosis (ADCP). All FcγRs bind the same region on Fc, at the N-terminal end of the Cg2 (CH2) domain and the preceding hinge. This interaction is well characterized structurally (Sondermann et al., 2001, J Mol Biol 309:737-749), and several structures of the human Fc bound to the extracellular domain of human FcγRIIIb have been solved (pdb accession code 1E4K) (Sondermann et al., 2000, Nature 406:267-273.) (pdb accession codes 1IIS and 1IIX) (Radaev et al., 2001, J Biol Chem 276:16469-16477.)

The different IgG subclasses have different affinities for the FcγRs, with IgG1 and IgG3 typically binding substantially better to the receptors than IgG2 and IgG4 (Jefferis et al., 2002, Immunol Lett 82:57-65). All FcγRs bind the same region on IgG Fc, yet with different affinities: the high affinity binder FcγRI has a K_{d} for IgG1 of 10⁻⁸ M⁻¹, whereas the low affinity receptors FcγRII and FcγRIII generally bind at 10⁻⁶ and 10⁻⁵ respectively. The extracellular domains of FcγRIIIa and FcγRIIIb are 96% identical; however, FcγRIIIb does not have an intracellular signaling domain. Furthermore, whereas FcγRI, FcγRIIa/c, and FcγRIIIa are positive regulators of immune complex-triggered activation, characterized by having an intracellular domain that has an immunoreceptor tyrosine-based activation motif (ITAM), FcγRIIb has an immunoreceptor tyrosine-based inhibition motif (ITIM) and is therefore inhibitory. Thus the former are referred to as activation receptors, and FcγRIIb is referred to as an inhibitory receptor. The receptors also differ in expression pattern and levels on different immune cells. Yet another level of complexity is the existence of a number of FcγR polymorphisms in the human proteome. A particularly relevant polymorphism with clinical significance is V158/F158 FcγRIIIa. Human IgG1 binds with greater affinity to the V158 allotype than to the F158 allotype. This difference in affinity, and presumably its effect on ADCC and/or ADCP, has been shown to be a significant determinant of the efficacy of the anti-CD20 antibody rituximab (Rituxan^{®}, a registered trademark of IDEC Pharmaceuticals Corporation). Subjects with the V158 allotype respond favorably to rituximab treatment; however, subjects with the lower affinity F158 allotype respond poorly (Cartron et al., 2002, Blood 99:754-758). Approximately 10-20% of humans are V158/V158 homozygous, 45% are V158/F158 heterozygous, and 35-45% of humans are F158/F158 homozygous (Lehrnbecher et al., 1999, Blood 94:4220-4232; Cartron et al., 2002, Blood 99:754-758). Thus 80-90% of humans are poor responders, that is, they have at least one allele of the F158 FcγRIIIa.

The Fc region is also involved in activation of the complement cascade. In the classical complement pathway, C1 binds with its C1q subunits to Fc fragments of IgG or IgM, which has formed a complex with antigen(s). In certain embodiments of the present disclosure, modifications to the Fc region comprise modifications that alter (either enhance or decrease) the ability of a Fzd-specific antibody as described herein to activate the complement system (see e.g., U.S. Patent No. 7,740,847). To assess complement activation, a complement-dependent cytotoxicity (CDC) assay may be performed (See, e.g., Gazzano-Santoro et al., J. Immunol. Methods, 202:163 (1996)).

Thus in certain embodiments, the present disclosure provides anti-Fzd antibodies having a modified Fc region with altered functional properties, such as reduced or enhanced CDC, ADCC, or ADCP activity, or enhanced binding affinity for a specific FcγR or increased serum half-life. Other modified Fc regions contemplated herein are described, for example, in issued U.S. Patent Nos. 7,317,091; 7,657,380; 7,662,925; 6,538,124; 6,528,624; 7,297,775; 7,364,731; published U.S. Application Nos. US2009092599; US20080131435; US20080138344; and published International Application Nos. WO2006/105338; WO2004/063351; WO2006/088494; WO2007/024249.

In certain embodiments, Wnt surrogate molecules comprise antibody variable domains with the desired binding specificities fused to immunoglobulin constant domain sequences. In certain embodiments, the fusion is with an Ig heavy chain constant domain, comprising at least part of the hinge, C_{H}2, and C_{H}3 regions. In particular embodiments, the first heavy-chain constant region (C_{H}1) containing the site necessary for light chain bonding, present in at least one of the fusions. DNAs encoding the immunoglobulin heavy chain fusions and, if desired, the immunoglobulin light chain, are inserted into separate expression vectors, and are co-transfected into a suitable host cell. This provides for greater flexibility in adjusting the mutual proportions of the three polypeptide fragments in embodiments when unequal ratios of the three polypeptide chains used in the construction provide the optimum yield of the desired bispecific antibody. It is, however, possible to insert the coding sequences for two or all three polypeptide chains into a single expression vector when the expression of at least two polypeptide chains in equal ratios results in high yields or when the ratios have no significant effect on the yield of the desired chain combination.

Wnt surrogate molecules disclosed herein may also be modified to include an epitope tag or label, e.g., for use in purification or diagnostic applications. There are many linking groups known in the art for making antibody conjugates, including, for example, those disclosed in U.S. Patent No. 5,208,020 or EP Patent 0 425 235 B1, and Chari et al., Cancer Research 52: 127-131 (1992). The linking groups include disulfide groups, thioether groups, acid labile groups, photolabile groups, peptidase labile groups, or esterase labile groups, as disclosed in the above-identified patents, disulfide and thioether groups being preferred.

In certain embodiments, anti-LRP5/6 antibodies and antigen-binding fragments thereof and/or anti-Fzd antibodies and antigen-binding fragments thereof present within a Wnt surrogate molecule are monoclonal. In certain embodiments, they are humanized.

The present disclosure further provides in certain embodiments an isolated nucleic acid encoding a polypeptide present in a Wnt surrogate molecule disclosed herein. Nucleic acids include DNA and RNA. These and related embodiments may include polynucleotides encoding antibody fragments that bind one or more Fzd receptors and/or LRP5 or LRP6 as described herein. The term "isolated polynucleotide" as used herein shall mean a polynucleotide of genomic, cDNA, or synthetic origin, or some combination thereof, which by virtue of its origin, the isolated polynucleotide: (1) is not associated with all or a portion of a polynucleotide in which the isolated polynucleotide is found in nature; (2) is linked to a polynucleotide to which it is not linked in nature, or (3) does not occur in nature as part of a larger sequence. An isolated polynucleotide may include naturally occurring and/or artificial sequences.

The term "operably linked" means that the components to which the term is applied are in a relationship that allows them to carry out their inherent functions under suitable conditions. For example, a transcription control sequence "operably linked" to a protein coding sequence is ligated thereto so that expression of the protein coding sequence is achieved under conditions compatible with the transcriptional activity of the control sequences.

The term "control sequence" as used herein refers to polynucleotide sequences that can affect expression, processing or intracellular localization of coding sequences to which they are ligated or operably linked. The nature of such control sequences may depend upon the host organism. In particular embodiments, transcription control sequences for prokaryotes may include a promoter, ribosomal binding site, and transcription termination sequence. In other particular embodiments, transcription control sequences for eukaryotes may include promoters comprising one or a plurality of recognition sites for transcription factors, transcription enhancer sequences, transcription termination sequences and polyadenylation sequences. In certain embodiments, "control sequences" can include leader sequences and/or fusion partner sequences.

The term "polynucleotide" as referred to herein means single-stranded or double-stranded nucleic acid polymers. In certain embodiments, the nucleotides comprising the polynucleotide can be ribonucleotides or deoxyribonucleotides or a modified form of either type of nucleotide. Said modifications include base modifications such as bromouridine, ribose modifications such as arabinoside and 2',3'-dideoxyribose and internucleotide linkage modifications such as phosphorothioate, phosphorodithioate, phosphoroselenoate, phosphorodiselenoate, phosphoroanilothioate, phoshoraniladate and phosphoroamidate. The term "polynucleotide" specifically includes single and double stranded forms of DNA.

The term "naturally occurring nucleotides" includes deoxyribonucleotides and ribonucleotides. The term "modified nucleotides" includes nucleotides with modified or substituted sugar groups and the like. The term "oligonucleotide linkages" includes oligonucleotide linkages such as phosphorothioate, phosphorodithioate, phosphoroselenoate, phosphorodiselenoate, phosphoroanilothioate, phoshoraniladate, phosphoroamidate, and the like. See, e.g., LaPlanche et al., 1986, Nucl. Acids Res., 14:9081; Stec et al., 1984, J. Am. Chem. Soc., 106:6077; Stein et al., 1988, Nucl. Acids Res., 16:3209; Zon et al., 1991, Anti-Cancer Drug Design, 6:539; Zon et al., 1991, OLIGONUCLEOTIDES AND ANALOGUES: A PRACTICAL APPROACH, pp. 87-108 (F. Eckstein, Ed.), Oxford University Press, Oxford England; Stec et al., U.S. Pat. No. 5,151,510; Uhlmann and Peyman, 1990, Chemical Reviews, 90:543, the disclosures of which are hereby incorporated by reference for any purpose. An oligonucleotide can include a detectable label to enable detection of the oligonucleotide or hybridization thereof.

The term "vector" is used to refer to any molecule (e.g., nucleic acid, plasmid, or virus) used to transfer coding information to a host cell. The term "expression vector" refers to a vector that is suitable for transformation of a host cell and contains nucleic acid sequences that direct and/or control expression of inserted heterologous nucleic acid sequences. Expression includes, but is not limited to, processes such as transcription, translation, and RNA splicing, if introns are present.

As will be understood by those skilled in the art, polynucleotides may include genomic sequences, extra-genomic and plasmid-encoded sequences and smaller engineered gene segments that express, or may be adapted to express, proteins, polypeptides, peptides and the like. Such segments may be naturally isolated, or modified synthetically by the skilled person.

As will be also recognized by the skilled artisan, polynucleotides may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide according to the present disclosure, and a polynucleotide may, but need not, be linked to other molecules and/or support materials. Polynucleotides may comprise a native sequence or may comprise a sequence that encodes a variant or derivative of such a sequence.

It will be appreciated by those of ordinary skill in the art that, as a result of the degeneracy of the genetic code, there are many nucleotide sequences that encodes an antibody as described herein. Some of these polynucleotides bear minimal sequence identity to the nucleotide sequence of the native or original polynucleotide sequence encoding a polypeptide within a Wnt surrogate molecule. Nonetheless, polynucleotides that vary due to differences in codon usage are expressly contemplated by the present disclosure. In certain embodiments, sequences that have been codon-optimized for mammalian expression are specifically contemplated.

Therefore, in another embodiment of the present disclosure, a mutagenesis approach, such as site-specific mutagenesis, may be employed for the preparation of variants and/or derivatives of the polypeptides described herein. By this approach, specific modifications in a polypeptide sequence can be made through mutagenesis of the underlying polynucleotides that encode them. These techniques provide a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments, the inventors contemplate the mutagenesis of the polynucleotide sequences that encode a polypeptide present in a Wnt surrogate molecule, to alter one or more properties of the encoded polypeptide, such as the binding affinity, or the function of a particular Fc region, or the affinity of the Fc region for a particular FcγR. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phages are readily commercially-available and their use is generally well-known to those skilled in the art. Double-stranded plasmids are also routinely employed in site directed mutagenesis that eliminates the step of transferring the gene of interest from a plasmid to a phage.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy et al., 1994; Segal, 1976; Prokop and Bajpai, 1991; Kuby, 1994; and Maniatis et al., 1982, each incorporated herein by reference, for that purpose.

In many embodiments, one or more nucleic acids encoding a polypeptide of a Wnt surrogate molecule are introduced directly into a host cell, and the cell incubated under conditions sufficient to induce expression of the encoded polypeptides. The Wnt surrogate polypeptides of this disclosure may be prepared using standard techniques well known to those of skill in the art in combination with the polypeptide and nucleic acid sequences provided herein. The polypeptide sequences may be used to determine appropriate nucleic acid sequences encoding the particular polypeptide disclosed thereby. The nucleic acid sequence may be optimized to reflect particular codon "preferences" for various expression systems according to standard methods well known to those of skill in the art.

According to certain related embodiments there is provided a recombinant host cell which comprises one or more constructs as described herein, e.g., a vector comprising a nucleic acid encoding a Wnt surrogate molecule or polypeptide thereof; and a method of production of the encoded product, which method comprises expression from encoding nucleic acid therefor. Expression may conveniently be achieved by culturing under appropriate conditions recombinant host cells containing the nucleic acid. Following production by expression, an antibody or antigen-binding fragment thereof, may be isolated and/or purified using any suitable technique, and then used as desired.

Polypeptides, and encoding nucleic acid molecules and vectors, may be isolated and/or purified, e.g., from their natural environment, in substantially pure or homogeneous form, or, in the case of nucleic acid, free or substantially free of nucleic acid or genes of origin other than the sequence encoding a polypeptide with the desired function. Nucleic acid may comprise DNA or RNA and may be wholly or partially synthetic. Reference to a nucleotide sequence as set out herein encompasses a DNA molecule with the specified sequence, and encompasses a RNA molecule with the specified sequence in which U is substituted for T, unless context requires otherwise.

Systems for cloning and expression of a polypeptide in a variety of different host cells are well known. Suitable host cells include bacteria, mammalian cells, yeast and baculovirus systems. Mammalian cell lines available in the art for expression of a heterologous polypeptide include Chinese hamster ovary cells, HeLa cells, baby hamster kidney cells, NSO mouse melanoma cells and many others. A common, preferred bacterial host is *E*. *coli.*

The expression of polypeptides, e.g., antibodies and antigen-binding fragments thereof, in prokaryotic cells such as *E*. *coli* is well established in the art. For a review, see for example Pluckthun, A. Bio/Technology 9: 545-551 (1991). Expression in eukaryotic cells in culture is also available to those skilled in the art as an option for production of antibodies or antigen-binding fragments thereof, see recent reviews, for example Ref, M. E. (1993) Curr. Opinion Biotech. 4: 573-576; Trill J. J. et al. (1995) Curr. Opinion Biotech 6: 553-560.

Suitable vectors can be chosen or constructed, containing appropriate regulatory sequences, including promoter sequences, terminator sequences, polyadenylation sequences, enhancer sequences, marker genes and other sequences as appropriate. Vectors may be plasmids, viral, e.g., phage, or phagemid, as appropriate. For further details see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Second Edition, Ausubel et al. eds., John Wiley & Sons, 1992, or subsequent updates thereto.

The term "host cell" is used to refer to a cell into which has been introduced, or which is capable of having introduced into it, a nucleic acid sequence encoding one or more of the herein described polypeptides, and which further expresses or is capable of expressing a selected gene of interest, such as a gene encoding any herein described polypeptide. The term includes the progeny of the parent cell, whether or not the progeny are identical in morphology or in genetic make-up to the original parent, so long as the selected gene is present. Accordingly there is also contemplated a method comprising introducing such nucleic acid into a host cell. The introduction may employ any available technique. For eukaryotic cells, suitable techniques may include calcium phosphate transfection, DEAE-Dextran, electroporation, liposome-mediated transfection and transduction using retrovirus or other virus, e.g. vaccinia or, for insect cells, baculovirus. For bacterial cells, suitable techniques may include calcium chloride transformation, electroporation and transfection using bacteriophage. The introduction may be followed by causing or allowing expression from the nucleic acid, e.g. by culturing host cells under conditions for expression of the gene. In one embodiment, the nucleic acid is integrated into the genome (e.g., chromosome) of the host cell. Integration may be promoted by inclusion of sequences which promote recombination with the genome, in accordance-with standard techniques.

The present disclosure also provides, in certain embodiments, a method which comprises using a construct as stated above in an expression system in order to express a particular polypeptide such as a Wnt mimetic molecule as described herein. The term "transduction" is used to refer to the transfer of genes from one bacterium to another, usually by a phage. "Transduction" also refers to the acquisition and transfer of eukaryotic cellular sequences by retroviruses. The term "transfection" is used to refer to the uptake of foreign or exogenous DNA by a cell, and a cell has been "transfected" when the exogenous DNA has been introduced inside the cell membrane. A number of transfection techniques are well known in the art and are disclosed herein. See, e.g., Graham et al., 1973, Virology 52:456; Sambrook et al., 2001, MOLECULAR CLONING, A LABORATORY MANUAL, Cold Spring Harbor Laboratories; Davis et al., 1986, BASIC METHODS IN MOLECULAR BIOLOGY, Elsevier; and Chu et al., 1981, Gene 13:197. Such techniques can be used to introduce one or more exogenous DNA moieties into suitable host cells.

The term "transformation" as used herein refers to a change in a cell's genetic characteristics, and a cell has been transformed when it has been modified to contain a new DNA. For example, a cell is transformed where it is genetically modified from its native state. Following transfection or transduction, the transforming DNA may recombine with that of the cell by physically integrating into a chromosome of the cell, or may be maintained transiently as an episomal element without being replicated, or may replicate independently as a plasmid. A cell is considered to have been stably transformed when the DNA is replicated with the division of the cell. The term "naturally occurring" or "native" when used in connection with biological materials such as nucleic acid molecules, polypeptides, host cells, and the like, refers to materials which are found in nature and are not manipulated by a human. Similarly, "non-naturally occurring" or "non-native" as used herein refers to a material that is not found in nature or that has been structurally modified or synthesized by a human.

The terms "polypeptide" "protein" and "peptide" and "glycoprotein" are used interchangeably and mean a polymer of amino acids not limited to any particular length. The term does not exclude modifications such as myristylation, sulfation, glycosylation, phosphorylation and addition or deletion of signal sequences. The terms "polypeptide" or "protein" means one or more chains of amino acids, wherein each chain comprises amino acids covalently linked by peptide bonds, and wherein said polypeptide or protein can comprise a plurality of chains non-covalently and/or covalently linked together by peptide bonds, having the sequence of native proteins, that is, proteins produced by naturally-occurring and specifically non-recombinant cells, or genetically-engineered or recombinant cells, and comprise molecules having the amino acid sequence of the native protein, or molecules having deletions from, additions to, and/or substitutions of one or more amino acids of the native sequence. The terms "polypeptide" and "protein" specifically encompass Wnt surrogate molecules, Fzd binding regions thereof, LRP5/6 binding regions thereof, antibodies and antigen-binding fragments thereof that bind to a Fzd receptor or a LRP5 or LRP6 receptor disclosed herein, or sequences that have deletions from, additions to, and/or substitutions of one or more amino acid of any of these polypeptides. Thus, a "polypeptide" or a "protein" can comprise one (termed "a monomer") or a plurality (termed "a multimer") of amino acid chains.

The term "isolated protein," "isolated Wnt surrogate molecule or "isolated antibody" referred to herein means that a subject protein, Wnt surrogate molecule, or antibody: (1) is free of at least some other proteins with which it would typically be found in nature; (2) is essentially free of other proteins from the same source, e.g., from the same species, (3) is expressed by a cell from a different species; (4) has been separated from at least about 50 percent of polynucleotides, lipids, carbohydrates, or other materials with which it is associated in nature; (5) is not associated (by covalent or noncovalent interaction) with portions of a protein with which the "isolated protein" is associated in nature; (6) is operably associated (by covalent or noncovalent interaction) with a polypeptide with which it is not associated in nature; or (7) does not occur in nature. Such an isolated protein can be encoded by genomic DNA, cDNA, mRNA or other RNA, or may be of synthetic origin, or any combination thereof. In certain embodiments, an isolated protein may comprise naturally-occurring and/or artificial polypeptide sequences. In certain embodiments, the isolated protein is substantially free from proteins or polypeptides or other contaminants that are found in its natural environment that would interfere with its use (therapeutic, diagnostic, prophylactic, research or otherwise).

Amino acid sequence modification(s) of any of the polypeptides (e.g., Wnt surrogate molecules or Fzd binding regions or LRP5/6 binding regions thereof) described herein are contemplated. For example, it may be desirable to improve the binding affinity and/or other biological properties of the Wnt surrogate molecule. For example, amino acid sequence variants of a Wnt surrogate molecule may be prepared by introducing appropriate nucleotide changes into a polynucleotide that encodes the antibody, or a chain thereof, or by peptide synthesis. Such modifications include, for example, deletions from, and/or insertions into and/or substitutions of, residues within the amino acid sequences of the antibody. Any combination of deletion, insertion, and substitution may be made to arrive at the final Wnt surrogate molecule, provided that the final construct possesses the desired characteristics (e.g., high affinity binding to one or more Fzd and/or LRP5/6 receptor). The amino acid changes also may alter post-translational processes of the antibody, such as changing the number or position of glycosylation sites. Any of the variations and modifications described above for polypeptides of the present disclosure may be included in antibodies of the present disclosure.

The present disclosure provides variants of any of the polypeptides (e.g., Wnt surrogate molecules or Fzd binding regions or LRP5/6 binding regions thereof, or antibodies or antigen-binding fragments thereof) disclosed herein. In certain embodiments, a variant has at least 90%, at least 95%, at least 98%, or at least 99% identity to a polypeptide disclosed herein. In certain embodiments, such variant polypeptides bind to one or more Fzd receptor, and/or to one or more LRP5/6 receptor, at least about 50%, at least about 70%, and in certain embodiments, at least about 90% as well as a Wnt surrogate molecule specifically set forth herein. In further embodiments, such variant Wnt surrogate molecules bind to one or more Fzd receptor, and/or to one or more LRP5/6 receptor, with greater affinity than the Wnt surrogate molecules set forth herein, for example, that bind quantitatively at least about 105%, 106%, 107%, 108%, 109%, or 110% as well as an antibody sequence specifically set forth herein.

In particular embodiments, the Wnt surrogate molecule or a binding region thereof, e.g., a Fab, scFv, or VHH or sdAb may comprise: a) a heavy chain variable region comprising: i. a CDR1 region that is identical in amino acid sequence to the heavy chain CDR1 region of a selected antibody described herein; ii. a CDR2 region that is identical in amino acid sequence to the heavy chain CDR2 region of the selected antibody; and iii. a CDR3 region that is identical in amino acid sequence to the heavy chain CDR3 region of the selected antibody; and/or b) a light chain variable domain comprising: i. a CDR1 region that is identical in amino acid sequence to the light chain CDR1 region of the selected antibody; ii. a CDR2 region that is identical in amino acid sequence to the light chain CDR2 region of the selected antibody; and iii. a CDR3 region that is identical in amino acid sequence to the light chain CDR3 region of the selected antibody; wherein the antibody specifically binds a selected target (e.g., one or more Fzd receptor epitopes or LRP5 or LRP6 receptors). In a further embodiment, the antibody, or antigen-binding fragment thereof, is a variant antibody or antigen-binding fragment thereof wherein the variant comprises a heavy and light chain identical to the selected antibody except for up to 8, 9, 10, 11, 12, 13, 14, 15, or more amino acid substitutions in the CDR regions of the VH and VL regions. In this regard, there may be 1, 2, 3, 4, 5, 6, 7, 8, or in certain embodiments, 9, 10, 11, 12, 13, 14, 15 more amino acid substitutions in the CDR regions of the selected antibody. Substitutions may be in CDRs either in the VH and/or the VL regions. (See, e.g., Muller, 1998, Structure 6:1153-1167).

In particular embodiments, the Wnt surrogate molecule or a binding region thereof, e.g., a Fab, scFv, or VHH or sdAb, may have: a) a heavy chain variable region having an amino acid sequence that is at least 80% identical, at least 95% identical, at least 90%, at least 95% or at least 98% or 99% identical, to the heavy chain variable region of an antibody or antigen-binding fragments thereof described herein; and/or b) a light chain variable region having an amino acid sequence that is at least 80% identical, at least 85%, at least 90%, at least 95% or at least 98% or 99% identical, to the light chain variable region of an antibody or antigen-binding fragments thereof described herein. The amino acid sequence of illustrative antigen-binding fragments thereof are set forth in SEQ ID NOs:1-97 and 109-157.

A polypeptide has a certain percent "sequence identity" to another polypeptide, meaning that, when aligned, that percentage of amino acids are the same when comparing the two sequences. Sequence similarity can be determined in a number of different manners. To determine sequence identity, sequences can be aligned using the methods and computer programs, including BLAST, available over the world wide web at ncbi.nlm.nih.gov/BLAST/. Another alignment algorithm is FASTA, available in the Genetics Computing Group (GCG) package, from Madison, Wis., USA, a wholly owned subsidiary of Oxford Molecular Group, Inc. Other techniques for alignment are described in Methods in Enzymology, vol. 266: Computer Methods for Macromolecular Sequence Analysis (1996), ed. Doolittle, Academic Press, Inc., a division of Harcourt Brace & Co., San Diego, Calif., USA. Of particular interest are alignment programs that permit gaps in the sequence. The Smith-Waterman is one type of algorithm that permits gaps in sequence alignments. See Meth. Mol. Biol. 70: 173-187 (1997). Also, the GAP program using the Needleman and Wunsch alignment method can be utilized to align sequences. See J. Mol. Biol. 48: 443-453 (1970)

Of interest is the BestFit program using the local homology algorithm of Smith and Waterman (Advances in Applied Mathematics 2: 482-489 (1981) to determine sequence identity. The gap generation penalty will generally range from 1 to 5, usually 2 to 4 and in many embodiments will be 3. The gap extension penalty will generally range from about 0.01 to 0.20 and in many instances will be 0.10. The program has default parameters determined by the sequences inputted to be compared. Preferably, the sequence identity is determined using the default parameters determined by the program. This program is available also from Genetics Computing Group (GCG) package, from Madison, Wis., USA.

Another program of interest is the FastDB algorithm. FastDB is described in Current Methods in Sequence Comparison and Analysis, Macromolecule Sequencing and Synthesis, Selected Methods and Applications, pp. 127-149, 1988, Alan R. Liss, Inc. Percent sequence identity is calculated by FastDB based upon the following parameters:
Mismatch Penalty: 1.00; Gap Penalty: 1.00; Gap Size Penalty: 0.33; and Joining Penalty: 30.0.

In particular embodiments, the Wnt surrogate molecule or a binding region thereof, e.g., a Fab, scFv, or VHH or sdAb may comprise: a) a heavy chain variable region comprising: i. a CDR1 region that is identical in amino acid sequence to the heavy chain CDR1 region of a selected antibody described herein; ii. a CDR2 region that is identical in amino acid sequence to the heavy chain CDR2 region of the selected antibody; and iii. a CDR3 region that is identical in amino acid sequence to the heavy chain CDR3 region of the selected antibody; and b) a light chain variable domain comprising: i. a CDR1 region that is identical in amino acid sequence to the light chain CDR1 region of the selected antibody; ii. a CDR2 region that is identical in amino acid sequence to the light chain CDR2 region of the selected antibody; and iii. a CDR3 region that is identical in amino acid sequence to the light chain CDR3 region of the selected antibody; wherein the antibody specifically binds a selected target (e.g., a Fzd receptor, such as Fzd1). In a further embodiment, the antibody, or antigen-binding fragment thereof, is a variant antibody wherein the variant comprises a heavy and light chain identical to the selected antibody except for up to 8, 9, 10, 11, 12, 13, 14, 15, or more amino acid substitutions in the CDR regions of the VH and VL regions. In this regard, there may be 1, 2, 3, 4, 5, 6, 7, 8, or in certain embodiments, 9, 10, 11, 12, 13, 14, 15 more amino acid substitutions in the CDR regions of the selected antibody. Substitutions may be in CDRs either in the VH and/or the VL regions. (See, e.g., Muller, 1998, Structure 6:1153-1167).

Determination of the three-dimensional structures of representative polypeptides (e.g., variant Fzd binding regions or LRP5/6 binding regions of Wnt surrogate molecules as provided herein) may be made through routine methodologies such that substitution, addition, deletion or insertion of one or more amino acids with selected natural or non-natural amino acids can be virtually modeled for purposes of determining whether a so derived structural variant retains the space-filling properties of presently disclosed species. See, for instance, Donate et al., 1994 Prot. Sci. 3:2378; Bradley et al., Science 309: 1868-1871 (2005); Schueler-Furman et al., Science 310:638 (2005); Dietz et al., Proc. Nat. Acad. Sci. USA 103:1244 (2006); Dodson et al., Nature 450:176 (2007); Qian et al., Nature 450:259 (2007); Raman et al. Science 327:1014-1018 (2010). Some additional non-limiting examples of computer algorithms that may be used for these and related embodiments, such as for rational design of binding regions include VMD which is a molecular visualization program for displaying, animating, and analyzing large biomolecular systems using 3-D graphics and built-in scripting (see the website for the Theoretical and Computational Biophysics Group, University of Illinois at Urbana-Champagne, at ks.uiuc.edu/Research/vmd/. Many other computer programs are known in the art and available to the skilled person and which allow for determining atomic dimensions from space-filling models (van der Waals radii) of energy-minimized conformations; GRID, which seeks to determine regions of high affinity for different chemical groups, thereby enhancing binding, Monte Carlo searches, which calculate mathematical alignment, and CHARMM (Brooks et al. (1983) J. Comput. Chem. 4:187-217) and AMBER (Weiner et al (1981) J. Comput. Chem. 106: 765), which assess force field calculations, and analysis (see also, Eisenfield et al. (1991) Am. J. Physiol. 261:C376-386; Lybrand (1991) J. Pharm. Belg. 46:49-54; Froimowitz (1990) Biotechniques 8:640-644; Burbam et al. (1990) Proteins 7:99-111; Pedersen (1985) Environ. Health Perspect. 61:185-190; and Kini et al. (1991) J. Biomol. Struct. Dyn. 9:475-488). A variety of appropriate computational computer programs are also commercially available, such as from Schrödinger (Munich, Germany).

### Compositions

Pharmaceutical compositions comprising a Wnt surrogate molecule described herein and one or more pharmaceutically acceptable diluent, carrier, or excipient are also disclosed. In particular embodiments, the pharmaceutical composition further comprises one or more Wnt polypeptides or Norrin polypeptides.

In further embodiments, pharmaceutical compositions comprising a polynucleotide comprising a nucleic acid sequence encoding a Wnt surrogate molecule described herein and one or more pharmaceutically acceptable diluent, carrier, or excipient are also disclosed. In particular embodiments, the pharmaceutical composition further comprises one or more polynucleotides comprising a nucleic acid sequence encoding a Wnt polypeptide or Norrin polypeptide. In certain embodiments, the polynucleotides are DNA or mRNA, e.g., a modified mRNA. In particular embodiments, the polynucleotides are modified mRNAs further comprising a 5' cap sequence and/or a 3' tailing sequence, e.g., a polyA tail. In other embodiments, the polynucleotides are expression cassettes comprising a promoter operatively linked to the coding sequences. In certain embodiments, the nucleic acid sequence encoding the Wnt surrogate molecule and the nucleic acid sequence encoding the Wnt polypeptide or Norrin polypeptide are present in the same polynucleotide.

In further embodiments, pharmaceutical compositions comprising an expression vector, e.g., a viral vector, comprising a polynucleotide comprising a nucleic acid sequence encoding a Wnt surrogate molecule described herein and one or more pharmaceutically acceptable diluent, carrier, or excipient are also disclosed. In particular embodiments, the pharmaceutical composition further comprises an expression vector, e.g., a viral vector, comprising a polynucleotide comprising a nucleic acid sequence encoding a Wnt polypeptide or Norrin polypeptide. In certain embodiments, the nucleic acid sequence encoding the Wnt surrogate molecule and the nucleic acid sequence encoding the Wnt polypeptide or Norrin polypeptide are present in the same polynucleotide, e.g., expression cassette.

The present disclosure further contemplates a pharmaceutical composition comprising a cell comprising an expression vector comprising a polynucleotide comprising a promoter operatively linked to a nucleic acid encoding a Wnt surrogate molecule and one or more pharmaceutically acceptable diluent, carrier, or excipient. In particular embodiments, the pharmaceutical composition further comprises a cell comprising an expression vector comprising a polynucleotide comprising a promoter operatively linked to a nucleic acid sequence encoding a Wnt polypeptide or a Norrin polypeptide. In certain embodiments, the nucleic acid sequence encoding the Wnt surrogate molecule and the nucleic acid sequence encoding the Wnt polypeptide or Norrin polypeptide are present in the same polynucleotide, e.g., expression cassette and/or in the same cell. In particular embodiments, the cell is a heterologous cell or an autologous cell obtained from the subject to be treated. In particular embodiments, the cell is a stem cell, e.g., an adipose-derived stem cell or a hematopoietic stem cell.

The present disclosure contemplates pharmaceutical compositions comprising a first molecule for delivery of a Wnt surrogate molecule as a first active agent and a second molecule for delivery of a Wnt polypeptide or Norrin polypeptide. The first and second molecule may be the same type of molecule or different types of molecules. For example, in certain embodiments, the first and second molecule may each be independently selected from the following types of molecules: polypeptides, small organic molecules, nucleic acids encoding the first or second active agent (optionally DNA or mRNA, optionally modified RNA), vectors comprising a nucleic acid sequence encoding the first or second active agent (optionally expression vectors or viral vectors), and cells comprising a nucleic acid sequence encoding the first or second active agent (optionally an expression cassette).

The subject molecules, alone or in combination, can be combined with pharmaceutically-acceptable carriers, diluents, excipients and reagents useful in preparing a formulation that is generally safe, non-toxic, and desirable, and includes excipients that are acceptable for mammalian, e.g., human or primate, use. Such excipients can be solid, liquid, semisolid, or, in the case of an aerosol composition, gaseous. Examples of such carriers, diluents and excipients include, but are not limited to, water, saline, Ringer's solutions, dextrose solution, and 5% human serum albumin. Supplementary active compounds can also be incorporated into the formulations. Solutions or suspensions used for the formulations can include a sterile diluent such as water for injection, saline solution, fixed oils, polyethylene glycols, glycerine, propylene glycol or other synthetic solvents; antibacterial compounds such as benzyl alcohol or methyl parabens; antioxidants such as ascorbic acid or sodium bisulfite; chelating compounds such as ethylenediaminetetraacetic acid (EDTA); buffers such as acetates, citrates or phosphates; detergents such as Tween 20 to prevent aggregation; and compounds for the adjustment of tonicity such as sodium chloride or dextrose. The pH can be adjusted with acids or bases, such as hydrochloric acid or sodium hydroxide. In particular embodiments, the pharmaceutical compositions are sterile.

Pharmaceutical compositions may further include sterile aqueous solutions or dispersions and sterile powders for the extemporaneous preparation of sterile injectable solutions or dispersion. For intravenous administration, suitable carriers include physiological saline, bacteriostatic water, or phosphate buffered saline (PBS). In some cases, the composition is sterile and should be fluid such that it can be drawn into a syringe or delivered to a subject from a syringe. In certain embodiments, it is stable under the conditions of manufacture and storage and is preserved against the contaminating action of microorganisms such as bacteria and fungi. The carrier can be, e.g., a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the internal compositions can be brought about by including in the composition an agent which delays absorption, for example, aluminum monostearate and gelatin.

Sterile solutions can be prepared by incorporating the anti-Fzd antibody or antigen-binding fragment thereof (or encoding polynucleotide or cell comprising the same) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, methods of preparation are vacuum drying and freeze-drying that yields a powder of the active ingredient plus any additional desired ingredient from a previously sterile-filtered solution thereof.

In one embodiment, the pharmaceutical compositions are prepared with carriers that will protect the antibody or antigen-binding fragment thereof against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially. Liposomal suspensions can also be used as pharmaceutically acceptable carriers. These can be prepared according to methods known to those skilled in the art.

It may be advantageous to formulate the pharmaceutical compositions in dosage unit form for ease of administration and uniformity of dosage. Dosage unit form as used herein refers to physically discrete units suited as unitary dosages for the subject to be treated; each unit containing a predetermined quantity of active antibody or antigen-binding fragment thereof calculated to produce the desired therapeutic effect in association with the required pharmaceutical carrier. The specification for the dosage unit forms are dictated by and directly dependent on the unique characteristics of the antibody or antigen-binding fragment thereof and the particular therapeutic effect to be achieved, and the limitations inherent in the art of compounding such an active antibody or antigen-binding fragment thereof for the treatment of individuals.

The pharmaceutical compositions can be included in a container, pack, or dispenser, e.g. syringe, e.g. a prefilled syringe, together with instructions for administration.

The pharmaceutical compositions of the present disclosure encompass any pharmaceutically acceptable salts, esters, or salts of such esters, or any other compound which, upon administration to an animal comprising a human, is capable of providing (directly or indirectly) the biologically active antibody or antigen-binding fragment thereof.

The present disclosure includes pharmaceutically acceptable salts of a Wnt surrogate molecule described herein. The term "pharmaceutically acceptable salt" refers to physiologically and pharmaceutically acceptable salts of the compounds of the present disclosure: i.e., salts that retain the desired biological activity of the parent compound and do not impart undesired toxicological effects thereto. A variety of pharmaceutically acceptable salts are known in the art and described, e.g., in "Remington's Pharmaceutical Sciences", 17th edition, Alfonso R. Gennaro (Ed.), Mark Publishing Company, Easton, PA, USA, 1985 (and more recent editions thereof), in the "Encyclopaedia of Pharmaceutical Technology", 3rd edition, James Swarbrick (Ed.), Informa Healthcare USA (Inc.), NY, USA, 2007, and in J. Pharm. Sci. 66: 2 (1977). Also, for a review on suitable salts, see "Handbook of Pharmaceutical Salts: Properties, Selection, and Use" by Stahl and Wermuth (Wiley-VCH, 2002).

Pharmaceutically acceptable base addition salts are formed with metals or amines, such as alkali and alkaline earth metals or organic amines. Metals used as cations comprise sodium, potassium, magnesium, calcium, and the like. Amines comprise N-N'-dibenzylethylenediamine, chloroprocaine, choline, diethanolamine, dicyclohexylamine, ethylenediamine, N-methylglucamine, and procaine (see, for example, Berge et al., "Pharmaceutical Salts," J. Pharma Sci., 1977, 66, 119). The base addition salts of said acidic compounds are prepared by contacting the free acid form with a sufficient amount of the desired base to produce the salt in the conventional manner. The free acid form may be regenerated by contacting the salt form with an acid and isolating the free acid in the conventional manner. The free acid forms differ from their respective salt forms somewhat in certain physical properties such as solubility in polar solvents, but otherwise the salts are equivalent to their respective free acid for purposes of the present disclosure.

In some embodiments, the pharmaceutical composition provided herein comprise a therapeutically effective amount of a Wnt surrogate molecule or pharmaceutically acceptable salt thereof in admixture with a pharmaceutically acceptable carrier, diluent and/or excipient, for example saline, phosphate buffered saline, phosphate and amino acids, polymers, polyols, sugar, buffers, preservatives and other proteins. Exemplary amino acids, polymers and sugars and the like are octylphenoxy polyethoxy ethanol compounds, polyethylene glycol monostearate compounds, polyoxyethylene sorbitan fatty acid esters, sucrose, fructose, dextrose, maltose, glucose, mannitol, dextran, sorbitol, inositol, galactitol, xylitol, lactose, trehalose, bovine or human serum albumin, citrate, acetate, Ringer's and Hank's solutions, cysteine, arginine, carnitine, alanine, glycine, lysine, valine, leucine, polyvinylpyrrolidone, polyethylene and glycol. Preferably, this formulation is stable for at least six months at 4° C.

In some embodiments, the pharmaceutical composition provided herein comprises a buffer, such as phosphate buffered saline (PBS) or sodium phosphate/sodium sulfate, tris buffer, glycine buffer, sterile water and other buffers known to the ordinarily skilled artisan such as those described by Good et al. (1966) Biochemistry 5:467. The pH of the buffer may be in the range of 6.5 to 7.75, preferably 7 to 7.5, and most preferably 7.2 to 7.4.

### Methods of Use

The present disclosure also provides methods for using the Wnt surrogate molecules disclosed herein, e.g., to modulate a Wnt signaling pathway, e.g., to increase Wnt signaling, and the administration of a Wnt surrogate molecule disclosed herein in a variety of therapeutic settings. Provided herein are methods of treatment using a Wnt surrogate molecule. In one embodiment, a Wnt surrogate molecule is provided to a subject having a disease involving inappropriate or deregulated Wnt signaling, e.g., reduced Wnt signaling.

### Agonizing Wnt Pathway Signaling and Related Therapeutic Methods

In certain embodiments, a Wnt surrogate molecule may be used to agonize a Wnt signaling pathway in a tissue or a cell. Agonizing the Wnt signaling pathway may include, for example, increasing Wnt signaling or enhancing Wnt signaling in a tissue or cell. Thus, in some aspects, the present disclosure provides a method for agonizing a Wnt signaling pathway in a cell, comprising contacting the tissue or cell with an effective amount of a Wnt surrogate molecule or pharmaceutically acceptable salt thereof disclosed herein, wherein the a Wnt surrogate molecule is a Wnt signaling pathway agonist. In some embodiments, contacting occurs in vitro, ex vivo, or in vivo. In particular embodiments, the cell is a cultured cell, and the contacting occurs in vitro. In certain embodiments, the method comprises further contacting the tissue or cell with one or more Wnt polypeptides or Norrin polypeptides.

In related aspects, the present disclosure provides a method for agonizing Wnt signaling in a tissue or cell, comprising contacting the tissue or cell with an effective amount of a polynucleotide comprising a Wnt surrogate molecule disclosed herein. In certain embodiments, the target tissue or cell is also contacted with a polynucleotide comprising a nucleic acid sequence that encodes a Wnt polypeptide or a Norrin polypeptide. In certain embodiments, the polynucleotides are DNA or mRNA, e.g., a modified mRNA. In particular embodiments, the polynucleotides are modified mRNAs further comprising a 5' cap sequence and/or a 3' tailing sequence, e.g., a polyA tail. In other embodiments, the polynucleotides are expression cassettes comprising a promoter operatively linked to the coding sequences. In certain embodiments, the nucleic acid sequence encoding the Wnt surrogate molecule and the nucleic acid sequence encoding the Wnt polypeptide or Norrin polypeptide are present in the same polynucleotide.

In related aspects, the present disclosure provides a method for agonizing Wnt signaling in a tissue or cell, comprising contacting the tissue or cell with an effective amount of a vector comprising a nucleic acid sequence encoding a Wnt surrogate molecule. In certain embodiments, the tissue or cell is also contacted with a vector comprising a nucleic acid sequence that encodes a Wnt polypeptide or a Norrin polypeptide. In certain embodiments, the vector is an expression vector, and may comprise a promoter operatively linked to the nucleic acid sequence. In particular embodiments, the vector is a viral vector. In certain embodiments, the nucleic acid sequence encoding a Wnt surrogate molecule and the nucleic acid sequence encoding the Wnt polypeptide or Norrin polypeptide are present in the same vector, e.g., in the same expression cassette.

In related aspects, the present disclosure provides a method for agonizing Wnt signaling in a tissue, comprising contacting the tissue with an effective amount of a cell comprising a nucleic acid sequence encoding a Wnt surrogate molecule of the present disclosure. In certain embodiments, the tissue is also contacted with a cell comprising a nucleic acid sequence that encodes a Wnt polypeptide or Norrin polypeptide. In certain embodiments, the nucleic acid sequence encoding the Wnt surrogate molecule and the nucleic acid sequence encoding the Wnt polypeptide or Norrin polypeptide are present in the same cell. In particular embodiments, the cell is a heterologous cell or an autologous cell obtained from the subject to be treated. In certain embodiments, the cell was transduced with a vector comprising an expression cassette encoding the Wnt surrogate molecule or the Wnt polypeptide or Norrin polypeptide. In particular embodiments, the cell is a stem cell, e.g., an adipose-derived stem cell or a hematopoietic stem cell.

Wnt surrogate molecules disclosed herein may be used in to treat a disease, disorder or condition, for example, by agonizing, e.g., increasing Wnt signaling in a targeted cell, tissue or organ. Thus, in some aspects, the present disclosure provides a method for treating a disease or condition in a subject in need thereof, e.g., a disease or disorder associated with reduced or impaired Wnt signaling, and/or for which increased Wnt signaling would provide a therapeutic benefit, comprising contacting the subject with an effective amount of a composition of the present disclosure. In particular embodiments, the composition is a pharmaceutical composition comprising any of: a Wnt surrogate molecule; a polynucleotide comprising a nucleic acid sequence encoding a Wnt surrogate molecule, e.g., a DNA or mRNA, optionally a modified mRNA; a vector comprising a nucleic acid sequence encoding a Wnt surrogate molecule, e.g., an expression vector or viral vector; or a cell comprising a nucleic acid sequence encoding a Wnt surrogate molecule, e.g., a cell transduced with an expression vector or viral vector encoding a Wnt surrogate molecule. In particular embodiments, the disease or condition is a pathological disease or disorder, or an injury, e.g., an injury resulting from a wound. In certain embodiments, the wound may be the result of another therapeutic treatment. In certain embodiments, the disease or condition comprises impaired tissue repair, healing or regeneration, or would benefit from increased tissue repair, healing or regeneration. In some embodiments, contacting occurs in vivo, i.e., the subject composition is administered to a subject.

In certain embodiments, the method comprises further contacting the subject with a pharmaceutical composition comprising one or more Wnt polypeptides or Norrin polypeptides. The present disclosure contemplates contacting a subject with a first molecule for delivery of a Wnt surrogate molecule as a first active agent and a second molecule for delivery of a Wnt polypeptide or Norrin polypeptide. The first and second molecule may be the same type of molecule or different types of molecules. For example, in certain embodiments, the first and second molecule may each be independently selected from the following types of molecules: polypeptides, small organic molecules, nucleic acids encoding the first or second active agent (optionally DNA or mRNA, optionally modified RNA), vectors comprising a nucleic acid sequence encoding the first or second active agent (optionally expression vectors or viral vectors), and cells comprising a nucleic acid sequence encoding the first or second active agent (optionally an expression cassette).

In related aspects, the present disclosure provides a method for treating a disease or condition, e.g., a disease or disorder associated with reduced Wnt signaling, or for which increased Wnt signaling would provide a therapeutic benefit, comprising contacting a subject in need thereof with a pharmaceutical composition comprising an effective amount of a polynucleotide comprising a nucleic acid sequence encoding a Wnt surrogate molecule disclosed herein. In certain embodiments, the subject is also contacted with a pharmaceutical composition comprising an effective amount of a polynucleotide comprising a nucleic acid sequence that encodes a Wnt polypeptide or a Norrin polypeptide. In certain embodiments, the polynucleotides are DNA or mRNA, e.g., a modified mRNA. In particular embodiments, the polynucleotides are modified mRNAs further comprising a 5' cap sequence and/or a 3' tailing sequence, e.g., a polyA tail. In other embodiments, the polynucleotides are expression cassettes comprising a promoter operatively linked to the coding sequences. In certain embodiments, the nucleic acid sequence encoding the Wnt surrogate molecule and the nucleic acid sequence encoding the Wnt polypeptide or Norrin polypeptide are present in the same polynucleotide.

**In** related aspects, the present disclosure provides a method for treating a disease or condition, e.g., a disease or disorder associated with reduced Wnt signaling, or for which increased Wnt signaling would provide a therapeutic benefit, comprising contacting a subject in need thereof with a pharmaceutical composition comprising an effective amount of a vector comprising a nucleic acid sequence encoding a Wnt surrogate molecule. **In** certain embodiments, the subject is also contacted with a pharmaceutical composition comprising an effective amount of a vector comprising a nucleic acid sequence that encodes a Wnt polypeptide or a Norrin polypeptide. **In** certain embodiments, the vector is an expression vector, and may comprise a promoter operatively linked to the nucleic acid sequence. **In** particular embodiments, the vector is a viral vector. **In** certain embodiments, the nucleic acid sequence encoding the Wnt surrogate molecule and the nucleic acid sequence encoding the Wnt polypeptide or Norrin polypeptide are present in the same vector, e.g., in the same expression cassette.

**In** related aspects, the present disclosure provides a method for treating a disease or condition, e.g., a disease or disorder associated with reduced Wnt signaling, or for which increased Wnt signaling would provide a therapeutic benefit, comprising contacting a subject in need thereof with a pharmaceutical composition comprising an effective amount of a cell comprising a nucleic acid sequence encoding a Wnt surrogate molecule. **In** certain embodiments, the subject is also contacted with a cell comprising a nucleic acid sequence that encodes a Wnt polypeptide or a Norrin polypeptide. **In** certain embodiments, the nucleic acid sequence encoding the Wnt surrogate molecule and the nucleic acid sequence encoding the Wnt polypeptide or Norrin polypeptide are present in the same cell. In particular embodiments, the cell is a heterologous cell or an autologous cell obtained from the subject to be treated. **In** certain embodiments, the cell was transduced with a vector comprising an expression cassette encoding the Wnt surrogate molecule or the Wnt polypeptide or Norrin polypeptide. **In** particular embodiments, the cell is a stem cell, e.g., an adipose-derived stem cell or a hematopoietic stem cell.

Wnt signaling plays key roles in the developmental process and maintenance of stem cells. Reactivation of Wnt signals is associated with regeneration and repair of most tissues after injuries and diseases. Wnt surrogate molecule molecules are expected to provide benefit of healing and tissue repair in response to injuries and diseases. Causes of tissue damage and loss include but are not limited to aging, degeneration, hereditary conditions, infection and inflammation, traumatic injuries, toxins/metabolic-induced toxicities, or other pathological conditions. Wnt signals and enhancers of Wnt signals have been shown to activate adult, tissue-resident stem cells. **In** some embodiments, the compounds of the present disclosure are administered for use in treating diseased or damaged tissue, for use in tissue regeneration and for use in cell growth and proliferation, and/or for use in tissue engineering.

Human diseases associated with mutations of the Wnt pathway provide strong evidence for enhancement of Wnt signals in the treatment and prevention of diseases. Preclinical in vivo and in vitro studies provide additional evidence of involvement of Wnt signals in many disease conditions and further support utilization of a Wnt surrogate molecule in various human diseases. For example, compositions of the present disclosure may be used to promote or increase bone growth or regeneration, bone grafting, healing of bone fractures, stress fractures, vertebral compression fractures, treatment of osteoporosis and osteoporotic fractures, spinal fusion, osseointegration of orthopedic devices, tendon-bone integration, tooth growth and regeneration, dental implantation, periodontal diseases, maxillofacial reconstruction, and osteonecrosis of the jaw. They may also be used in the treatment of alopecia; enhancing regeneration of sensory organs, e.g. treatment of hearing loss, treatment of vestibular hypofunction, treatment of macular degeneration, treatment of vitreoretinopathy, other diseases of retinal degeneration, Fuchs' dystrophy, other cornea disease, etc.; treatment of stroke, traumatic brain injury, Alzheimer's disease, multiple sclerosis, muscular dystrophy, muscle atrophy caused by sarcopenia or cachexia, and other conditions affecting the blood brain barrier; treatment of spinal cord injuries, other spinal cord diseases. The compositions of this present disclosure may also be used in treatment of oral mucositis, treatment of short bowel syndrome, inflammatory bowel diseases (IBD), other gastrointestinal disorders; treatment of metabolic syndrome; treatment of diabetes, dyslipidemia, treatment of pancreatitis, conditions where exocrine or endocrine pancreas tissues are damaged; conditions where enhanced epidermal regeneration is desired, e.g., epidermal wound healing, treatment of diabetic foot ulcers, syndromes involving tooth, nail, or dermal hypoplasia, etc., conditions where angiogenesis is beneficial; treatment of myocardial infarction, coronary artery disease, heart failure; enhanced growth of hematopoietic cells, e.g. enhancement of hematopoietic stem cell transplants from bone marrow, mobilized peripheral blood, treatment of immunodeficiencies, graft versus host diseases, etc.; treatment of acute kidney injuries, chronic kidney diseases; treatment of lung diseases, chronic obstructive pulmonary diseases (COPD), idiopathic pulmonary fibrosis (IPF), enhanced regeneration of lung tissues. The compositions of the present disclosure may also be used in enhanced regeneration of liver cells, e.g. liver regeneration, treatment of cirrhosis, enhancement of liver transplantations, treatment of acute liver failure, treatment of chronic liver diseases with hepatitis C or B virus infection or post-antiviral drug therapies, alcoholic liver diseases, alcoholic hepatitis, non-alcoholic liver diseases with steatosis or steatohepatitis (NASH), and the like. The compositions of this present disclosure may treat diseases and disorders including, without limitation, conditions in which regenerative cell growth is desired.

Human genetics involving loss-of-function or gain-of-function mutations in Wnt signaling components show strong evidence supporting enhancing Wnt signals for bone growth. Conditions in which enhanced bone growth is desired may include, without limitation, fractures, grafts, ingrowth around prosthetic devices, osteoporosis, osteoporotic fractures, spinal fusion, osteonecrosis of the jaw, dental implantation, periodontal diseases, maxillofacial reconstruction, and the like. Wnt surrogate molecules enhance and promotes Wnt signals which are critical in promoting bone regeneration. Methods for regeneration of bone tissues benefit from administration of the compounds of the present disclosure, which can be systemic or localized. In some embodiments, bone marrow cells are exposed to molecules of the present disclosure, such that stem cells within that marrow become activated.

In some embodiments, bone regeneration is enhanced by contacting a responsive cell population, e.g., bone marrow, bone progenitor cells, bone stem cells, etc. with an effective dose of a Wnt surrogate molecule disclosed herein. Methods for regeneration of bone tissues benefit from administration of the Wnt surrogate molecule which can be systemic or localized. In some such embodiments, the contacting is performed in vivo. In other such embodiments, the contacting is performed ex vivo. The molecule may be localized to the site of action, e.g. by loading onto a matrix, which is optionally biodegradable, and optionally provides for a sustained release of the active agent. Matrix carriers include, without limitation, absorbable collagen sponges, ceramics, hydrogels, polymeric microspheres, nanoparticles, bone cements, and the like.

In particular embodiments, compositions comprising one or more Wnt surrogate molecule disclosed herein (or a polynucleotide encoding a Wnt surrogate molecule, or a vector or cell comprising a polynucleotide encoding a Wnt surrogate molecule) are used to treat or prevent a bone disease or disorder, including but not limited to any of the following, or to treat or prevent an injury associated with, but not limited to, any of the following: osteoporosis, osteoporotic fractures, bone fractures, non-union fractures, delayed union fractures, spinal fusion, osteonecrosis, osteonecrosis of the jaw, hip, femoral head, etc., osseointegration of implants (e.g., to accelerate recovery following partial or total knee or hip replacement), osteogenesis imperfecta, bone grafts, tendon repair, maxillofacial surgery, dental implant, all other bone disorders or defects resulting from genetic diseases, degeneration, aging, drugs, or injuries. In one embodiment, Wnt surrogate molecules that bind Fzd1, Fzd 2, and Fzd 7, and also LRP5 and/or LRP6, are used to treat or prevent any bone disease or disorder. In one embodiment, Wnt surrogate molecules that bind Fzd1, Fzd 2, Fzd 5, Fzd 7 and Fzd 8, and also LRP5 and/or LRP6, are used to treat or prevent any bone disease or disorder.

In particular embodiments, compositions and methods disclosed herein may be used to: increase bone mineral density, increase bone volume (e.g., tibia and/or femur bone volume), increase cortical thickness (e.g., in trabecular region or in femur mid-diaphysis), increase mineral apposition rate, increase the number of osteblasts and/or decrease the number of osteoclasts (e.g., in bone), increase bone stiffness, increase the ultimate load to fracture point, improve bone resistance to fracture, decrease bone loss associated with osteoporosis, or increase biochemical strength of bone, in a subject. In one embodiment, Wnt surrogate molecules that bind Fzd1, Fzd 2, and Fzd 7 are used for any of these indicated uses. In one embodiment, Wnt surrogate molecules that bind Fzd1, Fzd 2, Fzd 5, Fzd 7 and Fzd 8 are used for any of these indicated uses.

Compositions comprising one or more Wnt surrogate molecule disclosed herein (or a polynucleotide encoding a Wnt surrogate molecule, or a vector or cell comprising a polynucleotide encoding a Wnt surrogate molecule) can be used for the in vivo treatment of skeletal tissue deficiencies. By "skeletal tissue deficiency", it is meant a deficiency in bone or other skeletal connective tissue at any site where it is desired to restore the bone or connective tissue, no matter how the deficiency originated, e.g. whether as a result of surgical intervention, removal of tumor, ulceration, implant, fracture, or other traumatic or degenerative conditions. The compositions of the present disclosure can be used as part of a regimen for restoring cartilage function to a connective tissue, for the repair of defects or lesions in cartilage tissue such as degenerative wear and arthritis, trauma to the tissue, displacement of torn meniscus, meniscectomy, a luxation of a joint by a torn ligament, malalignment of joints, bone fracture, or by hereditary disease.

A Wnt surrogate molecule may also be used for treatment of periodontal diseases. Periodontal diseases are a leading cause of tooth loss and are linked to multiple systemic conditions. In some embodiments, tooth or underlying bone regeneration is enhanced by contacting a responsive cell population. In some such embodiments, the contacting is performed in vivo. In other such embodiments, the contacting is performed ex vivo, with subsequent implantation of the activated stem or progenitor cells. The molecule may be localized to the site of action, e.g. by loading onto a matrix, which is optionally biodegradable, and optionally provides for a sustained release of the active agent. Matrix carriers include, without limitation, absorbable collagen sponges, ceramics, hydrogels, bone cements, polymeric microspheres, nanoparticles, and the like.

Studies have shown that biology of Wnt signaling and R-spondins are capable of promoting sensory hair cell regeneration in the inner ear following injuries, aging, or degeneration. Loss of sensory hair cells in the inner ear involved in hearing loss or vestibular hypofunction may also benefit from the compositions of the present disclosure. In the inner ear, the auditory organ houses mechanosensitive hair cells required for translating sound vibration to electric impulses. The vestibular organs, comprised of the semicircular canals (SSCs), the utricle, and the saccule, also contain sensory hair cells in order to detect head position and motion. Compositions of the present disclosure can be used, for example, in an infusion; in a matrix or other depot system; or other topical application to the ear for enhancement of auditory regeneration.

A Wnt surrogate molecule may also be used in regeneration of retinal tissue. In the adult mammalian retina, Muller glia cells are capable of regenerating retinal cells, including photoreceptors, for example after neurotoxic injury in vivo. Wnt signaling and enhancers of Wnt signals can promote proliferation of Muller glia-derived retinal progenitors after damage or during degeneration. The compositions of the present disclosure may also be used in the regeneration of tissues and other cell types in the eye. For examples age-related macular degeneration (AMD), other retina degenerative diseases, cornea diseases, Fuchs' dystrophy, vitreoretinopathy, hereditary diseases, etc. can benefit from the compositions of the present disclosure. AMD is characterized by progressively decreased central vision and visual acuity. Fuchs' dystrophy is characterized by progressive loss of cornea endothelial cells. Wnt signal and enhancing of Wnt signal can promote regeneration of cornea endothelium, retina epithelium, etc. in the eye tissue. In other embodiments, compositions of the present disclosure can be used, for example, in an infusion; in a matrix or other depot system; or other topical application to the eye for retinal regeneration and treatment of macular degeneration.

Specific populations of proliferating cells for homeostatic renewal of hepatocytes have been identified through lineage tracing studies, for example Axin2-positive cells in peri-central region. Lineage tracing studies also identified additional potential liver progenitor cells, including but not limited to Lgr-positive cells. The self-renewing liver cells and other populations of potential progenitor cells, including Lgr5-positive and Axin2-positive cells, are identified to be capable of regeneration responding to Wnt signals and/or R-spondins following injuries. Numerous preclinical models of acute liver injury and failure and chronic liver diseases showed recovery and regeneration of hepatocytes benefit from enhancing Wnt signals.

In certain embodiments, compositions comprising a Wnt surrogate molecule disclosed herein (or a polynucleotide encoding a Wnt surrogate molecule, or a vector or cell comprising a polynucleotide encoding a Wnt surrogate molecule) are used to promote liver regeneration, reduce fibrosis, and/or improve liver function. In certain embodiments, compositions and methods disclosed herein are used to: increase liver weight, increase the liver to body weight ratio, increase the number of PCNA and pH3 positive nuclei in liver, increase expression of Ki67 and/or Cyclin D1 in liver, increase liver cell proliferation and/or mitosis, decrease fibrosis following chronic liver injury, or increase hepatocyte function.

In particular embodiments, the compositions of this disclosure may be used in treatment of acute liver failure, acute alcoholic liver injuries, treatment of chronic liver diseases with hepatitis C or B virus infection or post-antiviral drug therapies, chronic alcoholic liver diseases, non-alcoholic fatty liver diseases and non-alcoholic steatohepatitis (NASH), treatment of cirrhosis and severe chronic liver diseases of all causes, and enhanced regeneration of liver cells. Methods for regeneration of liver tissue benefit from administration of the compounds of the present disclosure, which can be systemic or localized. These include, but are not limited to, methods of systemic administration and methods of localized administration e.g. by injection into the liver tissue, by injection into veins or blood vessels leading into the liver, by implantation of a sustained release formulation, and the like.

In particular embodiments, compositions comprising a Wnt surrogate molecule disclosed herein (or a polynucleotide encoding a Wnt surrogate molecule, or a vector or cell comprising a polynucleotide encoding a Wnt surrogate molecule) are used to treat or prevent a liver disease or disorder, including but not limited to, or to treat or prevent a liver injury or disorder resulting from any of the following: acute liver failure (all causes), chronic liver failure (all causes), cirrhosis, liver fibrosis (all causes), portal hypertension, nonalcoholic steatohepatisis (NASH), nonalcoholic fatty liver disease (NAFLD) (fatty liver), alcoholic hepatitis, hepatitis C virus-induced liver diseases (HCV), hepatitis B virus-induced liver diseases (HBV), other viral hepatitis (e.g., hepatitis A virus-induced liver diseases (HAV) and hepatitis D virus-induced liver diseases (HDV)), primary biliary cirrhosis, autoimmune hepatitis, livery surgery, liver injury, liver transplantation, "small for size" syndrome in liver surgery and transplantation, congenital liver disease and disorders, any other liver disorder or defect resulting from genetic diseases, degeneration, aging, drugs, or injuries.

Wnt signals play an important role in regeneration of various epithelial tissues. Various epidermal conditions benefit from treatment with the compounds of the present disclosure. Mucositis occurs when there is a breakdown of the rapidly divided epithelial cells lining the gastro-intestinal tract, leaving the mucosal tissue open to ulceration and infection. The part of the epithelial lining that covers the mouth, called the oral mucosa, is one of the most sensitive parts of the body and is particularly vulnerable to chemotherapy and radiation. Oral mucositis is probably the most common, debilitating complication of cancer treatments, particularly chemotherapy and radiation. In addition, the compositions of the present disclosure may also benefit treatment of short bowel syndrome, inflammatory bowel diseases (IBD), or other gastrointestinal disorders. Other epidermal conditions include epidermal wound healing, diabetic foot ulcers, syndromes involving tooth, nail, or dermal hypoplasia, and the like. Molecules of the present disclosure may be used in all these conditions, where regenerative cells are contacted with compounds of the present disclosure. Methods for regeneration of epithelial tissues benefit from administration of the compounds of the present disclosure, which can be systemic or localized. Contacting can be, for example, topical, including intradermal, subdermal, in a gel, lotion, cream etc. applied at targeted site, etc.

In addition to skin and gastrointestinal tract, Wnt signals and enhancement and promotion of Wnt signals also play an important role in repair and regeneration of tissues including pancreas, kidney, and lung in preclinical models. A Wnt surrogate molecule may benefit various disease conditions involving exocrine and endocrine pancreas, kidney, or lung. The Wnt surrogate molecules may be used in treatment of metabolic syndrome; treatment of diabetes, treatment of acute or chronic pancreatitis, exocrine pancreatic insufficiency, treatment of acute kidney injuries, chronic kidney diseases, treatment of lung diseases, including but not limited to chronic obstructive pulmonary diseases (COPD), other conditions that cause loss of lung epithelial tissues. Methods for regeneration of these tissues benefit from administration of the compounds of the present disclosure, which can be systemic or localized.

Epidermal Wnt signaling, in coordination with signaling via other development factors, is critical for adult hair follicle regeneration. Hair loss is a common problem, and androgenetic alopecia, often called male pattern baldness, is the most common form of hair loss in men. In some embodiments, hair follicle regeneration is enhanced by contacting a responsive cell population with a molecule of the present disclosure. In some such embodiments, the contacting is performed in vivo. In other such embodiments, the contacting is performed ex vivo. The molecule may be localized to the site of action, e.g. topical lotions, gels, creams and the like.

Stroke, traumatic brain injury, Alzheimer's disease, multiple sclerosis and other conditions affecting the blood brain barrier (BBB) may be treated with a Wnt surrogate molecule. Angiogenesis is critical to ensure the supply of oxygen and nutrients to many tissues throughout the body, and is especially important for the CNS as the neural tissue is extremely sensitive to hypoxia and ischemia. CNS endothelial cells which form the BBB differ from endothelial cells in non-neural tissue, in that they are highly polarized cells held together by tight junctions and express specific transporters. Wnt signaling regulates CNS vessel formation and/or function. Conditions in which the BBB is compromised can benefit from administration of the compounds of the present disclosure, which can be systemic or localized e.g. by direct injection, intrathecal administration, implantation of sustained release formulations, and the like. In addition, Wnt signal is actively involved in neurogenesis and plays a role of neuroprotection following injury. The compositions of the present disclosure may also be used in treatment of spinal cord injuries, other spinal cord diseases, stroke, traumatic brain injuries, etc.

Wnt signals also play a role in angiogenesis. A Wnt surrogate molecule may benefit conditions where angiogenesis is beneficial, treatment of myocardial infarction, coronary artery disease, heart failure, etc., and conditions from hereditary diseases. Methods for regeneration of these tissues benefit from administration of the compounds of the present disclosure, which can be systemic or localized.

In certain embodiments, methods of the present disclosure promote tissue regeneration, e.g., in a tissue subjected to damage or tissue or cell reduction or loss. The loss or damage can be anything which causes the cell number to diminish, including diseases or injuries. For example, an accident, an autoimmune disorder, a therapeutic side-effect or a disease state could constitute trauma. Tissue regeneration increases the cell number within the tissue and preferably enables connections between cells of the tissue to be re-established, and more preferably the functionality of the tissue to be regained.

The terms "administering" or "introducing" or "providing", as used herein, refer to delivery of a composition to a cell, to cells, tissues and/or organs of a subject, or to a subject. Such administering or introducing may take place in vivo, in vitro or ex vivo.

In particular embodiments, a pharmaceutical composition is administered parenterally, e.g., intravenously, orally, rectally, or by injection. In some embodiments, it is administered locally, e.g., topically or intramuscularly. In some embodiments, a composition is administered to target tissues, e.g., to bone, joints, ear tissue, eye tissue, gastrointestinal tract, skin, a wound site or spinal cord. Methods of the present disclosure may be practiced in vivo or ex vivo. In some embodiments, the contacting of a target cell or tissue with a Wnt surrogate molecule is performed ex vivo, with subsequent implantation of the cells or tissues, e.g., activated stem or progenitor cells, into the subject. The skilled artisan can determine an appropriate site of and route of administration based on the disease or disorder being treated.

The dose and dosage regimen may depend upon a variety of factors readily determined by a physician, such as the nature of the disease or disorder, the characteristics of the subject, and the subject's history. In particular embodiments, the amount of a Wnt surrogate molecule administered or provided to the subject is in the range of about 0.01 mg/kg to about 50 mg/kg, 0.1 mg/kg to about 500 mg/kg, or about 0.1 mg/kg to about 50 mg/kg of the subject's body weight.

The terms "treatment", "treating" and the like are used herein to generally mean obtaining a desired pharmacologic and/or physiologic effect. The effect may be prophylactic in terms of completely or partially preventing a disease or symptom thereof, e.g. reducing the likelihood that the disease or symptom thereof occurs in the subject, and/or may be therapeutic in terms of a partial or complete cure for a disease and/or adverse effect attributable to the disease. "Treatment" as used herein covers any treatment of a disease in a mammal, and includes: (a) preventing the disease from occurring in a subject which may be predisposed to the disease but has not yet been diagnosed as having it; (b) inhibiting the disease, i.e., arresting its development; or (c) relieving the disease, i.e., causing regression of the disease. The therapeutic agent (e.g., a Wnt surrogate molecule) may be administered before, during or after the onset of disease or injury. The treatment of ongoing disease, where the treatment stabilizes or reduces the undesirable clinical symptoms of the patient, is of particular interest. Such treatment is desirably performed prior to complete loss of function in the affected tissues. The subject therapy will desirably be administered during the symptomatic stage of the disease, and in some cases after the symptomatic stage of the disease. In some embodiments, the subject method results in a therapeutic benefit, e.g., preventing the development of a disorder, halting the progression of a disorder, reversing the progression of a disorder, etc. In some embodiments, the subject method comprises the step of detecting that a therapeutic benefit has been achieved. The ordinarily skilled artisan will appreciate that such measures of therapeutic efficacy will be applicable to the particular disease being modified, and will recognize the appropriate detection methods to use to measure therapeutic efficacy.

Other embodiments relate, in part, to the use of the Wnt surrogate molecules disclosed herein to promote or enhance the growth or proliferation of cells, tissues and organoids, for example, by contacting cells or tissue with one or more Wnt surrogate, optionally in combination with a Norrin or Rspondin polypeptide. In certain embodiments, the cells or tissue are contacted ex vivo, in vitro, or in vivo. Such methods may be used to generate cells, tissue or organoids for therapeutic use, e.g., to be transplanted or grafted into a subject. They may also be used to generate cells, tissue or organoids for research use. The Wnt surrogate molecules have widespread applications in non-therapeutic methods, for example in vitro research methods.

The present disclosure provides a method for tissue regeneration of damaged tissue, such as the tissues discussed above, comprising administering a Wnt surrogate molecule to cells. The Wnt surrogate molecule may be administered directly to the cells in vivo, administered to a subject orally, intravenously, or by other methods known in the art, or administered to ex vivo cells. In some embodiments where the Wnt surrogate molecule is administered to ex vivo cells, these cells may be transplanted into a subject before, after or during administration of the Wnt surrogate molecule.

Wnt signaling is a key component of stem cell culture. For example, the stem cell culture media as described in WO2010/090513, WO2012/014076, Sato et al., 2011 (GASTROENTEROLOGY 201 1; 141: 1762-1772) and Sato et al., 2009 (Nature 459, 262-5). The Wnt surrogate molecules disclosed herein are suitable alternatives to Rspondin for use in these stem cell culture media, or may be combined with Rspondin.

Accordingly, in one embodiment, the disclosure provides a method for enhancing the proliferation of stem cells comprising contacting stem cells with one or more Wnt surrogate molecules disclosed herein. In one embodiment, the disclosure provides a cell culture medium comprising one or more Wnt surrogate molecules disclosed herein. In some embodiments, the cell culture medium may be any cell culture medium already known in the art that normally comprises Wnt or Rspondin, but wherein the Wnt or Rspondin is replaced (wholly or partially) or supplemented by Wnt surrogate molecule(s) disclosed herein. For example, the culture medium may be as described in as described in WO2010/090513, WO2012/014076, Sato et al., 2011 (GASTROENTEROLOGY 201 1; 141: 1762-1772) and Sato et al., 2009 (Nature 459, 262-5), which are hereby incorporated by reference in their entirety.

Stem cell culture media often comprise additional growth factors. This method may thus additionally comprise supplying the stem cells with a growth factor. Growth factors commonly used in cell culture medium include epidermal growth factor (EGF, (Peprotech), Transforming Growth Factor-alpha (TGF-alpha, Peprotech), basic Fibroblast Growth Factor (bFGF, Peprotech), brain-derived neurotrophic factor (BDNF, R&D Systems), Hepatocyte Growth Factor (HGF) and Keratinocyte Growth Factor (KGF, Peprotech, also known as FGF7). EGF is a potent mitogenic factor for a variety of cultured ectodermal and mesodermal cells and has a profound effect on the differentiation of specific cells in vivo and in vitro and of some fibroblasts in cell culture. The EGF precursor exists as a membrane-bound molecule which is proteolytically cleaved to generate the 53-amino acid peptide hormone that stimulates cells. EGF or other mitogenic growth factors may thus be supplied to the stem cells. During culturing of stem cells, the mitogenic growth factor may be added to the culture medium every second day, while the culture medium is refreshed preferably every fourth day. In general, a mitogenic factor is selected from the groups consisting of: i) EGF, TGF-alpha, and KGF, ii) EGF, TGF-alpha, and FGF7; iii) EGF, TGF-alpha, and FGF; iv) EGF and KGF; v) EGF and FGF7; vi) EGF and a FGF; vii) TGF-alpha and KGF; viii) TGF-alpha, and FGF7; ix) or from TGF-alpha and a FGF. In certain embodiments, the disclosure includes a stem cell culture media comprising a Wnt surrogate molecule disclosed herein, e.g., optionally in combination with one or more of the growth factors or combinations thereof described herein.

These methods of enhancing proliferation of stem cells can be used to grow new organoids and tissues from stem cells, as for example described in WO2010/090513 WO2012/014076, Sato et al., 201 1 (GASTROENTEROLOGY 2011; 141: 1762-1772) and Sato et al., 2009 (Nature 459, 262-5).

In some embodiments, the Wnt surrogate molecules are used to enhance stem cell regeneration. Illustrative stem cells of interest include but are not limited to: muscle satellite cells; hematopoietic stem cells and progenitor cells derived therefrom (U.S. Patent No. 5,061,620); neural stem cells (see Morrison et al. (1999) Cell 96: 737-749); embryonic stem cells; mesenchymal stem cells; mesodermal stem cells; liver stem cells; adipose-tissue derived stem cells, etc.

Other embodiments of the present disclosure relate, in part, to diagnostic applications for detecting the presence of cells or tissues expressing one or more Fzd receptors or LRP5 or LRP6 receptors. Thus, the present disclosure provides methods of detecting one or more Fzd receptors or LRP5 or LRP6 receptors in a sample, such as detection of cells or tissues expressing Fzd1. Such methods can be applied in a variety of known detection formats, including, but not limited to immunohistochemistry (IHC), immunocytochemistry (ICC), *in situ* hybridization (ISH), whole-mount *in situ* hybridization (WISH), fluorescent DNA *in situ* hybridization (FISH), flow cytometry, enzyme immuno-assay (EIA), and enzyme linked immuno-assay (ELISA), e.g., by detecting binding of a Wnt surrogate molecule.

ISH is a type of hybridization that uses a labeled complementary DNA or RNA strand (i.e., primary binding agent) to localize a specific DNA or RNA sequence in a portion or section of a cell or tissue *(in situ),* or if the tissue is small enough, the entire tissue (whole mount ISH). One having ordinary skill in the art would appreciate that this is distinct from immunohistochemistry, which localizes proteins in tissue sections using an antibody as a primary binding agent. DNA ISH can be used on genomic DNA to determine the structure of chromosomes. Fluorescent DNA ISH (FISH) can, for example, be used in medical diagnostics to assess chromosomal integrity. RNA ISH (hybridization histochemistry) is used to measure and localize mRNAs and other transcripts within tissue sections or whole mounts.

In various embodiments, the Wnt surrogate molecules described herein are conjugated to a detectable label that may be detected directly or indirectly. In this regard, an antibody "conjugate" refers to a Wnt surrogate molecule that is covalently linked to a detectable label. In the present disclosure, DNA probes, RNA probes, monoclonal antibodies, antigen-binding fragments thereof, and antibody derivatives thereof, such as a single-chain-variable-fragment antibody or an epitope tagged antibody, may all be covalently linked to a detectable label. In "direct detection", only one detectable antibody is used, i.e., a primary detectable antibody. Thus, direct detection means that the antibody that is conjugated to a detectable label may be detected, per se, without the need for the addition of a second antibody (secondary antibody).

A "detectable label" is a molecule or material that can produce a detectable (such as visually, electronically or otherwise) signal that indicates the presence and/or concentration of the label in a sample. When conjugated to an antibody, the detectable label can be used to locate and/or quantify the target to which the specific antibody is directed. Thereby, the presence and/or concentration of the target in a sample can be detected by detecting the signal produced by the detectable label. A detectable label can be detected directly or indirectly, and several different detectable labels conjugated to different specific-antibodies can be used in combination to detect one or more targets.

Examples of detectable labels, which may be detected directly, include fluorescent dyes and radioactive substances and metal particles. In contrast, indirect detection requires the application of one or more additional antibodies, i.e., secondary antibodies, after application of the primary antibody. Thus, the detection is performed by the detection of the binding of the secondary antibody or binding agent to the primary detectable antibody. Examples of primary detectable binding agents or antibodies requiring addition of a secondary binding agent or antibody include enzymatic detectable binding agents and hapten detectable binding agents or antibodies.

In some embodiments, the detectable label is conjugated to a nucleic acid polymer which comprises the first binding agent (e.g., in an ISH, WISH, or FISH process). In other embodiments, the detectable label is conjugated to an antibody which comprises the first binding agent (e.g., in an IHC process).

Examples of detectable labels which may be conjugated to Wnt surrogate molecules used in the methods of the present disclosure include fluorescent labels, enzyme labels, radioisotopes, chemiluminescent labels, electrochemiluminescent labels, bioluminescent labels, polymers, polymer particles, metal particles, haptens, and dyes.

Examples of fluorescent labels include 5-(and 6)-carboxyfluorescein, 5- or 6-carboxyfluorescein, 6-(fluorescein)-5-(and 6)-carboxamido hexanoic acid, fluorescein isothiocyanate, rhodamine, tetramethylrhodamine, and dyes such as Cy2, Cy3, and Cy5, optionally substituted coumarin including AMCA, PerCP, phycobiliproteins including R-phycoerythrin (RPE) and allophycoerythrin (APC), Texas Red, Princeton Red, green fluorescent protein (GFP) and analogues thereof, and conjugates of R-phycoerythrin or allophycoerythrin, inorganic fluorescent labels such as particles based on semiconductor material like coated CdSe nanocrystallites.

Examples of polymer particle labels include micro particles or latex particles of polystyrene, PMMA or silica, which can be embedded with fluorescent dyes, or polymer micelles or capsules which contain dyes, enzymes or substrates.

Examples of metal particle labels include gold particles and coated gold particles, which can be converted by silver stains. Examples of haptens include DNP, fluorescein isothiocyanate (FITC), biotin, and digoxigenin. Examples of enzymatic labels include horseradish peroxidase (HRP), alkaline phosphatase (ALP or AP), β-galactosidase (GAL), glucose-6-phosphate dehydrogenase, β-N-acetylglucosamimidase, β-glucuronidase, invertase, Xanthine Oxidase, firefly luciferase and glucose oxidase (GO). Examples of commonly used substrates for horseradishperoxidase include 3,3'-diaminobenzidine (DAB), diaminobenzidine with nickel enhancement, 3-amino-9-ethylcarbazole (AEC), Benzidine dihydrochloride (BDHC), Hanker-Yates reagent (HYR), Indophane blue (IB), tetramethylbenzidine (TMB), 4-chloro-1-naphtol (CN), .alpha.-naphtol pyronin (.alpha.-NP), o-dianisidine (OD), 5-bromo-4-chloro-3-indolylphosp- hate (BCIP), Nitro blue tetrazolium (NBT), 2-(p-iodophenyl)-3-p-nitropheny- 1-5-phenyl tetrazolium chloride (INT), tetranitro blue tetrazolium (TNBT), 5-bromo-4-chloro-3-indoxyl-beta-D-galactoside/ferro-ferricyanide (BCIG/FF).

Examples of commonly used substrates for Alkaline Phosphatase include Naphthol-AS-B 1-phosphate/fast red TR (NABP/FR), Naphthol-AS-MX-phosphate/fast red TR (NAMP/FR), Naphthol-AS-B1-phosphate/- fast red TR (NABP/FR), Naphthol-AS-MX-phosphate/fast red TR (NAMP/FR), Naphthol-AS-B1-phosphate/new fuschin (NABP/NF), bromochloroindolyl phosphate/nitroblue tetrazolium (BCIP/NBT), 5-Bromo-4-chloro-3-indolyl-b-- d-galactopyranoside (BCIG).

Examples of luminescent labels include luminol, isoluminol, acridinium esters, 1,2-dioxetanes and pyridopyridazines. Examples of electrochemiluminescent labels include ruthenium derivatives. Examples of radioactive labels include radioactive isotopes of iodide, cobalt, selenium, tritium, carbon, sulfur and phosphorous.

Detectable labels may be linked to the antibodies described herein or to any other molecule that specifically binds to a biological marker of interest, e.g., an antibody, a nucleic acid probe, or a polymer. Furthermore, one of ordinary skill in the art would appreciate that detectable labels can also be conjugated to second, and/or third, and/or fourth, and/or fifth binding agents or antibodies, etc. Moreover, the skilled artisan would appreciate that each additional binding agent or antibody used to characterize a biological marker of interest may serve as a signal amplification step. The biological marker may be detected visually using, e.g., light microscopy, fluorescent microscopy, electron microscopy where the detectable substance is for example a dye, a colloidal gold particle, a luminescent reagent. Visually detectable substances bound to a biological marker may also be detected using a spectrophotometer. Where the detectable substance is a radioactive isotope detection can be visually by autoradiography, or non-visually using a scintillation counter. See, e.g., Larsson, 1988, Immunocytochemistry: Theory and Practice, (CRC Press, Boca Raton, Fla.); Methods in Molecular Biology, vol. 80 1998, John D. Pound (ed.) (Humana Press, Totowa, N.J.).

The present disclosure further provides kits for detecting one or more Fzd or LRP5/6 receptor or cells or tissues expressing one or more Fzd or LRP5/6 receptors in a sample, wherein the kits contain at least one antibody, polypeptide, polynucleotide, vector or host cell as described herein. In certain embodiments, a kit may comprise buffers, enzymes, labels, substrates, beads or other surfaces to which the antibodies of the present disclosure are attached, and the like, and instructions for use.

All of the above U.S. patents, U.S. patent application publications, U.S. patent applications, foreign patents, foreign patent applications and non-patent publications referred to in this specification and/or listed in the Application Data Sheet, are incorporated herein by reference, in their entirety.

From the foregoing it will be appreciated that, although specific embodiments of the present disclosure have been described herein for purposes of illustration, various modifications may be made without deviating from the spirit and scope of the present disclosure. Accordingly, the present disclosure is not limited except as by the appended claims.

### EXAMPLES

### Example 1

### Multispecific Wnt Surrogate Molecules

A number of multispecific Wnt surrogate molecules representing different configurations were produced, as further described in the following Examples. These included the Wnt surrogate molecules disclosed in Table 5 below, which comprise the sequences set forth in SEQ ID NOs:109-157. The specific Fzd and LRP binding elements used for Wnt surrogate molecules presented in these examples are listed in Tables 1A and 1B and 2A and 2B.

**Table 5. Wnt surrogate molecule sequences**

| Lrp VHH or sdAb = italics | | |
|---|---|---|
| anti-Fzd light chain = underline | | |
| anti-Fzd heavy chain = bold | | |
| **Name** | **SEQ ID NO** | **Sequence** |
| R2M3-3 LC | 109 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGQAVVLQEPSLSVSPG GTVTLTCGLSSGSVSTNYYPSWYQQTPGQAPRTLIYYTNTRSSDVPERFSGSIV GNKAALTITGAQPDDESVYFCLLYLGRGIWVFGGGTKLTVLGQPKAAPSVTLFP PSSEELQANKATLVCLISDFYPGAVTVAWKADSSPVKAGVETTTPSKQSNNKYA ASSYLSLTPEQWKSHRSYSCQVTHEGSTVEKTVAPTECS |
| R2M3-3 HC | 110 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYYGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGK |
| 1291-3 LC | 111 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDVVMSQSPSSLAVS VGEKVTMSCKSSQSLLYSSNQKNYLAWYQQKPGQSPKLLIYWASTRESGVPDR FTGSGSGTDFTLTISSVKAKDLAVYYCQQYYSYPTFGGGTKLEIKRTVAAPSVFIF PPSDEQLKSGTASVVCLLNNFYPREAKVQWKVDNALQSGNSQESVTEQDSKD STYSLSSTLTLSKADYEKHKVYACEVTHQGLSSPVTKSFNRGEC |
| 1291-3 HC | 112 | MDMRVPAQLLGLLLLWLRGARCEVQLVESGGGLVKPGGSLKLSCAASGFTFS SYAMSWVRQTPEKRLEWVATISDGGSYTRYPDKLKGRFTISRDNAKNNLYLQ MSHLKSEDTAMYYCARVGGRRDYFDYWGQGTTLTVSSASTKGPSVFPLAPSS KSTSGGTAALGCLVKDYFPEPVTVSWNSGALTSGVHTFPAVLQSSGLYSLSSV VTVPSSSLGTQTYICNVNHKPSNTKVDKKVEPKSCDKTHTCPPCPAPEAAGGP SVFLFPPKPKDTLMISRTPEVTCVVVDVSHEDPEVKFNWYVDGVEVHNAKTKP REEQYNSTYRVVSVLTVLHQDWLNGKEYKCKVSNKALGAPIEKTISKAKGQPR EPQVYTLPPSREEMTKNQVSLTCLVKGFYPSDIAVEWESNGQPENNYKTTPPV LDSDGSFFLYSKLTVDKSRWQQGNVFSCSVMHEALHNHYTQKSLSLSPGK |
| 1791-3 LC | 113 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDIVMTQSPKSMSMS VGERVTLRCKASENVLNYVSWYQQKPEQSPKLLIYGASNRYTGVPDRFTGSGS ATDFTLTISSVQAEDLADYHCGQSYRYPTFGAGTKLELKRTVAAPSVFIFPPSDE QLKSGTASVVCLLNNFYPREAKVQWKVDNALQSGNSQESVTEQDSKDSTYSLS STLTLSKADYEKHKVYACEVTHQGLSSPVTKSFNRGEC |
| 1791-3 HC | 114 | MDMRVPAQLLGLLLLWLRGARCEVQPVESGGGLVQPKGSLKLSCAASGFTFN TYAMHWVRQAPGKGLEWVARIRSKSNNYAKNYDDSVKDRFTISRDDSQSMLY LQMNNLKTEDTAMYYCVRENYGGRFDYWGQGTTLTVSSASTKGPSVFPLAPS SKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 4SD1-3 LC | 115 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDIQMTQSPSSLSASV GDRVTITCRASQSISSYLNWYQQKPGKAPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLSST LTLSKADYEKHKVYACEVTHQGLSSPVTKSFNRGEC |
| 4SD1-3HC | 116 | MDMRVPAQLLGLLLLWLRGARCEVQLVESGGGLVKPGGSLRLSCAASGFTFT NYAMSWVRQAPGKGLEWVSAISGSGGSTYYADSVKGRFTISRDDSKNTLYLQ MNSLKTEDTAVYYCARATGFGTVVFDYWGQGTLVTVSSASTKGPSVFPLAPS SKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| 14SB6-3 LC | 117 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDIQMTQSPSSLSASV GDRVTITCRASQSISSYLNWYQQKPGKAPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLSST LTLSKADYEKHKVYACEVTHQGLSSPVTKSFNRGEC |
| 14SB6-3 HC | 118 | MDMRVPAQLLGLLLLWLRGARCEVQLVQSGGGLVKPGGSLRLSCAASGFTFS SYSMNWVRQAPGKGLEWVSYIENDGSITTYADSVKGRFTISRDDSKNTLYLQM NSLKTEDTAVYYCARAPYYYGSGSLFRLDYWGQGTLVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGK |
| 4SD1-3 LC | 119 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDIQMTQSPSSLSASV GDRVTITCRASQSISSYLNWYQQKPGKAPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLSST LTLSKADYEKHKVYACEVTHQGLSSPVTKSFNRGEC |
| 4SD1-3 HC-holes | 120 | MDMRVPAQLLGLLLLWLRGARCEVQLVESGGGLVKPGGSLRLSCAASGFTFT NYAMSWVRQAPGKGLEWVSAISGSGGSTYYADSVKGRFTISRDDSKNTLYLQ MNSLKTEDTAVYYCARATGFGTVVFDYWGQGTLVTVSSASTKGPSVFPLAPS SKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGKG SGSGHHHHHH |
| 14SB6-3 LC | 121 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGDIQMTQSPSSLSASV GDRVTITCRASQSISSYLNWYQQKPGKAPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLSST LTLSKADYEKHKVYACEVTHQGLSSPVTKSFNRGEC |
| 14SB6-3 HC-knobs | 122 | MDMRVPAQLLGLLLLWLRGARCEVQLVQSGGGLVKPGGSLRLSCAASGFTFS SYSMNWVRQAPGKGLEWVSYIENDGSITTYADSVKGRFTISRDDSKNTLYLQM NSLKTEDTAVYYCARAPYYYGSGSLFRLDYWGQGTLVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| Fc-knobs | 123 | MDMRVPAQLLGLLLLWLRGARCEFDKTHTCPPCPAPEAAGGPSVFLFPPKPKD TLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSRE EMTKNQVSLWCLVKGFYPSDIAVEWESNGQPENNYKTTPPVLDSDGSFFLYSK LTVDKSRWQQGNVFSCSVMHEALHNHYTQKSLSLSPGKGGSGGDYKDDDDK |
| Fc-holes | 124 | MDMRVPAQLLGLLLLWLRGARCEFDKTHTCPPCPAPEAAGGPSVFLFPPKPKD TLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSRE EMTKNQVSLSCAVKGFYPSDIAVEWESNGQPENNYKTTPPVLDSDGSFFLVSKL TVDKSRWQQGNVFSCSVMHEALHNHYTQKSLSLSPGKGSGSGHHHHHH |
| 4SC10-3 LC | 125 | MDMRVPAQLLGLLLLWLRGARCDVQLVESGGGLVQPGGSLRLSCTSSANINSI ETLGWYRQAPGKQRELIANMRGGGYMKYAGSLKGRFTMSTESAKNTMYLQM NSLKPEDTAVYYCYVKLRDDDYVYRGQGTQVTVSSGGSGSDIQMTQSPSSLS ASVGDRVTITCRASQSISSYLNWYQQKPGKAPKLLIYAASSLQSGVPSRFSGSG SGTDFTLTISSLQPEDFATYYCQQSYSTPLTFGGGTKVEIKRTVAAPSVFIFPPSD EQLKSGTASVVCLLNNFYPREAKVQWKVDNALQSGNSQESVTEQDSKDSTYSL SSTLTLSKADYEKHKVYACEVTHQGLSSPVTKSFNRGEC |
| 4SC10-3 HC | 126 | MDMRVPAQLLGLLLLWLRGARCQVQLVQSGAEVKKPGASVKVSCKASGYIFT DYYMHWVRQAPGQGLEWMGLVDPEDGETIYAEKFQGRVTMTRDTSTSTVYM ELSSLRSEDTAVYYCAHSDFFSGLSFGDWGQGTLVTVSSASTKGPSVFPLAPS SKSTSGGTAALGCLVKDYFPEPVTVSWNSGALTSGVHTFPAVLQSSGLYSLSS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK |
| heterolg 4SC10-3+1RC07-3 LC1 | 127 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGGSGSDIQMTQSPSSLSASV GDRVTITCRASQSISSYLNWYQKKPGKKPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLEST LTLSKADYEKHKVYACEVTHQGLSSPVTKSFNRGEC |
| heterolg 4SC10-3+1RC07-3 HC1 | 128 | MDMRVPAQLLGLLLLWLRGARCQVQLVQSGAEVKKPGASVKVSCKASGYIFT DYYMHWVRDAPGQGLEWMGLVDPEDGETIYAEKFQGRVTMTRDTSTSTVYM ELSSLRSEDTAVYYCAHSDFFSGLSFGDWGDGTLVTVSSASTKGPSVFPLAPS SKSTSGGTAALGCLVKDYFPEPVTVSWNSGALTSGVHTFPAVLQSSGLYSLKS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGKG SGSGHHHHHH |
| heterolg 4SC10-3+1RC07-3 LC2 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| heterolg 4SC10-3+1RC07-3 HC2 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1 RC07-3 monovalent bispecific LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1 RC07-3 monovalent bispecific HC | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1 RC07-3 monovalent bispecific Fc | 131 | MDMRVPAQLLGLLLLWLRGARCEFDKTHTCPPCPAPEAAGGPSVFLFPPKPKD TLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSR EEMTKNQVSLSCAVKGFYPSDIAVEWESNGQPENNYKTTPPVLDSDGSFFLVS KLTVDKSRWQQGNVFSCSVMHEALHNHYTQKSLSLSPGKGSGSGHHHHHH |
| R2M3-26 LC | 132 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGQAVVLQEPSLSVS PGGTVTLTCGLSSGSVSTNYYPSWYQQTPGQAPRTLIYYTNTRSSDVPERFSG SIVGNKAALTITGAQPDDESVYFCLLYLGRGIWVFGGGTKLTVLGQPKAAPSVTL FPPSSEELQANKATLVCLISDFYPGAVTVAWKADSSPVKAGVETTTPSKQSNNK YAASSYLSLTPEQWKSHRSYSCQVTHEGSTVEKTVAPTECS |
| R2M3-26 HC | 110 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYYGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGK |
| R2M3mut-26 LC | 132 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGQAVVLQEPSLSVS PGGTVTLTCGLSSGSVSTNYYPSWYQQTPGQAPRTLIYYTNTRSSDVPERFSG SIVGNKAALTITGAQPDDESVYFCLLYLGRGIWVFGGGTKLTVLGQPKAAPSVTL FPPSSEELQANKATLVCLISDFYPGAVTVAWKADSSPVKAGVETTTPSKQSNNK YAASSYLSLTPEQWKSHRSYSCQVTHEGSTVEKTVAPTECS |
| R2M3mut-26 HC | 133 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYAGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGKGSGSGHHHHHH |
| R2M3-3 LC | 109 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGQAVVLQEPSLSVSPG GTVTLTCGLSSGSVSTNYYPSWYQQTPGQAPRTLIYYTNTRSSDVPERFSGSIV GNKAALTITGAQPDDESVYFCLLYLGRGIWVFGGGTKLTVLGQPKAAPSVTLFP PSSEELQANKATLVCLISDFYPGAVTVAWKADSSPVKAGVETTTPSKQSNNKYA ASSYLSLTPEQWKSHRSYSCQVTHEGSTVEKTVAPTECS |
| R2M3-3 HC | 110 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYYGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGK |
| R2M3mut-3 LC | 109 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGQAVVLQEPSLSVSPG GTVTLTCGLSSGSVSTNYYPSWYQQTPGQAPRTLIYYTNTRSSDVPERFSGSIV GNKAALTITGAQPDDESVYFCLLYLGRGIWVFGGGTKLTVLGQPKAAPSVTLFP PSSEELQANKATLVCLISDFYPGAVTVAWKADSSPVKAGVETTTPSKQSNNKYA ASSYLSLTPEQWKSHRSYSCQVTHEGSTVEKTVAPTECS |
| R2M3mut-3 HC | 133 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYAGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGKGSGSGHHHHHH |
| 1 RC07-3 (2:2) LC | 134 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQQKPGQSPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAS SYLSLTPEQWKSHRSYSCQVTHEGSTVEKTVAPTECS |
| 1 RC07-3 (2:2) HC | 135 | MDMRVPAQLLGLLLLWLRGARCQVQLQQWGAGLLKPSETLSLTCAVSGASFS GHYWTWIRQPPGKGLEWIGEIDHTGSTNYEPSLRSRVTISVDTSKNQFSLNLKS VTAADTAVYYCARGGQGGYDWGHYHGLDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLTCLVKGFYPSDIAVEWESNGQPENNY KTTPPVLDSDGSFFLYSKLTVDKSRWQQGNVFSCSVMHEALHNHYTQKSLSL SPGK |
| 1RC07-3 (2:1) LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1RC07-3 (2:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1RC07-3 (2:1) LC2 | 136 | MDMRVPAQLLGLLLLWLRGARCSYVLTQPPSVSVSPGQTASITCSGDKVGHKY ASWYQKKPGQKPVLVIYEDSQRPSGIPVRFSGSNSGNTATLTISGTQAMDEADY YCQAWDSSTDVVFGGGTKLTVLGQPKAAPSVTLFPPSSEELQANKATLVCLISD FYPGAVTVAWKADSSPVKAGVETTTPSKQSNNKYAAESYLSLTPEQWKSHRSY SCQVTHEGSTVEKTVAPTECS |
| 1RC07-3 (2:1) HC2 | 137 | MDMRVPAQLLGLLLLWLRGARCQVQLQQWGAGLLKPSETLSLTCAVSGASFS GHYWTWIRDPPGKGLEWIGEIDHTGSTNYEPSLRSRVTISVDTSKNQFSLNLKS VTAADTAVYYCARGGQGGYDWGHYHGLDVWGDGTTVTVSSASTKGPSVFPL APSSKSTSGGTAALGCLVKDYFPEPVTVSWNSGALTSGVHTFPAVLQSSGLY SLKSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLSCAVKGFYPSDIAVEWESNGQPENNY KTTPPVLDSDGSFFLVSKLTVDKSRWQQGNVFSCSVMHEALHNHYTQKSLSL SPGKGSGSGHHHHHH |
| R2M3-26NL-26NH (1:2) LC | 132 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGQAVVLQEPSLSVS PGGTVTLTCGLSSGSVSTNYYPSWYQQTPGQAPRTLIYYTNTRSSDVPERFSG SIVGNKAALTITGAQPDDESVYFCLLYLGRGIWVFGGGTKLTVLGQPKAAPSVTL FPPSSEELQANKATLVCLISDFYPGAVTVAWKADSSPVKAGVETTTPSKQSNNK YAASSYLSLTPEQWKSHRSYSCQVTHEGSTVEKTVAPTECS |
| R2M3-26NL-26NH (1:2) HC | 138 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGEVQLVQSGAEVK KPGASVKVSCKASGYTFTSYGISWVRQAPGQGLEWMGWISAYNGNTNYAQK LQGRVTMTTDTSTSTAYMELRSLRSDDTAVYYCASSKEKATYYYGMDVWGQG TTVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCK VSNKALGAPIEKTISKAKGQPREPQVYTLPPSREEMTKNQVSLWCLVKGFYPS DIAVEWESNGQPENNYKTTPPVLDSDGSFFLYSKLTVDKSRWQQGNVFSCSV MHEALHNHYTQKSLSLSPGKGGSGGDYKDDDDK |
| R2M3-26NL-26NH (1:2) Fc | 131 | MDMRVPAQLLGLLLLWLRGARCEFDKTHTCPPCPAPEAAGGPSVFLFPPKPKD TLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSR EEMTKNQVSLSCAVKGFYPSDIAVEWESNGQPENNYKTTPPVLDSDGSFFLVS KLTVDKSRWQQGNVFSCSVMHEALHNHYTQKSLSLSPGKGSGSGHHHHHH |
| R2M3-26NL-26CL (1:2) LC | 139 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGQAVVLQEPSLSVS PGGTVTLTCGLSSGSVSTNYYPSWYQQTPGQAPRTLIYYTNTRSSDVPERFSG SIVGNKAALTITGAQPDDESVYFCLLYLGRGIWVFGGGTKLTVLGQPKAAPSVTL FPPSSEELQANKATLVCLISDFYPGAVTVAWKADSSPVKAGVETTTPSKQSNNK YAASSYLSLTPEQWKSHRSYSCQVTHEGSTVEKTVAPTECSGSGSGDVQLVES GGGLVQAGGSLRLACAGSGRIFAIYDIAWYRHPPGNQRELVAMIRPVVTEIDYA DSVKGRFTISRNNAMKTVYLQMNNLKPEDTAVYYCNAKRPWGSRDEYWGQGT QVTVSS |
| R2M3-26NL-26CL (1:2) HC_Fab | 140 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYYGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCGSGSGHHHHHH |
| R2M3-26NL-26CH (1:2) LC | 132 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGQAVVLQEPSLSVS PGGTVTLTCGLSSGSVSTNYYPSWYQQTPGQAPRTLIYYTNTRSSDVPERFSG SIVGNKAALTITGAQPDDESVYFCLLYLGRGIWVFGGGTKLTVLGQPKAAPSVTL FPPSSEELQANKATLVCLISDFYPGAVTVAWKADSSPVKAGVETTTPSKQSNNK YAASSYLSLTPEQWKSHRSYSCQVTHEGSTVEKTVAPTECS |
| R2M3-26NL-26CH (1:2) HC | 141 | MDMRVPAQLLGLLLLWLRGARCEVQLVQSGAEVKKPGASVKVSCKASGYTFT SYGISWVRQAPGQGLEWMGWISAYNGNTNYAQKLQGRVTMTTDTSTSTAYM ELRSLRSDDTAVYYCASSKEKATYYYGMDVWGQGTTVTVSSASTKGPSVFPL APSSKSTSGGTAALGCLVKDYFPEPVTVSWNSGALTSGVHTFPAVLQSSGLY SLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGSGSGDVQLVESGGGLVQAGGSLRLACAGSGRIFAIYDIAWYRHPPGNQ RELVAMIRPVVTEIDYADSVKGRFTISRNNAMKTVYLQMNNLKPEDTAVYYCNA KRPWGSRDEYWGQGTQVTVSS |
| R2M3-26NL-26CH (1:2) Fc | 131 | MDMRVPAQLLGLLLLWLRGARCEFDKTHTCPPCPAPEAAGGPSVFLFPPKPKD TLMISRTPEVTCVVVDVSHEDPEVKFNWYVDGVEVHNAKTKPREEQYNSTYRV VSVLTVLHQDWLNGKEYKCKVSNKALGAPIEKTISKAKGQPREPQVYTLPPSR EEMTKNQVSLSCAVKGFYPSDIAVEWESNGQPENNYKTTPPVLDSDGSFFLVS KLTVDKSRWQQGNVFSCSVMHEALHNHYTQKSLSLSPGKGSGSGHHHHHH |
| 1 RC07-3+R2M3 (1:1:1) LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1RC07-3+R2M3 (1:1:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1RC07-3+R2M3 (1:1:1) LC2 | 142 | MDMRVPAQLLGLLLLWLRGARCQAVVLQEPSLSVSPGGTVTLTCGLSSGSVST NYYPSWYQKTPGQKPRTLIYYTNTRSSDVPERFSGSIVGNKAALTITGAQPDDE SVYFCLLYLGRGIWVFGGGTKLTVLGQPKAAPSVTLFPPSSEELQANKATLVCLI SDFYPGAVTVAWKADSSPVKAGVETTTPSKQSNNKYAAESYLSLTPEQWKSHR SYSCQVTHEGSTVEKTVAPTECS |
| 1 RC07-3+R2M3 (1:1:1) HC2 | 143 | MDMRVPAQLLGLLLLWLRGARCEVQLVQSGAEVKKPGASVKVSCKASGYTFT SYGISWVRDAPGQGLEWMGWISAYNGNTNYAQKLQGRVTMTTDTSTSTAYM ELRSLRSDDTAVYYCASSKEKATYYYGMDVWGDGTTVTVSSASTKGPSVFPL APSSKSTSGGTAALGCLVKDYFPEPVTVSWNSGALTSGVHTFPAVLQSSGLY SLKSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLSCAVKGFYPSDIAVEWESNGQPENNY KTTPPVLDSDGSFFLVSKLTVDKSRWQQGNVFSCSVMHEALHNHYTQKSLSL SPGKGSGSGHHHHHH |
| 1 RC07-3+R2M13 (1:1:1) LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1 RC07-3+R2M13 (1:1:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1 RC07-3+R2M13 (1:1:1) LC2 | 144 | MDMRVPAQLLGLLLLWLRGARCDIQMTQSPSSLSASVGDRVTITCRASQSISSY LNWYQKKPGKKPKLLIYAASSLQSGVPSRFSGSGSGTDFTLTISSLQPEDFATYY CQQSYSTPLTFGGGTKVEIKRTVAAPSVFIFPPSDEQLKSGTASVVCLLNNFYPR EAKVQWKVDNALQSGNSQESVTEQDSKDSTYSLESTLTLSKADYEKHKVYACE VTHQGLSSPVTKSFNRGEC |
| 1 RC07-3+R2M13 (1:1:1) HC2 | 145 | MDMRVPAQLLGLLLLWLRGARCEVQLLQSGAEVKKPGSSVKVSCKASGGTFT YRYLHWVRDAPGQGLEWMGGIIPIFGTGNYAQKFQGRVTITADESTSTAYMEL SSLRSEDTAVYYCASSMVRVPYYYGMDVWGDGTLVTVSSASTKGPSVFPLAP SSKSTSGGTAALGCLVKDYFPEPVTVSWNSGALTSGVHTFPAVLQSSGLYSLK SVVTVPSSSLGTQTYICNVNHKPSNTKVDKKVEPKSCDKTHTCPPCPAPEAAG GPSVFLFPPKPKDTLMISRTPEVTCVVVDVSHEDPEVKFNWYVDGVEVHNAKT KPREEQYNSTYRVVSVLTVLHQDWLNGKEYKCKVSNKALGAPIEKTISKAKGQ PREPQVYTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPGK GSGSGHHHHHH |
| 1 RC07-3+5SH5 (1:1:1) LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1 RC07-3+5SH5 (1:1:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1RC07-3+5SH5 (1:1:1) LC2 | 146 | MDMRVPAQLLGLLLLWLRGARCDIQMTQSPSSLSASVGDRVTITCRASQGISSA LAWYQKKPGKKPKLLIYAASSLQSGVPSRFSGSGSGTDFTLTISSLQPEDFATYY CQQTYSMPITFGQGTRLEIKRTVAAPSVFIFPPSDEQLKSGTASVVCLLNNFYPR EAKVQWKVDNALQSGNSQESVTEQDSKDSTYSLESTLTLSKADYEKHKVYACE VTHQGLSSPVTKSFNRGEC |
| 1 RC07-3+5SH5 (1:1:1) HC2 | 147 | MDMRVPAQLLGLLLLWLRGARCQVQLVQSGAEVKKPGASVKVSCKASGYTFT SYYMHWVRDAPGQGLEWMGRINPNSGGTNYAQKFQGRVTMTRDTSTSTVYM ELSSLRSEDTAVYYCARVPDFWSGYLDYWGDGTLVTVSSASTKGPSVFPLAP SSKSTSGGTAALGCLVKDYFPEPVTVSWNSGALTSGVHTFPAVLQSSGLYSLK SVVTVPSSSLGTQTYICNVNHKPSNTKVDKKVEPKSCDKTHTCPPCPAPEAAG GPSVFLFPPKPKDTLMISRTPEVTCVVVDVSHEDPEVKFNWYVDGVEVHNAKT KPREEQYNSTYRVVSVLTVLHQDWLNGKEYKCKVSNKALGAPIEKTISKAKGQ PREPQVYTLPPSREEMTKNQVSLSCAVKGFYPSDIAVEWESNGQPENNYKTTP PVLDSDGSFFLVSKLTVDKSRWQQGNVFSCSVMHEALHNHYTQKSLSLSPGK GSGSGHHHHHH |
| 1RC07-3-26 NL (2:1:1) LC1 | 129 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGSGSGSYVLTQPPSVSVSPG QTASITCSGDKVGHKYASWYQDKPGQDPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAK SYLSLTPEQWKSHRSYSCQVTHEGSTVEKTVAPTECS |
| 1RC07-3-26 NL (2:1:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1RC07-3-26 NL (2:1:1) LC2 | 148 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGGSGSSYVLTQPPSVSVS PGQTASITCSGDKVGHKYASWYQKKPGQKPVLVIYEDSQRPSGIPVRFSGSNS GNTATLTISGTQAMDEADYYCQAWDSSTDVVFGGGTKLTVLGQPKAAPSVTLF PPSSEELQANKATLVCLISDFYPGAVTVAWKADSSPVKAGVETTTPSKQSNNKY AAESYLSLTPEQWKSHRSYSCQVTHEGSTVEKTVAPTECS |
| 1RC07-3-26 NL (2:1:1) HC2 | 137 | MDMRVPAQLLGLLLLWLRGARCQVQLQQWGAGLLKPSETLSLTCAVSGASFS GHYWTWIRDPPGKGLEWIGEIDHTGSTNYEPSLRSRVTISVDTSKNQFSLNLKS VTAADTAVYYCARGGQGGYDWGHYHGLDVWGDGTTVTVSSASTKGPSVFPL APSSKSTSGGTAALGCLVKDYFPEPVTVSWNSGALTSGVHTFPAVLQSSGLY SLKSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLSCAVKGFYPSDIAVEWESNGQPENNY KTTPPVLDSDGSFFLVSKLTVDKSRWQQGNVFSCSVMHEALHNHYTQKSLSL SPGKGSGSGHHHHHH |
| 1RC07-3-26 NH (2:1:1) LC | 149 | MDMRVPAQLLGLLLLWLRGARCSYVLTQPPSVSVSPGQTASITCSGDKVGHKY ASWYQQKPGQSPVLVIYEDSQRPSGIPVRFSGSNSGNTATLTISGTQAMDEADY YCQAWDSSTDVVFGGGTKLTVLGQPKAAPSVTLFPPSSEELQANKATLVCLISD FYPGAVTVAWKADSSPVKAGVETTTPSKQSNNKYAASSYLSLTPEQWKSHRSY SCQVTHEGSTVEKTVAPTECS |
| 1RC07-3-26 NH (2:1:1) HC1 | 150 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGGSGSQVQLQQWGAGL LKPSETLSLTCAVSGASFSGHYWTWIRQPPGKGLEWIGEIDHTGSTNYEPSLR SRVTISVDTSKNQFSLNLKSVTAADTAVYYCARGGQGGYDWGHYHGLDVWG QGTTVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKKV EPKSCDKTHTCPPCPAPEAAGGPSVFLFPPKPKDTLMISRTPEVTCVVVDVSH EDPEVKFNWYVDGVEVHNAKTKPREEQYNSTYRVVSVLTVLHQDWLNGKEYK CKVSNKALGAPIEKTISKAKGQPREPQVYTLPPSREEMTKNQVSLWCLVKGFY PSDIAVEWESNGQPENNYKTTPPVLDSDGSFFLYSKLTVDKSRWQQGNVFSC SVMHEALHNHYTQKSLSLSPGKGGSGGDYKDDDDK |
| 1RC07-3-26 NH (2:1:1) HC2 | 151 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGGSGSQVQLQQWGAGLLK PSETLSLTCAVSGASFSGHYWTWIRQPPGKGLEWIGEIDHTGSTNYEPSLRSR VTISVDTSKNQFSLNLKSVTAADTAVYYCARGGQGGYDWGHYHGLDVWGQG TTVTVSSASTKGPSVFPLAPSSKSTSGGTAALGCLVKDYFPEPVTVSWNSGAL TSGVHTFPAVLQSSGLYSLSSVVTVPSSSLGTQTYICNVNHKPSNTKVDKKVEP KSCDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKEYKCK VSNKALGAPIEKTISKAKGQPREPQVYTLPPSREEMTKNQVSLSCAVKGFYPS DIAVEWESNGQPENNYKTTPPVLDSDGSFFLVSKLTVDKSRWQQGNVFSCSV MHEALHNHYTQKSLSLSPGKGSGSGHHHHHH |
| 1 RC07-26+4SD1-3_(1:1:1:1) LC1 | 152 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGGSGSSYVLTQPPSVSVS PGQTASITCSGDKVGHKYASWYQDKPGQDPVLVIYEDSQRPSGIPVRFSGSNS GNTATLTISGTQAMDEADYYCQAWDSSTDVVFGGGTKLTVLGQPKAAPSVTLF PPSSEELQANKATLVCLISDFYPGAVTVAWKADSSPVKAGVETTTPSKQSNNKY AAKSYLSLTPEQWKSHRSYSCQVTHEGSTVEKTVAPTECS |
| 1 RC07-26+4SD1-3_(1:1:1:1) HC1 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |
| 1 RC07-26+4SD1-3_(1:1:1:1) LC2 | 153 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGGSGSDIQMTQSPSSLSASV GDRVTITCRASQSISSYLNWYQKKPGKAPKLLIYAASSLQSGVPSRFSGSGSGT DFTLTISSLQPEDFATYYCQQSYSTPLTFGGGTKVEIKRTVAAPSVFIFPPSDEQL KSGTASVVCLLNNFYPREAKVQWKVDNALQSGNSQESVTEQDSKDSTYSLEST LTLSKADYEKHKVYACEVTHQGLSSPVTKSFNRGEC |
| 1 RC07-26+4SD1-3_(1:1:1:1) HC2 | 154 | MDMRVPAQLLGLLLLWLRGARCEVQLVESGGGLVKPGGSLRLSCAASGFTFT NYAMSWVRDAPGKGLEWVSAISGSGGSTYYADSVKGRFTISRDDSKNTLYLQ MNSLKTEDTAVYYCARATGFGTVVFDYWGDGTLVTVSSASTKGPSVFPLAPS SKSTSGGTAALGCLVKDYFPEPVTVSWNSGALTSGVHTFPAVLQSSGLYSLKS VVTVPSSSLGTQTYICNVNHKPSNTKVDKKVEPKSCDKTHTCPPCPAPEAAGG PSVFLFPPKPKDTLMISRTPEVTCVVVDVSHEDPEVKFNWYVDGVEVHNAKTK PREEQYNSTYRVVSVLTVLHQDWLNGKEYKCKVSNKALGAPIEKTISKAKGQP REPQVYTLPPSREEMTKNQVSLSCAVKGFYPSDIAVEWESNGQPENNYKTTPP VLDSDGSFFLVSKLTVDKSRWQQGNVFSCSVMHEALHNHYTQKSLSLSPGKG SGSGHHHHHH |
| R2M9-26+1 RC07-3(1:1:1:1) LC1 | 155 | MDMRVPAQLLGLLLLWLRGARCDVQLVESGGGLVQAGGSLRLACAGSGRIFAI YDIAWYRHPPGNQRELVAMIRPVVTEIDYADSVKGRFTISRNNAMKTVYLQMNN LKPEDTAVYYCNAKRPWGSRDEYWGQGTQVTVSSGSGSGDIQMTQSPSSLSA SVGDRVTITCRASQSISSYLNWYQDKPGKDPKLLIYAASSLQSGVPSRFSGSGS GTDFTLTISSLQPEDFATYYCQQSYRTPFTFGPGTKVDIKRTVAAPSVFIFPPSDE QLKSGTASVVCLLNNFYPREAKVQWKVDNALQSGNSQESVTEQDSKDSTYSLK STLTLSKADYEKHKVYACEVTHQGLSSPVTKSFNRGEC |
| R2M9-26+1 RC07-3(1:1:1:1) HC1 | 156 | MDMRVPAQLLGLLLLWLRGARCQVQLVQSGAEVKKPGSSVKVSCKASGYTFT NNFMHWVRKAPGQGLEWMGWINPNSGGTKYAQKFQGRVTITADESTSTAYM ELSSLRSEDTAVYYCARSVGEVGATMLGIGVWYWFDPWGKGTLVTVSSASTK GPSVFPLAPSSKSTSGGTAALGCLVKDYFPEPVTVSWNSGALTSGVHTFPAVL QSSGLYSLESVVTVPSSSLGTQTYICNVNHKPSNTKVDKKVEPKSCDKTHTCP PCPAPEAAGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALGAPI EKTISKAKGQPREPQVYTLPPSREEMTKNQVSLWCLVKGFYPSDIAVEWESNG QPENNYKTTPPVLDSDGSFFLYSKLTVDKSRWQQGNVFSCSVMHEALHNHYT QKSLSLSPGKGGSGGDYKDDDDK |
| R2M9-26+1 RC07-3(1:1:1:1) LC2 | 157 | MDMRVPAQLLGLLLLWLRGARCDVQLVESGGGLVQPGGSLRLSCTSSANINSIE TLGWYRQAPGKQRELIANMRGGGYMKYAGSLKGRFTMSTESAKNTMYLQMNS LKPEDTAVYYCYVKLRDDDYVYRGQGTQVTVSSGGSGSSYVLTQPPSVSVSPG QTASITCSGDKVGHKYASWYQKKPGQKPVLVIYEDSQRPSGIPVRFSGSNSGN TATLTISGTQAMDEADYYCQAWDSSTDVVFGGGTKLTVLGQPKAAPSVTLFPPS SEELQANKATLVCLISDFYPGAVTVAWKADSSPVKAGVETTTPSKQSNNKYAAE SYLSLTPEQWKSHRSYSCQVTHEGSTVEKTVAPTECS |
| R2M9-26+1 RC07-3(1:1:1:1) HC2 | 130 | MDMRVPAQLLGLLLLWLRGARCQVQLQQWGAGLLKPSETLSLTCAVSGASFS GHYWTWIRKPPGKGLEWIGEIDHTGSTNYEPSLRSRVTISVDTSKNQFSLNLKS VTAADTAVYYCARGGQGGYDWGHYHGLDVWGKGTTVTVSSASTKGPSVFPL APSSKSTSGGTAALGCLVKDYFPEPVTVSWNSGALTSGVHTFPAVLQSSGLY SLESVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALGAPIEKTISKA KGQPREPQVYTLPPSREEMTKNQVSLWCLVKGFYPSDIAVEWESNGQPENNY KTTPPVLDSDGSFFLYSKLTVDKSRWQQGNVFSCSVMHEALHNHYTQKSLSL SPGKGGSGGDYKDDDDK |

### Example 2

### Generation of Wnt Surrogate Molecules with Binding Specificity for the Fzd Receptor Hinge Region

Active Wnt surrogate molecules were generated comprising various combinations of Fzd binders that bind the Fzd receptor hinge region (see FIG. 2A) and LRP binders. Two antibodies that bind to the hinge region of Fzd7, anti-FZD7-1791 (SEQ ID NOS:70-71) and anti-FZD7-1291 (SEQ ID NOS:72-73), have been described in WO2016/205551 and WO2016/205566. These two antibodies were used to demonstrate that binders to the Fzd hinge region yield active Wnt surrogate molecules.

Both anti-FZD7-1791 and anti-FZD7-1291 were cloned into human IgG1 framework with LALA-PG mutations in Fc to reduce effector functions. LRP5 binder #3 (008S- D01; SEQ ID NO:97) was cloned in frame to the N-termini of the light chains of both antibodies as depicted in FIG. 2B. Both recombinant appended IgG proteins, named 1791-3 (SEQ ID NOs:113-114) and 1291-3 (SEQ ID NOs:111-112), respectively, were prepared by transfection of respective expression vectors into Expi293F cells (Thermo Fisher Scientific, Waltham, MA) according to the manufacturer's instructions. Briefly, four days after the transfection, cell culture medium was collected after spinning down the cell pellet. The media was incubated with Protein A resin (REPLIGEN, Waltham, MA) for collecting proteins containing human IgG-Fc portion. Proteins were eluted with 10 mM glycine, pH 3.5 from Protein A resin. Subsequently, the protein elutes were fractionated and further purified by size-exclusion chromatography (SEC). SEC was performed by a fast protein liquid chromatography using a Superdex 200 Increase 10/300 GL (GE Healthcare, Pittsburgh, PA) in HBS buffer (10 mM HEPES, 150 mM NaCl, pH7.4). The peak fractions were analyzed by SDS-Polyacrylamide Gel Electrophoresis (SDS-PAGE) to confirm the content.

Antibodies that bind to the hinge region of Fzd1 and Fzd2 were utilitized to demonstrate that, in addition to the Fzd7 hinge, other Fzd hinge regions also yield active Wnt surrogate molecules. Recombinant Fab fragments of these antibodies were produced from Expi293F cells (Thermo Fisher Scientific, Waltham, MA) via transient transfection. The Fabs were purified from the culture media with Nickel resin and further polished with size exclusion chromatography (SEC).

The anti-FZD1 hinge and anti-FZD2 hinge antibodies were cloned into human IgG1 framework with LALA-PG mutations in Fc to reduce effector functions. LRP5 binder #3 (008S-D01; SEQ ID NO:97) or LRP5/6 binder #36 (013S-D05; SEQ ID NOs: 91 and 96) were cloned in frame to the N-termini of the light chains of respective antibodies as depicted in FIG. 2B. The recombinant appended IgG proteins were prepared by transfection of respective expression vectors into Expi293F cells (Thermo Fisher Scientific, Waltham, MA) according to the manufacturer's instructions. Briefly, four days after the transfection, cell culture medium was collected after spinning down the cell pellet. The media was incubated with Protein A resin (REPLIGEN, Waltham, MA) for collecting proteins containing human IgG-Fc portion. Proteins were eluted with 10 mM glycine, pH 3.5 from Protein A resin. Subsequently, the protein elutes were fractionated and further purified by size-exclusion chromatography (SEC). SEC was performed by a fast protein liquid chromatography using a Superdex 200 Increase 10/300 GL (GE Healthcare, Pittsburgh, PA) in HBS buffer (10 mM HEPES, 150 mM NaCl, pH7.4). The peak fractions were analyzed by SDS-Polyacrylamide Gel Electrophoresis (SDS-PAGE) to confirm the content. The results indicated that these antibodies also yielded active Wnt surrogate molecules.

### Example 3

### Binding Kinetics of Hinge Region-Specific Wnt Surrogate Molecules

Binding kinetics of 1791-3 and 1291-3 to either Fzd7 CRD or Fzd7 CRD with the extracellular hinge region sequences (Fzd7 CRD+hinge) was determined by bio-layer interferometry (BLI) using Octet Red 96 (PALL ForteBio, Fremont, CA) instruments at 30° C, 1000 rpm with streptavidin (SA) biosensors. N-terminal biotinylated Fzd7 CRD and Fzd7 CRD+hinge proteins were captured on the SA biosensor. Following capture of biotinylated-Fzd7, the SA biosensor with captured biotinylated-Fzd7 was dipped into wells containing the relevant antibodies at 7 different concentrations in running buffer plus a well with only running buffer as a reference channel. KD was determined by global fitting. As shown in FIG. 2C, both of these antibody fusion proteins only bound to the Fzd7 protein with the hinge region, and not to the Fzd7 CRD domain alone.

### Example 4

### In Vitro Activity of Hinge Region-Specific Wnt Surrogate Molecules

The ability of 1791-3 and 1291-3 to activate Wnt signaling was assessed in the 293 STF cell line, where the β-Catenin luciferase reporter plasmid Super TOP Flash (STF) was stably integrated. As shown in FIG. 2D, both 1791-3 and 1291-3 activated Wnt signaling as judged by the induction of luciferase reporter in these cells in the present of 20 nM R-spondin 2 (RPSO). 1791-3 showed higher activity than 1291-3, even though 1291-3 had higher affinity to Fzd7 CRD+hinge, suggesting that potential differences in geometry between the two antibodies may contribute to differences in activity. Another Wnt surrogate molecule, R2M3-3 (Fzd binder 001 S-A04; Lrp binder 008S-D01; SEQ ID NOs:109-110), which can engage Fzd1, Fzd2, Fzd7, Fzd5, and Fzd8, was also tested. As shown in FIG. 2D, the maximal effect from 1791-3 is approaching that of the multifamily specific R2M3-3.

The ability of Wnt surrogates comprising antibodies that bind the Fzd1 or Fzd2 hinge regions to activate Wnt signaling was assessed in the 293 STF cell line overexpressing either Fzd1 or Fzd2, where the β-Catenin luciferase reporter plasmid Super TOP Flash (STF) was stably integrated. For the Luciferase reporter assays, in each 96 well plate, 1 million cells were seeded, IWP2 (a wnt signaling inhibitor) was added at 3 µM final concentration. 26 hours after seeding, compounds were added to the 96 well plates with triplicates and 10-fold series dilution from 1 OOnM, and the highest concentration is 500nM. 18 hours later, cells were lysed with 100 µl lysis buffer. From the above lysed cells, 20 ul samples were transferred to opaque 96- well plates. Toward each well, 10 µl of luciferase substrate was added. The plate was immediately placed in Molecular Device Lum96 plate reader and luciferase luminescence signals were collected. Data were processed with Prism7. These antibodies activated Wnt signaling as judged by the induction of luciferase reporter in these cells with either Fzd1 or Fzd 2 overexpression in the present of 20 nM R-spondin 2 (RPSO). These results demonstrate that Fzd hinge binding antibodies when assembled with LRP binders can induce Wnt signaling activation.

### Example 5

### Generation of Multispecific Wnt Surrogate Molecules

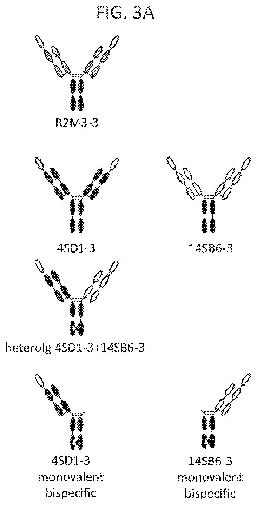

Sequences from monospecific Wnt surrogate molecules were combined to generate a multispecific Wnt surrogate molecule with a desired combination of Fzd binding specificity. As depicted in FIG. 3A, two monospecific Wnt surrogate molecules, 4SD1-3 (Fzd binder 004S-D01; Lrp binder 008S-D01; SEQ ID NOs:115-116), which binds Fzd4, and 14SB6-3 (Fzd binder 014S-B06; Lrp binder 008S-D01; SEQ ID NOs:117-118), which binds Fzd9, were combined using knobs-into-holes technology into a hetero-Ig Wnt surrogate molecule, hetero-Ig 4SD1-3+14SB6-3 (SEQ ID NOs:120 and122 ). Half of the hetero-Ig molecule came from 4SD1-3 with the introduction of the "holes" mutations in the CH3 domain (SEQ ID NO:120). This half of the molecule contained one binding arm to Fzd4 and one binding arm to LRP5. The other half of the hetero-Ig molecule came from 14SB6-3 with the introduction of the "knobs" mutations in the CH3 domain (SEQ ID NO:122). This half of the molecule contained one binding arm to Fzd9 and one binding arm to LRP5. The final Wnt surrogate, hetero-Ig 4SD1-3+14SB6-3, was a tetravalent tri-specific molecule containing one binding arm to Fzd4, one binding arm to Fzd9, and two binding arms to LRP5.

Two control molecules were also generated: 4SD1-3 monovalent bispecific (SEQ ID NO:120 and 123) and 14SB6-3 monovalent bispecific (FIG. 3A) SEQ ID NO:122 and 124). The 4SD1-3 monovalent bispecific molecule contained the 4SD1-3 half of the hetero-Ig with the "holes" mutations (SEQ ID NO:120) paired with an Fc with "knobs" mutations (SEQ ID NO:123), but without an Fab arm; therefore, this molecule was bispecific but monovalent against Fzd4 and LRP5. The 14SB6-3 monovalent bispecific contained the 14SB6-3 half of the hetero-Ig with the "knobs" mutations SEQ ID NO:122) paired with an Fc with "holes" mutations SEQ ID NO:124), but without an Fab arm; therefore, this molecule was bispecific but monovalent against Fzd9 and LRP5.

Hetero-Ig 4SD1-3+14SB6-3, 4SD1-3 monovalent bispecific, and 14SB6-3 monovalent bispecific were purified through a 4-step purification process. Since the Fc containing the "knobs" mutation also contains a FLAG tag, and the Fc containing the "holes" mutation also contains a His tag, these proteins were purified first by protein A and Ni-NTA affinity purification steps, followed by an SEC step; Anti-FLAG M2 beads were used as a final purification step.

### Example 6

### Characterization of Multispecific Wnt Surrogate Molecules

The ability of hetero-Ig 4SD1-3+14SB6-3, 4SD1-3 monovalent bispecific, and 14SB6-3 monovalent bispecific to activate Wnt signaling was assessed in Wnt-responsive 293STF reporter cells in the presence or absence of 20 nM RPSO (only data in the presence of RPSO is shown in FIGS. 3B-3E). R2M3-3, which can bind to Fzd1, Fzd2, Fzd7, Fzd5, and Fzd8, was also tested.

The 293STF cell line expresses low level of Fzd4 and no detectable Fzd9 expression by QPCR. All molecules other than R2M3-3 showed very little or no activity in the parental 293STF cells (FIG. 3B).

A retrovirus-based Fzd4 overexpression 293STF stable cell line was generated (293STF Fzd4OE). In this cell line, both R2M3-3 and 4SD1-3 (Fzd4 monospecific) potently activated Wnt signaling, while the hetero-Ig 4SD1-3+14SB6-3 showed very weak activity, and the Fzd9 monospecific molecule, 14SB6-3 was inactive (FIG. 3C).

Fzd9 was introduced into the parental 293STF cells through transient transfection to create the 293STF Fzd9OE cell line. In this cell line, both R2M3-3 and 14SB6-3 (Fzd9 monospecific) were able to activate Wnt signaling, while the hetero-Ig 4SD1-3+14SB6-3 showed very weak activity, and the Fzd4 monospecific molecule, 4SD1-3 was inactive (FIG. 3D).

Fzd4 and Fzd9 were co-introduced into 293STF cells through a combination of retroviral Fzd4 delivery and Fzd9 transient transfection to create the 293STF Fzd4OE + Fzd9OE cell line. In this cell line, R2M3-3, 4SD1-3, 14SB6-3, and hetero-Ig 4SD1-3+14SB6-3 were all fully active (FIG. 3E)
The two monovalent bispecific molecules, 4SD1-3 monovalent bispecific and 14SB6-3 monovalent bispecific, were inactive in all cells (FIGS. 3B-3E). These results demonstrated that for the two monospecific binders, the monovalent bispecific combination with a LRP5 binder is not able to induce or not able to efficiently induce Wnt signaling. Therefore, the activity observed with the hetero-Ig 4SD1-3+14SB6-3 was indeed the result of the engagement of both Fzd4 and Fzd9 proteins, together with LRP. These results further demonstrated that this approach can be applied to any combination of two or more Fzd binders of different specificities, thus enabling the generation of any desired combination of Fzd specificities through this process.

This example showed that two Fzd binders of different specificities can be combined in the hetero-Ig format to generate Wnt surrogate molecules of different specificities. This concept can also be extended to the use of two Fzd binders to the same Fzd on two different epitopes. The resulting hetero-Ig Wnt surrogate molecule would be a biparatopic molecule, which may be beneficial for further receptor clustering and activation of Wnt signaling.

### EXAMPLE 7

### Heterodimerization of Canonical and Noncanonical Signaling Fzd Receptors Together with LRP Receptor Binding Leads to Activation of Beta-catenin-dependent Signaling

Wnt signaling can be mediated through beta-catenin-dependent (canonical) and beta-catenin-independent (noncanonical) pathways. There are Fzd receptors that have been reported to activate beta-catenin-independent signaling such as Fzd6, while other Fzd receptors activate beta-catenin-dependent signaling. It has not been considered previously whether forced dimerization of a noncanonical Fzd receptor with Lrp or heterodimerizing a canonical and a noncanonical Fzd receptors with Lrp can lead to beta-catenin-dependent or beta-catenin-independent signaling. To test these ideas, the following examples were generated.

First, a multivalent molecule consisting of bivalent binding arms (004S-C10) toward Fzd6, a noncanonical receptor, was combined with bivalent binding arms (3; 004S-D01) toward Lrp5. The molecule is named 4SC10-3 (Fig. 5A top panel). Such a format has been shown to activate beta-catenin-dependent signaling when the Fzd binder is directed against a canonical Fzd receptor. However, as shown in Fig. 5A bottom panel, 4SC10-3 was not able to effectively activate beta-catenin-dependent signaling in 293STF reporter cells (in the presence of 20nM R-spondin), in contrast to the positive control molecule R2M3-26 (Fzd binder 001 S-A04; Lrp binder 009S-E04) that engages Fzd1,2,7,5,8.

Next, a multivalent molecule consisting of 4SC10 and another Fzd binding arm, 1 RC07 (001S- B03), which binds to Fzd1,2,7 (canonical Fzd receptors), was combined with bivalent binding arms (3) toward Lrp5. The molecule is named heterolg 4SC1 0-3+1 RC07-3 (Fig. 5B top panel), and it activated beta-catenin-dependent signaling in 293STF reporter cells. To rule out the possibility that the 1RC07-3 arm alone is able to activate signaling, the 1RC07-3 monovalent bispecific molecule shown in Fig. 5C top panel was also tested. As shown in Fig. 5C, 1RC07-3 monovalent bispecific molecule is not able to effectively activate signaling. These results demonstrated for the first time that combining a canonical and a noncanonical Fzd receptors with Lrp can lead to beta-catenin-dependent signaling.

### EXAMPLE 8

### Exploring Impact of Different Ratios of Fzd to Lrp Binders in Wnt Surrogate Molecules on Beta-catenin-dependent Signaling

Multivalent formats produced potent and highly efficacious Wnt signaling activators. Experiments were designed to test the impact of the formats on endogenous Wnt signaling. To understand the bivalent/multivalent Lrp binding arms impact on endogenous Wnt ligands, an inactive surrogate molecule was generated with a null mutant of R2M3, R2M3mut (Y9A), and fused to either Lrp binders 3 or 26, and named R2M3mut-3 or R2M3mut-26, respectively. As shown in Fig. 6A, while the wild-type R2M3 surrogates, R2M3-3 and R2M3-26, are fully active in 293STF Wnt reporter cell line in the presence of 20nM R-spondin, R2M3mut-3 and R2M3mut-26 are inactive. To test the impact of the bivalent Lrp arms in the surrogate molecules on endogenous Wnt signaling, we tested these two inactive surrogate molecules in 293STF Wnt reporter cell line in the presence of 20% conditioned media containing Wnt3A. As shown in Fig. 6B, while R2M3mut-3 and R2M3mut-26 are inactive on their own, R2M3mut-3 synergized with Wnt3a in a dose responsive manner, and R2M3mut-26 antagonized Wnt3a in a dose responsive manner. This demonstrated that multivalent formats might have potential to interact with endogenous Wnt ligands.

To understand formats that reduce the potential interaction with endogenous Wnts and to further understand the required number of receptor binders for active signaling, we explored different number and ratios of Fzd and Lrp binders in a multivalent surrogate molecule.

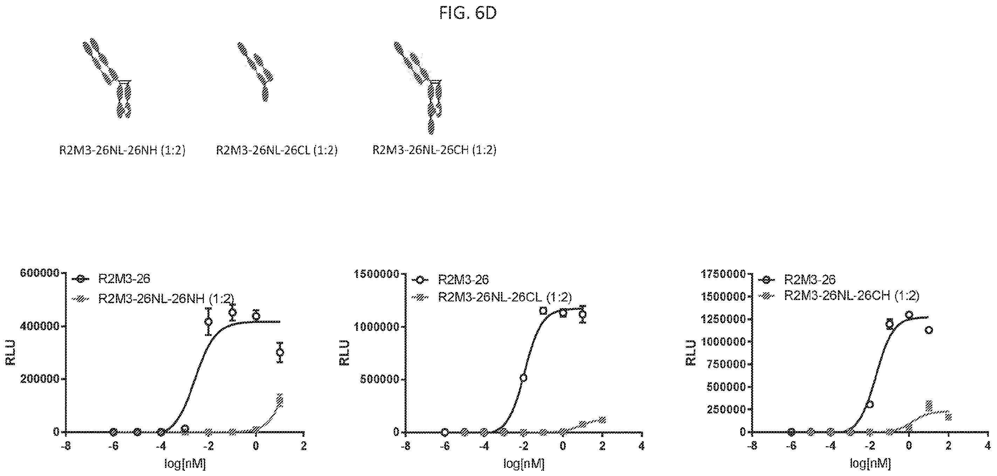

Since bivalency of the Lrp binding arms may lead to synergy with endogenous Wnts, removing one Lrp binding arm was tested, so in the final molecule there are two Fzd binders and one Lrp binder; this molecule is referred to as 2:1. The original molecule format would be 2:2 as there are two Fzd binders and two Lrp binding arms. One example of the 2:1 molecule, 1RC07-3 (2:1) is shown in Fig. 6C left panel, in comparison to the 2:2 format. As shown in Fig. 6C right panel, 1RC07-3 (2:1) is active and able to induce Wnt signaling in 293STF reporter cells. This format, in principle, should lack the ability to synergize with endogenous Wnt ligands via cross-linking of Lrp co-receptors. The reverse ratio, one Fzd binder and two Lrp binders, as exemplified by a few formats in Fig. 6D, has also been constructed. In general, these molecules at best, showed modest induction of signaling (Fig. 6D).

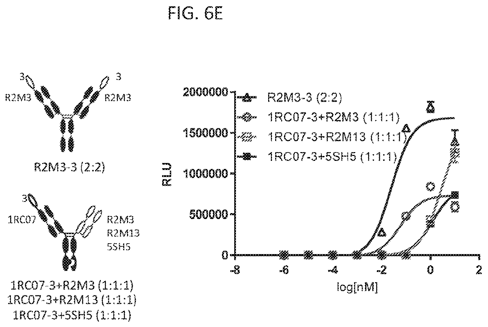

Since heterodimerizing Fzd in the 2:2 format can induce Wnt signaling (shown in Fig. 3 and Fig. 5), a molecules was constructed heterodimerizing two different Fzd binders with one Lrp binder (1:1:1) format and tested for the ability to confer Wnt signaling. As shown in Fig. 6E, several examples of 1:1:1 molecules all activated Wnt signaling in 293STF reporter cells. Fzd binders tested in this structure include R2M3 (001 S-A04),1 RC07 (001S- B03), R2M13 (004S- G06), and 5S-H5 (005S-H05)

### EXAMPLE 9

### Heterodimerization of Lrp Binders Together with Fzd Binding Leads to Activation of Wnt Signaling

In addition to heterodimerizing/multimerizing different Fzd receptor binders, the effects of heterodimerizing/multimerizing different Lrp co-receptor binders on Wnt signaling was also tested. As shown above, the two Fzd:one Lrp format alleviated synergy with endogenous Wnt ligands. To determine if heterodimerized/multimerized Lrp binders directed against two different regions on Lrp may also relieve synergistic effects of bivalent Lrp binder formats on endogenous Wnt ligands was tested. As shown in Fig. 7A, several examples were contstructed in a 2:1:1 ratio, where two different Lrp binders (one binds to Lrp5E1 E2, the other binds to Lrp6E3E4) were each fused to either the N-terminus of light chain of Fzd binder, 1RC01, as 1RC07-3-26 NL (2:1:1); or to the N-terminus of 1 RC01 heavy chain as 1RC07-3-26 **NH** (2:1:1). Both molecules are highly active in inducing Wnt signaling as detected in a 293STF Wnt responsive reporter cells (Fig. 7B).

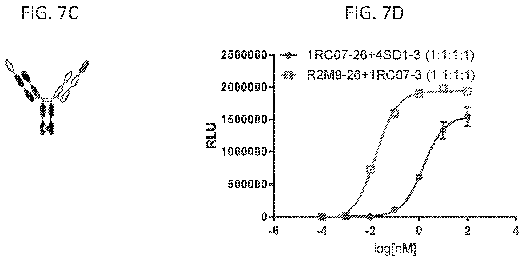

Finally, a multivalent molecule with two distinct Fzd binding arms and two distinct Lrp binding arms, termed 1:1:1:1 format as shown in Fig. 7C was generated. Both the 1 RC01 -26+4SD1-3 (1:1:1:1) and R2M9-26+1 RC07-3 (1:1:1:1) combinations resulted in strong induction of Wnt signaling (Fig. 7D). R2M9 structure contains the 003S-E07 Fzd binding element.

The examples shown in Figs. 5-7 illustrate the various structures for induction of canonical or non-canonical Wnt signaling (or both), as well as the ability to synergize with, antagonize, or leave unaffected signaling via endogenous Wnt ligands within target tissues. This versatile collection of soluble Wnt surrogate ligands could be used for therapies with tailored Wnt signaling that may allow for maximum therapeutic effect and minimal side effects.

### EXAMPLE 10

### Structure-Function Analysis of Wnt Surrogate Molecules Containing Ddifferent Stoichiometric Ratios of Fzd and LRP Binders

A surrogate WNT agonist generated by linking a FZD binder (18R5 antibody in scFv format) and the C-terminal portion of Dickkopf (DKK1c) into a single polypeptide chain (18R5-DKK1c) exhibited the ability to activate WNT/β-catenin signaling (Janda et al., 2017, Nature, 545(7653):234-237). Combination of various FZD and LRP binding antibody fragments were generated based on this concept.

The sequences for Fzd binding scFvs, referred to as F1, F2, and F3, and LRP scFvs, referred to as L1 and L2, as well as linker sequences used to combine them are shown in Table 6A. Since these molecules have a monovalent binding arm to each target, we refer to this format as bivalent bispecific format (denoted as 1:1 to represent the stoichiometry of binding to one FZD and one LRP molecule).

Additional stoichometries for the tandem scFv constructs were tested. A summary of Combinatorial Wnt surrogate constructs used for this analysis are shown in Table 6B.

**Table 6A: Wnt surrogate components used to stoichiometric analysis**

| Component | SEQ ID NO | Designation | Specificity (Clone name) | Sequence |
|---|---|---|---|---|
| signal peptide | 2234 | | | MDMRVPAQLLGLLLLWLRGARC |
| antiFZD_sc Fv | 2235 | F1: | antiFZ01, 2,7,5,8 (18R5) | EVQLVESGGGLVQPGGSLRLSCAASGFTFSHYTLSW VRQAPGKGLEWVSVISGD |
| | | | | GSYTYYADSVKGRFTISSDNSKNTLYLQMNSLRAEDT AVYYCARNFIKYVFANWG |
| | | | | QGTLVTVSSGGGGSGGGGSGGGGSDIELTQPPSVSV APGQTARISCSGDNIGSFY |
| | | | | VHWYQQKPGQAPVLVIYDKSNRPSGIPERFSGSNSG NTATLTISGTQAEDEADYY |
| | | | | CQSYANTLSLVFGGGTKLTVL |
| | 2236 | F2: | antiFZD1, 2,7 (R2H1) | EVQLVESGGGLVQPGGSLRLSCAASGFNISSSYIHWV RQAPGKGLEWVAYIYSSY |
| | | | | GSTYYADSVKGRFTISADTSKNTAYLQMNSLRAEDTA VYYCARASWYALDYWG |
| | | | | QGTLVTVSSGSAASGSSGGSSSGADIQMTQSPSSLS ASVGDRVTITCRASQSVSSAV |
| | | | | AWYQQKPGKAPKLLIYSASSLYSGVPSRFSGSRSGTD FTLTISSLQPEDFATYYCQQ |
| | | | | YWYGVAPITFGQGTKVEIK |
| | 2237 | F3: | antiFZ05, 8 (2919) | EVQLVESGGGLVQPGGSLRLSCAASGFNISYSYIHWV RQAPGKGLEWVASIYSSSGS |
| | | | | TSYADSVKGRFTISADTSKNTAYLQMNSLRAEDTAVY YCARGAIDYWGQGTLVTV |
| | | | | SSGGGGSGGGGSGGGGSDIQMTQSPSSLSASVGDR VTITCRASQSVSSAVAWYQQK |
| | | | | PGKAPKLLIYSASSLYSGVPSRFSGSRSGTDFTLTISS LQPEDFATYYCQQWYSSGHV |
| | | | | LITFGQGTKVEIK |
| antiLRP_sc Fv | 2238 | L1: | antiLRP6E 1E2 (1115.3) | DVKLVESGGGLVKLGGSLRLSCAASGFSFSTSYMSW VRQTPEKRLELVAAINLNGGS |
| | | | | TYYSDTVKGRFTISRDNAKNTLYLQMSSLKSEDTAFY YCASELAGYGTPFAYWGQGT |
| | | | | LVTVSAGSAASGSSGGSSSGADIVLTQSPATLSVTPG DSVSLSCKASQSISYNLHWYQQ |
| | | | | KSHESPRLLIKYTSQSISGIPSRFSGSGSGTDFTLTINN VETEDFGMYFCQQSNSWPLTFG |
| | | | | AGTKLEVK |
| | 2239 | L2: | antiLRP6E 3E4 (G211) | EVQLVESGGGLVQPGGSLRLSCAASGFTFTSYYISWV RQAPGKGLEWVAEISPYSGST |
| | | | | YYADSVKGRFTISADTSKNTAYLQMNSLRAEDTAVYY CALRARPPIRLHPRGSVMDY |
| | | | | WGQGTLVTVSSGSAASGSSGGSSSGADIQMTQSPSS LSASVGDRVTITCRASQDVSTA |
| | | | | VAWYQQKPGKAPKLLIYSASFLYSGVPSRFSGSGSGT DFTLTISSLQPEDFATYYCQQS |
| | | | | YTTPPTFGQGTKVEIK |
| antiGFP_sc Fv | 2240 | | | QVQLVESGGGLVQPGGSLRLSCAASGFT FSRYGMHWVRQAPGKGLEWVSGISSIGS NTYYADSVKGRFTISRDNSKNTLYLQMNS LRAEDTAVYYCARWYKTYIDVWGQGTLVT |
| | | | | VSSGGGGSGGGGSGGGGSDIELTQPPSV SVAPGQTARISCSGDNLGKKYVYWYQQK PGQAPVLVIYGDDERPSGIPERFSGSNSG NTATLTISGTQAEDEADYYCASYDSSHILIV FGGGTKLTVL |
| linker between tandem scFv | 2241 | 5-mer (5) | | GSGSG |
| | 2242 | 10-mer (10) | | GSGSGGSGSG |
| | 2243 | 15-mer (15) | | GSGSGGSGSGGSSGG |
| hlgG1_Fc_ LALAPG | 2244 | Fc | | DKTHTCPPCPAPEAAGGPSVFLFPPKPKDTLMISR TPEVTCVVVDVSHEDPEVKFNWYVDG |
| | | | | VEVHNAKTKPREEQYNSTYRVVSVLTVLHQDWLN GKEYKCKVSNKALGAPIEKTISKAKG |
| | | | | |
| | | | | |
| | 2245 | Fc knob-FLAG | | DKTHTCPPCPAPEAAGGPSVFLFPPKPKDTLMISR TPEVTCVVVDVSHEDPEVKFNWYVDGV |
| | | | | EVHNAKTKPREEQYNSTYRVVSVLTVLHQDWLNG KEYKCKVSNKALGAPIEKTISKAKGQP |
| | | | | REPQVYTLPPSREEMTKNQVSLWCLVKGFYPSDIA VEWESNGQPENNYKTTPPVLDSDGSFFL |
| | | | | YSKLTVDKSRWQQGNVFSCSVMHEALHNHYTQKS LSLSPGKGGSGGDYKDDDDK |
| | 2246 | Fc_hole-His | | DKTHTCPPCPAPEAAGGPSVFLFPPKPKDTLMISR TPEVTCVVVDVSHEDPEVKFNWYVDGVE |
| | | | | VHNAKTKPREEQYNSTYRVVSVLTVLHQDWLNGK EYKCKVSNKALGAPIEKTISKAKGQPRE |
| | | | | PQVYTLPPSREEMTKNQVSLSCAVKGFYPSDIAVE WESNGQPENNYKTTPPVLDSDGSFFLVSK |
| | | | | LTVDKSRWQQGNVFSCSVMHEALHNHYTQKSLSL SPGKGSGSGHHHHHH |
| linker before tags or Fc | 2241 | | | GSGSG |
| tags | 2247 | Flag | | DYKDDDDK |
| | 2248 | His | | HHHHHH |

**Table 6B: Combinatorial Wnt surrogate Constructs**

| **FZD:LRP ratio** | **Construct** |
|---|---|
| 1 FZD: 1 LRP | L1:5:F1 His |
| | L1:10:F1 His |
| | L1:15:F1 His |
| | F1:5:L1 His |
| | F1:10:L1 His |
| | F1:15:L1 His |
| | L2:5:F1 His |
| | L2:10:F1 His |
| | L2:15:F1 His |
| | F1:5:L2 His |
| | F1:10:L2 His |
| | F1:15:L2 His |
| | L2:5:F2 His |
| | L2:15:F2 His |
| | F2:5:L2 His |
| | F2:15:L2 His |
| | L1:5:F2 His |
| | L1:15:F2 His |
| | F2:5:L1 His |
| | F2:15:L1 His |
| | F3:5:L1 His |
| | L1:5:F3 His |
| | F3:5:L2 His |
| | L2:5:F3 His |
| | F1:5:Fc knob+L2:5:Fc hole |
| | F1:5:αGFP:Fc_knob+L2:5:αGFP:Fc hole |
| | F1:5:αGFP:Fc_knob+αGFP:5:L2:Fc hole |
| | αGFP:5:F1:Fc_knob+L2:5:αGFP:Fc hole |
| | F3:5:Fc knob+L2:5:Fc hole |
| | F3:5:αGFP:Fc_knob+αGFP:5:L2:Fc hole |
| | αGFP:5:F3:Fc_knob+L2:5:αGFP:Fc hole |
| | L2:5mer:αGFP:Fc_knob+αGFP:5mer:F2:Fc hole |
| | F2:5:Fc knob+L2:5:Fc hole |
| | F2:5:αGFP:Fc knob+αGFP:5:L2:Fc hole |
| 2FZD:2LRP | L1:5:F1:Fc |
| | L1:10:F1:Fc |
| | L1:15:F1:Fc |
| | F1:5:L1:Fc |
| | F1:10:L1:Fc |
| | F1:15:L1:Fc |
| | L2:5:F1:Fc |
| | L2:10:F1:Fc |
| | L2:15:F1:Fc |
| | F1 :5:L2:Fc |
| | F1:10:L2:Fc |
| | F1:15:L2:Fc |
| | L1:5:F2:Fc |
| | L1:15:F2:Fc |
| | F2:5:L1:Fc |
| | F2:15:L1:Fc |
| | L2:5:F2:Fc |
| | L2:15:F2:Fc |
| | F2:5: L2: Fc |
| | F2:15:L2:Fc |
| | F3:5:L1:Fc |
| | F3:5:L2:Fc |
| | L1:5:F3:Fc |
| | L2:5: F3: Fc |
| | F1:5:Fc:5:L1 |
| | L1:5:Fc:5:F1 |
| | F1:5:Fc:5:L2 |
| | L2:5:Fc:5:F1 |
| | F3:5:L2:Fc knob+F2:5:L2:Fc hole |
| | L2:5:F3:Fc knob+L2:5:F2:Fc hole |
| | L1:5:F3:Fc knob+L2:5:F2:Fc hole |
| | F3:5:L1:Fc knob+F2:5:L2:Fc hole |
| 0FZD:2LRP | aGFP:5:L1:Fc |
| | L1:5:αGFP:Fc |
| | αGFP:5:L2:Fc |
| | L2:5:αGFP:Fc |
| 2FZD:0LRP | αGFP:5:F1:Fc |
| | F1:5:αGFP:Fc |
| 2FZD:1LRP | F2:5:L2:Fc knob+F2:5:αGFP:Fc hole |
| | L2:5:F2:Fc knob+αGFP:5:F2:Fc hole |
| | αGFP:5:F2:Fc knob+L2:5:F2:Fc hole |
| | L2:5:F3:Fc knob+αGFP:5:F2:Fc hole |
| 1FZD:2LRP | F2:5:L2:Fc knob+αGFP:5:L2:Fc hole |
| | L2:5:F2:Fc knob+L2:5:αGFP:Fc hole |
| | F3:5:L2:Fc knob+αGFP:5:L2:Fc hole |
| | L2:5:F3:Fc knob+L2:5:αGFP:Fc hole |

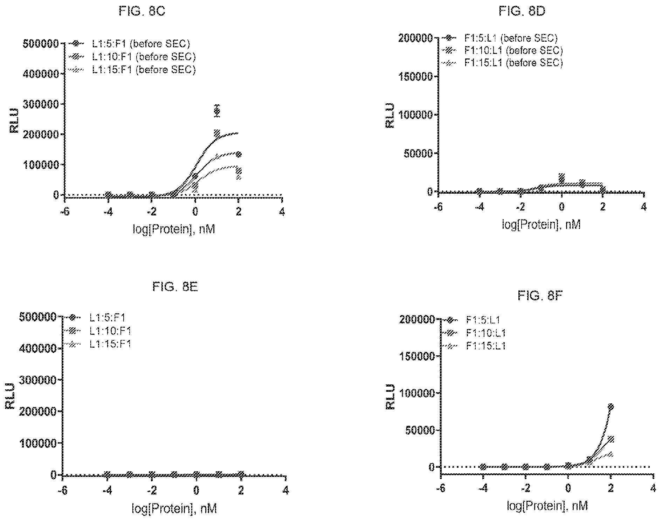

A set of molecules generated replaced the DKK1c component in the 18R5-DKK1c molecule (Janda et al., 2017, Nature, 545(7653):234-237) with LRP binding antibody fragments in scFv format to generate a tandem scFv molecule, depicted in Fig. 8A. The LRP binder is a LRP6 E1E2 domains binder, 1115.3 (US 8,715,941 B2), which is referred herein as L1. To test the impact of geometry to activity, L1 was fused to either the N- or C-terminus of 18R5 scFv (referred herein as F1) with 5, 10, or 15 amino acid linkers in between. This set of six proteins were purified to near homogeneity via Ni-affinity column (Fig. 8B) and tested in WNT responsive 293 STF reporter cells. While linker length did not seem to significantly affect activity, having the LRP6 binder fused to the N-terminus of the FZD binder, resulted in much higher activity than the other orientation (compare Fig. 8C to Fig. 8D, note the differences in the y-axis). All of these new surrogate molecules showed lower Emax (maximal response) than did both WNT3A and 18R5-DKK1c tested in parallel (Fig. 9).

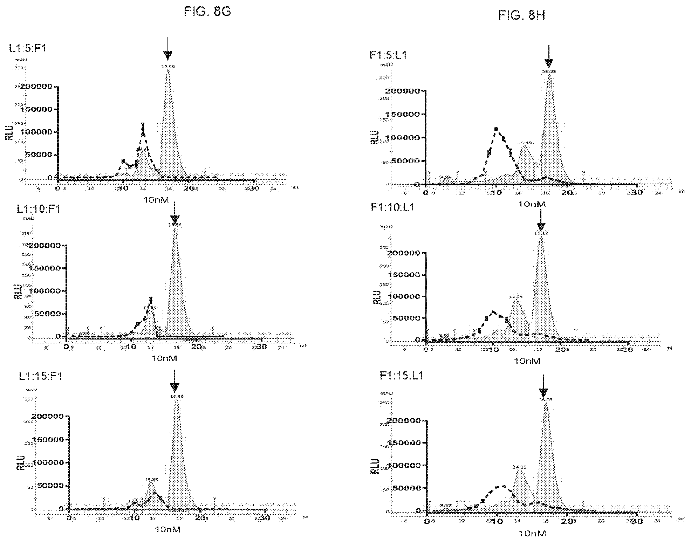

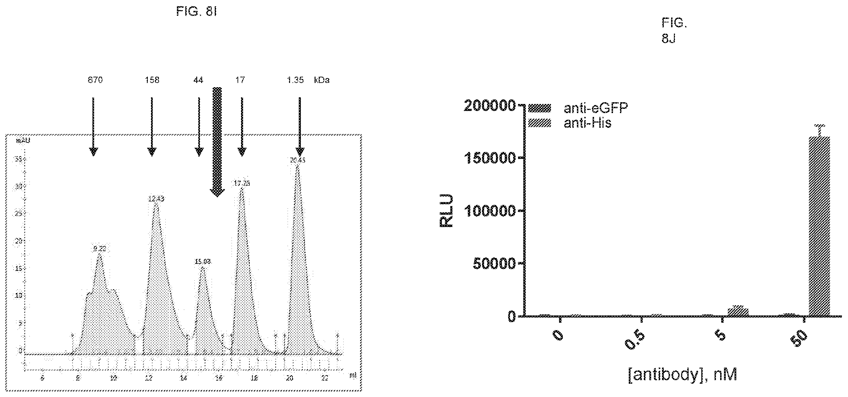

Since scFv fragments have tendencies to form higher molecular weight aggregates, to confirm whether the surrogate WNT activities came from the monomeric form of the tandem scFv molecules, these six proteins were purified on size-exclusion-chromatography (SEC) and the peak fractions corresponding to the monomeric forms were tested in the 293 STF reporter cells. In contrast to the Ni purified material before SEC, the SEC purified proteins significantly lost activity (compare Fig. 8E and 8F to 8C and 8D). The fractions across the SEC runs were assayed in the 293 STF reporter cells. Peak activities were identified across the column fractions; however, the STF activity peaks did not coincide with the protein peak of the monomeric molecules. Instead, in all cases, the STF activity peaked in the higher molecular weight fractions (Fig. 8G and 8H, SEC molecular weight standards shown in Fig. 8I), suggesting that the 1:1 tandem scFv molecules were ineffective in inducing β-catenin dependent signaling. These results suggest that efficient activation of WNT signaling requires multimerization of receptor complexes. The tandem scFv molecules, which contain a 6 x His tag, were artificially crosslinking with anti-His antibodies and tested in the 293 STF reporter assay. As shown in Fig. 8J, while L1:5:F1 from the monomeric faction alone was not effective in activating β-catenin dependent signaling in the presence of an isotype control (anti-GFP) antibody, the addition of anti-His antibody significantly induced signaling, supporting the idea that multimerization of receptor complexes may be required for signaling.

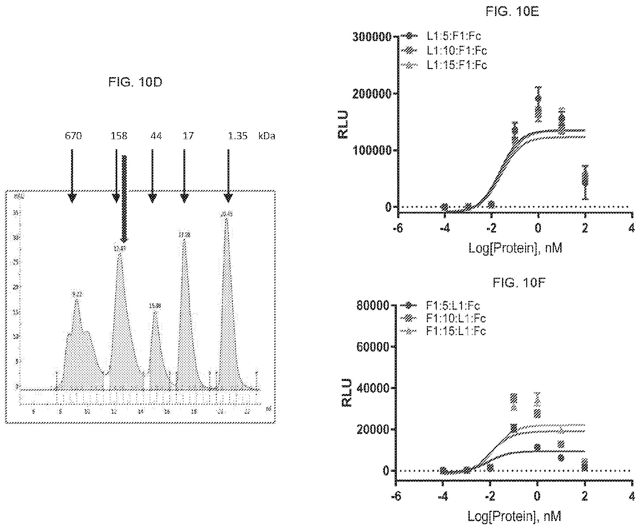

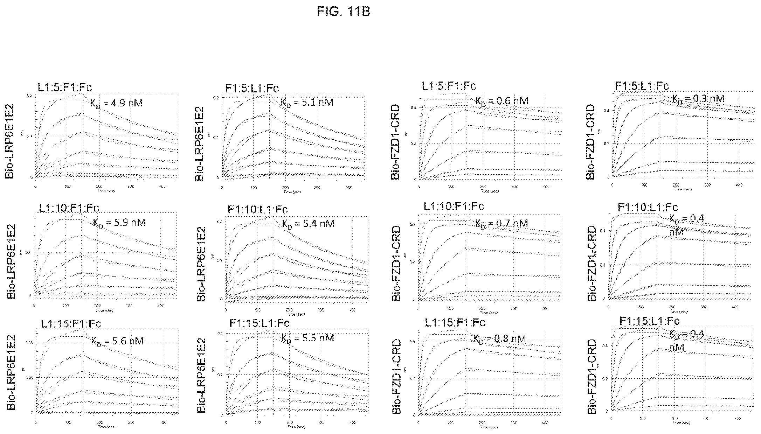

Multivalent molecules wre produced in a more defined and easily produced format by attaching L1 and F1 tandem scFv fusions to the N-terminus of an Fc fragment as depicted in Fig. 10A to generate a tetravalent bispecific format. In this format, there are two binding sites against each receptor target (also noted as 2:2 in later sections representing a stoichiometric ratio of two FZDs and two LRPs). These proteins were purified by Protein A affinity step followed by SEC. As shown in Fig. 10B and 10C, the activity peaks from the 293 STF assay across the SEC fractions coincided with the protein peaks that corresponded to the monomer species of the molecule shown in Fig. 10A. SEC molecular weight standards are shown in Fig. 10D. These new configurations demonstrated a correlation between STF and major protein peaks. Dose response curves of the peak fractions were also performed, while linker length did not seem to significantly affect activity, the relative orientation of the two binders did affect activity. Fusing L1 to the N-terminus of F1 is the preferred orientation for the combination of these two binders (compare Fig. 10E and 10F y-axis). The relative Emax of the activities of these multivalent bispecific FZD/LRP binding molecules as compared to WNT3A and 18R5-DKK1c are similar to that of the pre-SEC material shown in Fig. 8 (Fig. 11). The affinity of the binding arms to their respective receptors in this tetravalent bispecific format was determined using bio-layer interferometry on an Octet instrument. As shown in Fig. 11B the relative orientation and linker lengths had no impact on the affinity of the respective arms to their target receptors, suggesting that the effects of orientation on the reporter activity is not due to impact of format on binding but rather the geometry of the receptors assembled by the surrogate molecules.

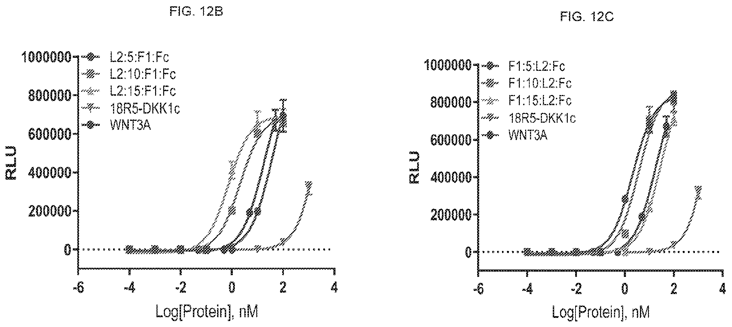

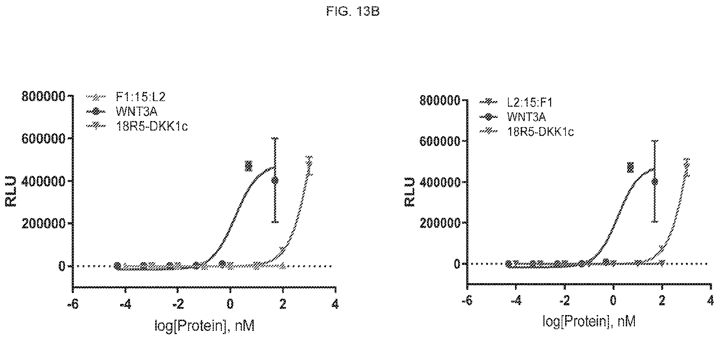

DKK1c predominantly binds LRP E3E4 domain (Ahn et al., 2011, Cheng et al., 2011). To understand the impact of LRP epitope on activity, the fusion of F1 with an LRP6E3E4 binder, YW211.31.57 (US 8,846,041 B2) referred herein as L2, was tested in the 1:1 tandem scFv format where L2 was fused to either the N- or C-terminus of F1 scFv with 5, 10, or 15 amino acid linkers in between. While these fusion proteins behaved less well and expressed at lower levels compared to the L1/F1 fusion proteins (Fig. 13A), similar to L1/F1 fusion proteins shown in Fig. 8, the 1:1 tandem scFv of L2/F1 fusions were also ineffective in inducing β-catenin dependent signaling in 293 STF reporter cells as seen both in activity tests across the SEC column (Fig. 13A) as well as titration of monomeric peak fractions from the SEC column (Fig. 13B). Fusion of the tandem scFv L2/F1 molecules to an Fc fragment to generate a 2:2 tetravalent bispecific molecule efficiently activated canonical WNT signaling. As shown in Fig. 12A, the peak from the 293 STF assay across the SEC fractions coincided with the protein peak that corresponded to the monomeric species. These L2/F1 fusion molecules are much more active than the L1/F1 fusion molecules with Emax in range similar to WNT3A and significantly improved potency beyond both WNT3A and 18R5-DKK1c (Fig. 12B). The L2 and F1 combinations are less sensitive to orientation; both orientations resulted in similar Emax, while having L2 on the N-terminus of F1 with a 15mer linker yielded higher potency (Fig. 12B and 12C).

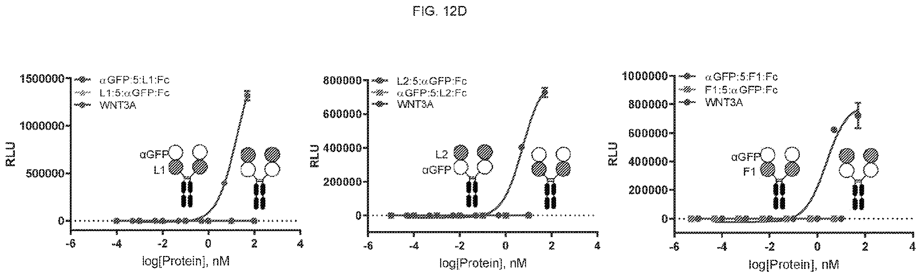

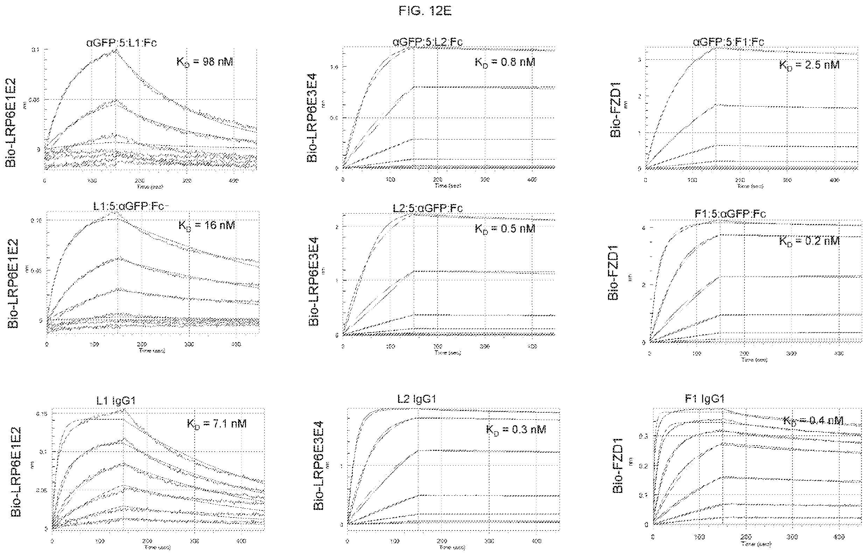

As a control to verify that the 2:2 tetravalent bispecific molecules activate WNT signaling requires binding to both FZD and LRP receptors, either the FZD binder (F1) or the LRP binders (L1 or L2) was replaced with an scFv fragment that binds GFP in the 2:2 tetravalent bispecific format. These moleclues are referred to as tetravalent monospecific or 2:0 or 0:2 formats. As shown in Fig. 12D and 12E, while all of these molecules bind to their respective receptors, none of these tetravalent monospecific molecules are active, demonstrating that the ability to activate WNT signaling requires the engagement of both FZD and LRP.

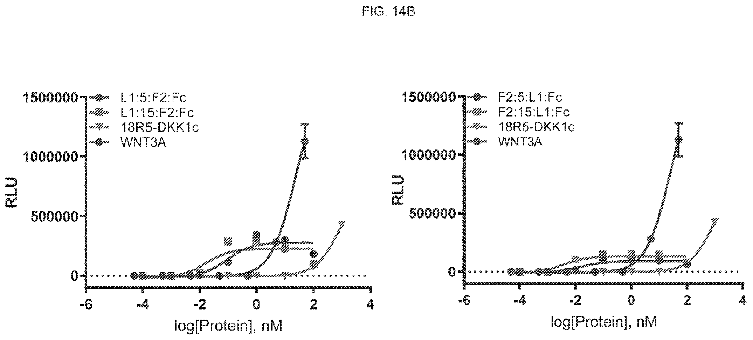

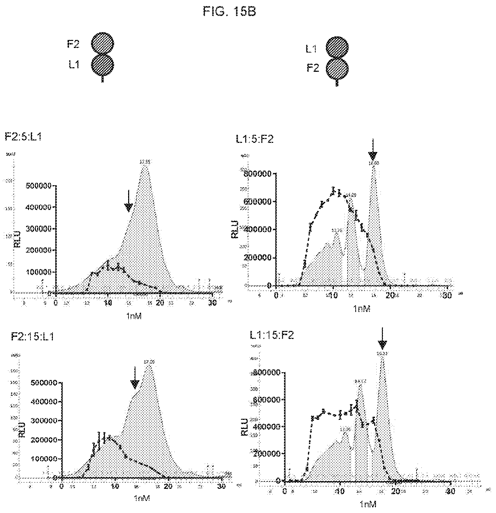

To generate WNT surrogate molecules with different FZD specificity and to understand whether the multivalency requirement for WNT signaling is a general requirement beyond the FZD binder F1 (which binds FZD1,2,7,5,8, Gurney et al., 2012), fusion proteins were generated between additional FZD binders, R2H1 (US 2016/0194394 A1) which binds to FZD1,2,7 referred herein as F2, and 2919 (WO 2017/127933 A1) which binds to FZD5,8 referred herein as F3, with the two LRP6 binders L1 and L2. The specificity of F2 and F3 were verified in Octet binding assays (Fig. 15A)
F2/L1 combination in the 1:1 tandem scFv format were contructed with 5 and 15mer linkers. Similar to the F1 fusion data in Fig. 8, the peak STF activities across the SEC column broadly distributed in the higher molecular weight fractions and did not coincide with the peak of the monomeric form of the molecules (Fig. 15B). In contrast, a STF peak activity across the SEC column coincided with the monomeric forms when the tandem scFvs were fused to Fc fragments in the 2:2 tetravalent bispecific format (Fig. 14A). A second STF activity peak on the SEC column was also observed in the higher molecular weight fractions that were not observed with the F1 fusion molecules (Fig. 14A). The dose response curves from the monomeric SEC peak fractions on 293 STF reporter cells showed slight preference for L1 fusion to the N-terminus of F2 for higher activity (Fig. 14B) similar to the fusions with F1 (Fig. 10).

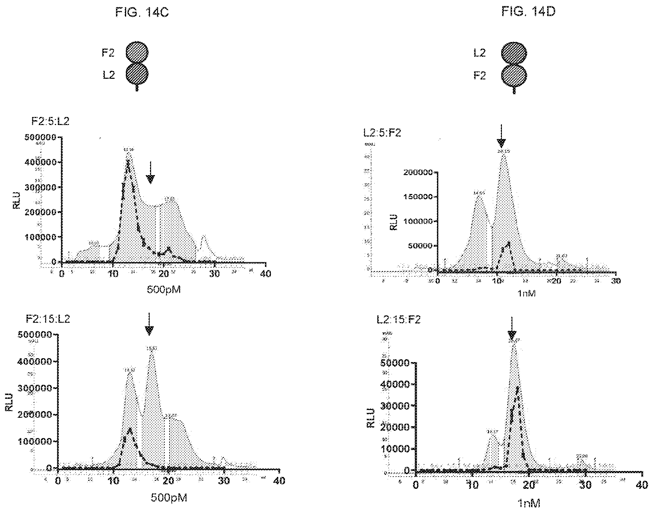

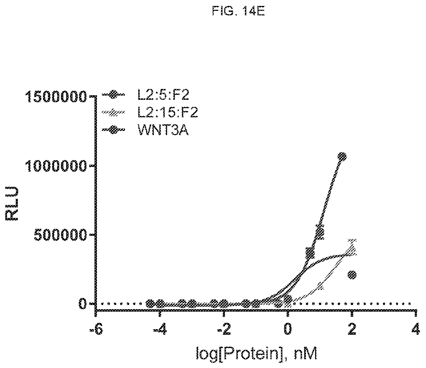

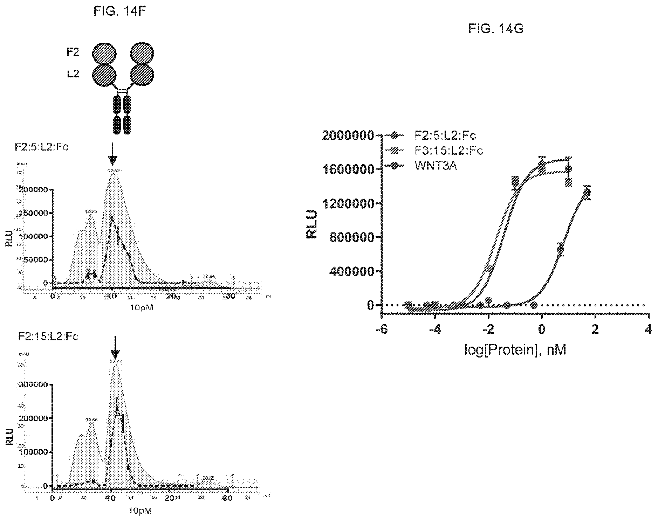

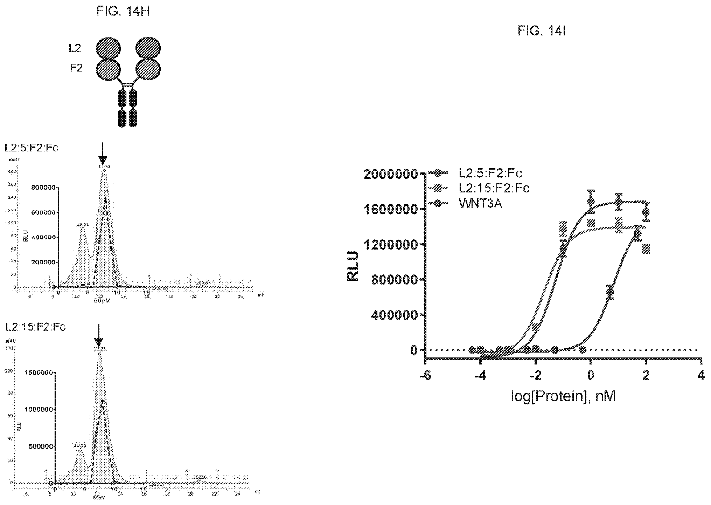

Fusions between F2 and L2 were also constructed in the various formats (Table 6B). While on SEC column, the peak STF activities did not coincide with the monomeric forms of F2 fused to the N-terminus of L2 in the 1:1 format (Fig. 14C), it appears that the monomeric 1:1 forms of the reverse orientation where L2 was fused to the N-terminus of F2 were active (Fig. 14D). Dose response of the peak fractions in this orientation showed weaker activity compared to WNT3A with a preference for the 5mer linker (Fig. 14E). The 2:2 tetravalent bispecific format from the fusion of these tandem scFvs to the N-terminus of Fc yielded highly potent molecules. On SEC column, the peak STF activities coincided with protein peak that's consistent with the monomeric forms (Fig. 14F and 14H), and dose responses in 293 STF reporter cells showed potent and efficacious molecules, exceeded the activities of the F1/L2 fusions shown in Fig. 3 (Fig. 14G and 14I).

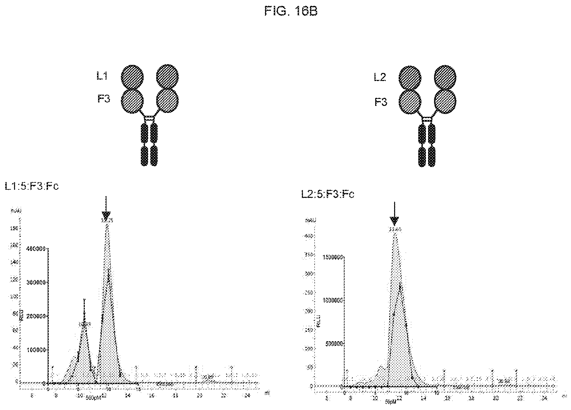

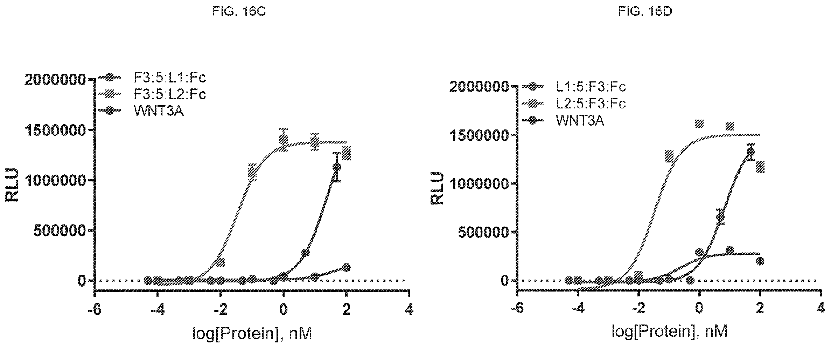

The 1:1 bivalent bispecific tandem scFv format and the 2:2 tetravalent bispecific fusions to Fc were also constructed between F3 and the two LRP binders. Similar to the observation with F1 fusions shown in Fig. 8, no robust activities were observed for the 1:1 tandem scFv fusion molecules between F3 and L1 or L2 (Fig. 17). In contrast, 293 STF reporter activities of the fusion molecules between F3, LRP binders, and Fc in the 2:2 tetravalent bispecific format from the SEC column fractions coincided to the protein peak of the monomeric forms of the molecules (Fig. 16A and 16B). It is interesting to note that similar to fusions with F1 and F2, the dose response of the peak SEC fractions show that the L2-F3 fusions are highly active in both orientations, while L1-F3 fusions are less active and show preference of L1 on the N-terminus of F3 (Fig. 16C and 16D). All of these results together suggest that multivalency of FZD and LRP binding with the stoichiometry of two FZDs and two LRPs are required for consistent and efficient activation of β-catenin dependent signaling, at least with the binders tested.

Having established the multivalency requirement for efficient induction of canonical WNT signaling, alternative 2:2 tetravalent bispecific format where the two scFv binders are attached two the two ends of a Fc fragment as shown in Fig. 18A and 18B were explored (termed dumbbell format). The fusion of F1 with L1 in this dumbbell format minimally activated WNT signaling in reporter assay (Fig. 18A). Interestingly, there is also a preference for the orientation of the L1 molecule where its attachment C-terminal to Fc affected activity. The fusions of F1 and L2 in this format also activated WNT signaling with similar preference in orientation for the LRP binding arm (Fig. 18B). The activity across the SEC columns were also tested for these molecules, and as shown in Fig. 18C, peak STF reporter activities coincided with the protein peak corresponding to the monovalent forms of the proteins.

To understand whether the activity from the 2:2 tetravalent bispecific format is due to the simultaneous binding of the same surrogate molecule to multiple FZDs and LRPs or whether it is due to a unique geometry provided by the format that does not require the multivalent binding to receptors, Octet binding studies to assess whether the 1:1 tandem scFv format allows simultaneous binding to both FZD and LRP. As shown in Fig. 19A, by sequential addition of FZD8 CRD to sensor surface, followed by 1:1 tandem scFv L2:5:F3 or F3:5:L2, and then LRP6 E3E4, a stepwise increase in binding signal was observed. This suggest that the 1:1 tandem scFv molecules are capable of binding to both receptors simultaneously, and therefore, the lack of activity from these molecules in inducing WNT signaling is not due to inability to crosslink FZD and LRP receptors.

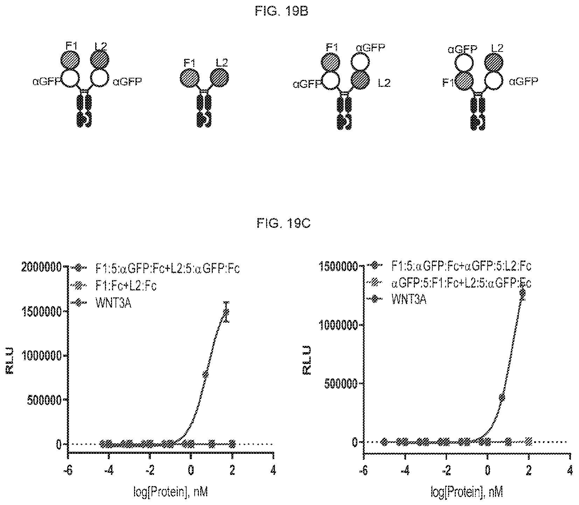

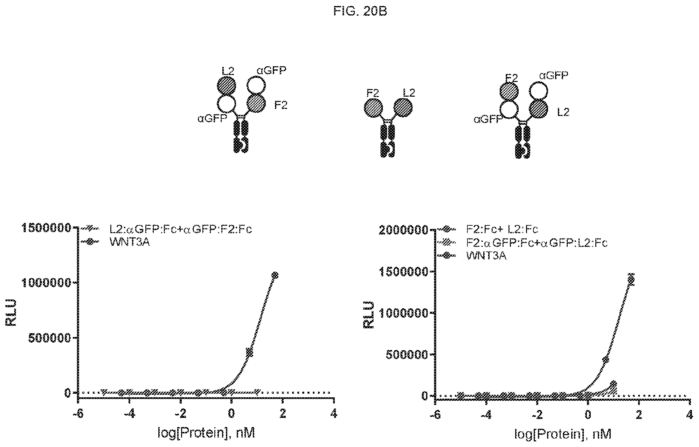

To determine if the 2:2 tetravalent bispecific format still engages the receptors in the 1:1 fashion, however, through a unique geometry that allows signaling not provided by the 1:1 tandem scFv format. We constructed 4 different variations of the possible 1:1 geometry on the framework of the 2:2 tetravalent bispecific molecule as depicted in Fig. 19B and Fig. 20. None of these 4 molecules induced WNT signaling (Fig. 19C and Fig. 20A) suggesting that crosslinking multiple copies of the receptors are required for efficient signaling.

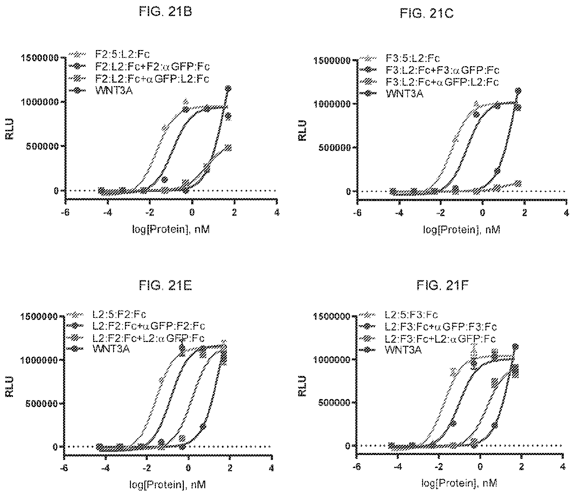

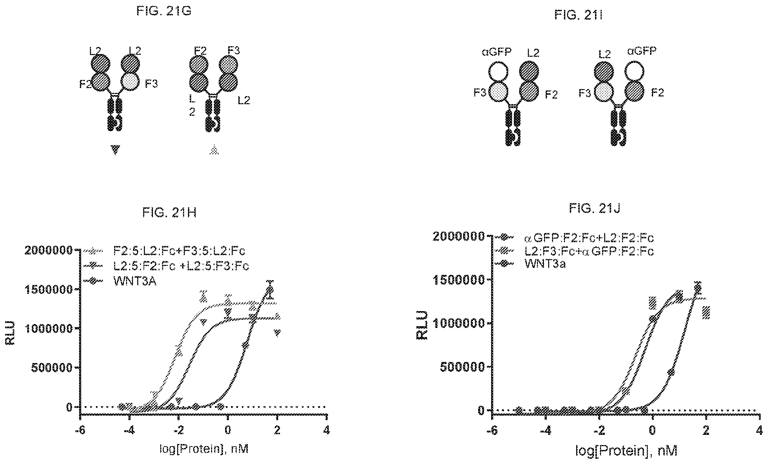

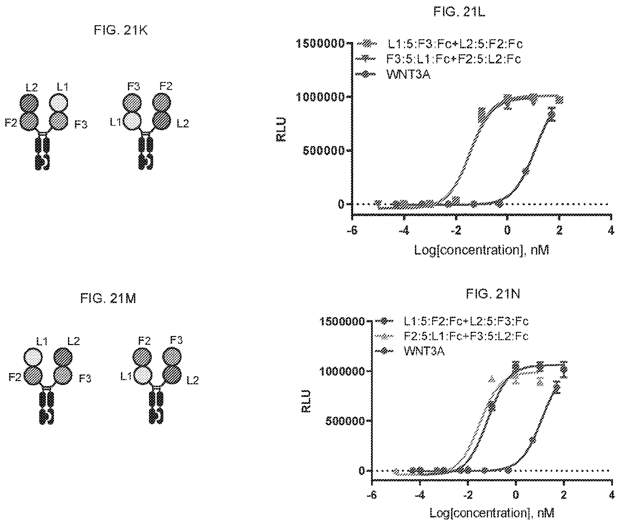

To further understand the required ratio of FZD to LRP receptors, either 2 FZD binders with 1 LRP binder (2:1) or 1 FZD binder combined with 2 LRP binders (1:2) were constructed as depicted in Fig. 21A and 21D. As shown in Fig. 21, removing either 1 FZD binder or 1 LRP binder from the 2:2 format still activated β-catenin dependent signaling. Since the 1:1 formats shown in Fig. 19 did not activate signaling, these results further supports the notion that simultaneous engagement of the multiple FZDs and LRPs are necessary for efficient signaling. As removing 1 LRP binder having much less impact on the activity than removing 1 FZD binder, these results also suggest that dimerization of FZD receptors is more important for signaling complex formation.

The multivalency requirement for the efficient induction of canonical WNT signaling is established, however, since each of the FZD binding arms in the surrogate molecules have the specificity to several different FZD receptors, it is not clear whether signaling can come from heterodimerization of different FZD receptors. To address this question, two FZD binders that have non-overlapping specificity, F2 and F3, were combined into one molecule as depicted in Fig. 21G. While either F2 or F3 in the 1:2 format (1 FZD binder combined with 2 copies of LRP binders, Fig. 21B and 21C) showed low to very low activities in the 293STF reporter cells, respectively, the combination of F2 and F3 together with 2 copies of LRP binders L2 into one molecule (we termed this 1:1:2 format with the stoichiometry of one FZD plus one different FZD plus two LRPs) yielded highly potent and efficacious surrogate molecules (Fig. 21H). To further confirm this, a molecule with F2 and F3 and one copy of LRP binder L2 was constructed (1:1:1:0 format with the stoichiometry of one FZD plus one different FZD plus one LRP binder, Table 6B and Fig. 21i). This molecule is also highly active (Fig. 21J), even though F2 or F3 alone with one copy of L2 showed no activity (Fig. 20). Since F2 and F3 bind to different FZDs, these results suggest that heterodimerization of FZD leads to β-catenin dependent signaling.

As a final configuration, we also constructed surrogate molecules with two different FZD binding arms and two different LRP binding arms (1:1:1:1 format) as depicted in Fig. 21K. It is interesting to note that although surrogate molecules containing the LRP binder L1 consistently yielded much lower Emax compared to WNT3A, when combined with L2 in the context of 1:1:1:1 format, yielded highly potent and efficacious molecules (Fig. 21L).

All the observation together demonstrate a new surrogate WNT platform that is flexible, can produce any FZD/LRP specificity combination, and can generate highly potent and active molecules.

The various embodiments described above can be combined to provide further embodiments. All of the U.S. patents, U.S. patent application publications, U.S. patent applications, foreign patents, foreign patent applications and non-patent publications referred to in this specification and/or listed in the Application Data Sheet are incorporated herein by reference, in their entirety. Aspects of the embodiments can be modified, if necessary to employ concepts of the various patents, applications and publications to provide yet further embodiments. These and other changes can be made to the embodiments in light of the above-detailed description.

In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### CLAUSES

Embodiments of the invention are defined in the clauses below::
1. A multispecific Wnt surrogate molecule, wherein the Wnt surrogate molecule comprises:
   (i) a plurality of regions that each specifically bind to a set of one or more Frizzled (Fzd) receptor epitopes (Fzd binding regions), wherein at least two Fzd binding regions bind to different sets of one or more Fzd receptor epitopes; and
   (ii) one or more regions that specifically bind to a Low-density lipoprotein (LDL) receptor-related protein 5 (LRP5) and/or a LDL receptor-related protein 6 (LRP6) (LRP5/6 binding regions).
2. The Wnt surrogate molecule of clause 1, wherein the at least two Fzd binding regions bind to different sets of one or more Fzd receptors, different sets of one or more epitopes within the same set of one or more Fzd receptors, or a combination thereof.
3. The Wnt surrogate molecule of any one of clauses 1-2, wherein each Fzd binding region binds to one or more of Frizzled 1 (Fzd1), Frizzled 2 (Fzd2), Frizzled 3 (Fzd3), Frizzled 4 (Fzd4), Frizzled 5 (Fzd5), Frizzled 6 (Fzd6), Frizzled 7 (Fzd7), Frizzled 8 (Fzd8), Frizzled 9 (Fzd9), and Frizzled 10 (Fzd10).
4. The Wnt surrogate molecule of any one of clauses 1-3, wherein at least one Fzd binding region binds to: (i) Fzd1, Fzd2, Fzd7, and Fzd9; (ii) Fzd1, Fzd2, and Fzd7; (iii) Fzd5 and Fzd8; (iv) Fzd5, Fzd7, and Fzd8; (v) Fzd1, Fzd4, Fzd5, and Fzd8; (vi) Fzd1, Fzd2, Fzd5, Fzd7, and Fzd8; (vii) Fzd4 and Fzd9; (viii) Fzd9 and Fzd10; (ix) Fzd5, Fzd8, and Fzd10; (x) Fzd4, Fzd5, and Fzd8; or (xi) Fzd1, Fzd5, Fzd7 and Fzd8.
5. The Wnt surrogate molecule of any one of clauses 1-4, wherein the plurality of Fzd binding regions comprises:
   (i) a first Fzd binding region that binds to a first set of one or more Fzd receptors, and
   (ii) a second Fzd binding region that binds to a second, different set of one or more Fzd receptors.
6. The Wnt surrogate molecule of clause 5, wherein:
   (i) the first Fzd binding region binds to one or more of Fzd1, Fzd2, Fzd3, Fzd4, Fzd5, Fzd6, Fzd7, Fzd8, Fzd9, and Fzd10, and
   (ii) the second Fzd binding region binds to one or more of Fzd1, Fzd2, Fzd3, Fzd4, Fzd5, Fzd6, Fzd7, Fzd8, Fzd9, and Fzd10.
7. The Wnt surrogate molecule of clause 5, wherein the first Fzd binding region binds to Fzd4 and the second Fzd binding region binds to Fzd9.
8. The Wnt surrogate molecule of any one of clauses 1-7, wherein the plurality of Fzd binding regions comprises:
   (i) a first Fzd binding region that binds to a first set of one or more epitopes within a set of one or more Fzd receptors, and
   (ii) a second Fzd binding region that binds to a second, different set of one or more epitopes within the same set of one or more Fzd receptors.
9. The Wnt surrogate of clause 8, wherein:
   a) the first Fzd binding region binds to at least one Fzd receptor that induces non-canonical Wnt signaling; and
   b) the second Fzd binding region binds to at least one Fzd receptor that induces canonical Wnt signaling.
10. The Wnt surrogate of clause 9, wherein binding of the first Fzd receptor and second Fzd receptor results in:
   a. canonical Wnt signaling; or
   b. non-canonical Wnt signaling.
11. The Wnt surrogate molecule of any one of clauses 1-10, wherein at least one Fzd binding region binds monospecifically to a single Fzd receptor.
12. The Wnt surrogate molecule of clause 11, wherein the at least one Fzd binding region binds monospecifically to Fzd1, Fzd2, Fzd3, Fzd4, Fzd5, Fzd6, Fzd7, Fzd8, Fzd9, or Fzd10.
13. The Wnt surrogate molecule of any one of clauses 1-12, wherein at least one Fzd binding region binds to a region of a Fzd receptor that (i) does not include the cysteine rich domain (CRD) of the Fzd receptor or (ii) includes less than the entire CRD of the FZD receptor or (iii) overlaps with the CRD of the Fzd receptor.
14. The Wnt surrogate molecule of clause 13, wherein the at least one Fzd binding region binds to a hinge region of the Fzd receptor.
15. The Wnt surrogate molecule of clause 14, wherein the hinge region comprises an amino acid sequence having at least 90% identity to any of the sequences set forth in SEQ ID NOs:98-107.
16. The Wnt surrogate molecule of clause 13 wherein the at least one Fzd binding region binds to an N-terminal region upstream of the CRD of the Fzd receptor.
17. The Wnt surrogate molecule of clause 16, wherein the N-terminal region comprises an amino acid sequence having at least 90% identity to SEQ ID NO:108.
18. The Wnt surrogate molecule of any one of clauses 1-17, wherein at least one of the Fzd binding regions comprises one or more antigen-binding fragments of an antibody.
19. The Wnt surrogate molecule of clause 18, wherein the one or more antigen-binding fragments are selected from the group consisting of: IgG, Fv, scFv, Fab, and VHH or sdAbs.
20. The Wnt surrogate molecule of any one of clauses 18-19, wherein the one or more antigen-binding fragments are humanized.
21. The Wnt surrogate molecule of any one of clauses 1-20, wherein at least one Fzd binding region comprises an amino acid sequence having at least 90% identity to any of the sequences set forth in Table 1A, Table 1B, or SEQ ID NOs:1-73, or an antigen-binding fragment thereof.
22. The Wnt surrogate molecule of any one of clauses 1-21, wherein the one or more LRP5/6 binding regions comprises one or more antigen-binding fragments of an antibody.
23. The Wnt surrogate molecule of clause 22, wherein the one or more antigen-binding fragments are selected from the group consisting of: IgG, Fv, scFv, Fab, and VHH or sdAb.
24. The Wnt surrogate molecule of any one of clauses 22-23, wherein the one or more antigen-binding fragments are humanized.
25. The Wnt surrogate molecule of any one of clauses 1-24, wherein the one or more LRP5/6 binding regions comprise an amino acid sequence having at least 90% identity to any of the sequences set forth in Table 2A, Table 2B, or SEQ ID NOs:74-97, or an antigen-binding fragment thereof.
26. The Wnt surrogate molecule of any one of clauses 1-25, comprising one or more LRP5/6 binding regions.
27. The Wnt surrogate molecule of any one of clauses 1-26, wherein the Fzd binding regions and the LRP5/6 binding regions are in a ratio of Fzdₙ:LRP5/6ₙ (Fₙ:Lₙ), wherein F and L are integers between 1 and 9, inclusive, and n is an integer between 1 and 4 inclusive.
28. The Wnt surrogate molecule of any one of clauses 1-27, wherein the Fzd binding regions and the LRP5/6 binding regions are in a ratio of Fzd:LRP5/6 selected from the group consisting of: 1:1, 2:1, 3:1, 4:1, 5:1, 6:1, 2:1 2:3, 2:5, 2:7, 7:2, 5:2, 3:2, 3:4, 3:5, 3:7, 3:8, 8:3, 7:3, 5:3, 4:3, 4:5, 4:7, 4:9, 9:4, 7:4, 5:4, 6:7, 7:6, 1:2, 1:3, 1:4, 1:5, 1:6, 2:1 (with two Fzd binders and one LRP binder), 1:2 (with one Fzd binder and two LRP binders), 2:1:1 (with two different LRP binders), 1:1:2 (with two different Fzd binders), 1:1:1 (two different Fzd binders and one LRP binder or one Fzd binder and two different LRP binders) and 1:1:1:1 (all different Fzd and LRP binders).
29. The Wnt surrogate molecule of clause 28, wherein the ratio of Fzd binding regions to LRP5/6 binding regions (Fzd:LRP5/6) comprises:
   a) 2 Fzd binding regions and 2 LRP5/6 binding regions;
   b) 2 Fzd binding regions and 1 LRP5/6 binding region; and
   c) 1 Fzd binding region and 2 LRP5/6 binding regions.
30. The Wnt surrogate molecule of clause 28, wherein the ratio of Fzd binding regions to LRP5/6 binding regions (Fzd:LRP5/6) comprises:
   a) a first Fzd binding region, a second Fzd binding region, and 1 LRP5/6 binding region; or
   b) a first Fzd binding regions, a second Fzd binding regions, a first LRP5/6 binding region, and a second LRP5/6 binding region,
   wherein the first Fzd and second Fzd binding regions bind to same or different Fzd receptors, or binding to the same Fzd receptor on different regions/epitope, and the first LRP5/6 and second LRP5/6 binding regions bind to different epitopes or to same or different LRP proteins.
31. The Wnt surrogate molecule of any one of clauses 1-30, wherein two or more LRP binding regions comprise:
   (i) a first LRP binding region that binds to a first set of one or more LRP receptors, and
   (ii) a second LRP binding region that binds to a second, different set of one or more LRP receptors.
32. The Wnt surrogate molecule of any one of clauses 1-31, comprising a structural format selected from the group consisting of: hetero-Ig, diabody (DART), tandem diabody (DART), diabody-Fc, Fabs-in-tandem, Fabs-in-tandem IgG (FIT-Ig), Fv-IgG, and tandem scFv.
33. The Wnt surrogate molecule of any one of clauses 1-32, comprising (i) a first light chain and a first heavy chain forming a first Fzd binding region, and (ii) a second light chain and a second heavy chain forming a second Fzd binding region, wherein the first and second Fzd binding regions bind to same or different sets of one or more Fzd receptor epitopes.
34. The Wnt surrogate molecule of clause 33, comprising a first LRP5/6 binding region fused to an N-terminus of the first light chain, a C-terminus of the first light chain, an N-terminus of the first heavy chain, or a C-terminus of the first heavy chain.
35. The Wnt surrogate molecule of any one of clauses 33-34, comprising a second LRP5/6 binding region fused to an N-terminus of the second light chain, a C-terminus of the second light chain, an N-terminus of the second heavy chain, or a C-terminus of the second heavy chain.
36. The Wnt surrogate molecule of any one of clauses 33-35, wherein the first and second heavy chains are connected to each other.
37. The Wnt surrogate molecule of clause 36, wherein the first heavy chain comprises a first CH3 domain, the second heavy chain comprises a second CH3 domain, and the first and second CH3 domains are connected to/interact with each other.
38. The Wnt surrogate molecule of clause 37, wherein the first and second CH3 domains are connected to each other via knobs-into-holes mutations.
39. The Wnt surrogate molecule of any one of clauses 33-38, wherein: (i) the first heavy chain and/or the second heavy chain comprise an amino acid sequence having at least 90% identity to any of the sequences set forth in SEQ ID NOs:110, 112, 114, 116, 118, 120, and 122, and (ii) the first light chain and/or the second light chain comprise an amino acid sequence having at least 90% identity to any of the sequences set forth in SEQ ID NOs:109, 111, 113, 115, 117, 119, and 121.
40. The Wnt surrogate molecule of clause 1, comprising one or more sequences set forth in Table 5 or Table 6A.
41. The Wnt surrogate molecule of clause 41, having a structure as set forth in Table 6B.
42. The Wnt surrogate molecule of any of clauses 1-41, which modulates a Wnt signaling pathway in a cell, optionally a mammalian cell.
43. The Wnt surrogate molecule of clause 42, which increases signaling via the Wnt signaling pathway in the cell.
44. The Wnt surrogate molecule of any one of clauses 42-43, wherein the Wnt signaling pathway is a canonical Wnt signaling pathway.
45. The Wnt surrogate molecule of any one of clauses 42-43, wherein the Wnt signaling pathway is a non-canonical Wnt signaling pathway.
46. A pharmaceutical composition comprising a pharmaceutically acceptable excipient, diluent, or carrier, and the Wnt surrogate molecule according to any of clauses 1-45.
47. A method for agonizing a Wnt signaling pathway in a cell, comprising contacting the cell with the Wnt surrogate molecule according to any of clauses 1-45 or the pharmaceutical composition of clause 45, wherein the Wnt surrogate molecule is an agonist of a Wnt signaling pathway.
48. A method for treating a subject having a disease or disorder, comprising administering to the subject an effective amount of the Wnt surrogate molecule according to any of clauses 1-45 or the pharmaceutical composition of clause 44, wherein the Wnt surrogate molecule is an agonist of a Wnt signaling pathway.
49. The method of clause 48, wherein the disease or disorder is associated with reduced or impaired Wnt signaling, and/or wherein the subject would benefit from increased Wnt signaling.
50. The method of any one of clauses 48-49, wherein the disease or disorder is selected from the group consisting of: bone fractures, stress fractures, vertebral compression fractures, osteoporosis, osteoporotic fractures, non-union fractures, delayed union fractures, spinal fusion, pre-operative optimization for spine surgeries, osteonecrosis, osseointegration of implants or orthopedic devices, osteogenesis imperfecta, bone grafts, tendon repair, tendon-bone integration, tooth growth and regeneration, maxillofacial surgery, dental implantation, periodontal diseases, maxillofacial reconstruction, osteonecrosis of the jaw, hip or femoral head, avascular necrosis, alopecia, hearing loss, vestibular hypofunction, macular degeneration, age-related macular degeneration (AMD), vitreoretinopathy, retinopathy, diabetic retinopathy, diseases of retinal degeneration, Fuchs' dystrophy, cornea diseases, stroke, traumatic brain injury, Alzheimer's disease, multiple sclerosis, muscular dystrophy, muscle atrophy caused by sarcopenia or chachexia, diseases affecting blood brain barrier (BBB), spinal cord injuries, spinal cord diseases, oral mucositis, short bowel syndrome, inflammatory bowel diseases (IBD), metabolic syndrome, diabetes, dyslipidemia, pancreatitis, exocrine pancreatic insufficiency, wound healing, diabetic foot ulcers, pressure sores, venous leg ulcers, epidermolysis bullosa, dermal hypoplasia, myocardial infarction, coronary artery disease, heart failure, hematopoietic cell disorders, immunodeficiencies, graft versus host diseases, acute kidney injuries, chronic kidney diseases, chronic obstructive pulmonary diseases (COPD), idiopathic pulmonary fibrosis, acute liver failure of all causes, acute liver failure drug-induced, alcoholic liver diseases, chronic liver failure of all causes, cirrhosis, liver fibrosis of all causes, portal hypertension, chronic liver insufficiency of all causes, nonalcoholic steatohepatitis (NASH), nonalcoholic fatty liver disease (NAFLD) (fatty liver), alcoholic hepatitis, hepatitis C virus-induced liver diseases (HCV), hepatitis B virus-induced liver diseases (HBV), other viral hepatitis (e.g., hepatitis A virus-induced liver diseases (HAV) and hepatitis D virus-induced liver diseases (HDV)), primary biliary cirrhosis, autoimmune hepatitis, livery surgery, liver injury, liver transplantation, "small for size" syndrome in liver surgery and transplantation, congenital liver disease and disorders, any other liver disorder or defect resulting from genetic diseases, degeneration, aging, drugs, and injuries.
51. The method of any one of clauses 48-49, wherein the disease or disorder is a bone disease or disorder.
52. The method of clause 51, wherein the Wnt surrogate molecule binds: (i) Fzd1, Fzd2, and Fzd7; or (ii) Fzd1, Fzd2, Fzd5, Fzd7, and Fzd8.

## Claims

1. A multispecific Wnt surrogate molecule, wherein the Wnt surrogate molecule comprises:
(i) two regions that each specifically bind to a set of one or more Frizzled (Fzd) receptor epitopes (Fzd binding regions), wherein the two Fzd binding regions bind to different sets of one or more Fzd receptor epitopes;
wherein the Wnt surrogate molecule comprises a first light chain and a first heavy chain forming a first Fzd binding region, and a second light chain and a second heavy chain forming a second Fzd binding region; and
(ii) one region that specifically binds to a Low-density lipoprotein (LDL) receptor-related protein 5 (LRP5) and/or a LDL receptor-related protein 6 (LRP6) (LRP5/6 binding region);
wherein the Wnt surrogate molecule comprises a first LRP5/6 binding region fused to an N-terminus of the first light chain or an N-terminus of the first heavy chain or to an N-terminus of the second light chain or an N-terminus of the second heavy chain.

2. The Wnt surrogate molecule of claim 1, wherein the two Fzd binding regions bind to different sets of one or more Fzd receptors, different sets of one or more epitopes within the same set of one or more Fzd receptors, or a combination thereof.

3. The Wnt surrogate molecule of any one of claims 1-2, wherein each Fzd binding region binds to one or more of Frizzled 1 (Fzd1), Frizzled 2 (Fzd2), Frizzled 3 (Fzd3), Frizzled 4 (Fzd4), Frizzled 5 (Fzd5), Frizzled 6 (Fzd6), Frizzled 7 (Fzd7), Frizzled 8 (Fzd8), Frizzled 9 (Fzd9), and Frizzled 10 (Fzd10).

4. The Wnt surrogate molecule of any one of claims 1-3, wherein at least one Fzd binding region binds to: (i) Fzd1, Fzd2, Fzd7, and Fzd9; (ii) Fzd1, Fzd2, and Fzd7; (iii) Fzd5 and Fzd8; (iv) Fzd5, Fzd7, and Fzd8; (v) Fzd1, Fzd4, Fzd5, and Fzd8; (vi) Fzd1, Fzd2, Fzd5, Fzd7, and Fzd8; (vii) Fzd4 and Fzd9; (viii) Fzd9 and Fzd10; (ix) Fzd5, Fzd8, and Fzd10; (x) Fzd4, Fzd5, and Fzd8; or (xi) Fzd1, Fzd5, Fzd7 and Fzd8.

5. The Wnt surrogate molecule of any one of claims 1-4, wherein the two Fzd binding regions comprise:
(i) a first Fzd binding region that binds to a first set of one or more Fzd receptors, and
(ii) a second Fzd binding region that binds to a second, different set of one or more Fzd receptors.

6. The Wnt surrogate molecule of claim 5, wherein:
(i) the first Fzd binding region binds to one or more of Fzd1, Fzd2, Fzd3, Fzd4, Fzd5, Fzd6, Fzd7, Fzd8, Fzd9, and Fzd10, and
(ii) the second Fzd binding region binds to one or more of Fzd1, Fzd2, Fzd3, Fzd4, Fzd5, Fzd6, Fzd7, Fzd8, Fzd9, and Fzd10.

7. The Wnt surrogate molecule of any one of claims 1-4, wherein the two Fzd binding regions comprise:
(i) a first Fzd binding region that binds to a first set of one or more epitopes within a set of one or more Fzd receptors, and
(ii) a second Fzd binding region that binds to a second, different set of one or more epitopes within the same set of one or more Fzd receptors.

8. The Wnt surrogate molecule of claim 1-7, wherein at least one Fzd binding region binds monospecifically to Fzd1, Fzd2, Fzd3, Fzd4, Fzd5, Fzd6, Fzd7, Fzd8, Fzd9, or Fzd10.

9. The Wnt surrogate molecule of any one of claims 1-8, wherein at least one Fzd binding region binds to a region of a Fzd receptor that (i) does not include the cysteine rich domain (CRD) of the Fzd receptor or (ii) includes less than the entire CRD of the FZD receptor or (iii) overlaps with the CRD of the Fzd receptor.

10. The Wnt surrogate molecule of claim 9, wherein at least one Fzd binding region binds to a hinge region of the Fzd receptor, wherein the hinge region comprises an amino acid sequence having at least 90% identity to any of the sequences set forth in SEQ ID NOs: 98-107.

11. The Wnt surrogate molecule of claim 9, wherein at least one Fzd binding region binds to an N-terminal region upstream of the CRD of the Fzd receptor, wherein the N-terminal region comprises an amino acid sequence having at least 90% identity to SEQ ID NO: 108.

12. The Wnt surrogate molecule of claims 1-11, wherein the two Fzd binding regions and the LRP5/6 binding regions are in a ratio of Fzd:LRP5/6 selected from the group consisting of: 2:1 (with two Fzd binders and one LRP binder) and 1:1:1 (two different Fzd binders and one LRP binder) .

13. The Wnt surrogate molecule of claim 12, wherein the ratio of Fzd binding regions to LRP5/6 binding region (Fzd:LRP5/6) comprises 2 Fzd binding regions and 1 LRP5/6 binding region.

14. The Wnt surrogate molecule of claims 1-13, wherein the first and second heavy chains are connected to each other;
wherein the first heavy chain comprises a first CH3 domain, the second heavy chain comprises a second CH3 domain, and the first and second CH3 domains are connected to/interact with each other;
wherein the first and second CH3 domains are connected to each other via knobs-into-holes mutations.

15. The Wnt surrogate molecule of claims 1-14, which modulates a Wnt signaling pathway in a cell, optionally a mammalian cell.

16. A pharmaceutical composition comprising a pharmaceutically acceptable excipient, diluent, or carrier, and the Wnt surrogate molecule according to any of claims 1-15.
